# EUROPÄISCHE PATENTSCHRIFT

(11) **EP 0 825 982 B1**
(45) Veröffentlichungstag und Bekanntmachung des Hinweises auf die Patenterteilung: **27.11.2002**
(21) Anmeldenummer: 96914146.4
(22) Anmeldetag: 29.04.1996
(51) Int. Cl.: C07D 207/38, C07D 209/54, C07D 307/60, C07D 307/94, C07D 333/32, C07D 333/50, C07D 309/38, C07D 279/06, A01N 43/36, A01N 43/02, C07D 491/10

(54) **ALKYL-DIHALOGENPHENYLSUBSTITUIERTE KETOENOLE ALS SCHÄDLINGSBEKÄMPFUNGSMITTEL UND HERBIZIDE**
ALKYL DIHALOGENATED PHENYL-SUBSTITUTED KETOENOLS USEFUL AS PESTICIDES AND HERBICIDES
ENOLS CETONIQUES SUBSTITUES PAR DES PHENYLES DIHALOGENES D'ALKYLE SERVANT DE PESTICIDES ET HERBICIDES

(30) Priorität: 09.05.1995 DE 19516258; 06.12.1995 DE 19545467
(43) Veröffentlichungstag der Anmeldung: 04.03.1998
(73) Patentinhaber: Bayer CropScience AG, 40789 Monheim (DE)
(72) Erfinder: LIEB, Folker, D-51375 Leverkusen (DE); HAGEMANN, Hermann, D-51375 Leverkusen (DE); WIDDIG, Arno, D-51519 Odenthal (DE); RUTHER, Michael, D-40789 Monheim (DE); FISCHER, Reiner, D-40789 Monheim (DE); BRETSCHNEIDER, Thomas, D-53797 Lohmar (DE); ERDELEN, Christoph, D-42799 Leichlingen (DE); WACHENDORFF-NEUMANN, Ulrike, D-56566 Neuwied (DE); DAHMEN, Peter, D-41470 Neuss (DE); DOLLINGER, Markus, D-51381 Leverkusen (DE); SANTEL, Hans-Joachim, D-51371 Leverkusen (DE); GRAFF, Alan, D-51427 Bergisch Gladbach (DE); ANDERSCH, Wolfram, D-51469 Bergisch Gladbach (DE); MENCKE, Norbert, D-51381 Leverkusen (DE); TURBERG, Andreas, D-40699 Erkrath (DE)
(86) Internationale Anmeldenummer: EP9601781
(87) Internationale Veröffentlichungsnummer: WO96035664

(56) Entgegenhaltungen:
- EP-A- 0 355 599
- EP-A- 0 423 482
- EP-A- 0 442 077
- EP-A- 0 508 126
- EP-A- 0 521 334
- EP-A- 0 528 156
- EP-A- 0 588 137
- DE-A- 4 413 669

## Beschreibung

Die Erfindung betrifft neue phenylsubstituierte cyclische Ketoenole, mehrere Verfahren und Zwischenprodukte zu ihrer Herstellung und ihre Verwendung als Schädlingsbekämpfungsmittel und Herbizide.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599 und EP-A-415 211) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, WO 94/01 997, WO 93/26954 und WO 95/01 358).

Es ist bekannt, daß bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-dihydrofuranon-Derivate mit herbiziden, akariziden und insektiziden Eigenschaften aus EP-A-528 156 bekannt, jedoch ist die dort beschriebene Wirkung nicht immer ausreichend. Thiotetronsäuren sind aus der WO 95/26345 bekannt.

Aus der Literatur sind ferner bestimmte 3H-Pyrazol-3-on-Derivate, wie beispielsweise 1,2-Diethyl-1,2-dihydro-5-hydroxy-4-phenyl-3H-pyrazol-3-on oder {[5-Oxo-1,2-diphenyl-4-(p-sulfophenyl)-3-pyrazolin-3-yl]-oxy}-dinatriumsalz oder p-(3-Hydroxy-5-oxo-1,2-diphenyl-3-pyrazolin-4-yl)-benzolsulfonsäure bekannt (vgl. J. Heterocycl. Chem., 25(5), 1301-1305, 1988 oder J. Heterocycl. Chem., 25(5), 1307-1310, 1988 oder Zh. Obshch. Khim., 34(7), 2397-2402, 1964). Eine biologische Wirkung dieser Verbindungen wird aber nicht beschrieben.

Weiterhin ist bekannt, daß das Trinatriumsalz der 4,4',4"-(5-Hydroxy-3-oxo-1Hpyrazol-1,2,4(3H)-triyl)-tris-benzolsulfonsäure pharmakologische Eigenschaften besitzt (vgl. Farmakol. Toksikol. (Moscow), 38(2), 180-186, 1976). Seine Verwendung im Pflanzenschutz ist aber nicht bekannt.

Außerdem sind in EP-A-508 126 und in WO 92/16 510 4-Arylpyrazolidin-3,5-dion-Derivate mit herbiziden, akariziden und insektiziden Eigenschaften beschrieben.

Bestimmte, im Phenylring unsubstituierte Phenyl-pyron-Derivate sind bereits bekannt geworden (vgl. A.M. Chirazi, T. Kappe und E. Ziegler, Arch. Pharm. 309, 558 (1976) und K.-H. Boltze und K. Heidenbluth, Chem. Ber. 91, 2849), wobei für diese Verbindungen eine mögliche Verwendbarkeit als Schädlingsbekämpfungsmittel nicht angegeben wird. Im Phenylring substituierte Phenyl-pyron-Derivate mit herbiziden, akariziden und insektiziden Eigenschaften sind in EP-A-588 137 beschrieben.

Bestimmte, im Phenylring unsubstituierte 5-Phenyl-1,3-thiazin-Derivate sind bereits bekannt geworden (vgl. E. Ziegler und E. Steiner, Monatsh. 95, 147 (1964), R. Ketcham, T. Kappe und E. Ziegler, J. Heterocycl. Chem. 10, 223 (1973)), wobei für diese Verbindungen eine mögliche Anwendung als Schädlingsbekämpfungsmittel nicht angegeben wird. Im Phenylring substituierte 5-Phenyl-1,3-thiazin-Derivate mit herbizider, akarizider und insektizider Wirkung sind in WO 94/14 785 beschrieben.

Die Wirksamkeit und Wirkungsbreite dieser Verbindungen ist jedoch insbesondere bei niedrigen Aufwandmengen und Konzentrationen nicht immer voll zufriedenstellend. Weiterhin ist die Pflanzenverträglichkeit der bekannten Verbindungen nicht immer ausreichend.

Es wurden nun neue Verbindungen der Formel (I) 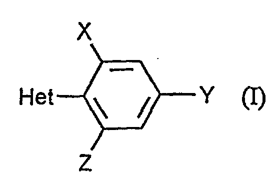 gefunden,
in welcher 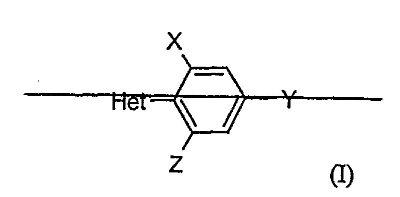
- X: für Halogen,
- Y: für Halogen oder C₁-C₆-Alkyl,
- Z: für Halogen oder C₁-C₆-Alkyl steht,
wobei immer einer der Substituenten Y und Z fiir Halogen und der andere fiir Alkyl steht,
- Het: für eine der Gruppen 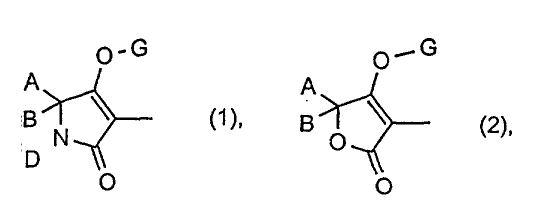 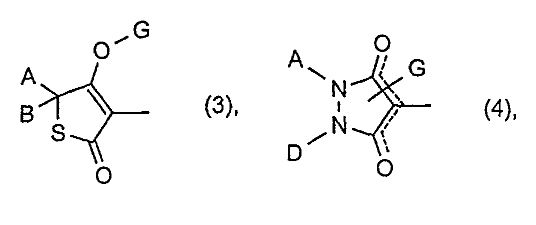 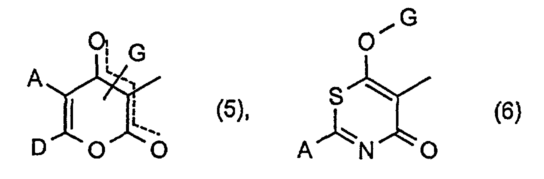
steht,
- A: für Wasserstoff, für jeweils gegebenenfalls durch Halogen substituiertes C₁-C₁₂-Alkyl, C₂-C₈-Alkenyl, C₁-C₁₀-Alkoxy-C₁-C₈-alkyl, Poly-C₁-C₈-alkoxy-C₁-C₈-alkyl oder C₁-C₁₀-Alkylthio-C₁-C₆-alkyl, fiir jeweils gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogen-alkoxy, Cyano oder Nitro substituiertes Phenyl, Naphthyl, Phenyl-C₁-C₆-alkyl, Naphthyl-C₁-C₆-alkyl oder Hetaryl mit 5 oder 6 Ringatomen und ein bis drei Heteroatomen aus der Reihe Sauerstoff, Schwefel und Stickstoff steht,
- B: für Wasserstoff, C₁-C₁₂-Alkyl oder C₁-C₈-Alkoxy-C₁-C₆-alkyl steht oder
- A, B: und das Kohlenstoffatom, an das sie gebunden sind, für C₃-C₁₀-Cycloalkyl oder C₅-C₁₀-Cycloalkenyl stehen, worin jeweils gegebenenfalls eine Methylengruppe durch Sauerstoff oder Schwefel ersetzt ist und welche gegebenenfalls durch C₁-C₈-Alkyl, C₃-C₁₀-Cycloalkyl, C₁-C₈-Halogenalkyl, C₁-C₈-Alkoxy, C₁-C₈-Alkylthio, Halogen oder Phenyl substituiert sind oder
- A, B: und das Kohlenstoffatom, an das sie gebunden sind, für C₅-C₆-Cycloalkyl stehen, welches durch eine gegebenenfalls ein oder zwei Sauerstoff- und/oder Schwefelatome enthaltende Alkylendiyl-, oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Ring bildet oder
- A, B: und das Kohlenstoffatom, an das sie gebunden sind, für C₃-C₈-Cycloalkyl oder C₅-C₈-Cycloalkenyl stehen, in welchen zwei Substituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₃-C₆-Alkandiyl, C₃-C₆-Alkendiyl oder C₄-C₆-Alkandiendiyl stehen, worin jeweils gegebenenfalls eine Methylengruppe durch Sauerstoff oder Schwefel ersetzt ist,
- D: für Wasserstoff, für jeweils gegebenenfalls durch Halogen substituiertes C₁-C₁₂-Alkyl, C₃-C₈-Alkenyl, C₃-C₈-Alkinyl, C₁-C₁₀-Alkoxy-C₂-C₈-alkyl, Poly-C₁-C₈-alkoxy-C₂-C₈-alkyl oder C₁-C₁₀-Alkylthio-C₂-C₈-alkyl, für gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls eine oder zwei nicht direkt benachbarte Methylengruppen durch Sauerstoff und/oder Schwefel ersetzt sind oder fiir jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Hetaryl mit 5 bis 6 Ringatomen und ein oder zwei Heteroatomen aus der Reihe Sauerstoff, Schwefel und Stickstoff, Phenyl-C₁-C₆-alkyl oder Hetaryl-C₁-C₆-alkyl mit 5 bis 6 Ringatomen und ein oder zwei Heteroatomen aus der Reihe Sauerstoff, Schwefel und Stickstoff steht oder
- A und D: gemeinsam für eine C₃-C₆-Alkandiyl-, C₃-C₆-Alkendiyl- oder C₄-C₆-Alkandiendiylgruppe stehen, in welchen jeweils gegebenenfalls eine Methylengruppe durch Sauerstoff oder Schwefel ersetzt ist und welche jeweils gegebenenfalls substituiert sind durch Halogen, Hydroxy, Mercapto oder jeweils gegebenenfalls durch Halogen substituiertes C₁-C₁₀-Alkyl, C₁-C₆-Alkoxy, C₁-C₆-Alkylthio, C₃-C₇-Cycloalkyl, Phenyl oder Benzyloxy oder durch eine weitere, einen ankondensierten Ring bildende C₃-C₆-Alkandiyl-, C₃-C₆-Alkendiyl- oder C₄-C₆-Alkandien-diylgruppe, in welchen gegebenenfalls jeweils eine Methylengruppe durch Sauerstoff oder Schwefel ersetzt ist und welche gegebenenfalls durch C₁-C₆-Alkyl substituiert sind oder in welchen gegebenenfalls zwei benachbarte Substituenten gemeinsam mit den Kohlenstoffatomen, an die sie gebunden sind, einen weiteren gesättigten oder ungesättigten Carbocyclus mit 5 oder 6 Ringatomen bilden oder
- A und D: gemeinsam für eine C₃-C₆-Alkandiyl- oder C₃-C₆-Alkendiylgruppe stehen, worin jeweils gegebenenfalls eine der folgenden Gruppen 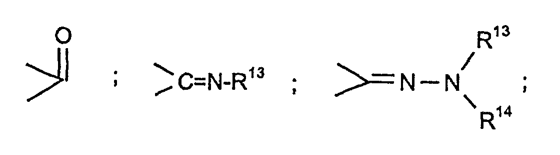 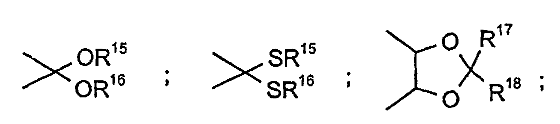 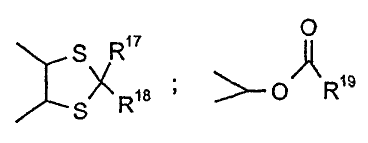 oder 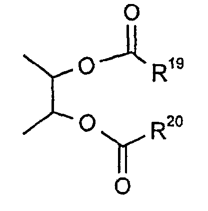 enthalten ist,
- G: im Fall, daß Het fiir einen der Reste (1), (2), (3), (5) oder (6) steht, für Wasserstoff (a) oder, im Fall, daß Het für einen der Reste (1), (2), (3), (4), (5) oder (6) steht, für eine der Gruppen 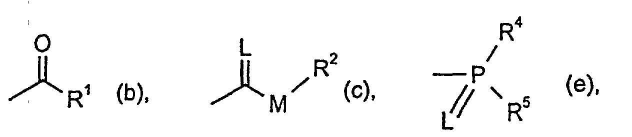 E (f) oder 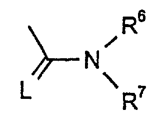 (g) steht, oder im Fall, dass
Het für einen der Reste (2), (3), (4), (5) oder (6) steht, fiir die Gruppe -SO₂-R³(d) steht, in welchen
E für ein Metallionäquivalent oder ein Ammoniumion steht,
L fiir Sauerstoff oder Schwefel steht und
M für Sauerstoff oder Schwefel steht,
- R¹: für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈--alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈--Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5-oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff steht,
- R²: fiir jeweils gegebenenfalls durch Halogen substituiertes C₁-C₂₀-Alkyl, C₂-C₂₀-Alkenyl, C₁-C₈-Alkoxy-C₂-C₈-alkyl oder Poly-C₁-C₈-alkoxy-C-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht,
- R³: für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl,
- R⁴ und R⁵: unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio oder C₂-C₈-Alkenylthio oder fiir jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen,
- R⁶ und R⁷: unabhängig voneinander für Wasserstoff, fiir jeweils gegebenenfalls durch Halogen substituiertes C₁-C₈-Alkyl, C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Alkylenrest stehen, in welchem gegebenenfalls eine Methylengruppe durch Sauerstoff oder Schwefel ersetzt ist,
- R¹³: für Wasserstoff, für jeweils gegebenenfalls durch Halogen substituiertes C₁-C₈-Alkyl oder C₁-C₈-Alkoxy, für gegebenenfalls durch Halogen, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt ist, oder fiir jeweils gegebenenfalls durch Halogen, C₁-C₆-Alkyl, C₁-C₆-Alkoxy, C₁-C₄-Halogenalkyl, C₁-C₄-Halogen-alkoxy, Nitro oder Cyano substituiertes Phenyl, Phenyl-C₁-C₄-alkyl oder Phenyl-C₁-C₄-alkoxy steht,
- R¹⁴: für Wasserstoff oder C₁-C₈-Alkyl steht oder
- R¹³ und R¹⁴: gemeinsam für C₄-C₆-Alkandiyl stehen,
- R¹⁵ und R¹⁶: gleich oder verschieden sind und für C₁-C₆-Alkyl stehen oder
- R¹⁵ und R¹⁶: gemeinsam fiir einen C₂-C₄-Alkandiylrest stehen, der gegebenenfalls durch C₁-C₆-Alkyl oder durch gegebenenfalls durch Halogen, C₁-C₄-Alkyl, C₁-C₄-Halogenalkyl, C₁-C₄-Alkoxy, C₁-C₄-Halogenalkoxy, Nitro oder Cyano substituiertes Phenyl substituiert ist,
- R¹⁷ und R¹⁸: unabhängig voneinander für Wasserstoff, für gegebenenfalls durch Halogen substituiertes C₁-C₈-Alkyl oder für gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl stehen oder
- R¹⁷ und R¹⁸: gemeinsam mit dem Kohlenstoffatom, an das sie gebunden sind, für gegebenenfalls durch C₁-C₄-Alkyl oder C₁-C₄-Alkoxy substituiertes C₅-C₇-Cycloalkyl stehen, in dem gegebenenfalls eine Methylengruppe durch Sauerstoff oder Schwefel ersetzt ist und
- R¹⁹ und R²⁰: unabhängig voneinander für C₁-C₁₀-Alkyl, C₂-C₁₀-Alkenyl, C₁-C₁₀-Alkoxy, C₁-C₁₀-Alkylamino, C₃-C₁₀-Alkenylamino, Di-(C₁-C₁₀-alkyl)amino oder Di-(C₃-C₁₀-alkenyl)amino stehen.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der Bedeutungen (1) bis (6) der Gruppe Het ergeben sich folgende hauptsächliche Strukturen (I-1) bis (I-6): 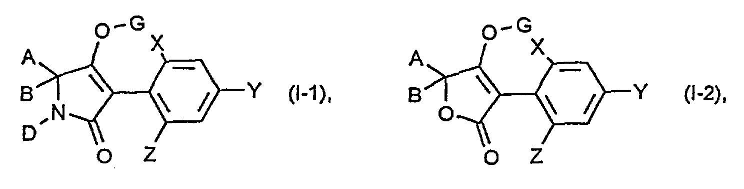 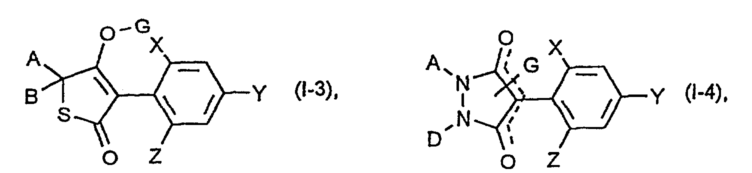 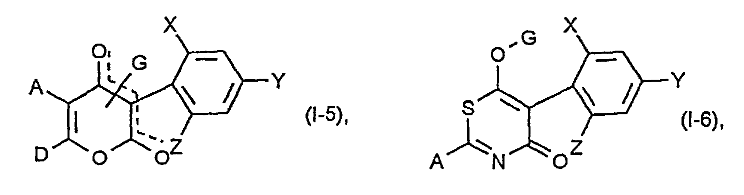 worin
A, B, D, G, X, Y und Z die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-1-a) bis (I-1-g), wenn Het für die Gruppe (1) steht, 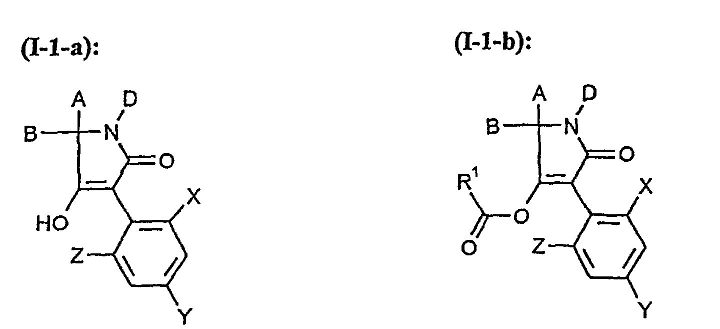 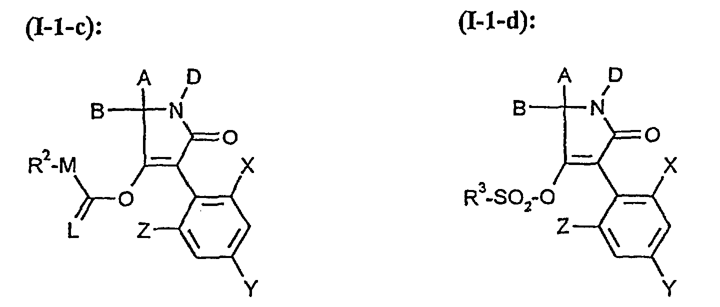 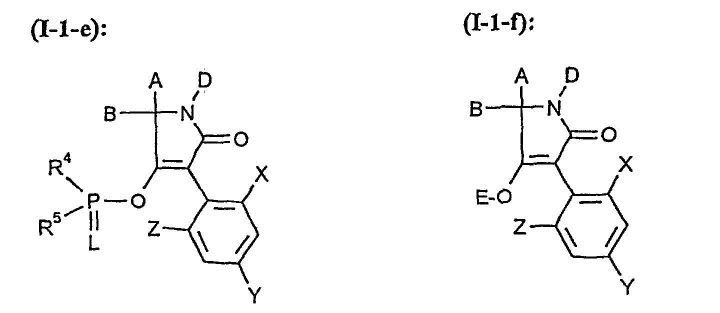 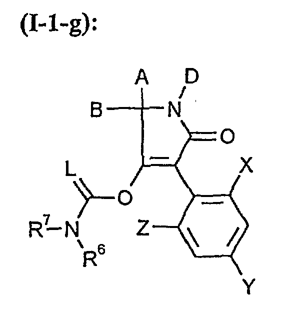 worin
A, B, D, E, L, M, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-2-a) bis (I-2-g), wenn Het für die Gruppe (2) steht, 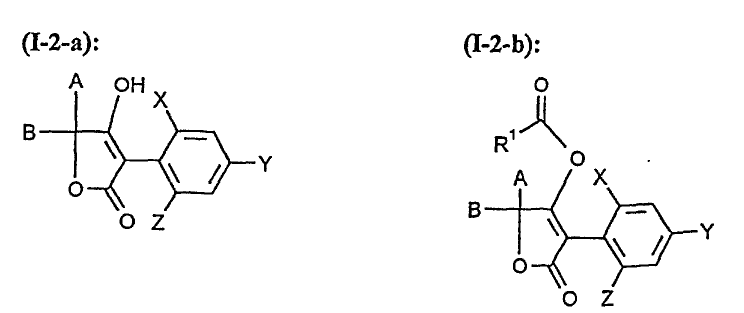 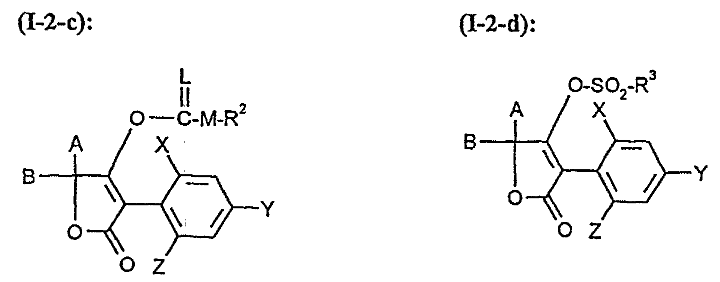 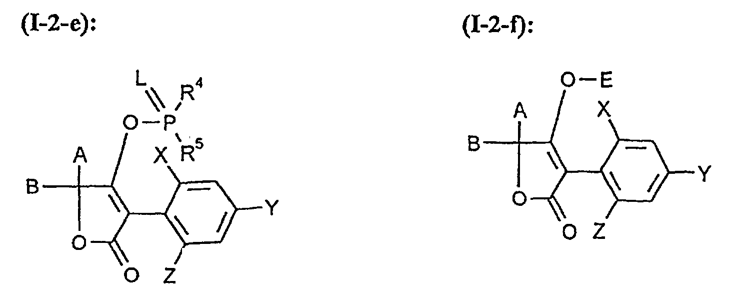 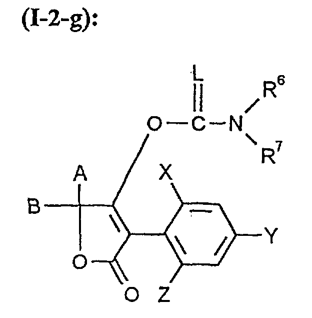 worin
A, B, E, L, M, X, Y, Z, R¹, R², R³, R⁴, R⁵, R⁶ und R⁷ die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-3-a) bis (I-3-g), wenn Het für die Gruppe (3) steht, 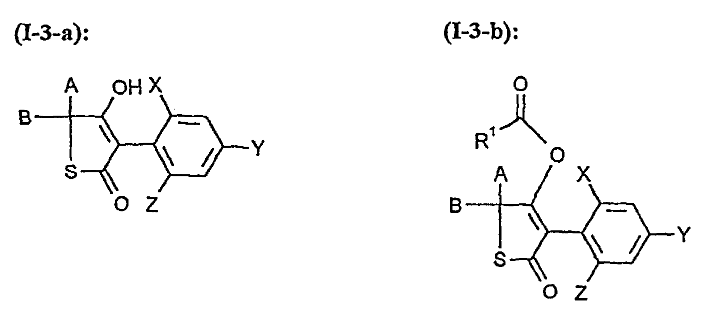 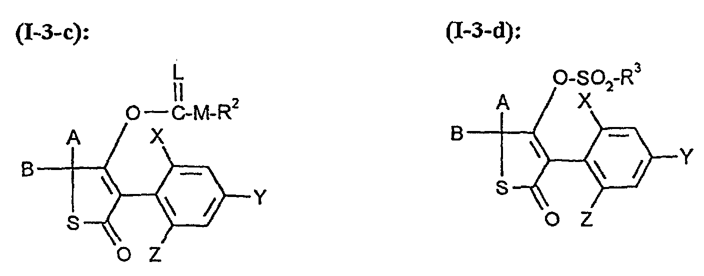 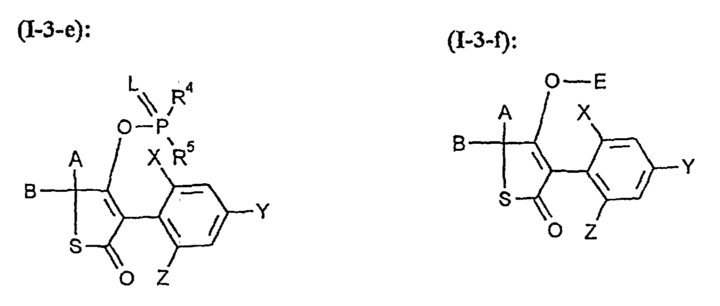 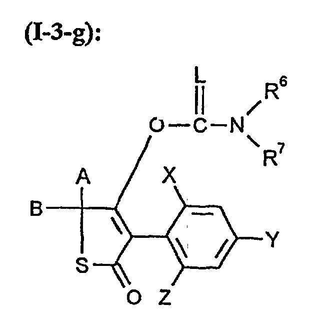 worin
A, B, E, L, M, X, Y, Z, R¹, R², R³, R⁴, R⁵, R⁶ und R⁷ die oben angegebenen Bedeutung besitzen.

Die Verbindungen der Formel (I-4) können in Abhängigkeit von der Stellung des Substituenten G in den zwei isomeren Formeln (I-4)ₐ und (I-4)_{b} vorliegen, 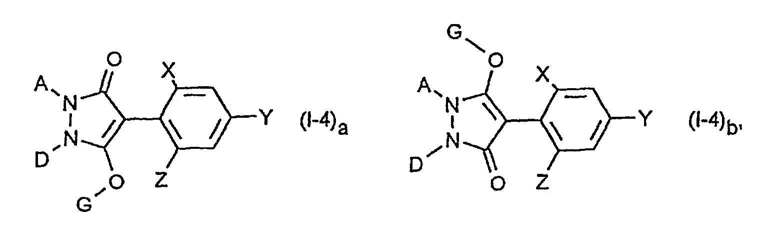 was durch die gestrichelte Linie in der Formel (I-4) zum Ausdruck gebracht werden soll:

Die Verbindungen der Formeln (I-4)ₐ und (I-4)_{b} können sowohl als Gemische als auch in Form ihrer reinen Isomeren vorliegen. Gemische der Verbindungen der Formel (I-4)ₐ und (I-4)_{b} lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, daß die Verbindungen gegebenenfalls in Form der Isomerengemische oder in der jeweils anderen isomeren Form vorliegen können.

Unter Einbeziehung der verschiedenen Bedeutungen (b), (c), (d), (e), (f) und (g) der Gruppe G ergeben sich folgende hauptsächliche Strukturen (I-4-b) bis (I-4-g), wenn Het für die Gruppe (4) steht, 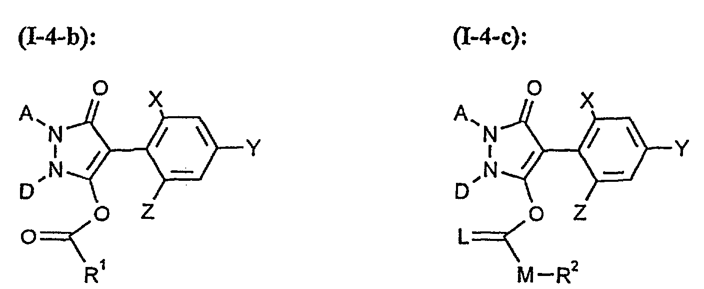 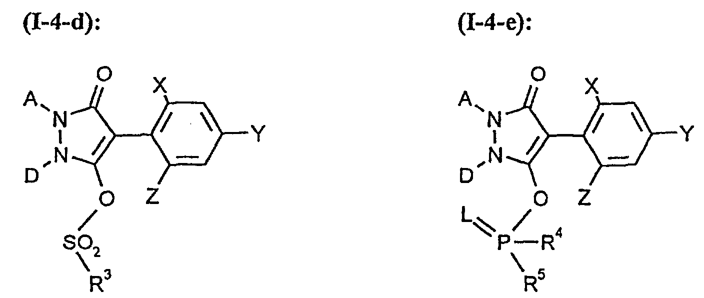 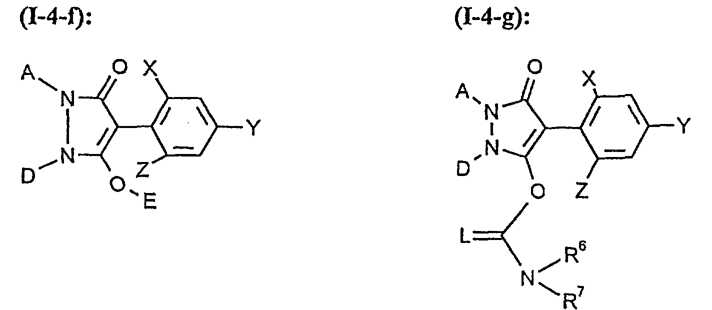 worin
A, D, E, L, M, X, Y, Z, R¹, R², R³, R⁴, R⁵, R⁶ und R⁷ die oben angegebenen Bedeutungen besitzen.

Die Verbindungen der Formel (I-5) können in Abhängigkeit von der Stellung des Substituenten G in den zwei isomeren Formen der Formeln (I-5)ₐ und (I-5)_{b} vorliegen, 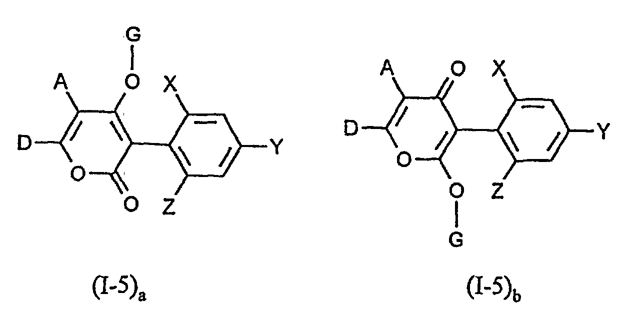 was durch die gestrichelte Linie in der Formel (I-5) zum Ausdruck gebracht werden soll.

Die Verbindungen der Formeln (I-5)ₐ und (I-5)_{b} können sowohl als Gemische als auch in Form ihrer reinen Isomeren vorliegen. Gemische der Verbindungen der Formeln (I-5)ₐ und (I-5)_{b} lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, daß die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächliche Strukturen (I-5-a) bis (I-5-g), wenn Het für die Gruppe (5) steht, 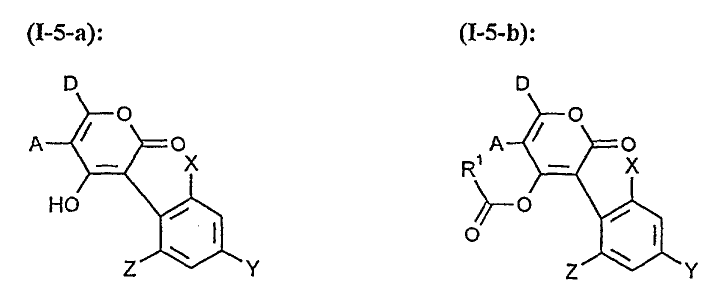 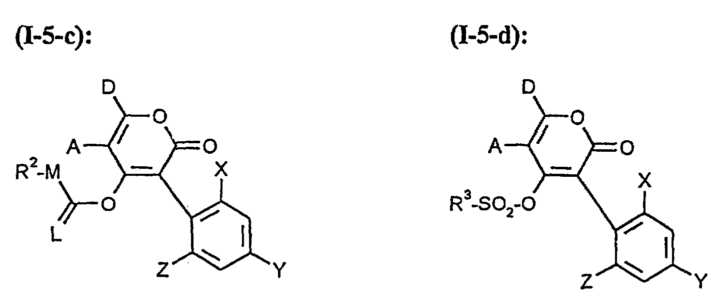 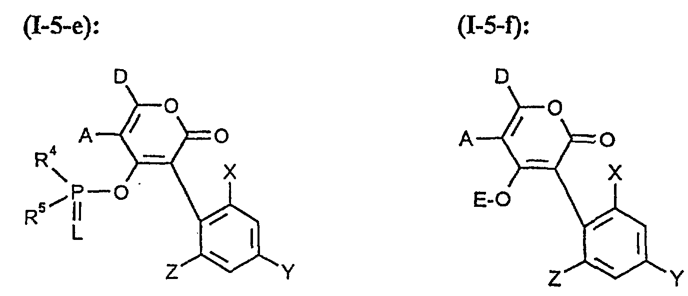 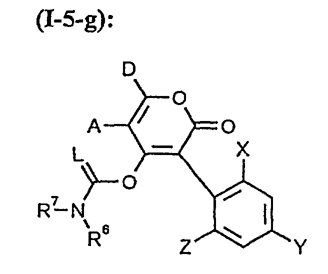 worin
A, D, E, L, M, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-6-a) bis (I-6-g), wenn Het für die Gruppe (6) steht, 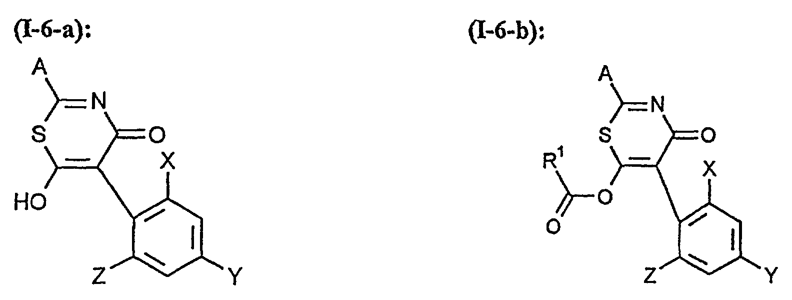 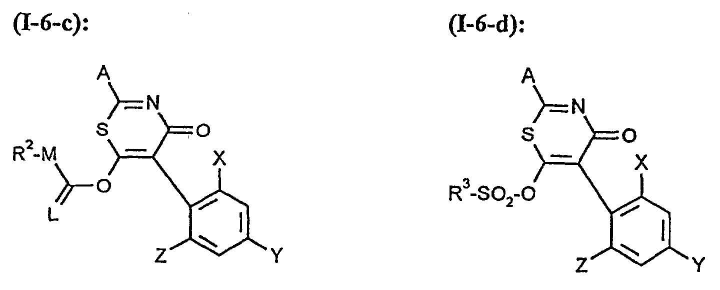 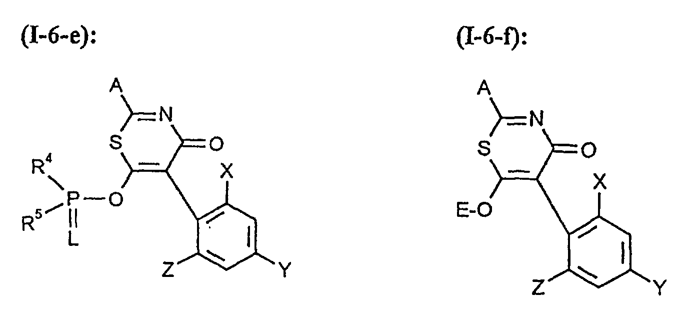 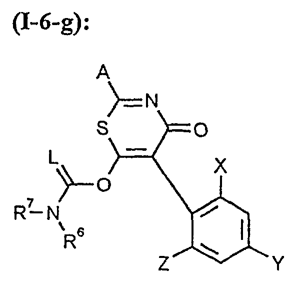 worin
A, E, L, M, X, Y, Z, R¹, R², R³, R⁴, R⁵, R⁶ und R⁷ die oben angegebenen Bedeutungen besitzen.

Weiterhin wurde gefunden, daß man die neuen Verbindungen der Formel (I) nach einem der im folgenden beschriebenen Verfahren erhält:
(A) Man erhält Verbindungen der Formel (I-1-a) 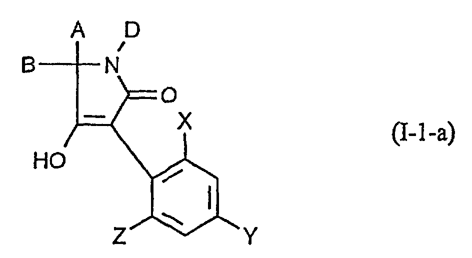 in welcher
   A, B, D, X, Y und Z die oben angegebenen Bedeutungen haben,
   wenn man
   Verbindungen der Formel (II) 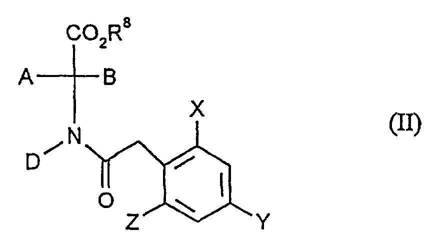 in welcher
   - A, B,: D, X, Y und Z die oben angegebenen Bedeutungen haben,
   und
   - R⁸: für Alkyl (bevorzugt C₁-C₆-Alkyl) steht,
   in Gegenwart eines Verdünnungsmittels und in Gegenwart einer Base intramolekular kondensiert.
(B) Außerdem wurde gefunden, daß man Verbindungen der Formel (I-2-a) 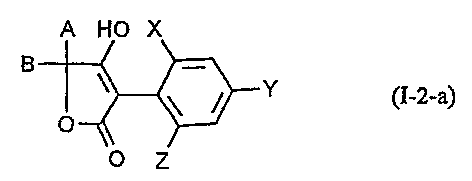 in welcher
   A, B, X, Y und Z die oben angegebenen Bedeutungen haben,
   erhält, wenn man
   Verbindungen der Formel (III) 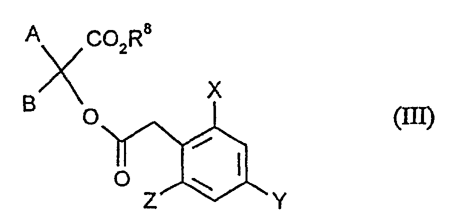 in welcher
   A, B, X, Y, Z und R⁸ die oben angegebenen Bedeutungen haben,
   in Gegenwart eines Verdünnungsmittels und in Gegenwart einer Base intramolekular kondensiert.
(C) Weiterhin wurde gefunden, daß man Verbindungen der Formel (I-3-a) 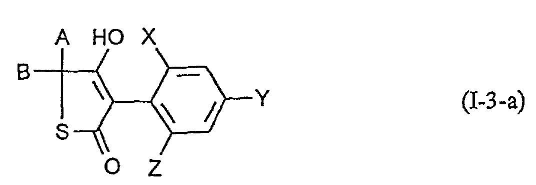 in welcher
   A, B, X, Y und Z die oben angegebenen Bedeutungen haben,
   erhält, wenn man
   Verbindungen der Formel (IV) 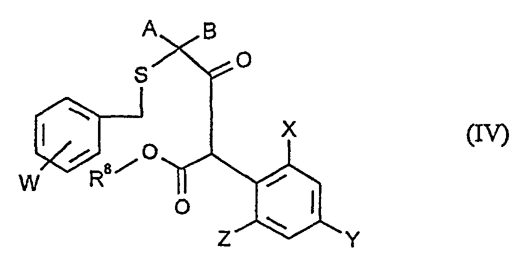 in welcher
   - A, B, X, Y, Z und R⁸: die oben angegebenen Bedeutungen haben und
   - W: für Wasserstoff, Halogen, Alkyl (bevorzugt C₁-C₆-Alkyl) oder Alkoxy (bevorzugt C₁-C₈-Alkoxy) steht,
   gegebenenfalls in Gegenwart eines Verdünnungsmittels und in Gegenwart einer Säure intramolekular cyclisiert.
(E) Weiterhin wurde gefunden, daß man die Verbindungen der Formel (I-5-a) 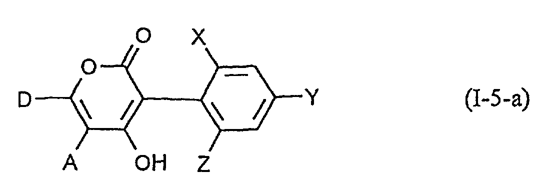 in welcher
   A, D, X, Y und Z die oben angegebenen Bedeutungen haben,
   erhält, wenn man
   Verbindungen der Formel (VIII) 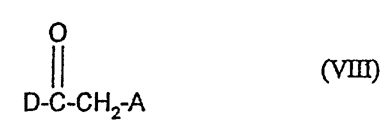 in welcher
   - A und D: die oben angegebenen Bedeutungen haben,
   oder deren Silylenolether der Formel (VIIIa) 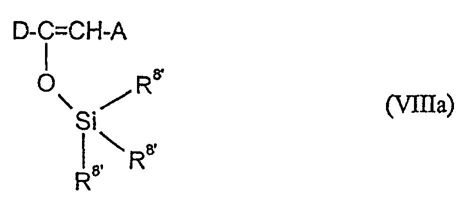 in welcher
   - A und D: die oben angegebene Bedeutung haben und
   - R^{8'}: für Alkyl (bevorzugt Methyl) steht,
   mit Verbindungen der Formel (V) 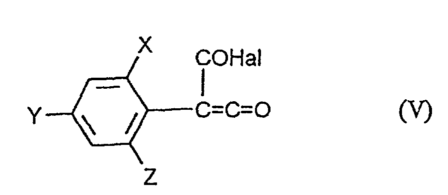 in welcher
   - X, Y und Z: die oben angegebenen Bedeutungen haben und
   - Hal: für Halogen (vorzugsweise für Chlor oder Brom) steht,
   gegebenenfalls in Gegenwart eines Verdünnungsmittels und gegebenenfalls in Gegenwart eines Säureakzeptors umsetzt.
(F) Weiterhin wurde gefunden, daß man die Verbindungen der Formel (I-6-a) 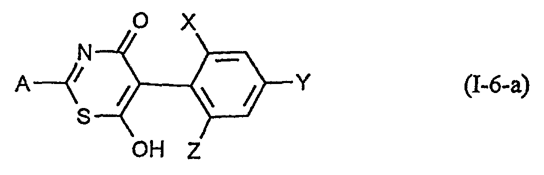 in welcher
   A, X, Y und Z die oben angegebenen Bedeutungen haben,
   erhält, wenn man Verbindungen der Formel (IX) 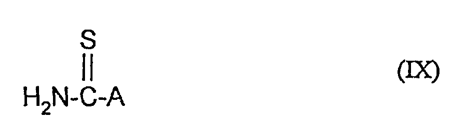 in welcher
   - A: die oben angegebene Bedeutung hat,
   mit Verbindungen der Formel (V) 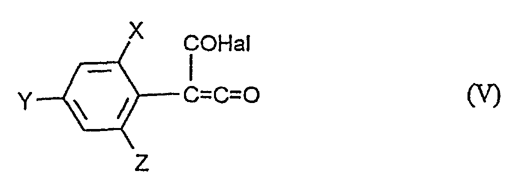 in welcher
   Hal, X, Y und Z die oben angegebenen Bedeutungen haben,
   gegebenenfalls in Gegenwart eines Verdünnungsmittels und gegebenenfalls in Gegenwart eines Säureakzeptors umsetzt.
   Außerdem wurde gefunden,
(G) daß man die Verbindungen der oben gezeigten Formeln (I-1-b) bis (I-3-b), (I-5-b) und (I-6-b), in welchen A, B, D, R¹, X, Y und Z die oben angebenen Bedeutungen haben, erhält, wenn man Verbindungen der oben gezeigten Formeln (I-1-a) bis (I-3-a), (I-5-a) und (I-6-a), in welchen A, B, D, X, Y und Z die oben angegebenen Bedeutungen haben, und daß man Verbindungen der oben gezeigten Formel (I-4-b), in welcher A, D, R¹, X, Y und Z die oben angegebene Bedeutung haben, erhält, wenn man Verbindungen der Formel (I-4-a) 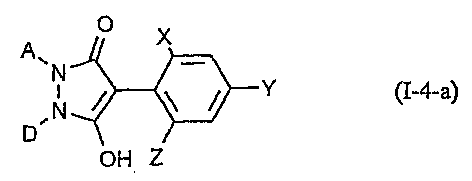 in welcher
   A, D, X, Y und Z die oben angegebene Bedeutung haben, jeweils
   α) mit Säurehalogeniden der Formel (X) 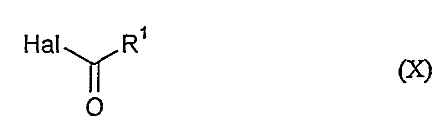 in welcher
      - R¹: die oben angegebene Bedeutung hat und
      - Hal: für Halogen (insbesondere Chlor oder Brom) steht
      oder
   β) mit Carbonsäureanhydriden der Formel (XI)

      R¹-CO-O-CO-R¹ (XI)

      in welcher
      - R¹: die oben angegebene Bedeutung hat,
   gegebenenfalls in Gegenwart eines Verdünnungsmittels und gegebenenfalls in Gegenwart eines Säurebindemittels umsetzt;
(H) daß man die Verbindungen der oben gezeigten Formeln (I-1-c) bis (I-6-c), in welchen A, B, D, R², M, X, Y und Z die oben angegebenen Bedeutungen haben und L für Sauerstoff steht, erhält, wenn man Verbindungen der oben gezeigten Formeln (I-1-a) bis (I-6-a), in welchen A, B, D, X, Y und Z die oben angegebenen Bedeutungen haben, jeweils
   mit Chlorameisensäureestern oder Chlorameisensäurethioestern der Formel (XII)

   R²-M-CO-Cl (XII)

   in welcher
   - R² und M: die oben angegebenen Bedeutungen haben,
   gegebenenfalls in Gegenwart eines Verdünnungsmittels und gegebenenfalls in Gegenwart eines Säurebindemittels umsetzt;
(I) daß man Verbindungen der oben gezeigten Formeln (I-1-c) bis (I-6-c), in welchen A, B, D, R², M, X, Y und Z die oben angegebenen Bedeutungen haben und L für Schwefel steht, erhält, wenn man Verbindungen der oben gezeigten Formeln (I-1-a) bis (I-6-a), in welchen A, B, D, X, Y und Z die oben angegebenen Bedeutungen haben, jeweils
   α) mit Chlormonothioameisensäureestern oder Chlordithioameisensäureestern der Formel (XIII) 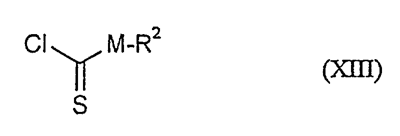 in welcher
      - M und R²: die oben angegebenen Bedeutungen haben,
      gegebenenfalls in Gegenwart eines Verdünnungsmittels und gegebenenfalls in Gegenwart eines Säurebindemittels umsetzt
      oder
   β) mit Schwefelkohlenstoff und anschließend mit Verbindungen der Formel (XIV)

      R²-Hal (XIV)

      in welcher
      - R²: die oben angegebene Bedeutung hat und
      - Hal: für Chlor, Brom oder Iod steht,
      gegebenenfalls in Gegenwart eines Verdünnungsmittels und gegebenenfalls in Gegenwart einer Base umsetzt,
(J) daß man Verbindungen der oben gezeigten Formeln (I-1-d) bis (I-6-d), in welchen A, B, D, R³, X, Y und Z die oben angegebenen Bedeutungen haben, erhält, wenn man Verbindungen der oben gezeigten Formeln (I-1-a) bis (I-6-a), in welchen A, B, D, X, Y und Z die oben angegebenen Bedeutungen haben, jeweils
   mit Sulfonsäurechloriden der Formel (XV)

   R³-SO₂-Cl (XV)

   in welcher
   - R³: die oben angegebene Bedeutung hat,
   gegebenenfalls in Gegenwart eines Verdünnungsmittels und gegebenenfalls in Gegenwart eines Säurebindemittels umsetzt,
(K) daß man Verbindungen der oben gezeigten Formeln (I-1-e) bis (I-6-e), in welchen A, B, D, L, R⁴, R⁵, X, Y und Z die oben angegebenen Bedeutungen haben, erhält, wenn man Verbindungen der oben gezeigten Formeln (I-1-a) bis (I-6-a), in welchen A, B, D, X, Y und Z die oben angegebenen Bedeutungen haben, jeweils
   mit Phosphorverbindungen der Formel (XVI) 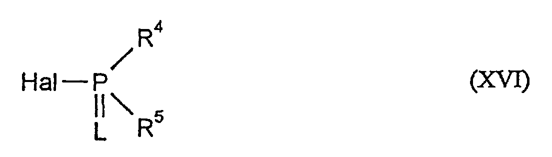 in welcher
   - L, R⁴ und R⁵: die oben angegebenen Bedeutungen haben und
   - Hal: für Halogen (insbesondere Chlor oder Brom) steht,
   gegebenenfalls in Gegenwart eines Verdünnungsmittels und gegebenenfalls in Gegenwart eines Säurebindemittels umsetzt,
(L) daß man Verbindungen der oben gezeigten Formeln (I-1-f) bis (I-6-f), in welchen A, B, D, E, X, Y und Z die oben angegebenen Bedeutungen haben, erhält, wenn man Verbindungen der Formeln (I-1-a) bis (I-6-a), in welchen A, B, D, X, Y und Z die oben angegebenen Bedeutungen haben, jeweils
   mit Metallverbindungen oder Aminen der Formeln (XVII) oder (XVIII)

   Me(OR¹⁰)ₜ (XVII)

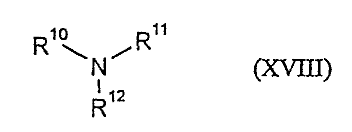

   in welchen
   - Me: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   - R¹⁰, R¹¹, R¹²: unabhängig voneinander für Wasserstoff oder Alkyl (bevorzugt C₁-C₈-Alkyl) stehen,
   gegebenenfalls in Gegenwart eines Verdünnungsmittels umsetzt,
(M) daß man Verbindungen der oben gezeigten Formeln (I-1-g) bis (I-6-g), in welchen A, B, D, L, R⁶, R⁷, X, Y und Z die oben angegebenen Bedeutungen haben, erhält, wenn man Verbindungen der oben gezeigten Formeln (I-1-a) bis (I-6-a), in welchen A, B, D, X, Y und Z die oben angegebenen Bedeutungen haben, jeweils
   α) mit Isocyanaten oder Isothiocyanaten der Formel (XIX)

      R⁶-N=C=L (XIX)

      in welcher
      - R⁶ und L: die oben angegebenen Bedeutungen haben,
      gegebenenfalls in Gegenwart eines Verdünnungsmittels und gegebenenfalls in Gegenwart eines Katalysators umsetzt oder
   β) mit Carbamidsäurechloriden oder Thiocarbamidsäurechloriden der Formel (XX) 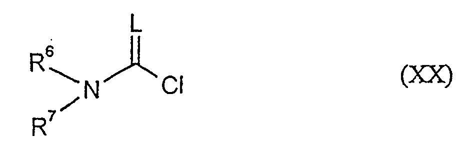 in welcher
      - L, R⁶ und R⁷: die oben angegebenen Bedeutungen haben,
      gegebenenfalls in Gegenwart eines Verdünnungsmittels und gegebenenfalls in Gegenwart eines Säurebindemittels, umsetzt.

Weiterhin wurde gefunden, daß die neuen Verbindungen der Formel (I) eine sehr gute Wirksamkeit als Schädlingsbekämpfungsmittel, vorzugsweise als Insektizide, Akarizide und auch als Herbizide aufweisen.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:
- X: steht bevorzugt für Fluor, Chlor oder Brom.
- Y: steht bevorzugt für Fluor, Chlor, Brom oder C₁-C₄-Alkyl.
- Z: steht bevorzugt für Fluor, Chlor, Brom oder C₁-C₄-Alkyl.

Dabei gilt, daß immer einer der Reste Y und Z für Halogen und der andere für Alkyl steht.
- Het: steht bevorzugt für eine der Gruppen 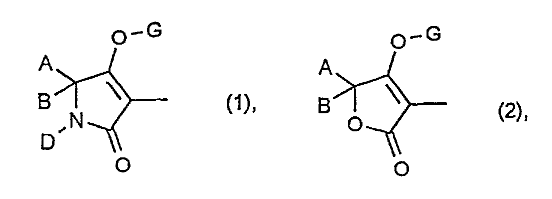 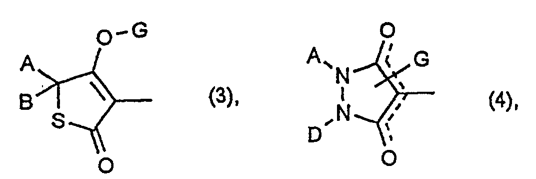 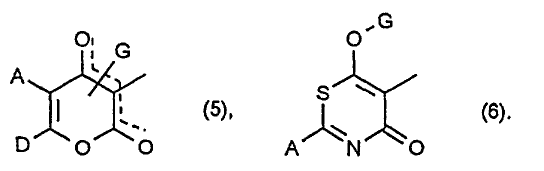
- A: steht bevorzugt für Wasserstoff, für jeweils gegebenenfalls durch Fluor oder Chlor substituiertes C₁-C₁₀-Alkyl, C₂-C₆-Alkenyl, C₁-C₈-Alkoxy-C₁-C₆-alkyl, Poly-C₁-C₆-alkoxy-C₁-C₆-alkyl oder C₁-C₈-Alkylthio-C₁-C₆-alkyl oder für gegebenenfalls durch Fluor, Chlor, C₁-C₄-Alkyl oder C₁-C₄-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, C₁-C₄-Alkyl, C₁-C₄-Halogenalkyl, C₁-C₄-Alkoxy, C₁-C₄-Halogenalkoxy, Cyano oder Nitro substituiertes Phenyl, Furanyl, Pyridyl, Imidazolyl, Triazolyl, Pyrazolyl, Indolyl, Thiazolyl, Thienyl oder Phenyl-C₁-C₄-alkyl.
- B: steht bevorzugt für Wasserstoff, C₁-C₁₀-Alkyl oder C₁-C₆-Alkoxy-C₁-C₄-alkyl oder
- A, B: und das Kohlenstoffatom an das sie gebunden sind, stehen bevorzugt für C₃-C₈-Cycloalkyl oder C₅-C₈-Cycloalkenyl, worin jeweils gegebenenfalls eine Methylengruppe durch Sauerstoff oder Schwefel ersetzt ist und welche gegebenenfalls durch C₁-C₆-Alkyl, C₃-C₈-Cycloalkyl, C₁-C₃-Halogenalkyl, C₁-C₆-Alkoxy, C₁-C₆-Alkylthio, Fluor, Chlor oder Phenyl substituiert sind oder
- A, B: und das Kohlenstoffatom, an das sie gebunden sind, stehen bevorzugt für C₅-C₆-Cycloalkyl, welches durch eine gegebenenfalls ein oder zwei Sauerstoff- oder Schwefelatome enthaltende Alkylendiyl- oder durch eine Alkylendioxyl- oder durch eine Alkylendithiol-Gruppe substituiert ist, die mit dem Kohlenstoffatom, an das sie gebunden ist, einen weiteren fünf- bis siebengliedrigen Ring bildet oder
- A, B: und das Kohlenstoffatom, an das sie gebunden sind, stehen bevorzugt für C₃-C₆-Cycloalkyl oder C₅-C₆-Cycloalkenyl, in welchen zwei Substituenten gemeinsam mit den Kohlenstoffatomen, an die sie gebunden sind, für jeweils gegebenenfalls durch C₁-C₅-Alkyl, C₁-C₅-Alkoxy, Fluor, Chlor oder Brom substituiertes C₃-C₅-Alkandiyl, C₃-C₅-Alkendiyl oder Butadiendiyl stehen, worin jeweils gegebenenfalls eine Methylengruppe durch Sauerstoff oder Schwefel ersetzt ist.
- D: steht bevorzugt für Wasserstoff, für jeweils gegebenenfalls durch Fluor oder Chlor substituiertes C₁-C₁₀-Alkyl, C₃-C₆-Alkenyl, C₃-C₆-Alkinyl, C₁-C₈-Alkoxy-C₂-C₆-alkyl, Poly-C₁-C₆-alkoxy-C₂-C₆-alkyl oder C₁-C₈-Alkylthio-C₂-C₆-alkyl, für gegebenenfalls durch Fluor, Chlor, C₁-C₄-Alkyl, C₁-C₄-Alkoxy oder C₁-C₂-Halogenalkyl substituiertes C₃-C₇-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, C₁-C₄-Alkyl, C₁-C₄-Halogenalkyl, C₁-C₄-Alkoxy, C₁-C₄-Halogenalkoxy, Cyano oder Nitro substituiertes Phenyl, Furanyl, Imidazolyl, Pyridyl, Thiazolyl, Pyrazolyl, Pyrimidyl, Pyrrolyl, Thienyl, Triazolyl oder Phenyl-C₁-C₄-alkyl oder
- A und D: stehen gemeinsam bevorzugt für eine C₃-C₅-Alkandiyl- oder C₃-C₅-Alkendiylgruppe, worin jeweils gegebenenfalls eine Methylengruppe durch Sauerstoff oder Schwefel ersetzt ist und welche gegebenenfalls substituiert sind durch Fluor, Chlor, Hydroxy, Mercapto oder durch jeweils gegebenenfalls durch Fluor oder Chlor substituiertes C₁-C₆-Alkyl, C₁-C₄-Alkoxy, C₁-C₄-Alkylthio, C₃-C₆-Cycloalkyl, Phenyl oder Benzyloxy oder
worin jeweils gegebenenfalls eine der folgenden Gruppen: 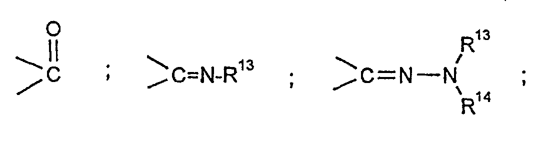 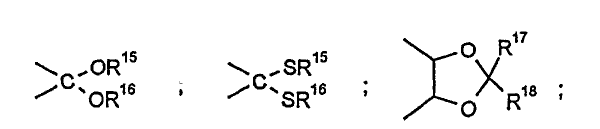 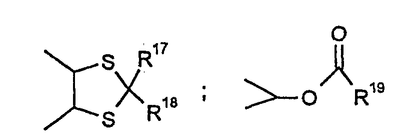 oder 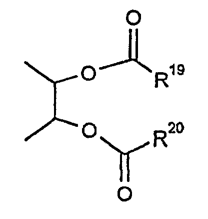 enthalten ist;
oder A und D stehen (im Fall der Verbindungen der Formel (I-1)) gemeinsam mit den Atomen, an die sie gebunden sind, für eine der Gruppen AD-1 bis AD-27 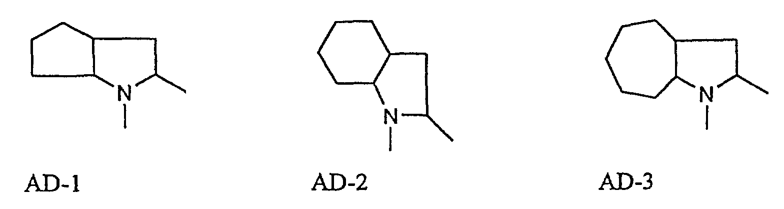 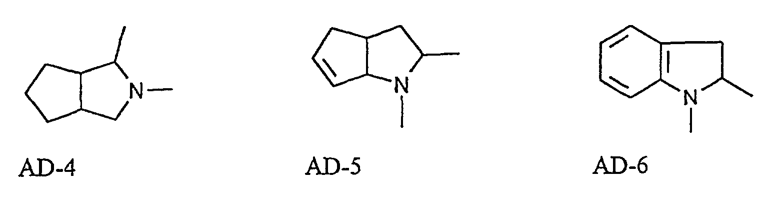 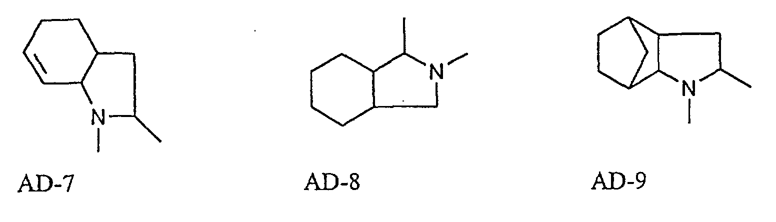 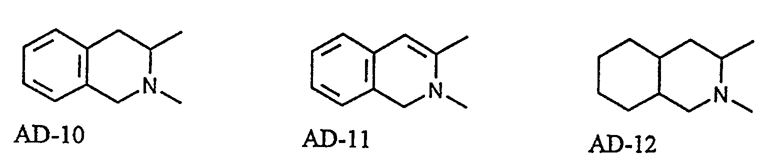 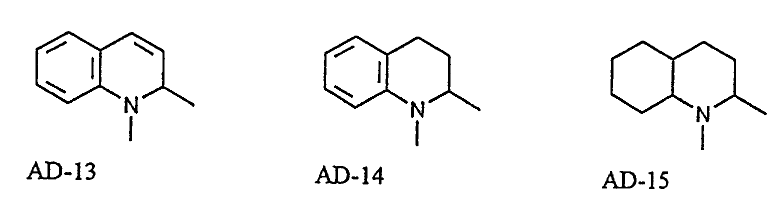 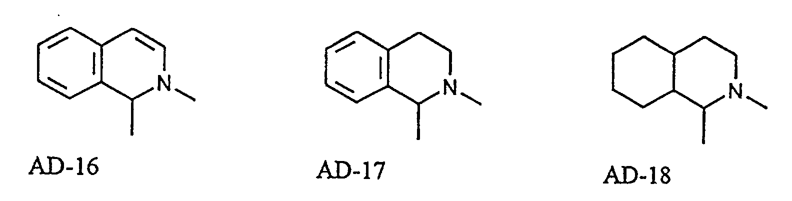 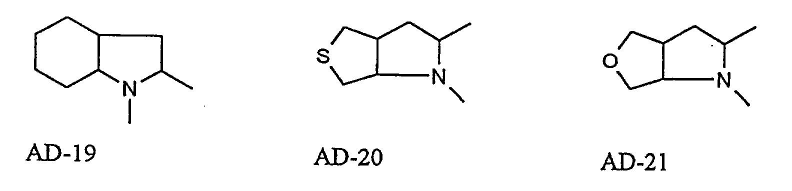 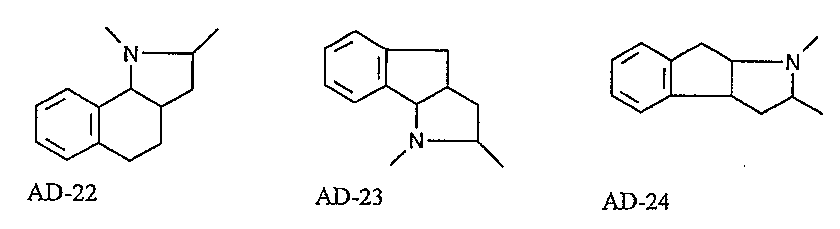 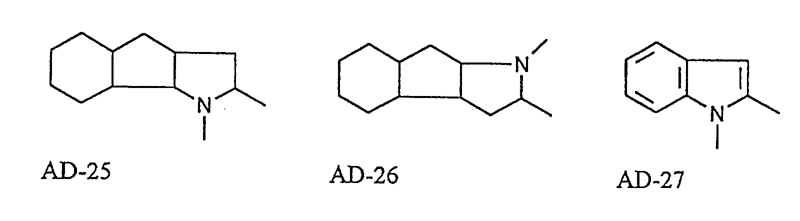
- G: steht bevorzugt im Fall, daß Het für einen der Reste (1), (2), (3), (5) oder (6) steht, für Wasserstoff (a) oder im Fall, daß Het für einen der Reste (1), (2), (3), (4), (5) oder (6) steht, für eine der Gruppen 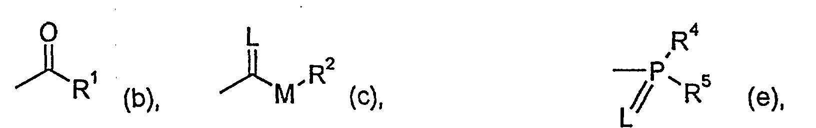 E (f) oder 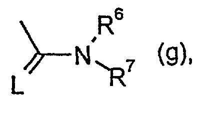 oder im Fall, daß Het für einen der Reste (2), (3), (4), (5) oder (6) steht, für die grüppe -SO₂-R³ (d),
in welchen
E für ein Metallionäquivalent oder ein Ammoniumion steht,
L für Sauerstoff oder Schwefel steht und
M für Sauerstoff oder Schwefel steht.
- R¹: steht bevorzugt für jeweils gegebenenfalls durch Fluor oder Chlor substituiertes C₁-C₁₆-Alkyl, C₂-C₁₆-Alkenyl, C₁-C₆-Alkoxy-C₁-C₆-alkyl, C₁-C₆-Alkylthio-C₁-C₆-alkyl oder Poly-C₁-C₆-alkoxy-C₁-C₆-alkyl oder für gegebenenfalls durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls durch Fluor, Chlor, Brom oder C₁-C₄-Alkyl substituiertes Phenoxy-C₁-C₅-alkyl oder
für jeweils gegebenenfalls durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl.
- R²: steht bevorzugt für jeweils gegebenenfalls durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls durch Fluor, Chlor, C₁-C₄-Alkyl oder C₁-C₄-Alkoxy substituiertes C₃-C₇-Cycloalkyl oder
für jeweils gegebenenfalls durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl.
- R³: steht bevorzugt für gegebenenfalls durch Fluor oder Chlor substituiertes C₁-C₆-Alkyl oder für jeweils gegebenenfalls durch Fluor, Chlor, Brom, C₁-C₅-Alkyl, C₁-C₅-Alkoxy, C₁-C₃-Halogenalkyl, C₁-C₃-Halogenalkoxy, Cyano oder Nitro substituiertes Phenyl oder Benzyl.
- R⁴ und R⁵: stehen unabhängig voneinander bevorzugt für jeweils gegebenenfalls durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio oder C₃-C₄-Alkenylthio oder für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio.
- R⁶ und R⁷: stehen unabhängig voneinander bevorzugt für Wasserstoff, für jeweils gegebenenfalls durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest, in welchem gegebenenfalls eine Methylengruppe durch Sauerstoff oder Schwefel ersetzt ist.
- R¹³: steht bevorzugt für Wasserstoff, für jeweils gegebenenfalls durch Fluor oder Chlor substituiertes C₁-C₆-Alkyl oder C₁-C₆-Alkoxy, für gegebenenfalls durch Fluor, C₁-C₂-Alkyl oder C₁-C₂-Alkoxy substituiertes C₃-C₇-Cycloalkyl, in welchem gegebenenfalls eine Methylengruppe durch Sauerstoff oder Schwefel ersetzt ist oder für jeweils gegebenenfalls durch Fluor, Chlor, Brom, C₁-C₅-Alkyl, C₁-C₅-Alkoxy, C₁-C₂-Halogenalkyl, C₁-C₂-Halogenalkoxy, Nitro oder Cyano substituiertes Phenyl, Phenyl-C₁-C₃-alkyl oder Phenyl-C₁-C₂-alkyloxy.
- R¹⁴: steht bevorzugt für Wasserstoff oder C₁-C₆-Alkyl oder
- R¹³ und R¹⁴: stehen gemeinsam bevorzugt für C₄-C₆-Alkandiyl.
- R¹⁵ und R¹⁶: sind gleich oder verschieden und stehen bevorzugt für C₁-C₄-Alkyl oder
- R¹⁵ und R¹⁶: stehen zusammen bevorzugt für einen C₂-C₃-Alkandiylrest, der gegebenenfalls durch C₁-C₄-Alkyl oder durch gegebenenfalls durch Fluor, Chlor, Brom, C₁-C₂-Alkyl, C₁-C₂-Halogenalkyl, C₁-C₂-Alkoxy, C₁-C₂-Halogenalkoxy, Nitro oder Cyano substituiertes Phenyl substituiert ist.
- R¹⁷ und R¹⁸: stehen unabhängig voneinander bevorzugt für Wasserstoff, für gegebenenfalls durch Fluor oder Chlor substituiertes C₁-C₈-Alkyl oder für gegebenenfalls durch Fluor, Chlor, C₁-C₄-Alkyl, C₁-C₄-Alkoxy, C₁-C₂-Halogenalkyl, C₁-C₂-Halogenalkoxy, Nitro oder Cyano substituiertes Phenyl oder
- R¹⁷ und R¹⁸: stehen bevorzugt gemeinsam mit dem Kohlenstoffatom, an das sie gebunden sind, für gegebenenfalls durch C₁-C₃-Alkyl oder C₁-C₃-Alkoxy substituiertes C₅-C₆-Cycloalkyl, in dem gegebenenfalls eine Methylengruppe durch Sauerstoff oder Schwefel ersetzt ist.
- R¹⁹ und R²⁰: stehen unabhängig voneinander bevorzugt für C₁-C₆-Alkyl, C₂-C₆-Alkenyl, C₁-C₆-Alkoxy, C₁-C₆-Alkylamino, C₃-C₆-Alkenylamino, Di-(C₁-C₆-alkyl)amino oder Di-(C₃-C₆-alkenyl)amino.
- X: steht besonders bevorzugt für Fluor, Chlor oder Brom.
- Y: steht besonders bevorzugt für Fluor, Chlor, Brom, Methyl, Ethyl, n-Propyl oder iso-Propyl.
- Z: steht besonders bevorzugt für Fluor, Chlor, Brom, Methyl, Ethyl, n-Propyl oder iso-Propyl.

Dabei gilt, daß immer einer der Reste Y und Z für Halogen und der andere für Alkyl steht.
- Het: steht besonders bevorzugt für eine der Gruppen 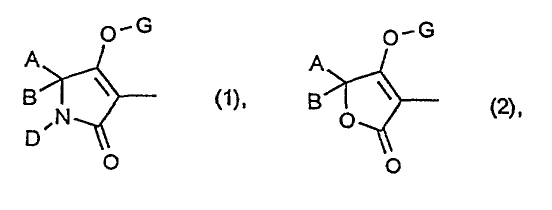 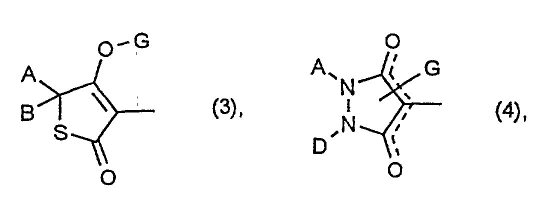 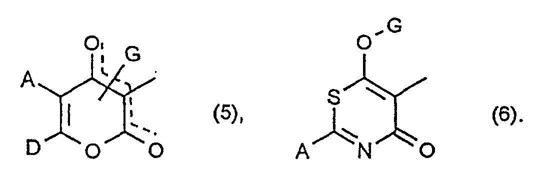
- A: steht besonders bevorzugt für Wasserstoff, für jeweils gegebenenfalls durch Fluor oder Chlor substituiertes C₁-C₈-Alkyl, C₂-C₄-Alkenyl, C₁-C₆-Alkoxy-C₁-C₄-alkyl, Poly-C₁-C₄-alkoxy-C₁-C₄-alkyl oder C₁-C₆-Alkylthio-C₁-C₄-alkyl, oder für gegebenenfalls durch Fluor, Chlor, Methyl oder Methoxy substituiertes C₃-C₆-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Pyridyl oder Benzyl.
- B: steht besonders bevorzugt für Wasserstoff, C₁-C₈-Alkyl oder C₁-C₄-Alkoxy-C₁-C₂-alkyl oder
- A, B: und das Kohlenstoffatom an das sie gebunden sind, stehen besonders bevorzugt für C₃-C₈-Cycloalkyl oder C₅-C₈-Cycloalkenyl, worin jeweils gegebenenfalls eine Methylengruppe durch Sauerstoff oder Schwefel ersetzt ist und welche gegebenenfalls durch Methyl, Ethyl, n-Propyl, iso-Ppropyl, Butyl, iso-Butyl, sec.-Butyl, tert.-Butyl, Cyclohexyl, Trifluormethyl, Methoxy, Ethoxy, n-Propoxy, iso-Propoxy, Butoxy, iso-Butoxy, sek.-Butoxy, tert.-Butoxy, Methylthio, Ethylthio, Fluor, Chlor oder Phenyl substituiert sind oder
- A, B: und das Kohlenstoffatom, an das sie gebunden sind, stehen besonders bevorzugt für C₅-C₆-Cycloalkyl, welches durch eine gegebenenfalls ein Sauerstoff- oder Schwefelatom enthaltende Alkylendiyl- oder durch eine Alkylendioxyl-Gruppe substituiert ist, die mit dem Kohlenstoffatom, an das sie gebunden ist, einen weiteren fünf- oder sechsgliedrigen Ring bildet oder
- A, B: und das Kohlenstoffatom, an das sie gebunden sind, stehen besonders bevorzugt für C₃-C₆-Cycloalkyl oder C₅-C₆-Cycloalkenyl, in welchen zwei Substituenten gemeinsam mit den Kohlenstoffatomen, an die sie gebunden sind, für C₃-C₄-Alkandiyl, C₃-C₄-Alkendiyl oder Butadiendiyl stehen, worin jeweils gegebenenfalls eine Methylengruppe durch Sauerstoff oder Schwefel ersetzt ist.
- D: steht besonders bevorzugt für Wasserstoff, für jeweils gegebenenfalls durch Fluor oder Chlor substituiertes C₁-C₈-Alkyl, C₃-C₄-Alkenyl, C₃-C₄-Alkinyl, C₁-C₆-Alkoxy-C₂-C₄-alkyl, Poly-C₁-C₄-alkoxy-C₂-C₄-alkyl, C₁-C₄-Alkylthio-C₂-C₄-alkyl oder C₃-C₆-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Furanyl, Pyridyl, Thienyl oder Benzyl,
oder
- A und D: stehen gemeinsam besonders bevorzugt für eine C₃-C₅-Alkandiyl- oder C₃-C₅-Alkendiylgruppe, worin jeweils gegebenenfalls eine Methylengruppe durch Sauerstoff oder Schwefel ersetzt ist und welche gegebenenfalls durch Fluor, Chlor, Hydroxy, Mercapto oder durch jeweils gegebenenfalls durch Fluor oder Chlor substituiertes C₁-C₆-Alkyl, C₁-C₄-Alkoxy, C₁-C₄-Alkylthio, C₃-C₆-Cycloalkyl, Phenyl oder Benzyloxy substituiert sind oder
worin jeweils gegebenenfalls eine der folgenden Gruppen 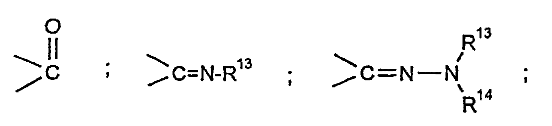 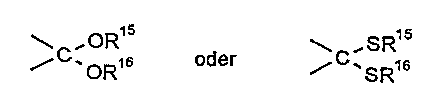 enthalten ist,
oder A und D stehen im Fall der Verbindungen der Formel (I-1) gemeinsam mit den Atomen, an die sie gebunden sind, für eine der folgenden Gruppen: 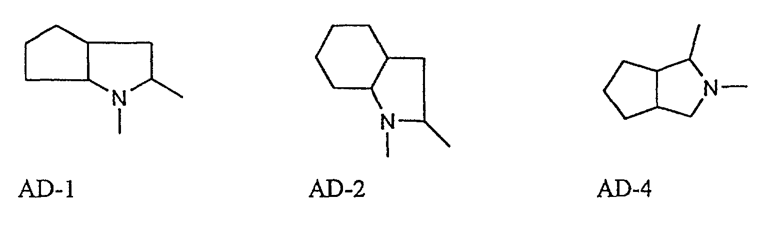 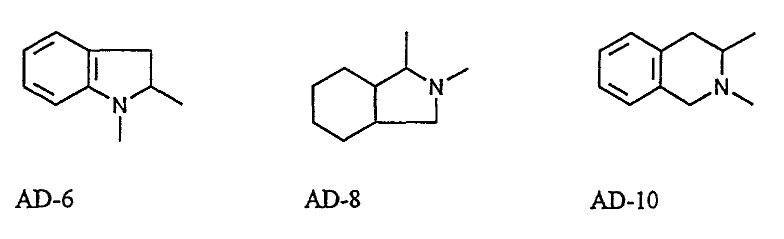 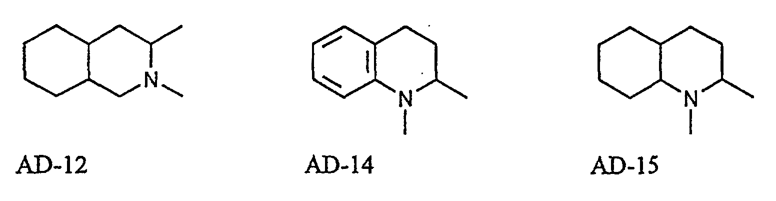 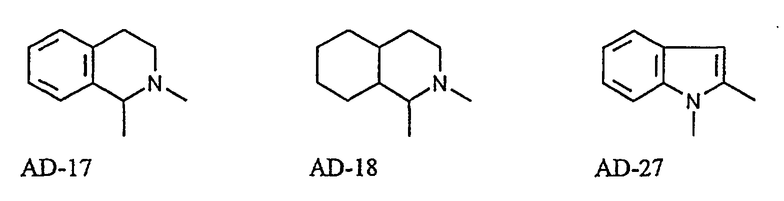
- G: steht besonders bevorzugt, im Fall, daß Het für einen der Reste (1), (2), (3), (5) oder (6) steht, für Wasserstoff (a) oder, im Fall, daß Het für einen der Reste (1), (2), (3), (4), (5) oder (6) steht, für eine der Gruppen 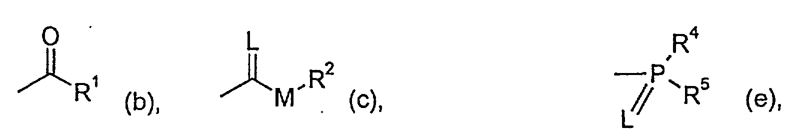 E (f) oder 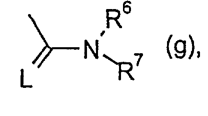 oder im Fall, daß Het für einen der Reste (2), (3), (4), (5) oder (6) steht, für die grüppe - SO₂-R³ (d),
in welchen
E für ein Metallionäquivalent oder ein Ammoniumion steht,
L für Sauerstoff oder Schwefel steht und
M für Sauerstoff oder Schwefel steht.
- R¹: steht besonders bevorzugt für jeweils gegebenenfalls durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, Poly-C₁-C₄-alkoxy-C₁-C₄-alkyl oder für gegebenenfalls durch Fluor, Chlor, Methyl, Ethyl, n-Propyl, i-Propyl, n-Butyl, i-Butyl, tert.-Butyl, Methoxy, Ethoxy, n-Propoxy oder iso-Propoxy substituiertes C₃-C₆-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, Methyl, Ethyl, n-Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für gegebenenfalls durch Fluor, Chlor, Brom, Methyl, Ethyl, n-Propyl, i-Propyl, Methoxy, Ethoxy, Trifluormethyl oder Trifluormethoxy substituiertes Benzyl,
für jeweils gegebenenfalls durch Fluor, Chlor, Brom, Methyl oder Ethyl substituiertes Furanyl, Thienyl oder Pyridyl,
für gegebenenfalls durch Fluor, Chlor, Methyl oder Ethyl substituiertes Phenoxy-C₁-C₄-alkyl oder
für jeweils gegebenenfalls durch Fluor, Chlor, Amino, Methyl oder Ethyl substituiertes Pyridyloxy-C₁-C₄-alkyl, Pyrimidyloxy-C₁-C₄-alkyl oder Thiazolyloxy-C₁-C₄-alkyl.
- R²: steht besonders bevorzugt für jeweils gegebenenfalls durch Fluor oder Chlor substituiertes C₁-C₁₄-Alkyl, C₂-C₁₄-Alkenyl, C₁-C₄-Alkoxy-C₂-C₆-alkyl oder Poly-C₁-C₄-alkoxy-C₂-C₆-alkyl,
für gegebenenfalls durch Fluor, Chlor, Methyl, Ethyl, n-Propyl, iso-Propyl oder Methoxy substituiertes C₃-C₆-Cycloalkyl,
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl.
- R³: steht besonders bevorzugt für gegebenenfalls durch Fluor oder Chlor substituiertes Methyl, Ethyl, Propyl, Isopropyl oder jeweils gegebenenfalls durch Fluor, Chlor, Brom, Methyl, Ethyl, Propyl, iso-Propyl, tert.-Butyl, Methoxy, Ethoxy, Isopropoxy, tert.-Butoxy, Trifluormethyl, Trifluormethoxy, Cyano oder Nitro substituiertes Phenyl oder Benzyl.
- R⁴ und R⁵: stehen unabhängig voneinander besonders bevorzugt für jeweils gegebenenfalls durch Fluor oder Chlor substituiertes C₁-C₄-Alkyl, C₁-C₄-Alkoxy, C₁-C₄-Alkylamino, Di-(C₁-C₄-alkyl)amino oder C₁-C₄-Alkylthio oder für jeweils gegebenenfalls durch Fluor, Chlor, Brom, Nitro, Cyano, Methyl, Methoxy, Trifluormethyl oder Trifluormethoxy substituiertes Phenyl, Phenoxy oder Phenylthio.
- R⁶ und R⁷: stehen unabhängig voneinander besonders bevorzugt für Wasserstoff, für jeweils gegebenenfalls durch Fluor oder Chlor substituiertes C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für jeweils gegebenenfalls durch Fluor, Chlor, Brom, Methyl, Methoxy oder Trifluormethyl substituiertes Phenyl oder Benzyl, oder zusammen für einen gegebenenfalls durch Methyl oder Ethyl substituierten C₅-C₆-Alkylenrest, in welchem gegebenenfalls eine Methylengruppe durch Sauerstoff oder Schwefel ersetzt ist.
- R¹³: steht besonders bevorzugt für Wasserstoff, für jeweils gegebenenfalls durch Fluor oder Chlor substituiertes C₁-C₄-Alkyl oder C₁-C₄-Alkoxy, für C₃-C₆-Cycloalkyl oder für jeweils gegebenenfalls durch Fluor, Chlor, Brom, Methyl, Ethyl, iso-Propyl, tert.-Butyl, Methoxy, Ethoxy, iso-Propoxy, tert.-Butoxy, Trifluormethyl, Trifluormethoxy, Nitro oder Cyano substituiertes Phenyl, Phenyl-C₁-C₂-alkyl oder Benzyloxy.
- R¹⁴: steht besonders bevorzugt für Wasserstoff oder C₁-C₄-Alkyl oder
- R¹³ und R¹⁴: stehen gemeinsam besonders bevorzugt für C₄-C₆-Alkandiyl.
- R¹⁵ und R¹⁶: sind gleich oder verschieden und stehen besonders bevorzugt für Methyl oder Ethyl oder
- R¹⁵ und R¹⁶: stehen zusammen besonders bevorzugt für einen C₂-C₃-Alkandiylrest, der gegebenenfalls durch Methyl, Ethyl, n-Propyl, iso-Propyl, n-Butyl, iso-Butyl, sek.-Butyl, tert.-Butyl oder durch gegebenenfalls durch Fluor, Chlor, Methoxy, Trifluormethyl, Trifluormethoxy, Nitro oder Cyano substituiertes Phenyl substituiert ist.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie fiir die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1-a) genannt:

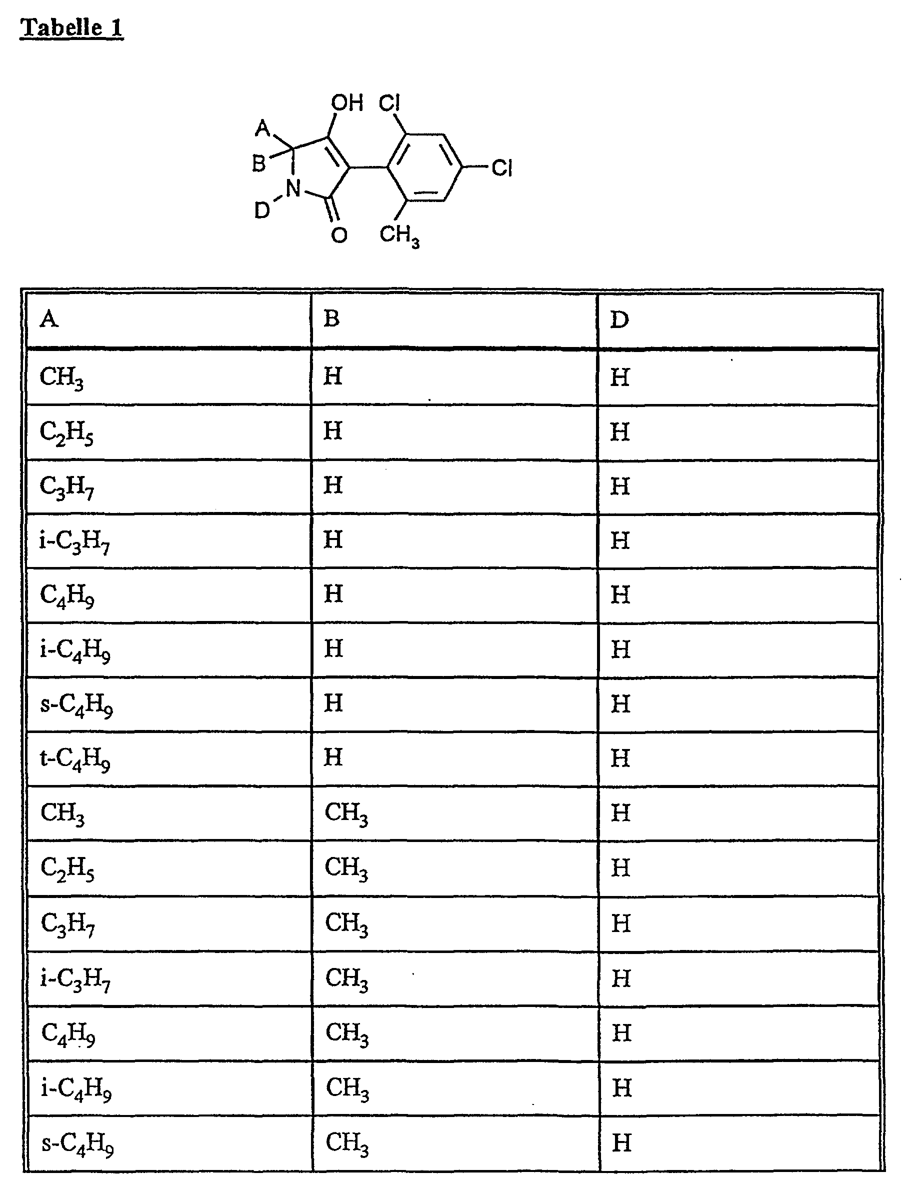

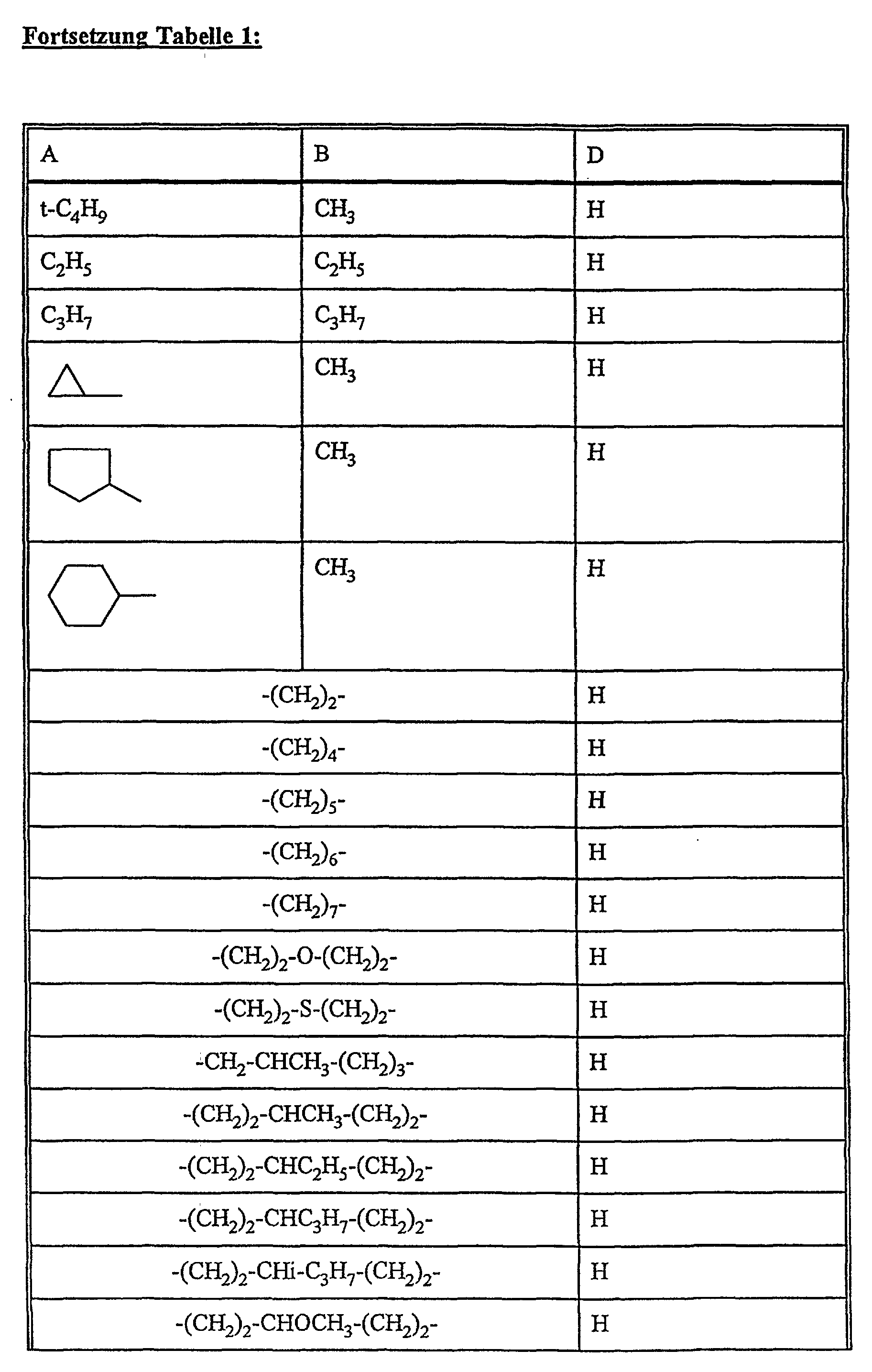

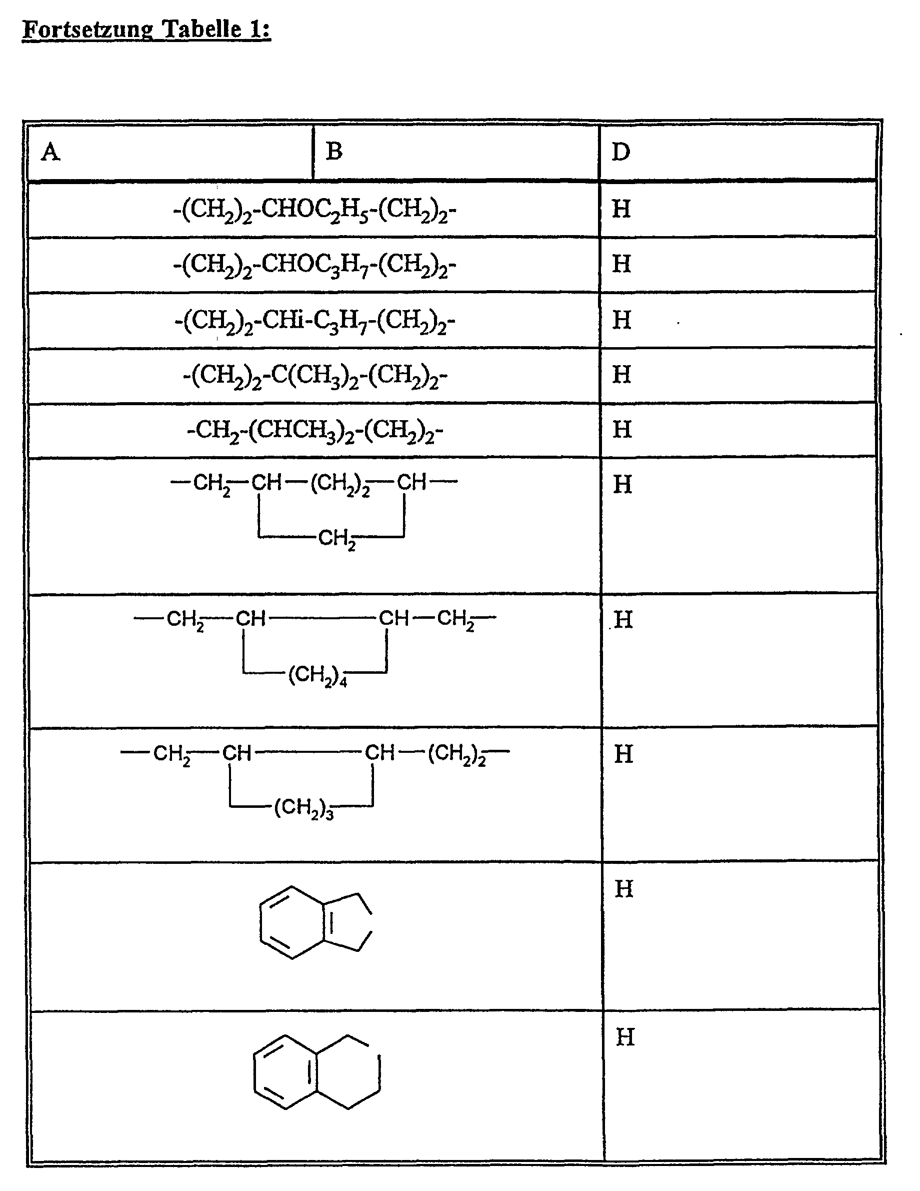

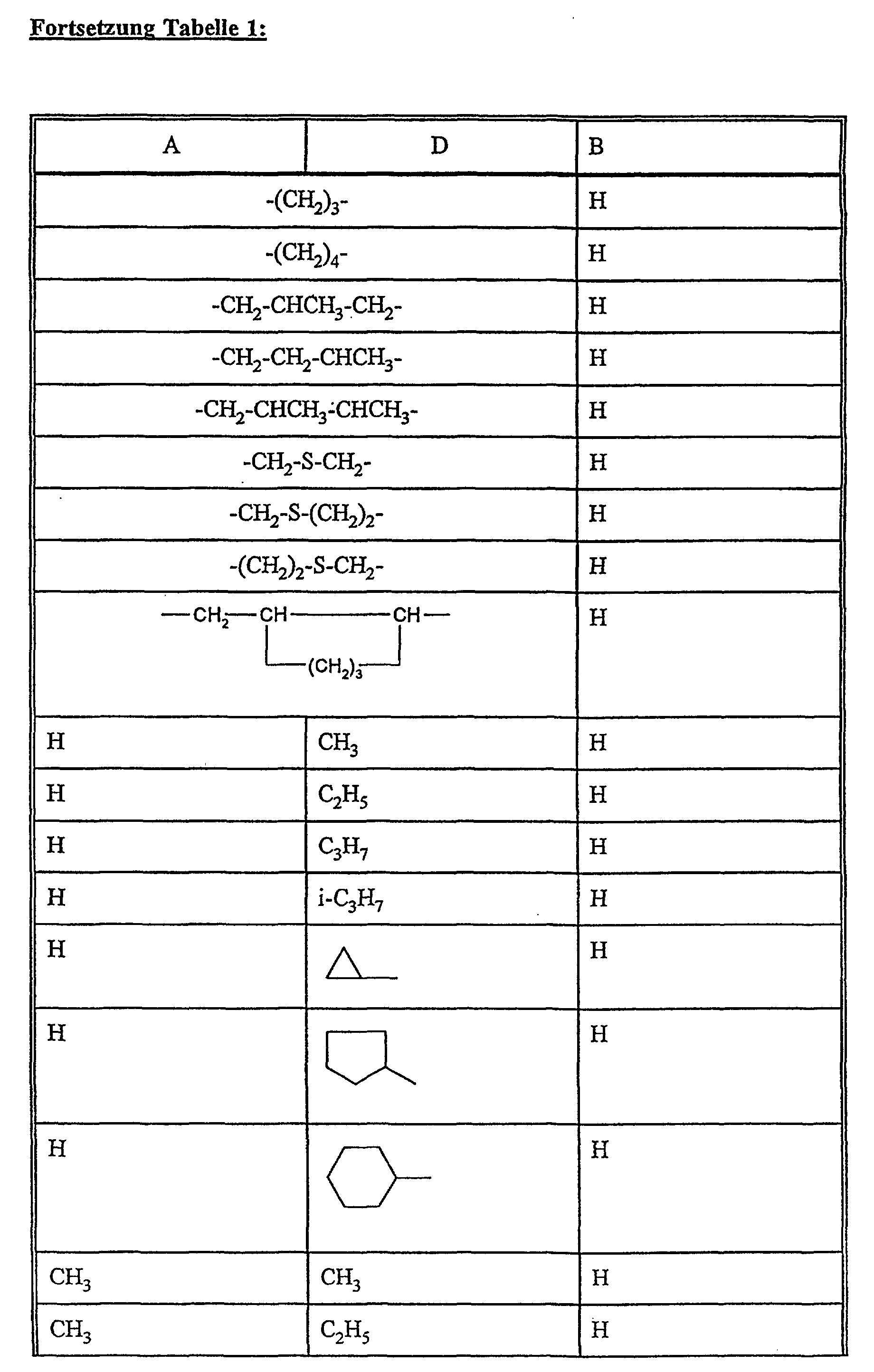

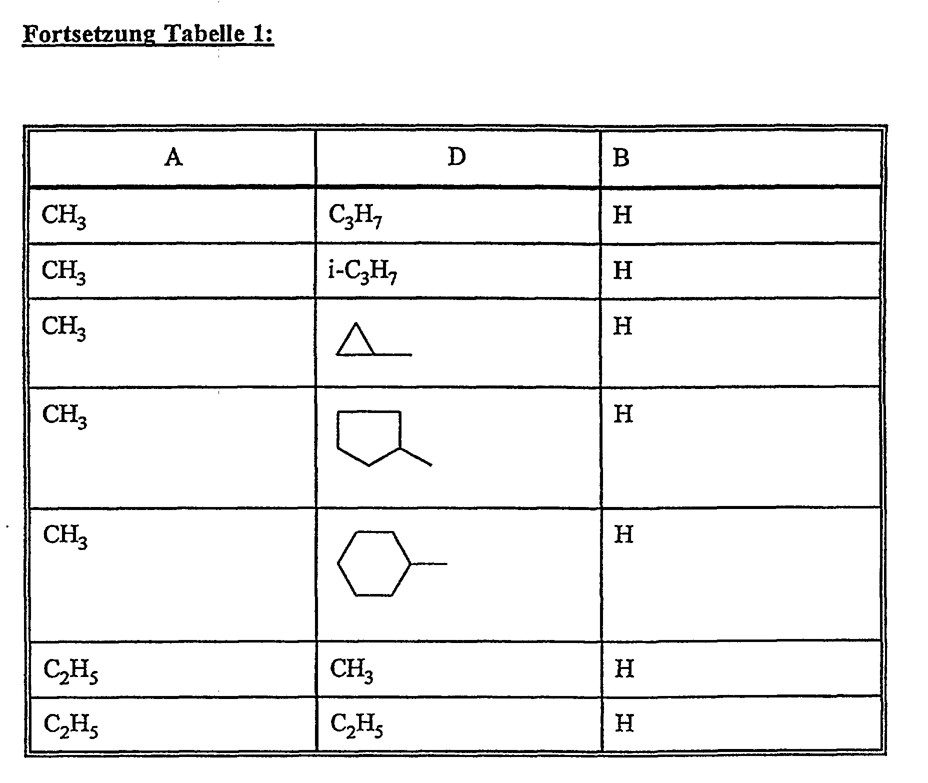

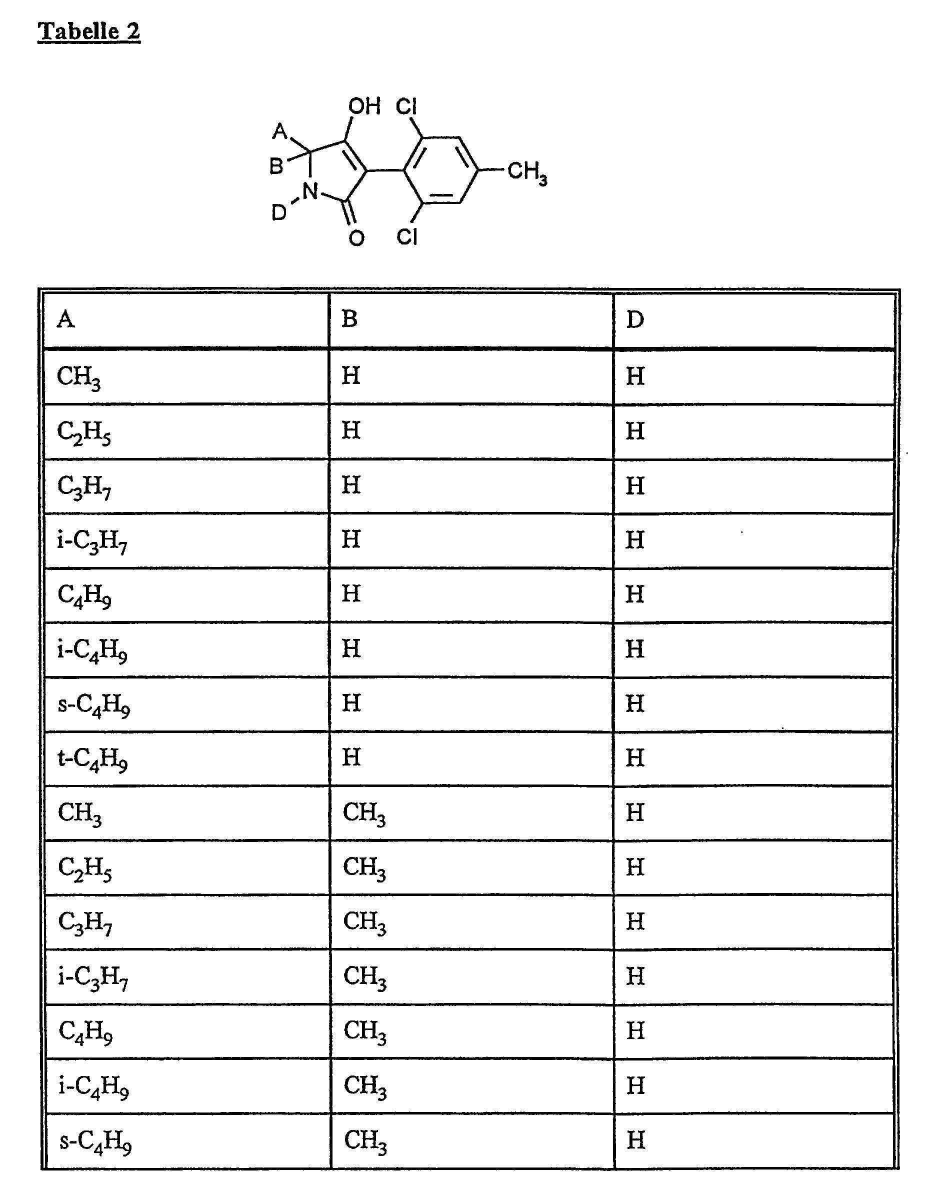

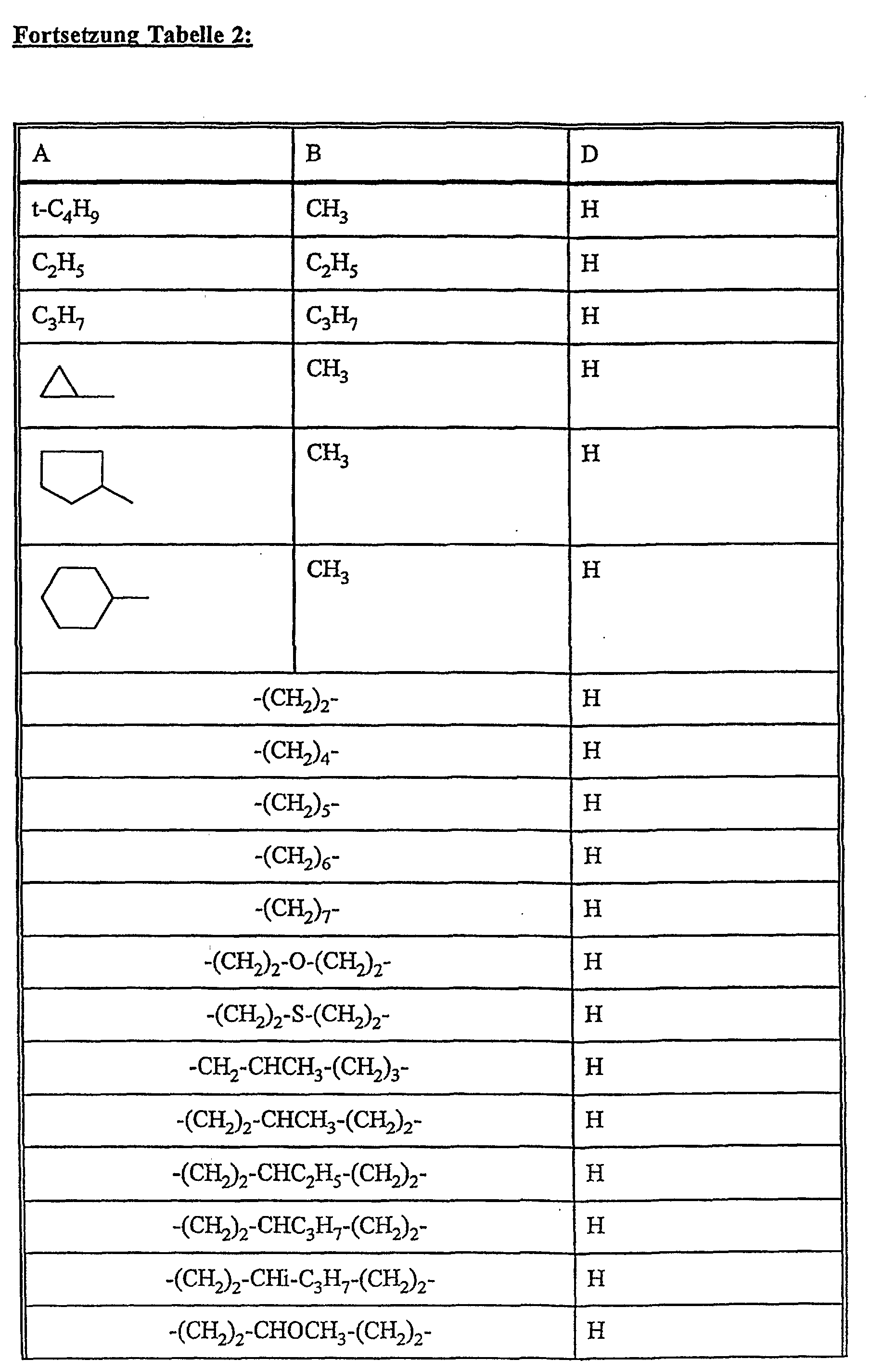

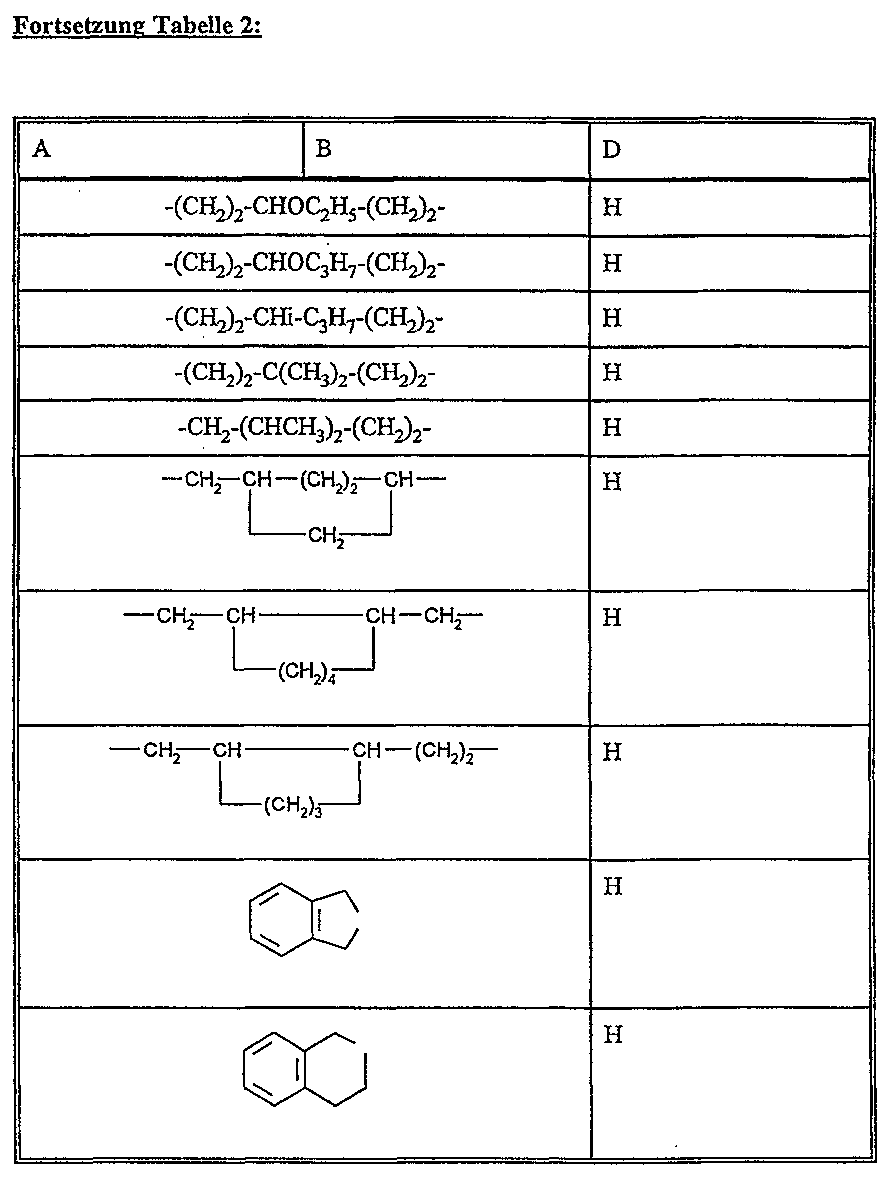

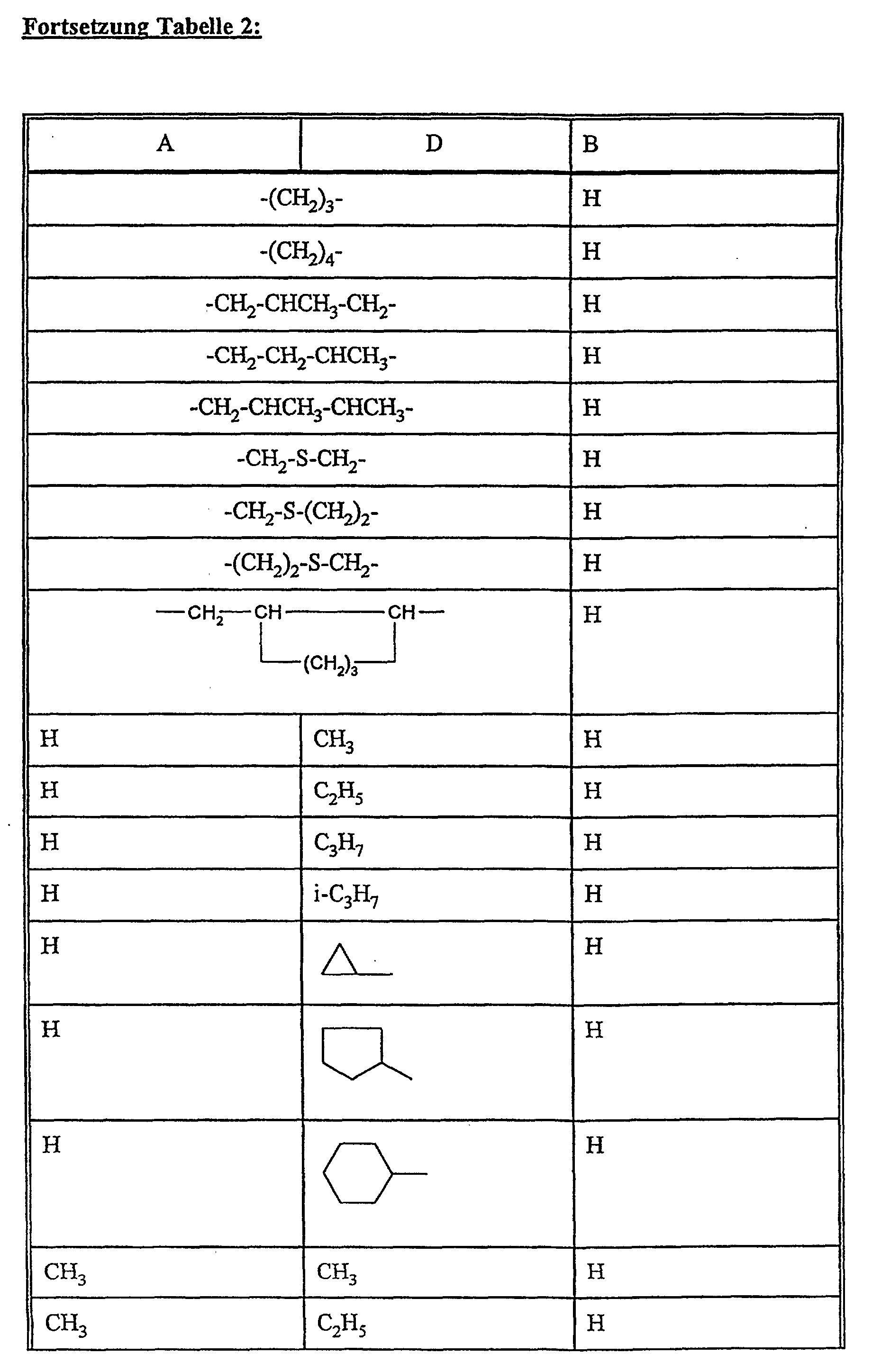

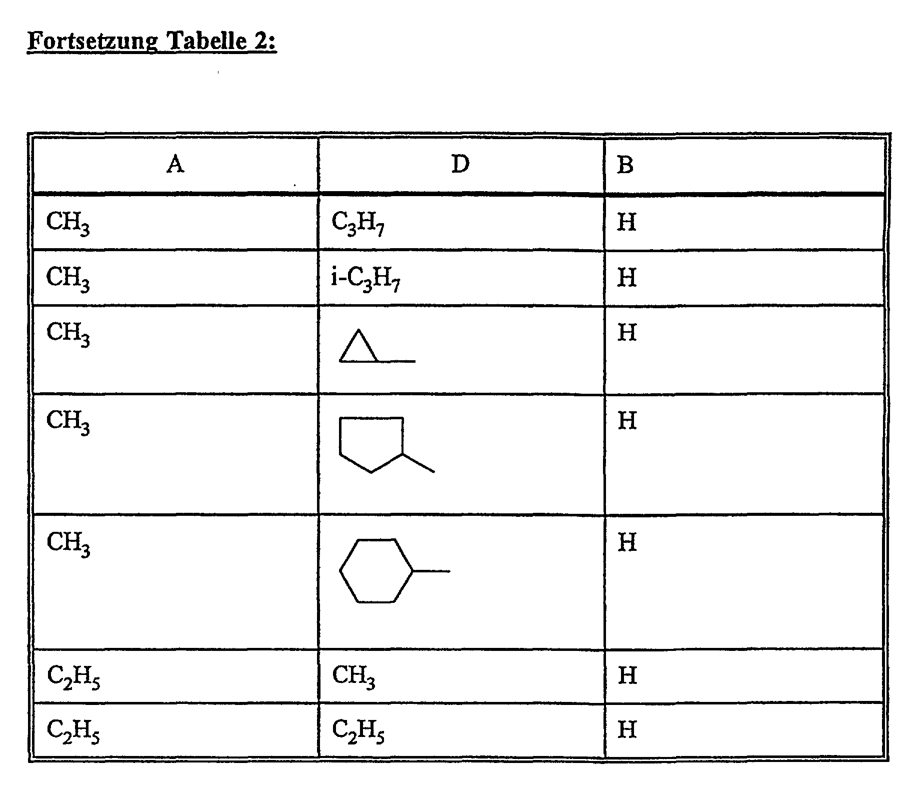

Im einzelnen seien außer den bei den Herstellungsbeispielen genannten Verbindungen die folgenden Verbindungen der Formel (I-2-a) genannt:

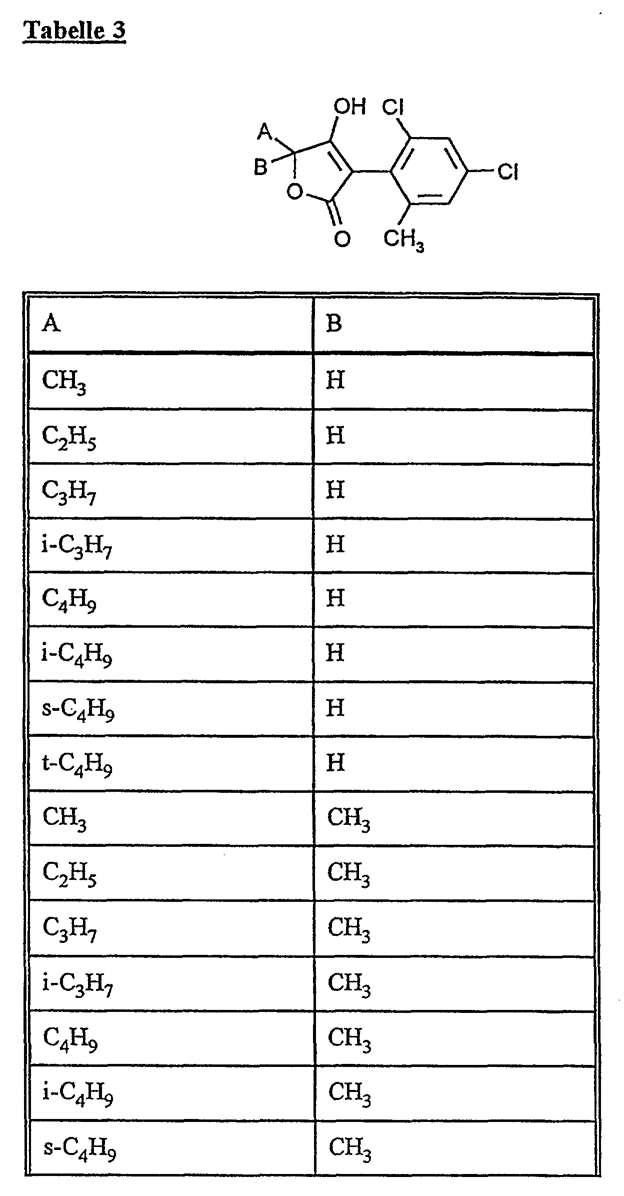

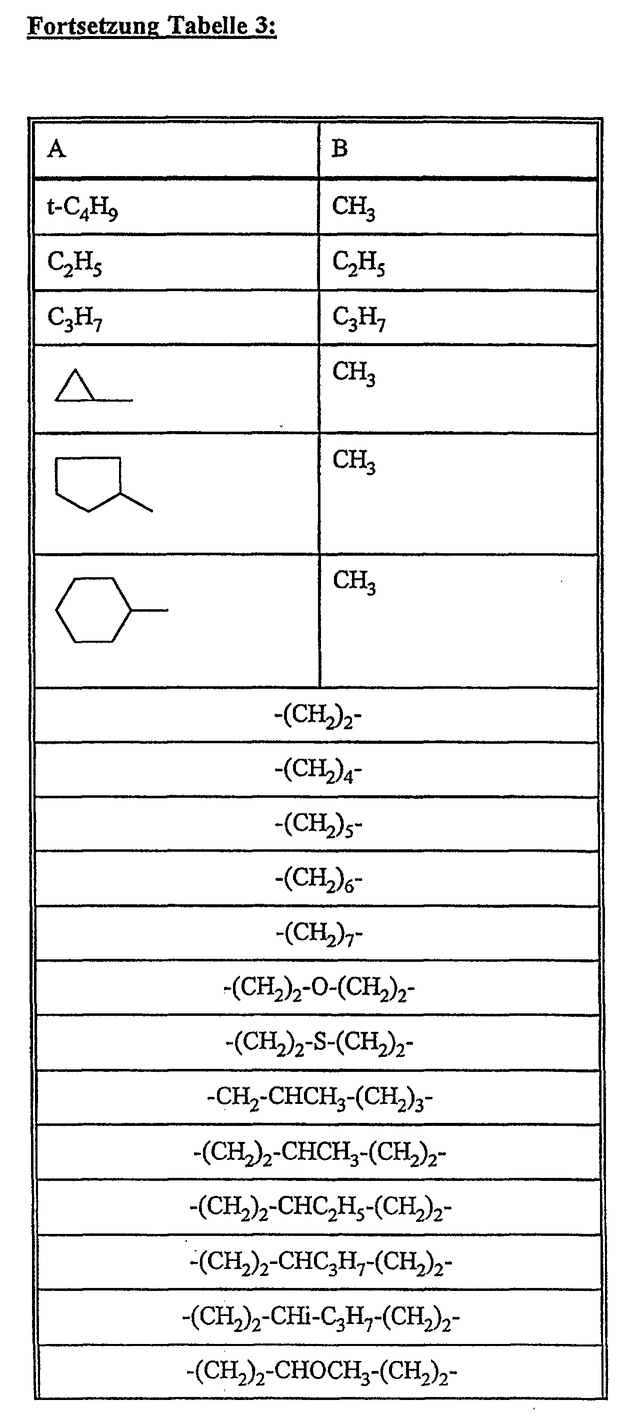

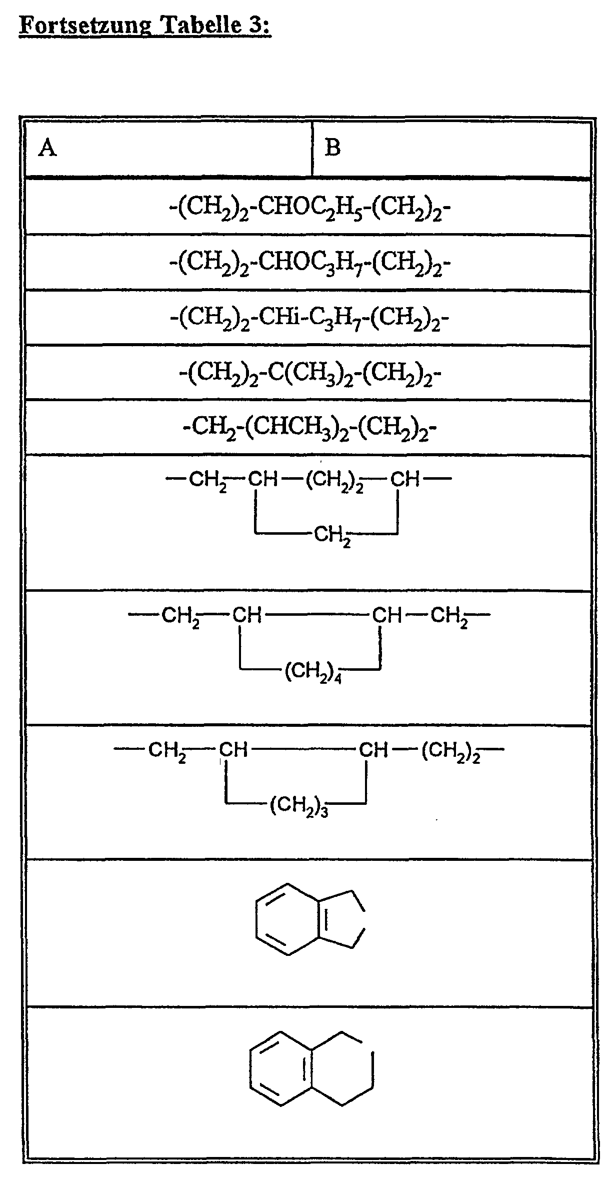

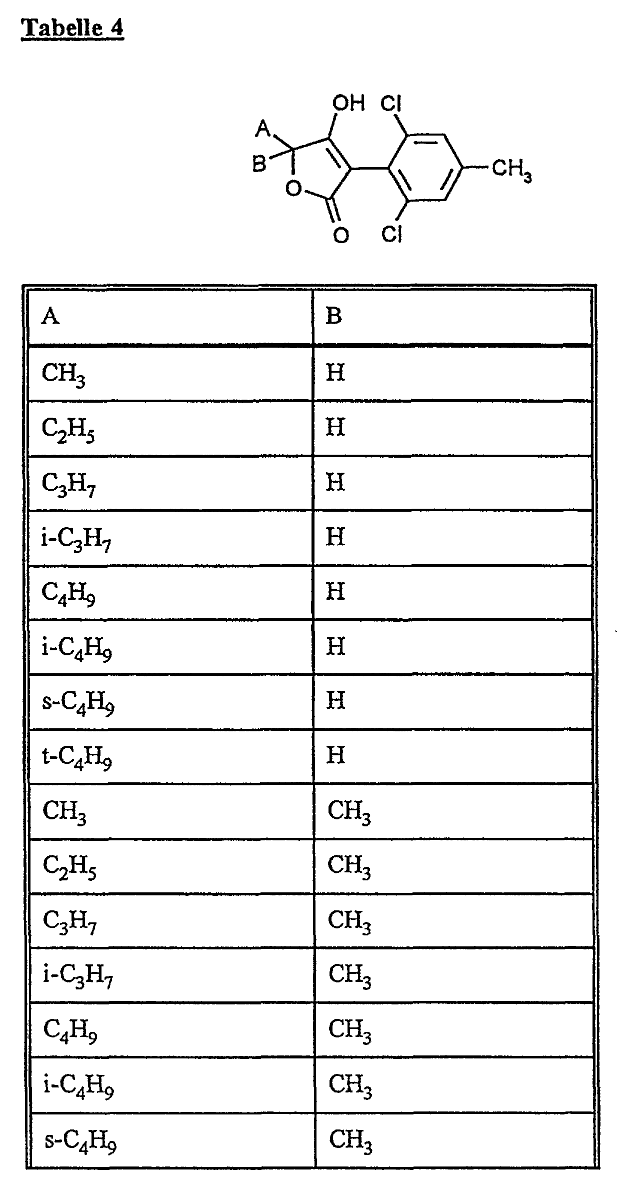

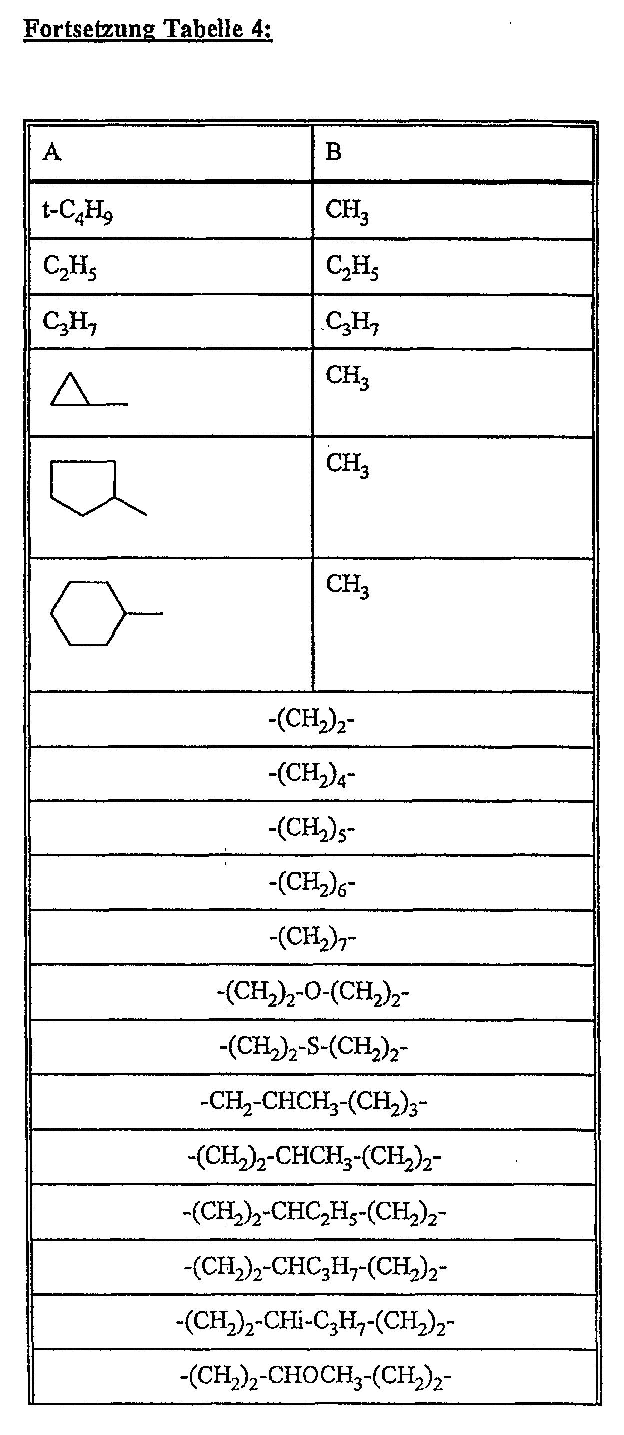

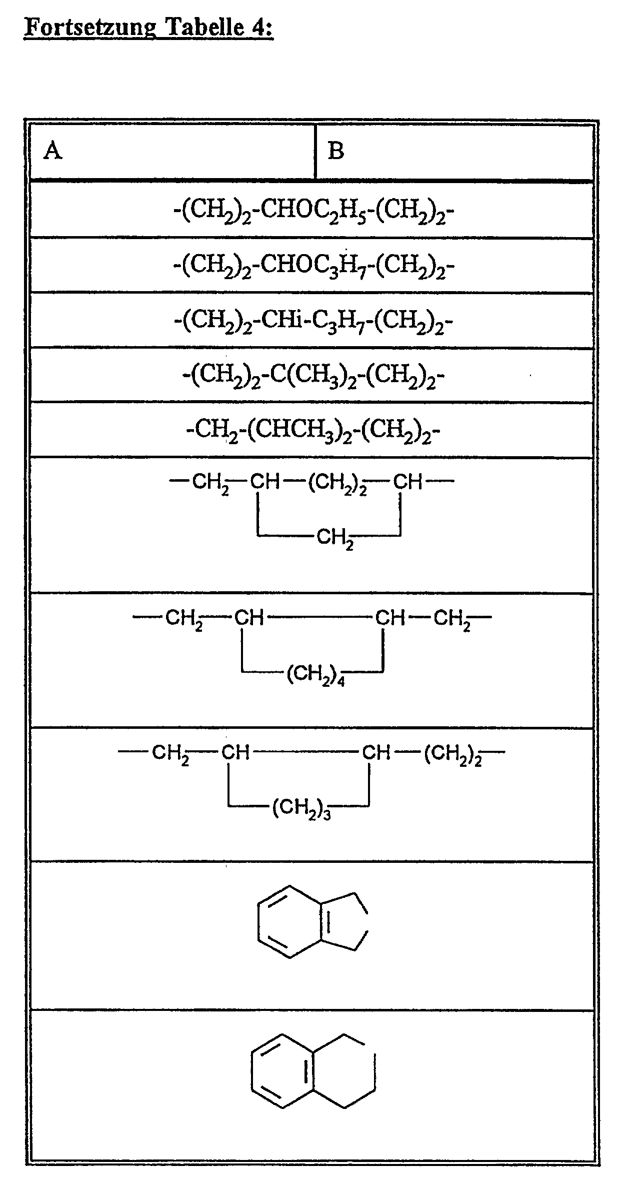

Im einzelnen seien außer den bei den Herstellungsbeispielen genannten Verbindungen die folgenden Verbindungen der Formel (I-3-a) genannt:

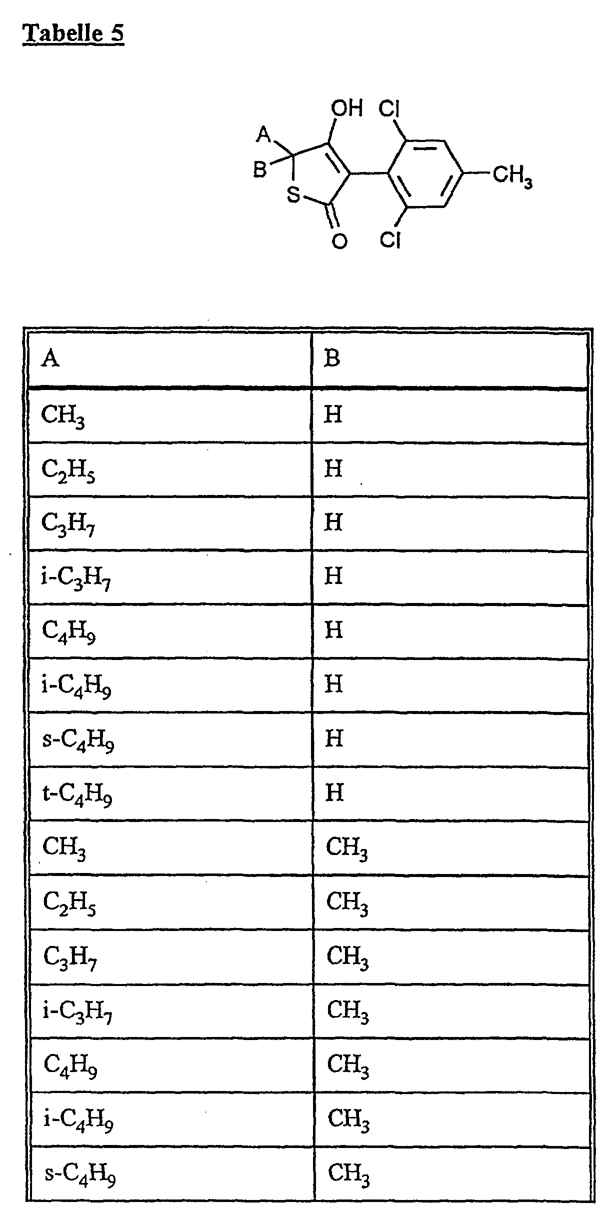

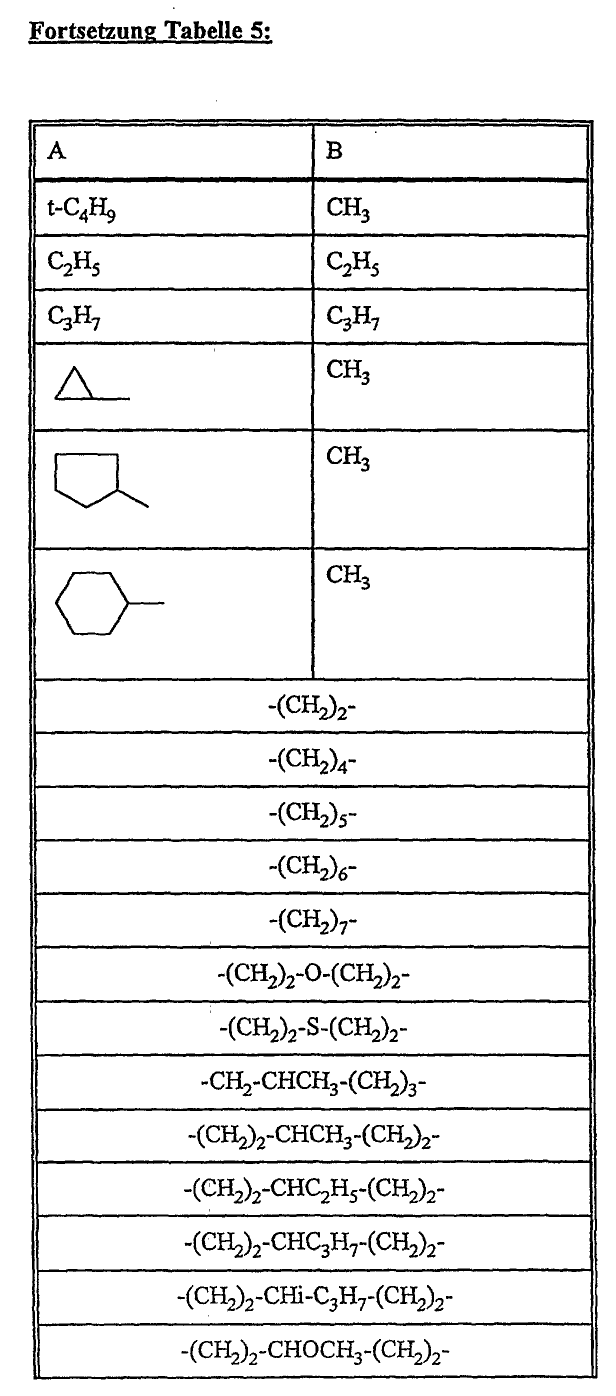

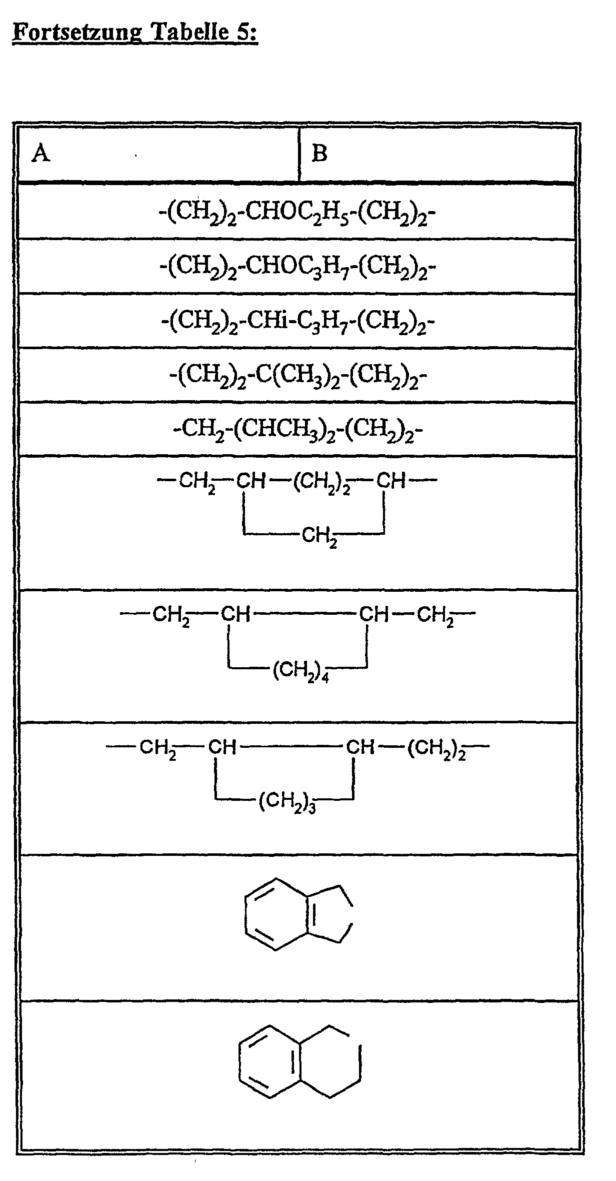

Im einzelnen seien außer den bei den Herstellungsbeispielen genannten Verbindungen die folgenden Verbindungen der Formel (I-5-a) genannt:

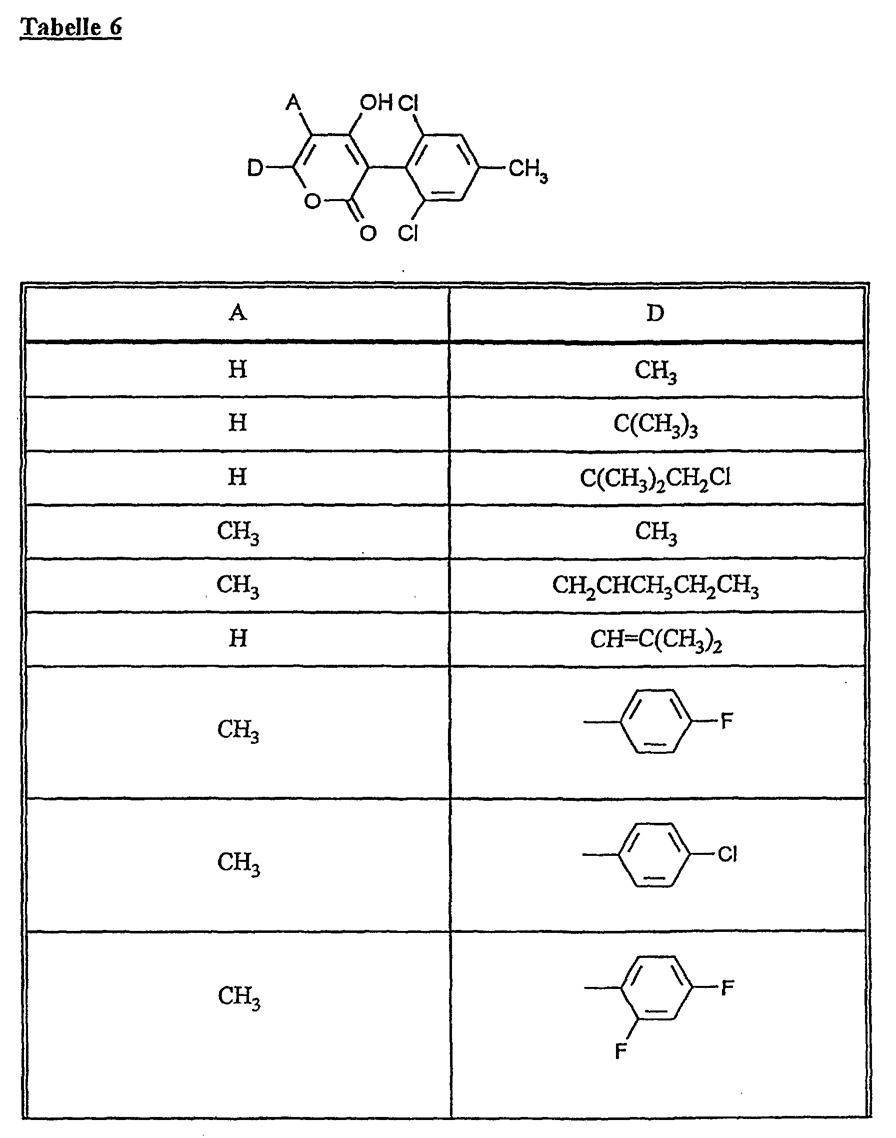

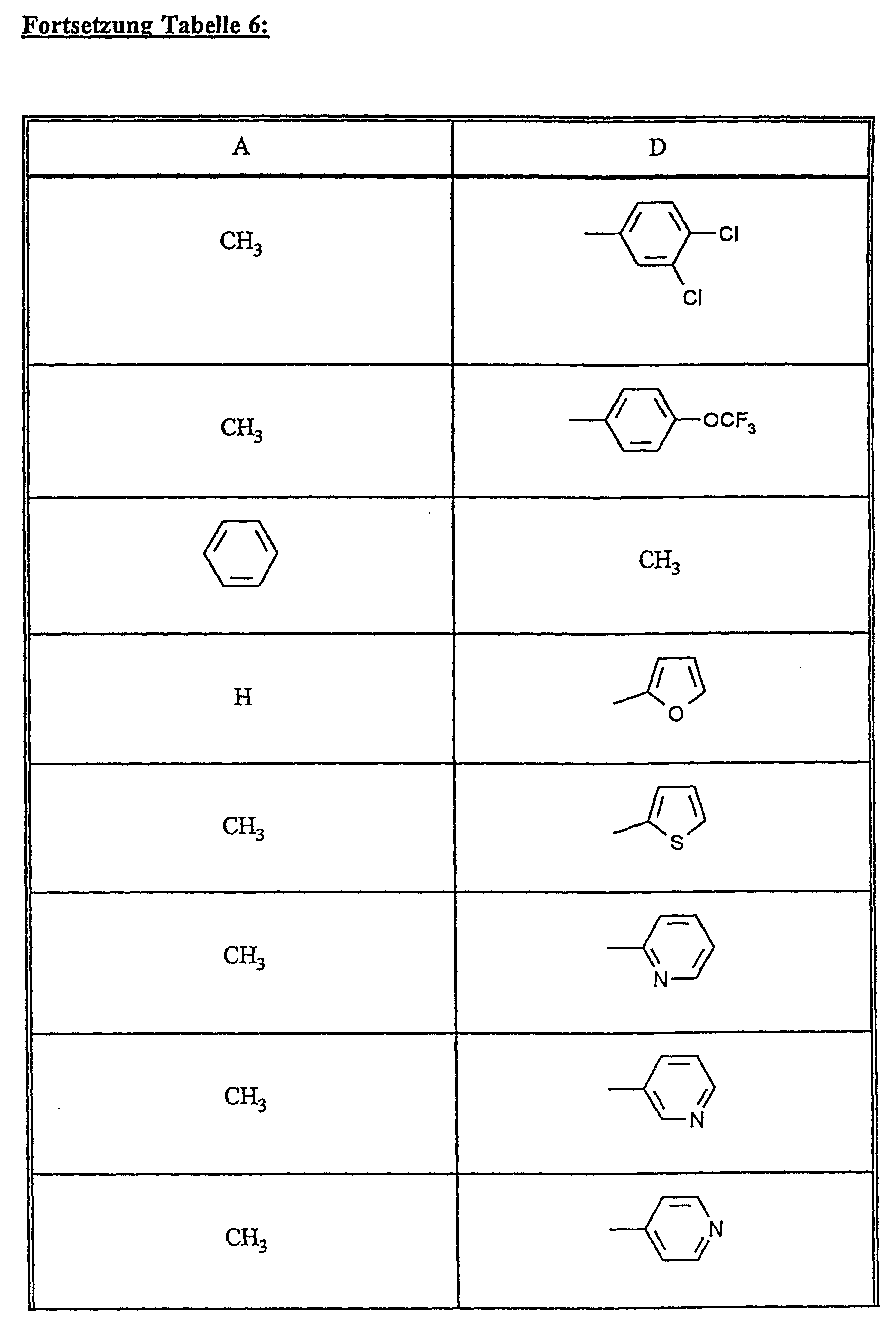

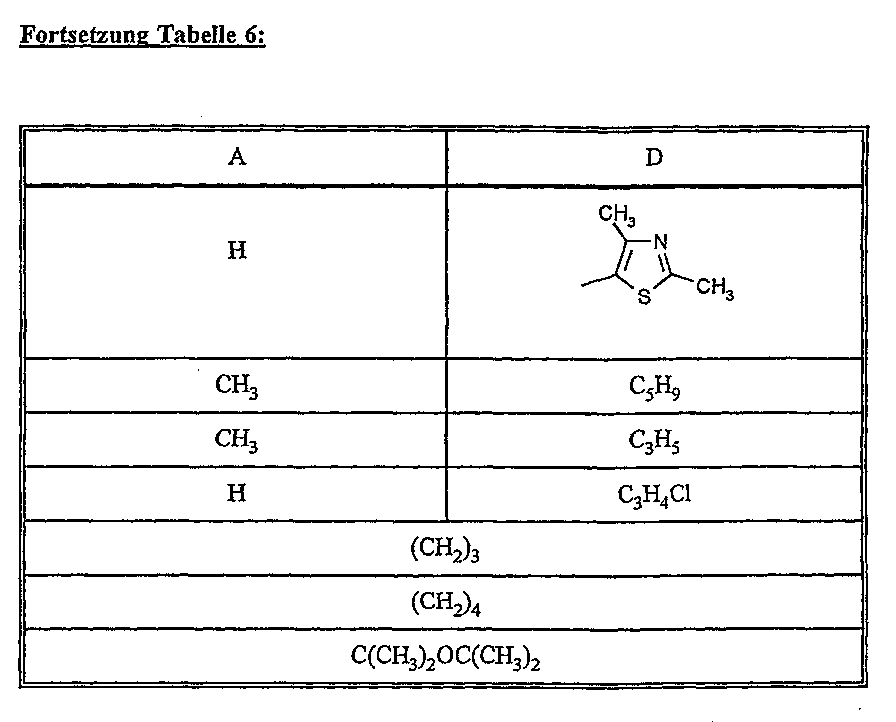

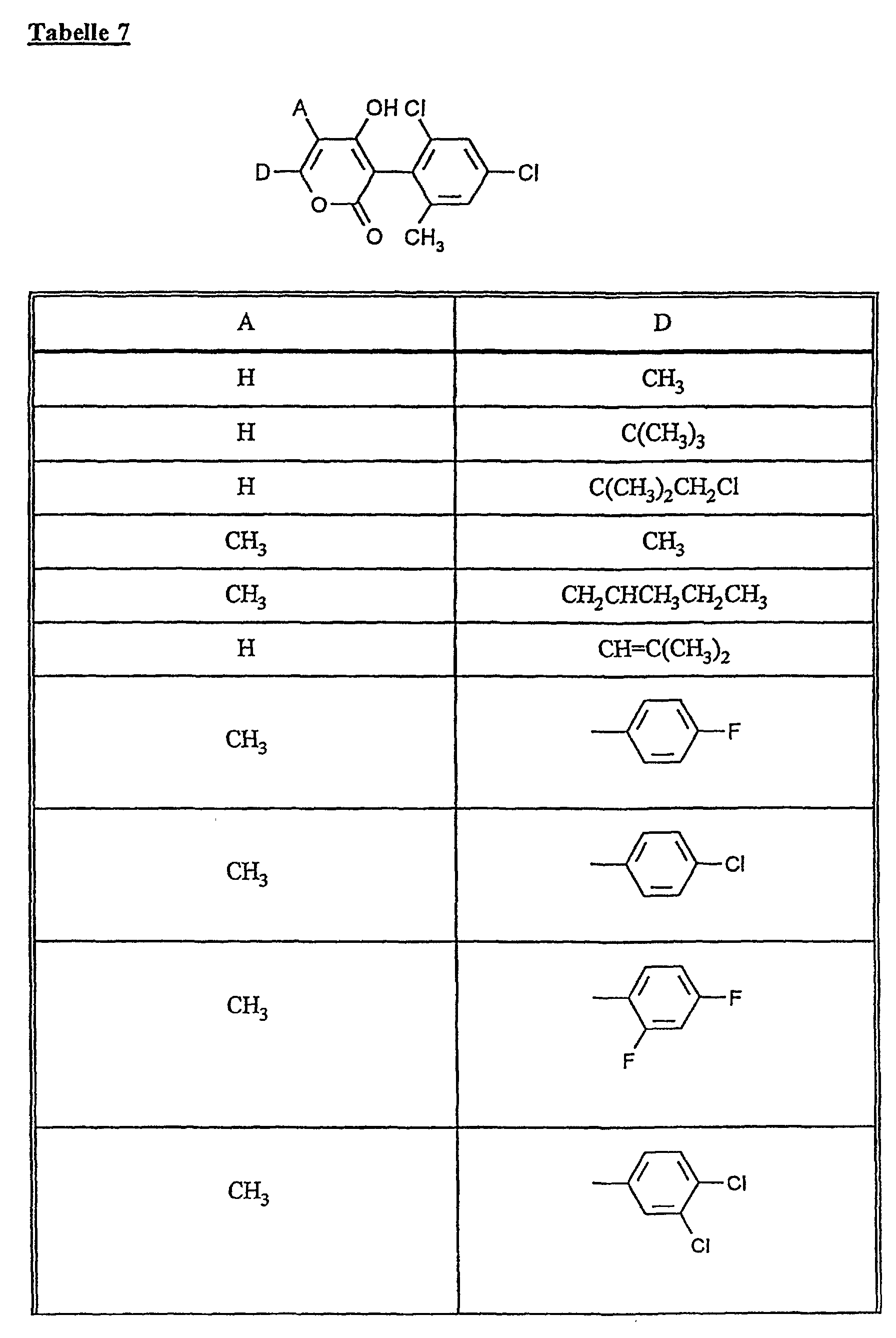

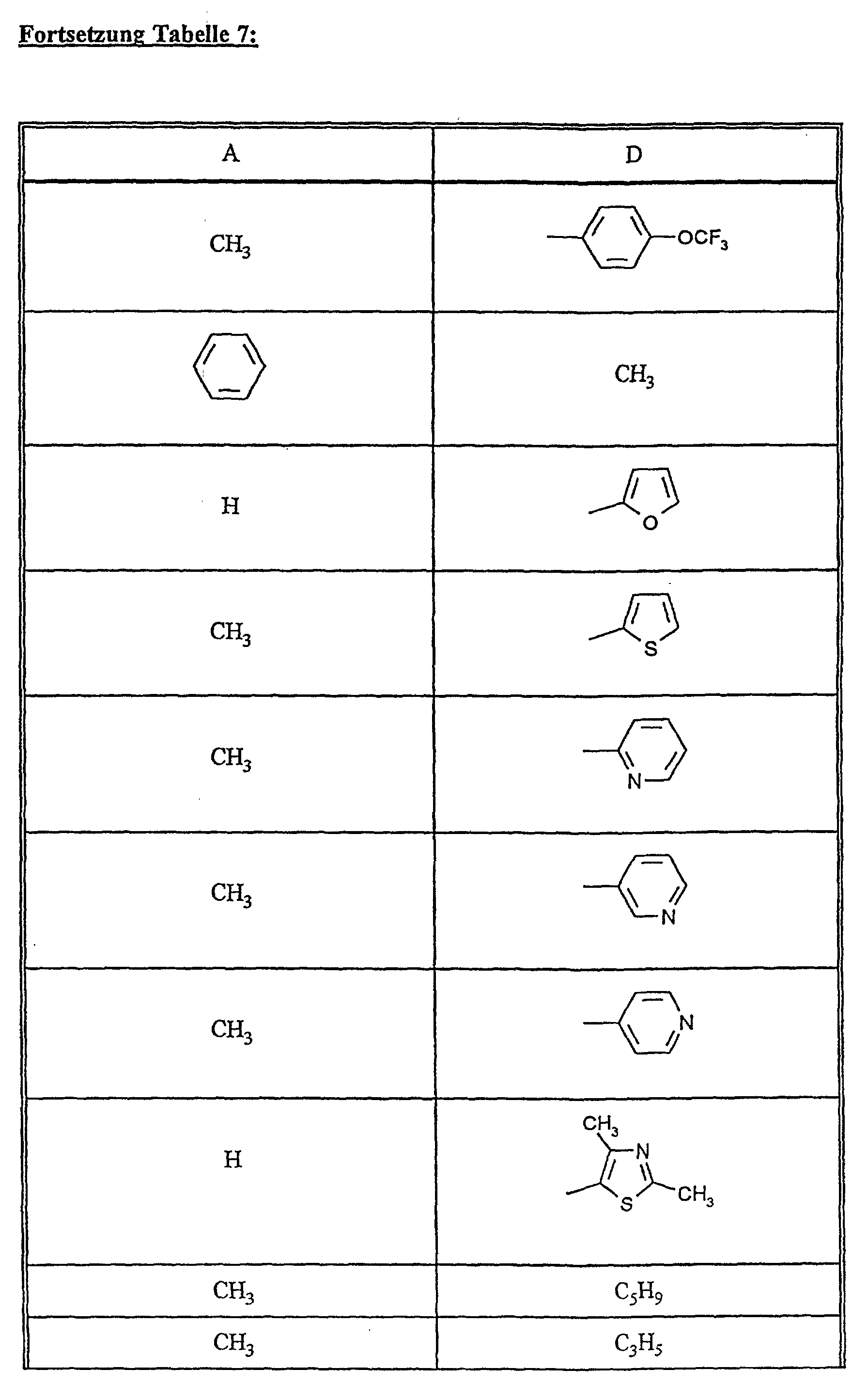

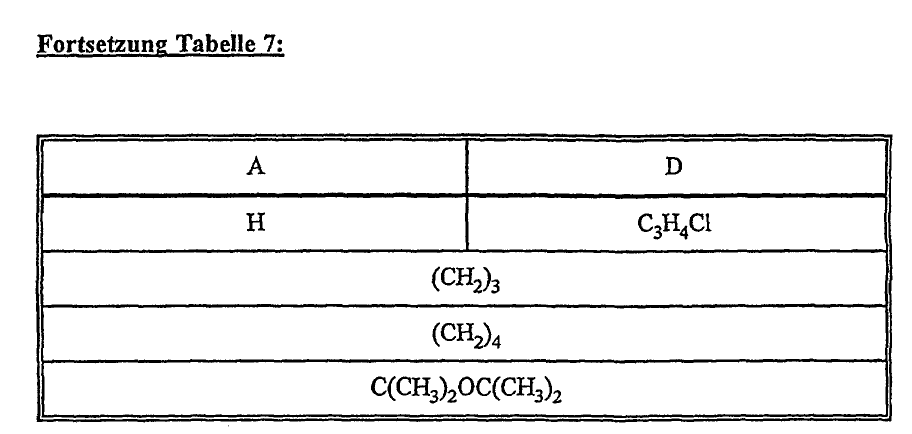

Im einzelnen seien außer den bei den Herstellungsbeispielen genannten Verbindungen die folgenden Verbindungen der Formel (I-6-a) genannt:

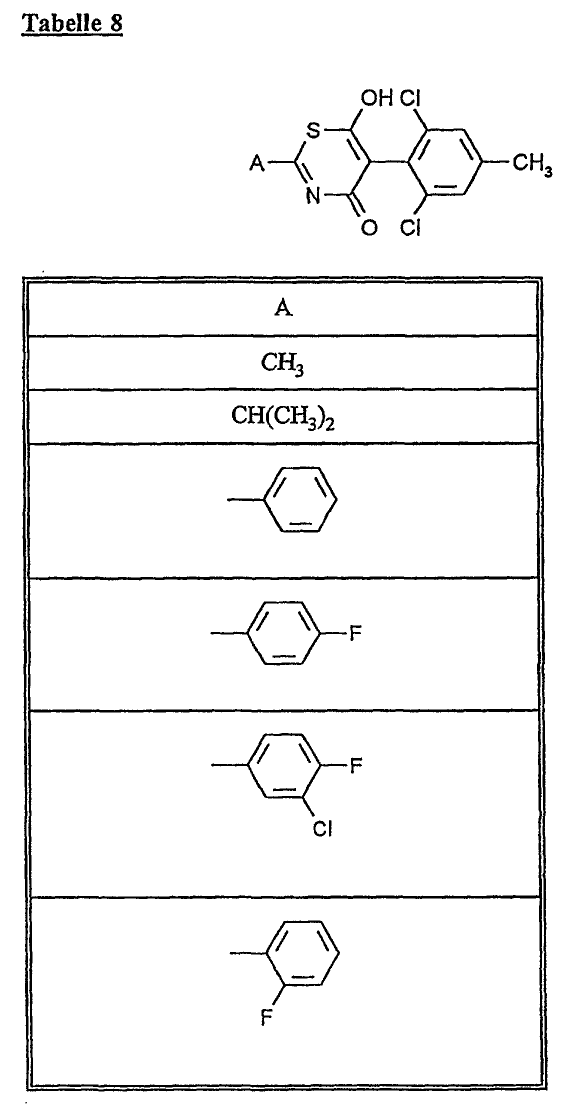

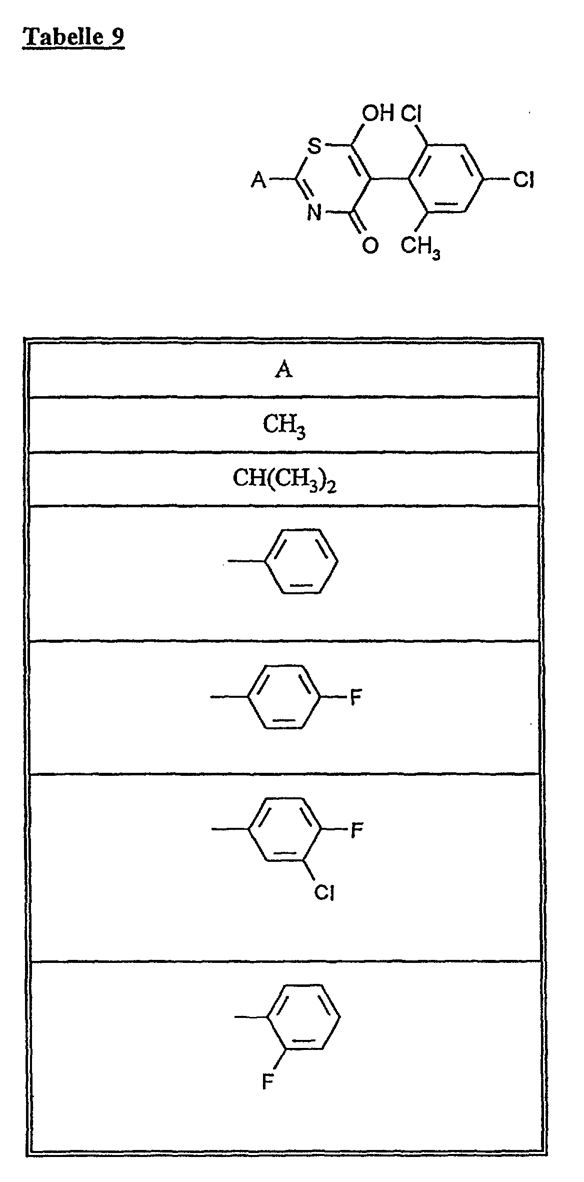

Verwendet man gemäß Verfahren (A) N-[(2,4-Dichlor-6-methyl)-phenylacetyl]-1-amino-4-ethyl-cyclohexan-carbonsäureethylester als Ausgangsstoff, so kann der Verlauf des erfindungsgemäßen Verfahrens durch folgendes Reaktionsschema wiedergegeben werden:

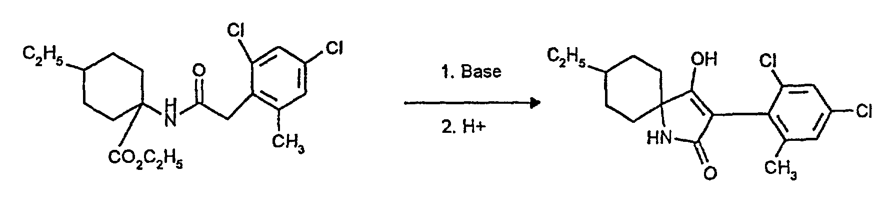

Verwendet man gemäß Verfahren (B) O-[(2,6-Dichlor-4-methyl)-phenylacetyl]-hydroxyessigsäureethylester, so kann der Verlauf des erfindungsgemäßen Verfahrens durch folgendes Reaktionsschema wiedergegeben werden:

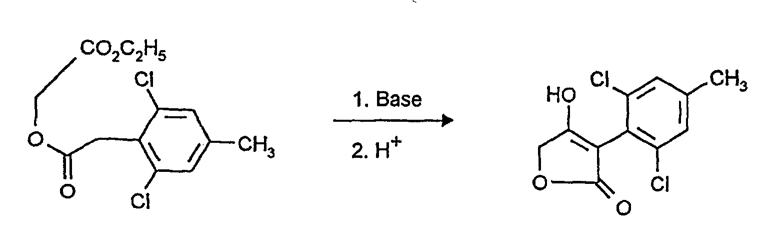

Verwendet man gemäß Verfahren (C) 2-[(2-Chlor-6-fluor-4-methyl)-phenyl]-4-(4-methoxy)-benzylmercapto-4-methyl-3-oxo-valeriansäure-ethylester, so kann der Verlauf des erfindungsgemäßen Verfahrens durch folgendes Reaktionsschema wiedergegeben werden:

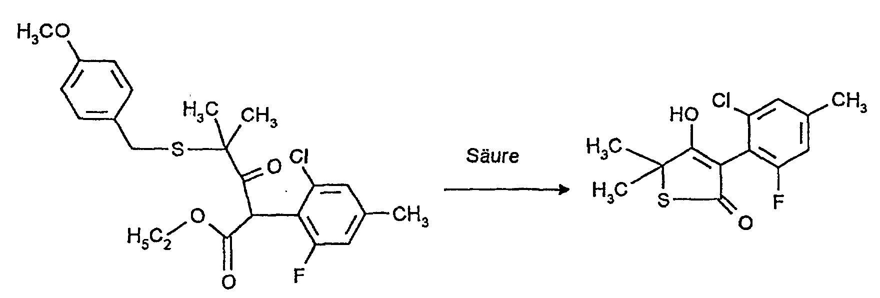

Verwendet man beispielsweise gemäß Verfahren (E) (Chlorcarbonyl)-2-[(2,4-Dichlor-6-methyl)-phenyl]-keten und Aceton als Ausgangsverbindungen, so kann der Verlauf des erfindungsgemäßen Verfahrens durch das folgende Reaktionsschema wiedergegeben werden:

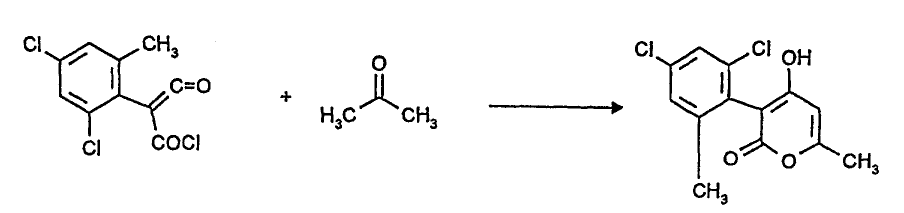

Verwendet man beispielsweise gemäß Verfahren (F) (Chlorcarbonyl)-2-[(2,4-Dichlor-6-methyl)-phenyl]-keten und Thiobenzamid als Ausgangsverbindungen, so kann der Verlauf des erfindungsgemäßen Verfahrens durch das folgende Reaktionsschema wiedergegeben werden:

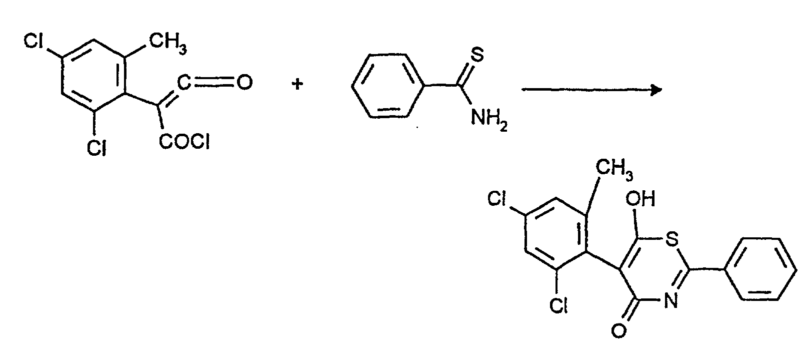

Verwendet man gemäß Verfahren (Gα) 3-[(2,6-Dichlor-4-methyl)-phenyl]-5,5-dimethyl-pyrrolidin-2,4-dion und Pivaloylchlorid als Ausgangsstoffe, so kann der Verlauf des erfindungsgemäßen Verfahrens durch folgendes Reaktionsschema wiedergegeben werden:

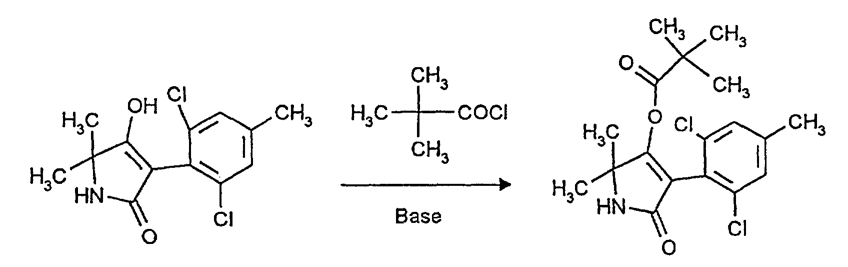

Verwendet man gemäß Verfahren (G) (Variante β) 3-[(2,4-Dichlor-6-methyl)-phenyl]-4-hydroxy-5-phenyl-Δ³-dihydrofuran-2-on und Acetanhydrid als Ausgangsverbindungen, so kann der Verlauf des erfindungsgemäßen Verfahrens durch folgendes Reaktionsschema wiedergegeben werden:

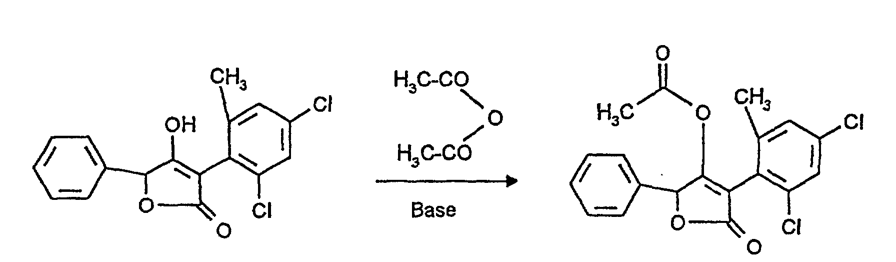

Verwendet man gemäß Verfahren (H) 8-[(2,4-Dichlor-6-methyl)-phenyl]-1,6-diazabicyclo-(4,3,0^{1,6})-nonan-7,9-dion und Chlorameisensäureethoxyethylester als Ausgangsverbindungen, so kann der Verlauf des erfindungsgemäßen Verfahrens durch folgendes Reaktionsschema wiedergegeben werden:

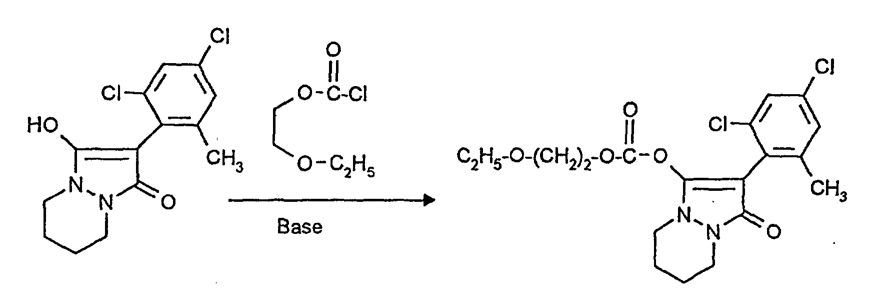

Verwendet man gemäß Verfahren (I), Variante α 3-[(2,6-Dichlor-4-methyl)-phenyl]-4-hydroxy-6-(3-pyridyl)-pyron und Chlormonothioameisensäuremethylester als Ausgangsprodukte, so kann der Reaktionsverlauf folgendermaßen wiedergegeben werden:

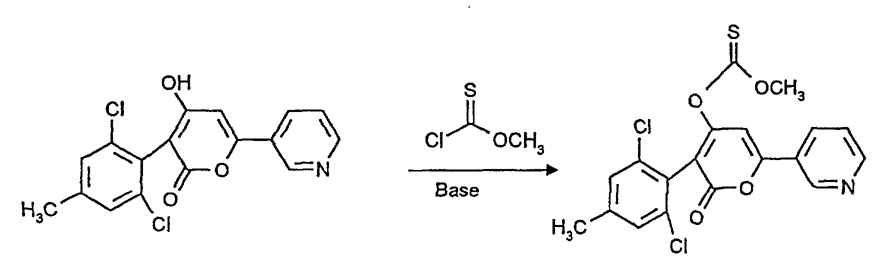

Verwendet man gemäß Verfahren (K), Variante β 5-[(2-Chlor-6-fluor-4-methyl)-phenyl]-6-hydroxy-2-(4-chlorphenyl)-thiazin-4-on, Schwefelkohlenstoff und Methyliodid als Ausgangskomponenten, so kann der Reaktionsverlauf wie folgt wiedergegeben werden:

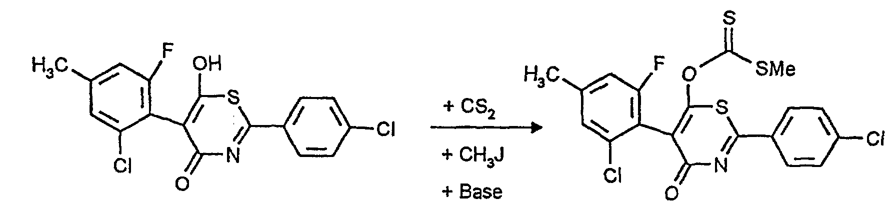

Verwendet man gemäß Verfahren (J) 2-[(2,4-Dichlor-6-methyl)-phenyl]-1,5-trimethylen-pyrrolidin-2,4-dion und Methansulfonsäurechlorid als Ausgangsprodukt, so kann der Reaktionsverlauf durch folgendes Reaktionsschema wiedergegeben werden:

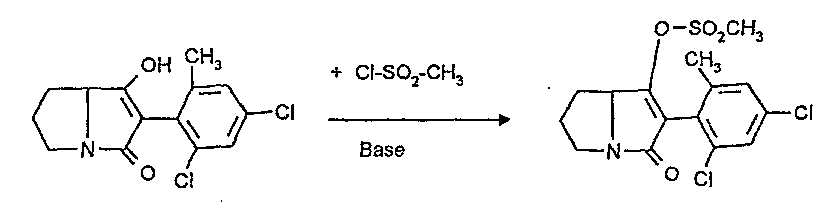

Verwendet man gemäß Verfahren (K) 2-[(2,6-Dichlor-4-methyl)-phenyl]-4-hydroxy-5-methyl-6-(2-pyridyl)-pyron und Methanthio-phosphonsäurechlorid-(2,2,2-trifluorethylester) als Ausgangsprodukte, so kann der Reaktionsverlauf durch folgendes Reaktionsschema wiedergegeben werden:

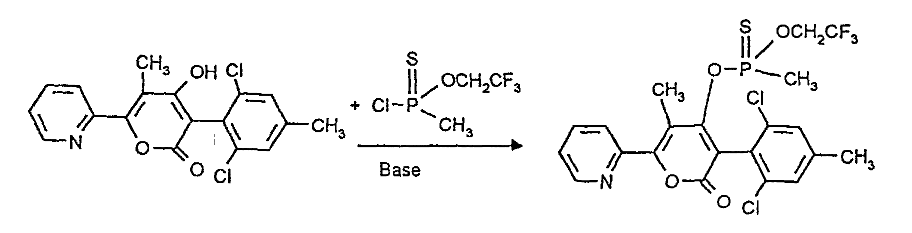

Verwendet man gemäß Verfahren (L) 3-[(2,4-Dichlor-6-methyl)-phenyl]-5-cyclopropyl-5-methyl-pyrrolidin-2,4-dion und NaOH als Komponenten, so kann der Verlauf des erfindungsgemäßen Verfahrens durch folgendes Reaktionsschema wiedergegeben werden:

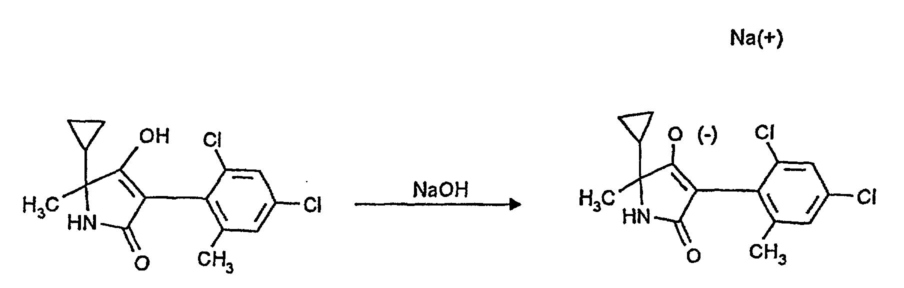

Verwendet man gemäß Verfahren (M) Variante α 3-[(2,6-Dichlor-4-methyl)-phenyl]-4-hydroxy-5,5-tetramethylen-Δ³-dihydro-furan-2-on und Ethylisocyanat als Ausgangsprodukte, so kann der Reaktionsverlauf durch folgendes Reaktionsschema wiedergegeben werden:

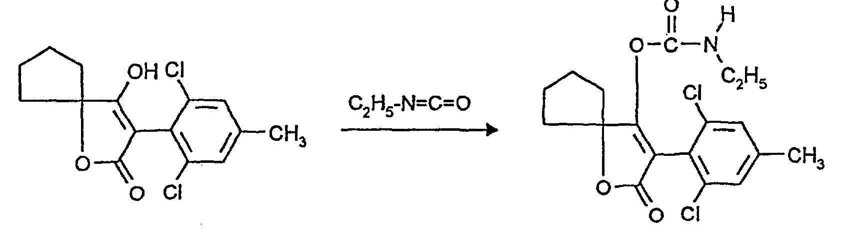

Verwendet man gemäß Verfahren (M) Variante β 3-[(2,6-Dichlor-4-methyl)-phenyl]-5-methyl-pyrrolidin-2,4-dion und Dimethylcarbamidsäurechlorid als Ausgangsprodukte, so kann der Reaktionsverlauf durch folgendes Schema wiedergegeben werden:

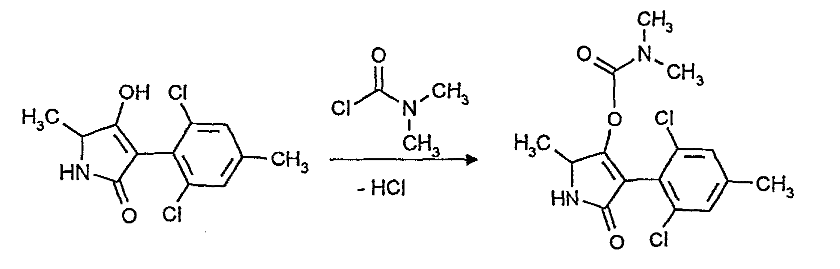

Die beim erfindungsgemäßen Verfahren (A) als Ausgangsstoffe benötigten Verbindungen der Formel (II) 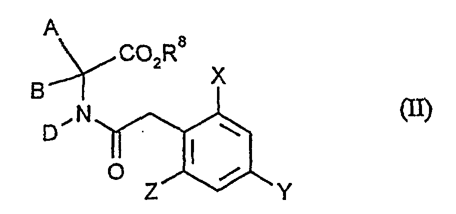 in welcher
- A, B, D, X, Y, Z und R⁸: die oben angegebene Bedeutungen haben,
sind neu.

Man erhält die Acylaminosäureester der Formel (II) beispielsweise, wenn man Aminosäurederivate der Formel (XXI) 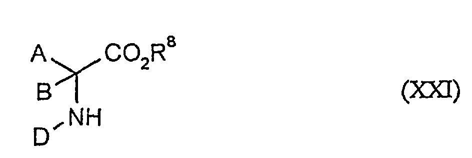 in welcher
- A, B, R⁸ und D: die oben angegebenen Bedeutungen haben,
mit substituierten Phenylessigsäurehalogeniden der Formel (XXII) 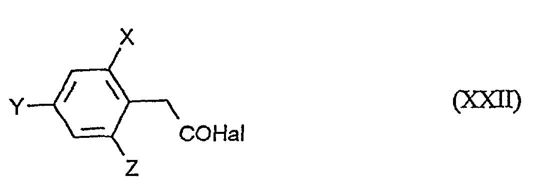 in welcher
- X, Y und Z: die oben angegebenen Bedeutungen haben und
- Hal: fiir Chlor oder Brom steht,
acyliert (Chem. Reviews 52, 237-416 (1953); Bhattacharya, Indian J. Chem. 6, 341-5, 1968)
oder wenn man Acylaminosäuren der Formel (XXIII) 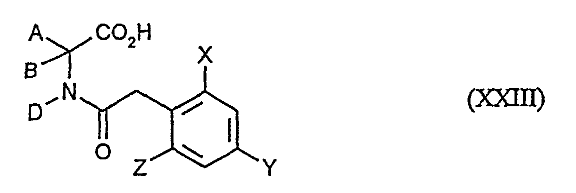 in welcher
- A, B, D, X, Y und Z: die oben angegebenen Bedeutungen haben,
verestert (Chem. Ind. (London) 1568 (1968)).

Die Verbindungen der Formel (XXIII) 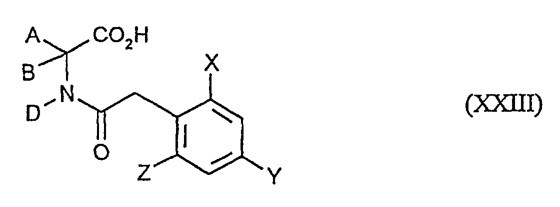 in welcher
- A, B, D, X, Y und Z: die oben angegebenen Bedeutungen haben,
sind neu.

Man erhält die Verbindungen der Formel (XXIII), wenn man Aminosäuren der Formel (XXIV) 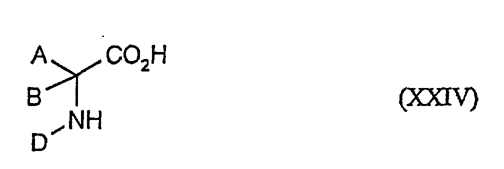 in welcher
- A, B und D: die oben angegebenen Bedeutungen haben,
mit substituierten Phenylessigsäurehalogeniden der Formel (XXII) 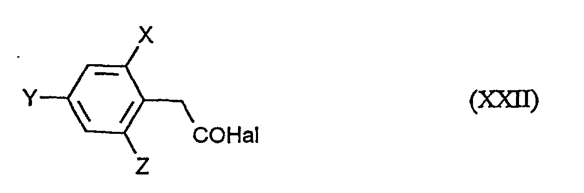 in welcher
- X, Y und Z: die oben angegebenen Bedeutungen haben und
- Hal: fiir Chlor oder Brom steht,
nach Schotten-Baumann acyliert (Organikum, VEB Deutscher Verlag der Wissenschaften, Berlin 1977, S. 505).

Die Verbindungen der Formel (XXII) sind neu.

Man erhält die Verbindungen der Formel (XXII) beispielsweise, indem man substituierte Phenylessigsäuren der Formel (XXV) 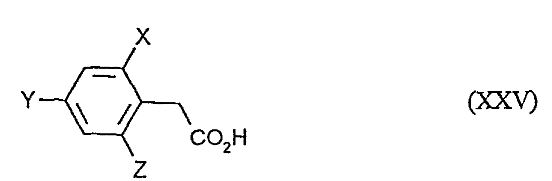 in welcher
- X, Y und Z: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Verbindungen der Formel (XXV) sind neu, ausgenommen 2,,4-Dichlor-6-methyl-phenylessigsäure (siehe Crosby et al., J. Agric. Food Chem. 33 569-73 (1985).

Man erhält die Verbindungen der Formel (XXV) beispielsweise, indem man substituierte Phenylessigsäureester der Formel (XXVI) 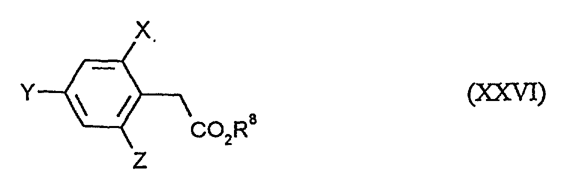 in welcher
- X, Y, Z und R⁸: die oben angegebene Bedeutung haben,
in Gegenwart einer Säure (z.B. einer anorganischen Säure wie Chlorwasserstoffsäure) oder einer Base (z.B. eines Alkalihydroxids wie Natrium- oder Kaliumhydroxid) und gegebenenfalls eines Verdünnungsmittels (z.B. eines wässrigen Alkohols wie Methanol oder Ethanol) bei Temperaturen zwischen 0°C und 150°C, bevorzugt zwischen 20°C und 100°C, hydrolysiert.

Die Verbindungen der Formel (XXVI) sind neu.

Man erhält die Verbindungen der Formel (XXVI) beispielsweise, indem man substituierte 1,1,1-Trichlor-2-phenylethane der Formel (XXVII) 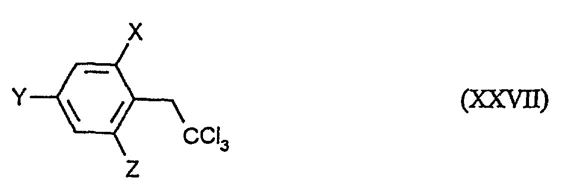 in welcher
- X, Y und Z: die oben angegebene Bedeutung haben,
zunächst mit Alkoholaten (z.B. Alkalimetallalkoholaten wie Natriummethylat oder Natriumethylat) in Gegenwart eines Verdünnungsmittels (z.B. dem vom Alkoholat abgeleiteten Alkohol) bei Temperaturen zwischen 0°C und 150°C, bevorzugt zwischen 20°C und 120°C, und anschließend mit einer Säure (bevorzugt eine anorganische Säure wie z.B. Schwefelsäure) bei Temperaturen zwischen -20°C und 150°C, bevorzugt 0°C und 100°C, umsetzt.

Die Verbindungen der Formel (XXVII) sind neu.

Man erhält die Verbindungen der Formel (XXVII) beispielsweise, wenn man Aniline der Formel (XXVIII) 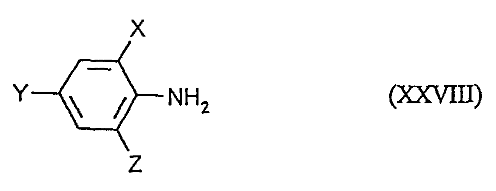 in welcher
- X, Y und Z: die oben angegebene Bedeutung haben,
in Gegenwart eines Alkylnitrits der Formel (XXIX)

R²¹-ONO (XXIX)

in welcher
- R²¹: für Alkyl, bevorzugt C₁-C₆-Alkyl steht,
in Gegenwart von Kupfer(II)-chlorid und gegebenenfalls in Gegenwart eines Verdünnungsmittels (z.B. eines aliphatischen Nitrils wie Acetonitril) bei einer Temperatur von -20°C bis 80°C, bevorzugt 0°C bis 60°C, mit Vinylidenchlorid (CH₂=CCl₂) umsetzt.

Die Verbindungen der Formel (XXVIII) und (XXIX) sind bekannte Verbindungen der Organischen Chemie. Kupfer(II)-chlorid und Vinylidenchlorid sind lange bekannt und käuflich.

Die Verbindungen der Formel (XXI) und (XXIV) sind teilweise bekannt und/oder lassen sich nach bekannten Verfahren darstellen (siehe z.B. Compagnon, Miocque Ann. Chim. (Paris) [14] 5, S. 11-22, 23-27 (1970)).

Die substituierten cyclischen Aminocarbonsäuren der Formel (XXIVa), in der A und B einen Ring bilden, sind im allgemeinen nach der Bucherer-Bergs-Synthese oder nach der Strecker-Synthese erhältlich und fallen dabei jeweils in unterschiedlichen Isomerenformen an. So erhält man nach den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 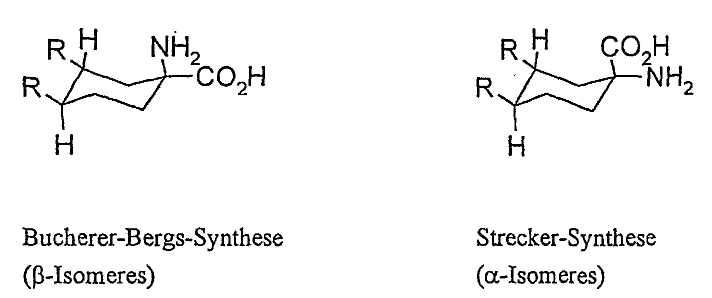 (L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 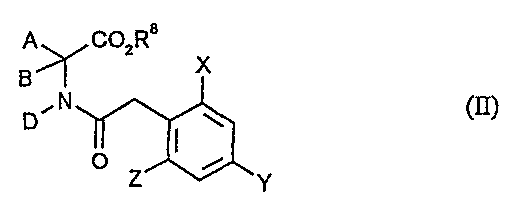 in welcher
- A, B, D, X, Y, Z und R⁸: die oben angegebenen Bedeutungen haben,
herstellen, wenn man Aminonitrile der Formel (XXX) 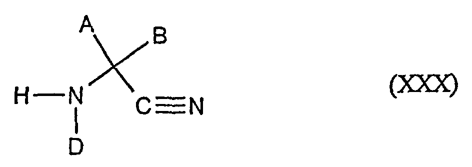 in welcher
- A, B und D: die oben angegebenen Bedeutungen haben,
mit substituierten Phenylessigsäurehalogeniden der Formel (XXII) 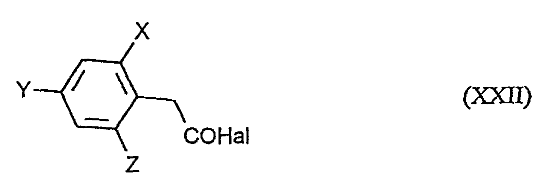 in welcher
- X, Y, Z und Hal: die oben angegebenen Bedeutungen haben,
zu Verbindungen der Formel (XXXI) 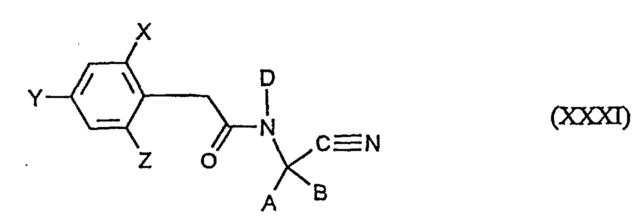 in welcher
- A, B, D, X, Y und Z: die oben angegebenen Bedeutungen haben,
umsetzt,
und diese anschließend einer sauren Alkoholyse unterwirft.

Die Verbindungen der Formel (XXXI) sind ebenfalls neu.

Die bei dem erfindungsgemäßen Verfahren (B) als Ausgangstoffe benötigten Verbindungen der Formel (III) 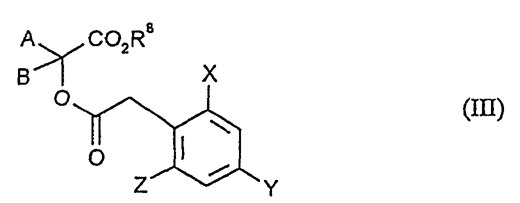 in welcher
- A, B, X, Y, Z und R⁸: die oben angegebenen Bedeutungen haben,
sind neu.

Sie lassen sich nach im Prinzip bekannten Methoden in einfacher Weise herstellen.

Man erhält die Verbindungen der Formel (III) beispielsweise, wenn man 2-Hydroxycarbonsäureester der Formel (XXXII) 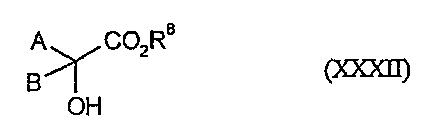 in welcher
- A, B und R⁸: die oben angegebenen Bedeutungen haben,
mit substituierten Phenylessigsäurehalogeniden der Formel (XXII) 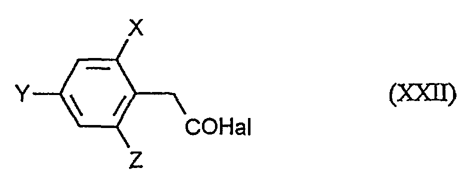 in welcher
- X, Y, Z und Hal: die oben angegebenen Bedeutungen haben,
acyliert (Chem. Reviews 52, 237-416 (1953)).

Weiterhin erhält man Verbindungen der Formel (III), wenn man
substituierte Phenylessigsäuren der Formel (XXV) 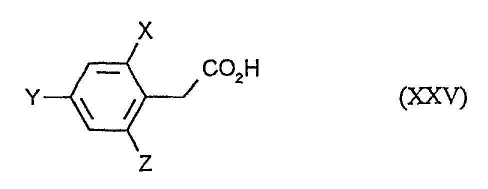 in welcher
- X, Y und Z: die oben angegebenen Bedeutungen haben,
mit a-Halogencarbonsäureestern der Formel (XXXIII) 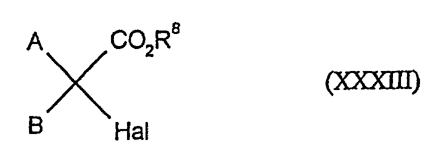 in welcher
- A, B und R⁸: die oben angegebenen Bedeutungen haben und
- Hal: für Chlor oder Brom steht,
alkyliert.

Die Verbindungen der Formel (XXXIII) sind käuflich oder in einfacher Weise nach bekannten Methoden herstellbar.

Die bei dem obigen Verfahren (C) als Ausgangsstoffe benötigten Verbindungen der Formel (IV) 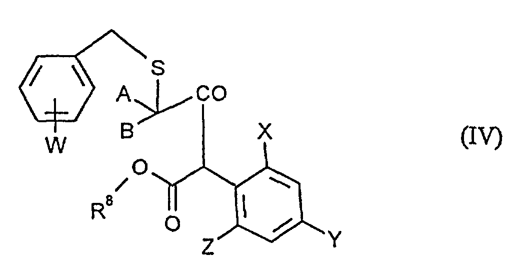 in welcher
- A, B, W, X, Y, Z und R⁸: die oben angegebenen Bedeutungen haben,
sind neu.

Sie lassen sich nach im Prinzip bekannten Methoden herstellen.

Man erhält die Verbindungen der Formel (IV) beispielsweise, wenn man substituierte Phenylessigsäureester der Formel (XXVI) 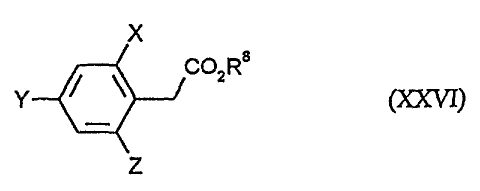 in welcher
- X, Y, R⁸ und Z: die oben angegebenen Bedeutungen haben,
mit 2-Benzylthio-carbonsäurehalogeniden der Formel (XXXIV) 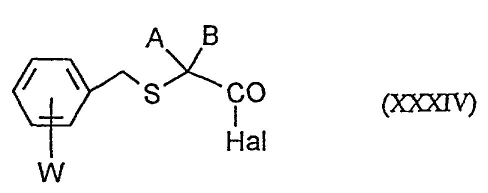 in welcher
- A, B und W: die oben angegebenen Bedeutungen haben und
- Hal: für Halogen (insbesondere Chlor oder Brom) steht,
in Gegenwart von starken Basen acyliert (siehe z.B. M.S. Chambers, E.J. Thomas, D.J. Williams, J. Chem. Soc. Chem. Commun., (1987), 1228).

Die Benzylthio-carbonsäurehalogenide der Formel (XXXIV) sind teilweise bekannt und/oder lassen sich nach bekannten Verfahren herstellen (J. Antibiotics (1983), 26, 1589).

Die beim Verfahren (E) als Ausgangsstoffe benötigten Halogencarbonylketene der Formel (V) sind neu. Sie lassen sich nach im Prinzip bekannten Methoden in einfacher Weise herstellen (vgl. beispielsweise Org. Prep. Proced. Int., 7, (4), 155-158, 1975 und DE 1 945 703). Man erhält die Verbindungen der Formel (V) 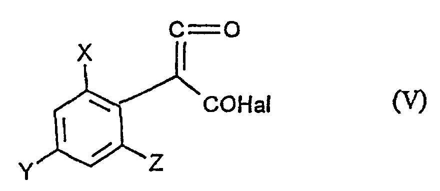 in welcher
- X, Y und Z: die oben angegebenen Bedeutungen haben und
- Hal: für Chlor oder Brom steht,
wenn man
substituierte Phenylmalonsäuren der Formel (XXXV) 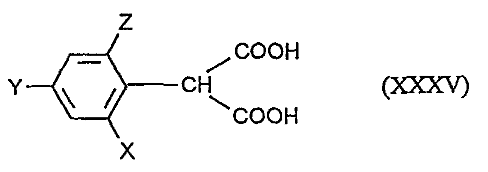 in welcher
- X, Y und Z: die oben angegebenen Bedeutungen haben,
mit Säurehalogeniden, wie beispielsweise Thionylchlorid, Phosphor(V)chlorid, Phosphor(III)chlorid, Oxalylchlorid, Phosgen oder Thionylbromid gegebenenfalls in Gegenwart von Katalysatoren, wie beispielsweise Diethylformamid, Methyl-Sterylformamid oder Triphenylphosphin und gegebenenfalls in Gegenwart von Basen wie z.B. Pyridin oder Triethylamin, bei einer Temperatur zwischen -20°C und 200°C, bevorzugt zwischen 0°C und 150°C, umsetzt.

Die substituierten Phenylmalonsäuren der Formel (XXXV) sind neu. Sie lassen sich aber in einfacher Weise nach bekannten Verfahren herstellen (vgl. z.B. Organikum, VEB Deutscher Verlag der Wissenschaften, Berlin 1977, S. 517 ff).

Die für das erfindungsgemäße Verfahren (E) als Ausgangsstoffe benötigten Carbonylverbindungen der Formel (VIII) oder deren Silylenolether der Formel (VIIIa) 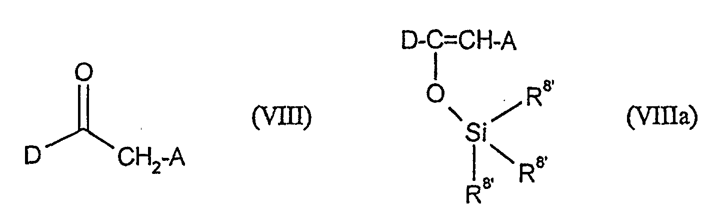 in welchen
- A, D und R^{8'}: die oben angegebenen Bedeutungen haben,
sind käufliche, allgemeine bekannte oder nach bekannten Verfahren zugängliche Verbindungen.

Die Herstellung der zur Durchführung des erfindungsgemäßen Verfahrens (F) als Ausgangsstoffe benötigten Ketensäurechloride der Formel (V) wurden bereits beim erfindungsgemäßen Verfahren (E) beschrieben. Die zur Durchführung des erfindungsgemäßen Verfahrens (F) benötigten Thioamide der Formel (IX) 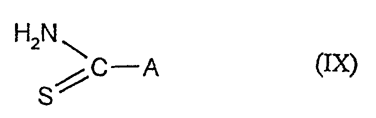 in welcher
- A: die oben angegebene Bedeutung hat,
sind allgemein in der Organischen Chemie bekannte Verbindungen.

Die beim Verfahren (G) als Ausgangsstoffe benötigten Verbindungen der Formel (I-4-a) sind bekannt und/oder lassen sich in einfacher Weise nach bekannten Methoden herstellen (vgl. auch WO 92/16510).

Man erhält die Verbindungen der Formel (I-4-a) beispielsweise, wenn man
Verbindungen der Formel (V) 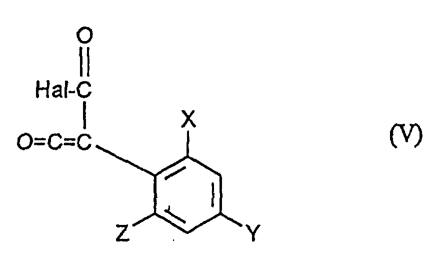 in welcher
- X, Y und Z: die oben angegebenen Bedeutungen haben
und
- Hal: für Halogen (insbesondere Chlor oder Brom) steht,
oder
Verbindungen der Formel (VI) 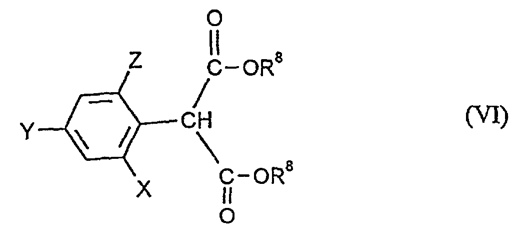 in welcher
- R⁸, X, Y und Z: die oben angegebenen Bedeutungen haben,
mit Hydrazinen der Formel (VII)

A-NH-NH-D (VII)

in welcher
- A und D: die oben angegebenen Bedeutungen haben,
gegebenenfalls in Gegenwart eines Verdünnungsmittels,
wobei verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie, nur im Fall, daß Verbindungen der Formel (VI) eingesetzt werden, Alkohole wie Methanol, Ethanol, Propanol, Iso-Propanol, Butanol, Iso-Butanol und tert.-Butanol, und gegebenenfalls in Gegenwart einer Base, wobei in dem Fall, daß Verbindungen der Formel (V) eingesetzt werden, anorganische Basen, insbesondere Alkali- oder Erdalkalicarbonate wie Natriumcarbonat, Kaliumcarbonat oder Calciumcarbonat sowie organische Basen wie beispielsweise Pyridin oder Triethylamin in Betracht kommen und in dem Fall, daß Verbindungen der Formel (VI) eingesetzt werden, Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können, Alkalimetalle wie Natrium oder Kalium, Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat in Betracht kommen, bei Temperaturen zwischen -20°C und 250°C, vorzugsweise zwischen 0°C und 150°C umsetzt.

Die Malonsäureester der Formel (VI) 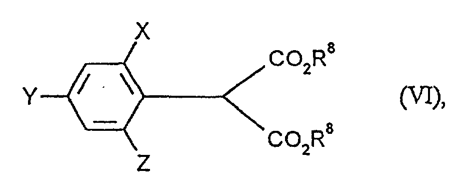 in welcher
- R⁸, X, Y und Z: die oben angegebenen Bedeutungen haben,
sind neu. Sie lassen sich nach allgemein bekannten Methoden der Organischen Chemie darstellen (vgl. z.B. Tetrahedron Lett. 27, 2763 (1986) und Organikum VEB Deutscher Verlag der Wissenschaften, Berlin 1977, S. 587 ff.).

Die Hydrazine der Formel (VII)

A-NH-NH-D (VII),

in welcher
- A und D: die oben angegebenen Bedeutungen haben,
sind teilweise bekannt und/oder nach literaturbekannten Methoden herstellbar (vgl. beispielsweise Liebigs Ann. Chem. 585, 6 (1954); Reaktionen der organischen Synthese, C. Ferri, Seite 212, 513; Georg Thieme Verlag Stuttgart, 1978; Liebigs Ann. Chem. 443, 242 (1925); Chem. Ber. 98, 2551 (1965), EP 508 126).

Die zur Durchführung der erfindungsgemäßen Verfahren (G), (H), (I), (J), (K), (L) und (M) außerdem als Ausgangsstoffe benötigten Säurehalogenide der Formel (X), Carbonsäureanhydride der Formel (XI), Chlorameisensäureester oder Chlorameisensäurethioester der Formel (XII), Chlormonothioameisensäureester oder Chlordithioameisensäureester der Formel (XIII), Alkylhalogenide der Formel (XIV), Sulfonsäurechloride der Formel (XV), Phosphorverbindungen der Formel (XVI) und Metallhydroxide, Metallalkoxide oder Amine der Formel (XVII) und (XVIII) und Isocyanate der Formel (XIX) und Carbamidsäurechloride der Formel (XX) sind allgemein bekannte Verbindungen der organischen bzw. anorganischen Chemie.

Die Verbindungen der Formeln (VII), (VIII), (IX) bis (XXI), (XXIV) und (XXXII) bis (XXXIV) sind darüber hinaus aus den eingangs zitierten Patentanmeldungen bekannt und/oder lassen sich nach den dort angegebenen Methoden herstellen.

Das Verfahren (A) ist dadurch gekennzeichnet, daß man Verbindungen der Formel (II), in welcher A, B, D, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponente der Formel (II) und die deprotonierende Base im allgemeinen in äquimolaren bis etwa doppeltäquimolaren Mengen ein. Es ist jedoch auch möglich, die eine oder andere Komponente in einem größeren Überschuß (bis zu 3 Mol) zu verwenden.

Das Verfahren (B) ist dadurch gekennzeichnet, daß man Verbindungen der Formel (III), in welcher A, B, X, Y, Z und R⁸ die oben angegebenen Bedeutungen haben, in Gegenwart eines Verdünnungsmittels und in Gegenwart einer Base intramolekular kondensiert.

Als Verdünnungsmittel können bei dem erfindungsgemäßen Verfahren (B) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (B) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (B) wird im allgemeinen unter Normaldruck durchgeführt.

Bei der Durchführung des erfindungsgemäßen Verfahrens (B) setzt man die Reaktionskomponenten der Formel (III) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (C) ist dadurch gekennzeichnet, daß man Verbindungen der Formel (IV) in welcher A, B, W, X, Y, Z und R⁸ die oben angegebene Bedeutung haben, in Gegenwart einer Säure und gegebenenfalls in Gegenwart eines Verdünnungsmittels intramolekular cyclisiert.

Als Verdünnungsmittel können bei dem erfindungsgemäßen Verfahren (C) alle gegenüber den Reaktionsteilnehmern inerten organischen Solventien eingesetzt werden. Vorzugsweise verwendbar sind Kohlenwasserstoffe, wie Toluol und Xylol, ferner halogenierte Kohlenwasserstoffe wie Dichlormethan, Chloroform, Ethylenchlorid, Chlorbenzol, Dichlorbenzol, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Gegebenenfalls kann auch die eingesetzte Säure als Verdünnungsmittel dienen.

Als Säure können bei dem erfindungsgemäßen Verfahren (C) alle üblichen anorganischen und organischen Säuren eingesetzt werden, wie z.B. Halogenwasserstoffsäuren, Schwefelsäure, Alkyl-, Aryl- und Haloalkylsulfonsäuren, insbesondere werden halogenierte Alkylcarbonsäuren wie z.B. Trifluoressigsäure verwendet.

Die Reaktionstemperatur kann bei der Durchführung des erfindungsgemäßen Verfahrens (C) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (C) wird im allgemeinen unter Normaldruck durchgeführt.

Bei der Durchführung des erfindungsgemäßen Verfahrens (C) setzt man die Reaktionskomponenten der Formeln (IV) und die Säure z.B. in äquimolaren Mengen ein. Es ist jedoch gegebenenfalls auch möglich, die Säure in katalytischen Mengen einzusetzen.

Das erfindungsgemäße Verfahren (E) ist dadurch gekennzeichnet, daß man Carbonylverbindungen der Formel (VIII) oder deren Silylenolether der Formel (VIIIa) mit Ketensäurehalogeniden der Formel (V) gegebenenfalls in Gegenwart eines Verdünnungsmittels und gegebenenfalls in Gegenwart eines Säureakzeptors umsetzt.

Als Verdünnungsmittel können bei dem erfindungsgemäßen Verfahren (E) alle gegenüber den Reaktionsteilnehmern inerten organischen Solventien eingesetzt werden. Vorzugsweise verwendbar sind Kohlenwasserstoffe, wie o-Dichlorbenzol, Tetralin, Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid oder N-Methyl-pyrrolidon.

Als Säureakzeptoren können bei der Durchführung des erfindungsgemäßen Verfahrens (E) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base oder N,N-Dimethyl-anilin.

Die Reaktionstemperatur kann bei der Durchführung des erfindungsgemäßen Verfahrens (E) innerhalb eines größeren Bereiches variiert werden. Zweckmäßigerweise arbeitet man bei Temperaturen zwischen 0°C und 250°C, vorzugsweise zwischen 50°C und 220°C.

Das erfindungsgemäße Verfahren (E) wird vorzugsweise unter Normaldruck durchgeführt.

Bei der Durchführung des erfindungsgemäßen Verfahrens (E) setzt man die Reaktionskomponenten der Formeln (VIII) oder (VIIIa) und (V) und gegebenenfalls den Säureakzeptor im allgemeinen in etwa äquimolaren Mengen ein. Es ist jedoch auch möglich, die eine oder andere Komponente in einem größeren Überschuß (bis zu 5 Mol) zu verwenden.

Das erfindungsgemäße Verfahren (F) ist dadurch gekennzeichnet, daß man Thioamide der Formel (IX) mit Ketensäurehalogeniden der Formel (V) gegebenenfalls in Gegenwart eines Verdünnungsmittels und gegebenenfalls in Gegenwart eines Säureakzeptors umsetzt.

Als Verdünnungsmittel können bei der erfindungsgemäßen Verfahrensvariante (F) alle inerten organischen Solventien eingesetzt werden. Vorzugsweise verwendbar sind Kohlenwasserstoffe, wie o-Dichlorbenzol, Tetralin, Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid und N-Methyl-pyrrolidon.

Als Säureakzeptoren können bei der Durchführung des erfindungsgemäßen Verfahrens (F) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperatur kann bei der Durchführung des erfindungsgemäßen Verfahrens (F) innerhalb eines größeren Bereiches variiert werden. Zweckmäßigerweise arbeitet man bei Temperaturen zwischen 0°C und 250°C, vorzugsweise zwischen 20°C und 220°C.

Das erfindungsgemäße Verfahren (F) wird zweckmäßigerweise unter Normaldruck durchgeführt.

Bei der Durchführung des erfindungsgemäßen Verfahrens (F) setzt man die Reaktionskomponenten der Formeln (IX) und (V) und gegebenenfalls die Säureakzeptoren im allgemeinen in etwa äquimolaren Mengen ein. Es ist jedoch auch möglich, die eine oder andere Komponente in einem größeren Überschuß (bis zu 5 Mol) zu verwenden.

Das Verfahren (Gα) ist dadurch gekennzeichnet, daß man Verbindungen der Formeln (I-1-a) bis (I-6-a) jeweils mit Carbonsäurehalogeniden der Formel (X) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Gα) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgerührt werden.

Als Säurebindemittel kommen bei der Umsetzung nach dem erfindungsgemäßen Verfahren (Gα) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren (Gα) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Gα) werden die Ausgangsstoffe der Formeln (I-1-a) bis (I-6-a) und das Carbonsäurehalogenid der Formel (X) im allgemeinen jeweils in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Gβ) ist dadurch gekennzeichnet, daß man Verbindungen der Formeln (I-1-a) bis (I-6-a) jeweils mit Carbonsäureanhydriden der Formel (XI) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Gβ) vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Als gegebenenfalls zugesetzte Säurebindemittel kommen beim Verfahren (Gβ) vorzugsweise diejenigen Säurebindemittel in Frage, die auch bei der Verwendung von Säurehalogeniden vorzugsweise in Betracht kommen.

Die Reaktionstemperatur kann bei dem erfindungsgemäßen Verfahren (Gβ) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Gβ) werden die Ausgangsstoffe der Formeln (I-1-a) bis (I-6-a) und das Carbonsäureanhydrid der Formel (XI) im allgemeinen in jeweils angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (H) ist dadurch gekennzeichnet, daß man Verbindungen der Formeln (I-1-a) bis (I-6-a) jeweils mit Chlorameisensäureestern oder Chlorameisensäurethiolestern der Formel (XII) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (H) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (H) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperatur kann bei der Durchführung des erfindungsgemäßen Verfahrens (H) innerhalb eines größeren Bereiches variiert werden. Die Reaktionstemperatur liegt im allgemeinen zwischen -20°C und +100°C, vorzugsweise zwischen 0°C und 50°C.

Das erfindungsgemäße Verfahren (H) wird im allgemeinen unter Normaldruck durchgeführt.

Bei der Durchführung des erfindungsgemäßen Verfahrens (H) werden die Ausgangsstoffe der Formeln (I-1-a) bis (I-6-a) und der entsprechende Chlorameisensäureester bzw. Chlorameisensäurethiolester der Formel (XII) im allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Das erfindungsgemäße Verfahren (I) ist dadurch gekennzeichnet, daß man Verbindungen der Formeln (I-1-a) bis (I-6-a) jeweils mit (Iα) Verbindungen der Formel (XIII) in Gegenwart eines Verdünnungsmittels und gegebenenfalls in Gegenwart eines Säurebindemittels oder (Iβ) Schwefelkohlenstoff und anschließend mit Alkylhalogeniden der Formel (XIV) gegebenenfalls in Gegennwart eines Verdünnungsmittels und gegebenenfalls in Gegenwart einer Base umsetzt.

Beim Herstellungsverfahren (Iα) setzt man pro Mol Ausgangsverbindung der Formeln (I-1-a) bis (I-6-a) ca. 1 Mol Chlormonothioameisensäureester bzw. Chlordithioameisensäureester der Formel (XIII) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) bis (I-6-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (Iβ) setzt man pro Mol Ausgangsverbindungen der Formeln (I-1-a) bis (I-6-a) jeweils die äquimolare Menge bzw. einen Überschuß Schwefelkohlenstoff zu. Man arbeitet hierbei vorzugsweise bei Temperaturen von 0 bis 50°C und insbesondere bei 20 bis 30°C.

Oft ist es zweckmäßig zunächst aus den Verbindungen der Formeln (I-1-a) bis (I-6-a) durch Zusatz einer Base (wie z.B. Kaliumtertiärbutylat oder Natriumhydrid) das entsprechende Salz herzustellen. Man setzt die Verbindungen (I-1-a) bis (I-6-a) jeweils so lange mit Schwefelkohlenstoff um, bis die Bildung der Zwischenverbindung abgeschlossen ist, z.B. nach mehrstündigem Rühren bei Raumtemperatur.

Als Basen können beim Verfahren (Iβ) alle üblichen Protonenakzeptoren eingesetzt werden. Vorzugsweise verwendbar sind Alkalimetallhydride, Alkalimetallalkoholate, Alkali- oder Erdalkalimetallcarbonate oder -hydrogencarbonate oder Stickstoffbasen. Genannt seien beispielsweise Natriumhydrid, Natriummethanolat, Natriumhydroxid, Calciumhydroxid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Als Verdünnungsmittel können bei diesem Verfahren alle üblichen Lösungsmittel verwendet werden.

Vorzugsweise sind verwendbar aromatische Kohlenwasserstoffe wie Benzol oder Toluol, Alkohole wie Methanol, Ethanol, Isopropanol oder Ethylenglykol, Nitrile wie Acetonitril, Ether wie Tetrahydrofuran oder Dioxan, Amide wie Dimethylformamid oder andere polare Lösungsmittel wie Dimethylsulfoxid oder Sulfolan.

Die weitere Umsetzung mit dem Alkylhalogenid der Formel (XIV) erfolgt vorzugsweise bei 0 bis 70°C und insbesondere bei 20 bis 50°C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Das erfindungsgemäße Verfahren (J) ist dadurch gekennzeichnet, daß man Verbindungen der Formeln (I-1-a) bis (I-6-a) jeweils mit Sulfonsäurechloriden der Formel (XV) gegebenenfalls in Gegenwart eines Verdünnungsmittels und gegebenenfalls in Gegenwart eines Säurebindemittels umsetzt.

Beim Herstellungsverfahren (J) setzt man pro Mol Ausgangsverbindung der Formel (I-1-a bis I-6-a) ca. 1 Mol Sulfonsäurechlorid der Formel (XV) bei -20 bis 150°C, vorzugsweise bei 20 bis 70°C um.

Das Verfahren (J) wird vorzugsweise in Gegenwart eines Verdünnungsmittels durchgeführt.

Als Verdünnungsmittel kommen alle inerten polaren organischen Lösungsmittel in Frage wie Ether, Amide, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) bis (I-6-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (K) ist dadurch gekennzeichnet, daß man Verbindungen der Formeln (I-1-a) bis (I-6-a) jeweils mit Phosphorverbindungen der Formel (XVI) gegebenenfalls in Gegenwart eines Verdünnungsmittels und gegebenenfalls in Gegenwart eines Säurebindemittels umsetzt.

Beim Herstellungsverfahren (K) setzt man zum Erhalt von Verbindungen der Formeln (I-1-e) bis (I-6-e) auf 1 Mol der Verbindungen (I-1-a) bis (I-6-a), 1 bis 2, vorzugsweise 1 bis 1,3 Mol der Phosphorverbindung der Formel (XVI) bei Temperaturen zwischen -40°C und 150°C, vorzugsweise zwischen -10 und 110°C um.

Das Verfahren (K) wird vorzugsweise in Gegenwart eines Verdünnungsmittels durchgeführt.

Als Verdünnungsmittel kommen alle inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin und Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise durch Kristallisation, chromatographische Reinigung oder durch sogenanntes "Andestillieren", d.h. Entfernung der flüchtigen Bestandteile im Vakuum gereinigt.

Das Verfahren (L) ist dadurch gekennzeichnet, daß man Verbindungen der Formeln (I-1-a) bis (I-6-a) jeweils mit Metallhydroxiden bzw. Metallalkoxiden der Formel (XVII) oder Aminen der Formel (XVIII), gegebenenfalls in Gegenwart eines Verdünnungsmittels, umsetzt.

Als Verdünnungsmittel können bei dem erfindungsgemäßen Verfahren (L) vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (L) wird im allgemeinen unter Normaldruck durchgerührt. Die Reaktionstemperatur liegt im allgemeinen zwischen -20°C und 100°C, vorzugsweise zwischen 0°C und 50°C.

Das erfindungsgemäße Verfahren (M) ist dadurch gekennzeichnet, daß man Verbindungen der Formeln (I-1-a) bis (I-6-a) jeweils mit (Mα) Verbindungen der Formel (XIX) gegebenenfalls in Gegenwart eines Verdünnungsmittels und gegebenenfalls in Gegenwart eines Katalysators oder (Mβ) mit Verbindungen der Formel (XX) gegebenenfalls in Gegenwart eines Verdünnungsmittels und gegebenenfalls in Gegenwart eines Säurebindemittels umsetzt.

Bei Herstellungsverfahren (Mα) setzt man pro Mol Ausgangsverbindung der Formeln (I-1-a) bis (I-6-a) ca. 1 Mol Isocyanat der Formel (XIX) bei 0 bis 100°C, vorzugsweise bei 20 bis 50°C um.

Das Verfahren (Mα) wird vorzugsweise in Gegenwart eines Verdünnungsmittels durchgeführt.

Als Verdünnungsmittel kommen alle inerten organischen Lösungsmittel in Frage, wie Ether, Amide, Nitrile, Sulfone oder Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden.

Es wird vorzugsweise bei Normaldruck gearbeitet.

Beim Herstellungsverfahren (Mβ) setzt man pro Mol Ausgangsverbindung der Formeln (I-1-a) bis (I-6-a) ca. 1 Mol Carbamidsäurechlorid der Formel (XX) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-1-a) bis (I-6-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus
   Aus der Ordnung der Chilopoda z.B. Geophilus carpophagus, Scutigera spec.
   Aus der Ordnung der Symphyla z.B. Scutigerella immaculata.
   Aus der Ordnung der Thysanura z.B. Lepisma saccharina.
   Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
   Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp..
   Aus der Ordnung der Mallophaga z.B. Trichodectes spp., Damalinea spp.
   Aus der Ordnung der Thysanoptera z.B. Hercinothrips femoralis, Thrips tabaci.
   Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
   Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Larven der grünen Reiszikade (Nephotettix cincticeps) oder gegen die Raupen der Kohlschabe (Plutella maculipennis).

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die zur Unkrautbekämpfung notwendigen Dosierungen der erfindungsgemäßen Wirkstoffe liegen zwischen 0,001 und 10 kg/ha, vorzugsweise zwischen 0,005 und 5 kg/ha.

Die erfindungsgemäßen Wirkstoffe können z.B. bei den folgenden Pflanzen verwendet werden:
Dikotyle Unkräter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Sachharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfruchtund Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachlaufverfahren. Sie können beispielsweise in Baumwolle oder Zuckerrüben mit sehr gutem Erfolg zur Bekämpfung von Schadgräser eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

**Fungizide**:
2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil, Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon, Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Primiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen, Quinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

### Herbizide:

beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4-D, 2,4-DB, 2,4-DP, Fluroxypyr, MCPA, MCPP und Triclopyr, Aryloxyphenoxy-alkansäureester, wie z.B. Diclofop-methyl, Fenoxaprop-ethyl, Fluazifopbutyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygieneund Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen Boophilus microplus und Lucilia cuprina.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
   Sirex juvencus, Urocerus gigas, Urocerus gigas taignus, Urocerus augur.
Termiten wie
   Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze
   wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise a-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3 -on,
sein.

Die Herstellung und die Verwendung der erfindungsgemäßen Wirkstoffe gehen aus den nachfolgenden Beispielen hervor.

### Herstellungsbeispiele

### Beispiel (I-1-a-1)

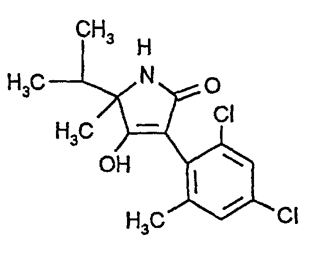

Zu 17,36 g (0,159 Mol) Kalium-tert.-butylat in 55 ml wasserfreiem Tetrahydrofuran (THF) tropft man bei Rückflußtemperatur 25,1 g (0,072 Mol) der Verbindung gemäß Beispiel (II-1) in 160 ml wasserfreiem Toluol und rührt 1,5 Stunden unter Rückfluß. Zur Aufarbeitung gibt man 230 ml Wasser zu, trennt die wäßrige Phase ab, extrahiert die Toluolphase mit 120 ml Wasser, vereinigt die wäßrigen Phasen, wäscht sie mit Toluol und säuert bei 10 bis 20°C mit etwa 25 ml konz. HCl an. Das Produkt wird abgesaugt, gewaschen, getrocknet und durch Verrühren in Methyl-tert.-butyl(MTB)-ether/n-Hexan gewaschen.
Ausbeute: 16,7 g (73 % der Theorie), Fp.: 159°C.
Analog bzw. gemäß den allgemeinen Angaben zur Herstellung erhält man die folgenden Verbindungen der Formel (I-1-a):

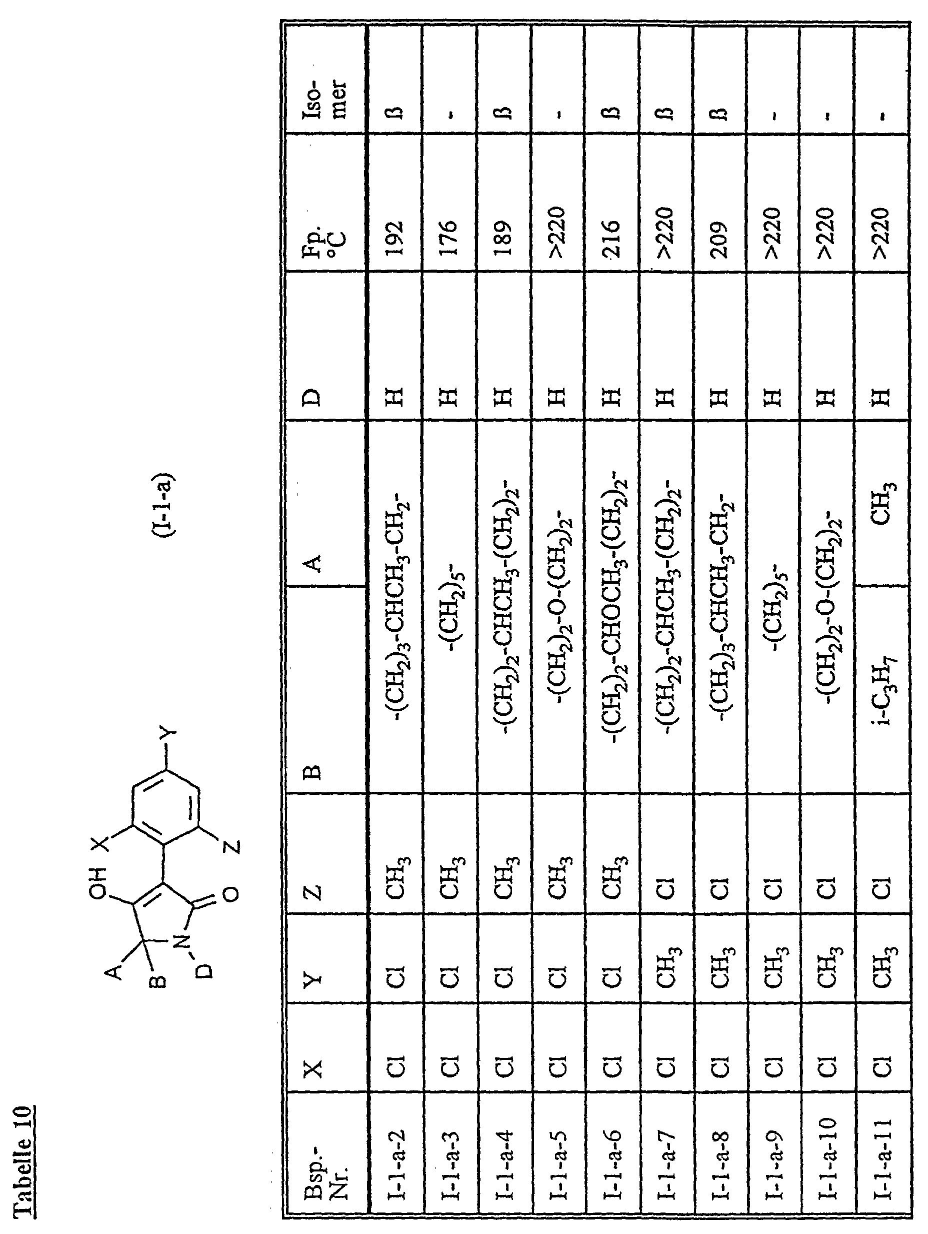

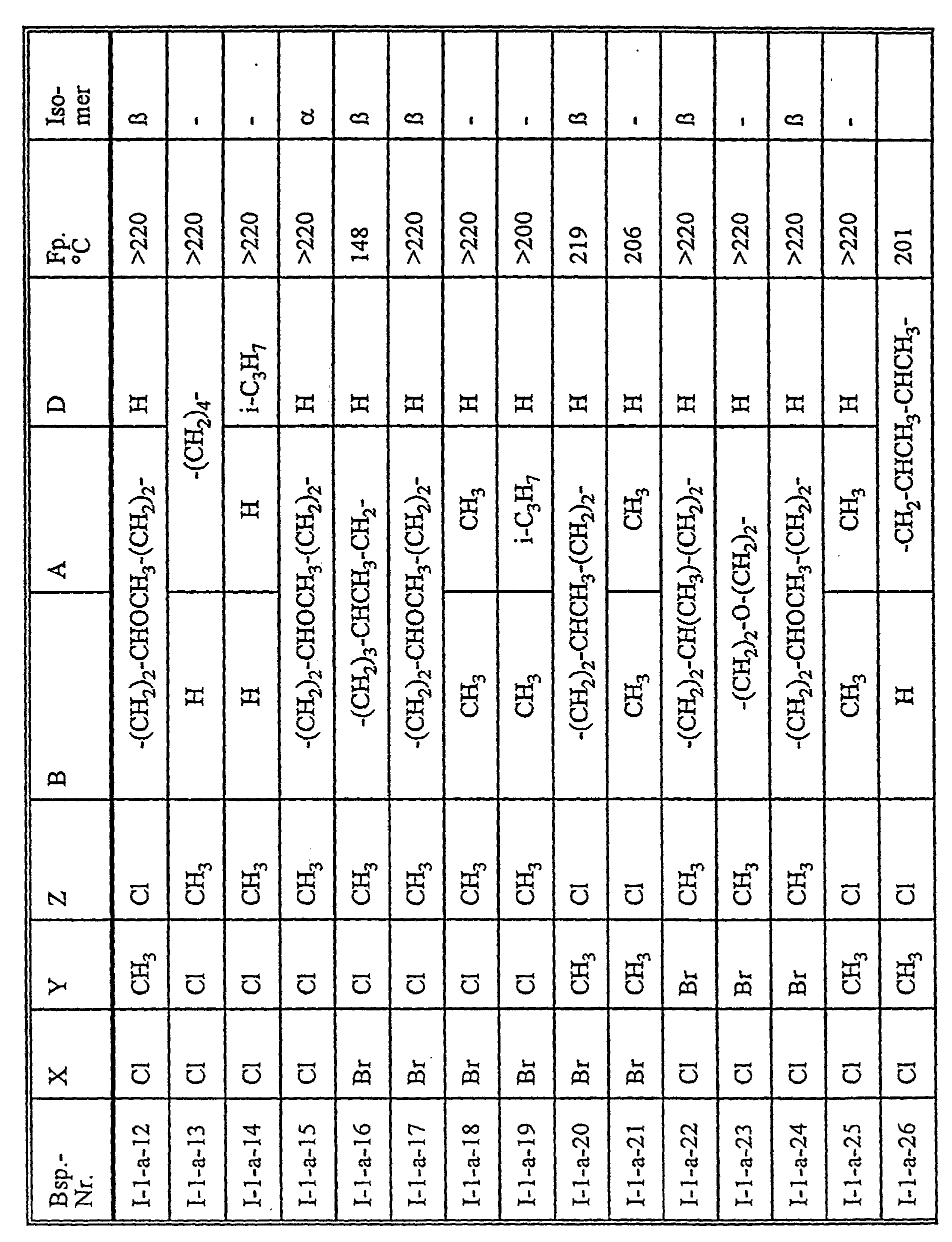

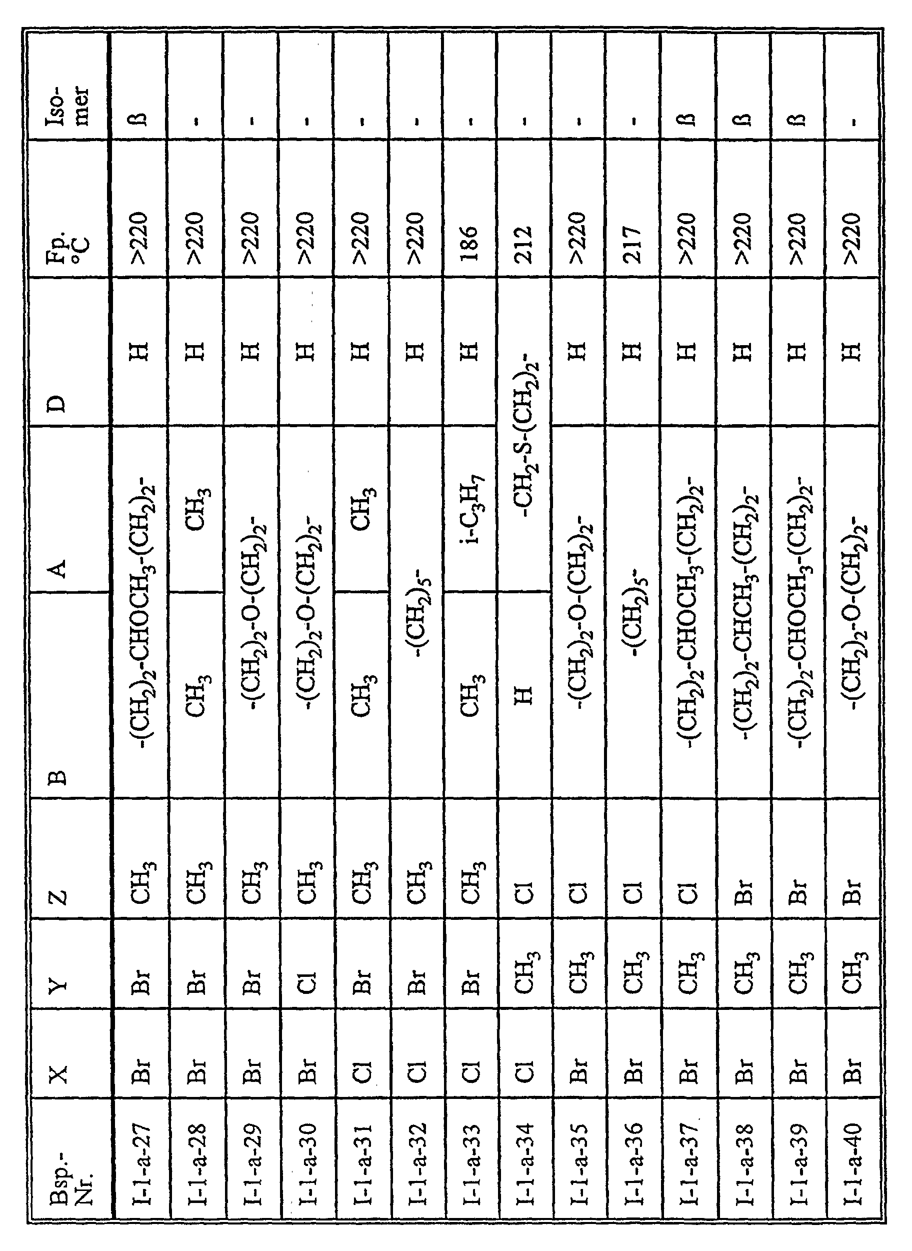

### Beispiel (I-1-b-1)

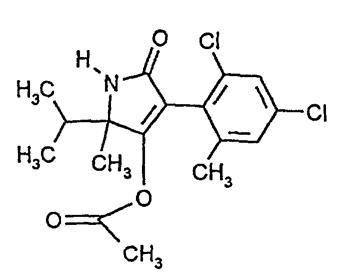

4,7 g (0,015 Mol) der Verbindung gemäß Beispiel (I-1-a-1) und 4,2 ml (30 mMol) Triethylamin in 70 ml absolutem Methylenchlorid werden bei 0 bis 10°C mit 2,14 ml (0,03 Mol) Acetylchlorid in 5 ml absolutem Methylenchlorid versetzt. Bei Raumtemperatur wird gerührt, bis die Reaktion nach dünnschichtchromatographischer Kontrolle beendet ist. Zur Aufarbeitung wird 2 mal mit 50 ml 0,5 N Natronlauge gewaschen, über Magnesiumsulfat getrocknet und eingedampft. Das Rohprodukt wird aus MTB-Ether/n-Hexan umkristallisiert.
Ausbeute: 2,30 g (43 % der Theorie), Fp.: 211°C.
Analog bzw. gemäß den allgemeinen Angaben zur Herstellung erhält man die folgenden Verbindungen der Formel (I-b-1):

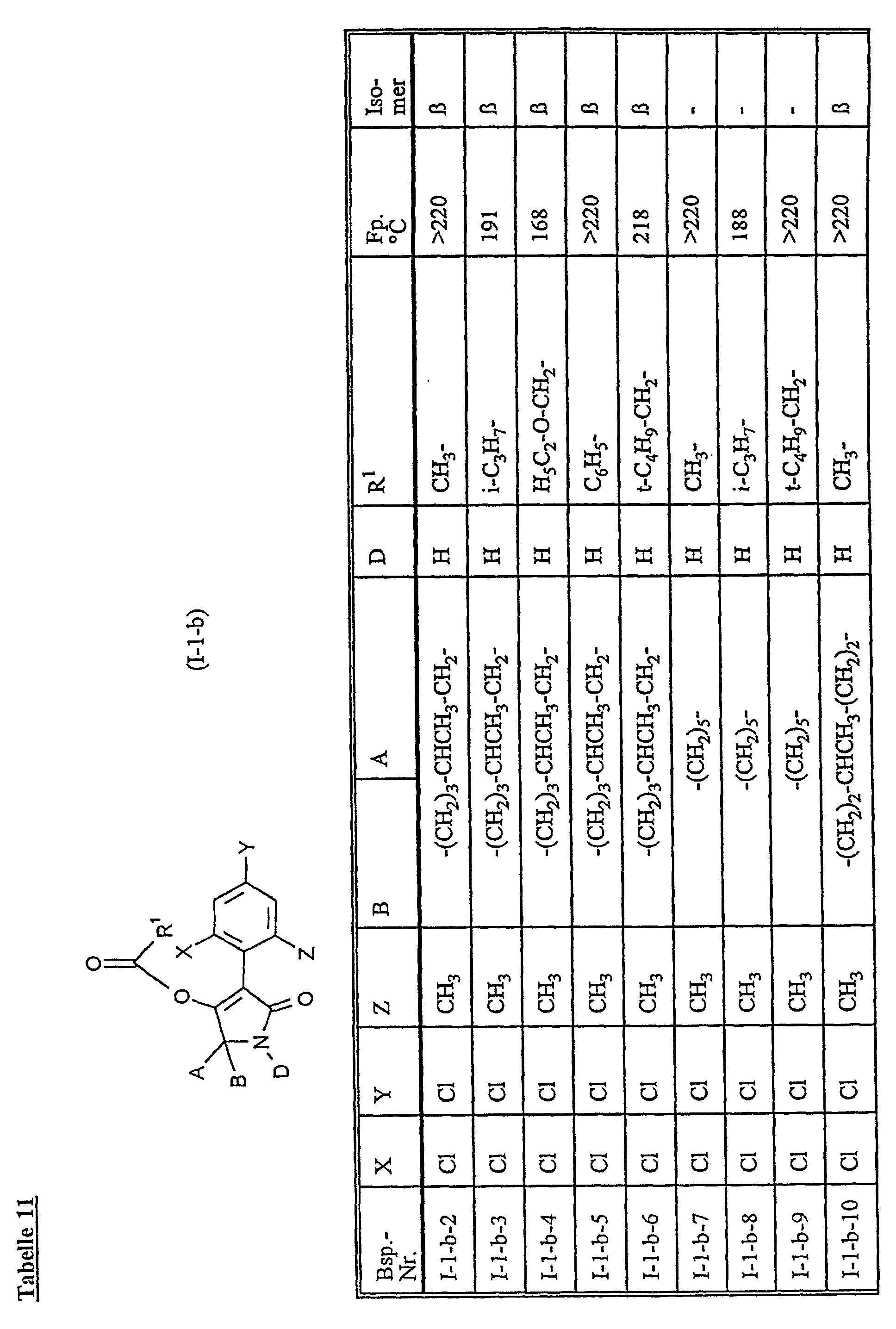

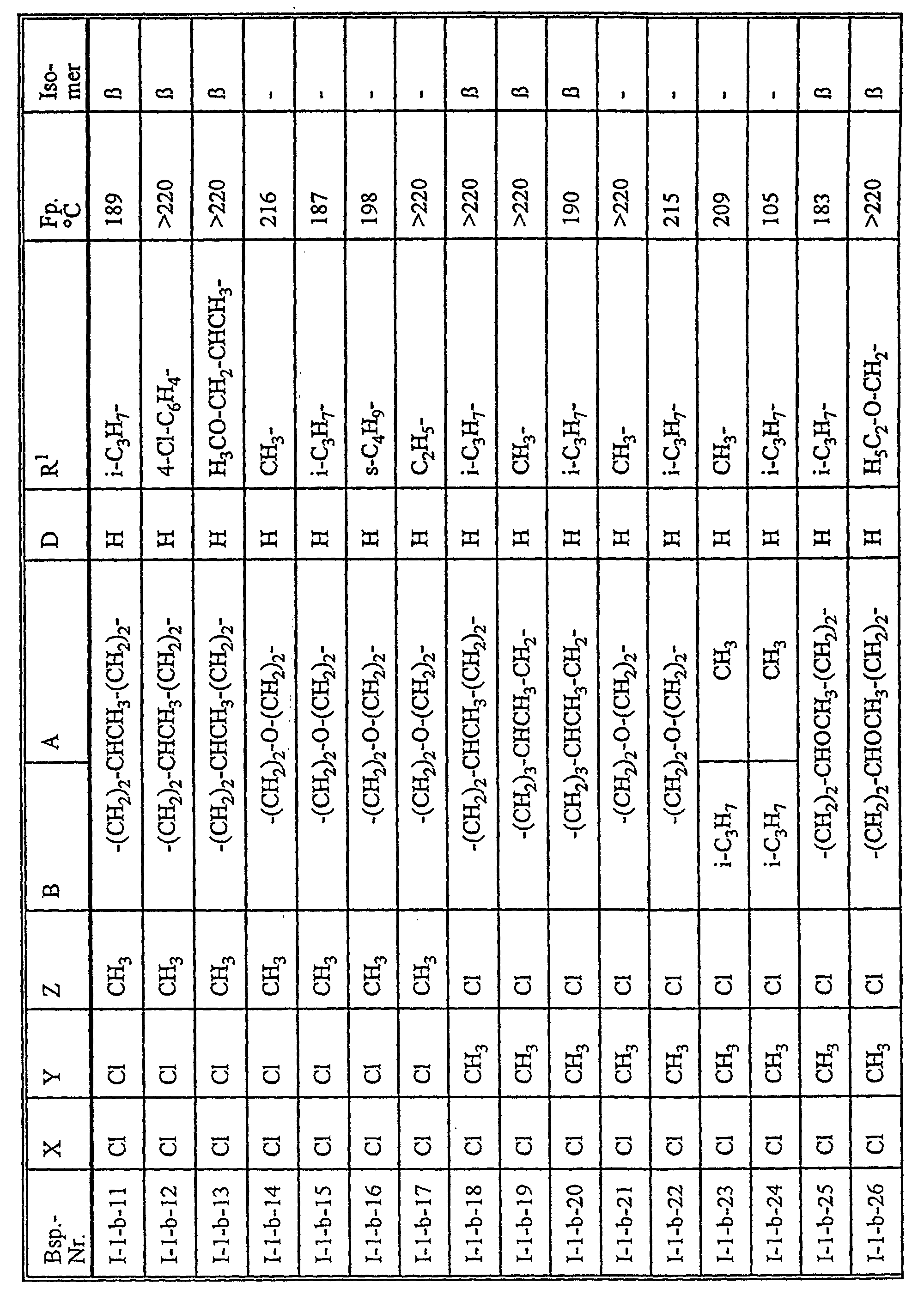

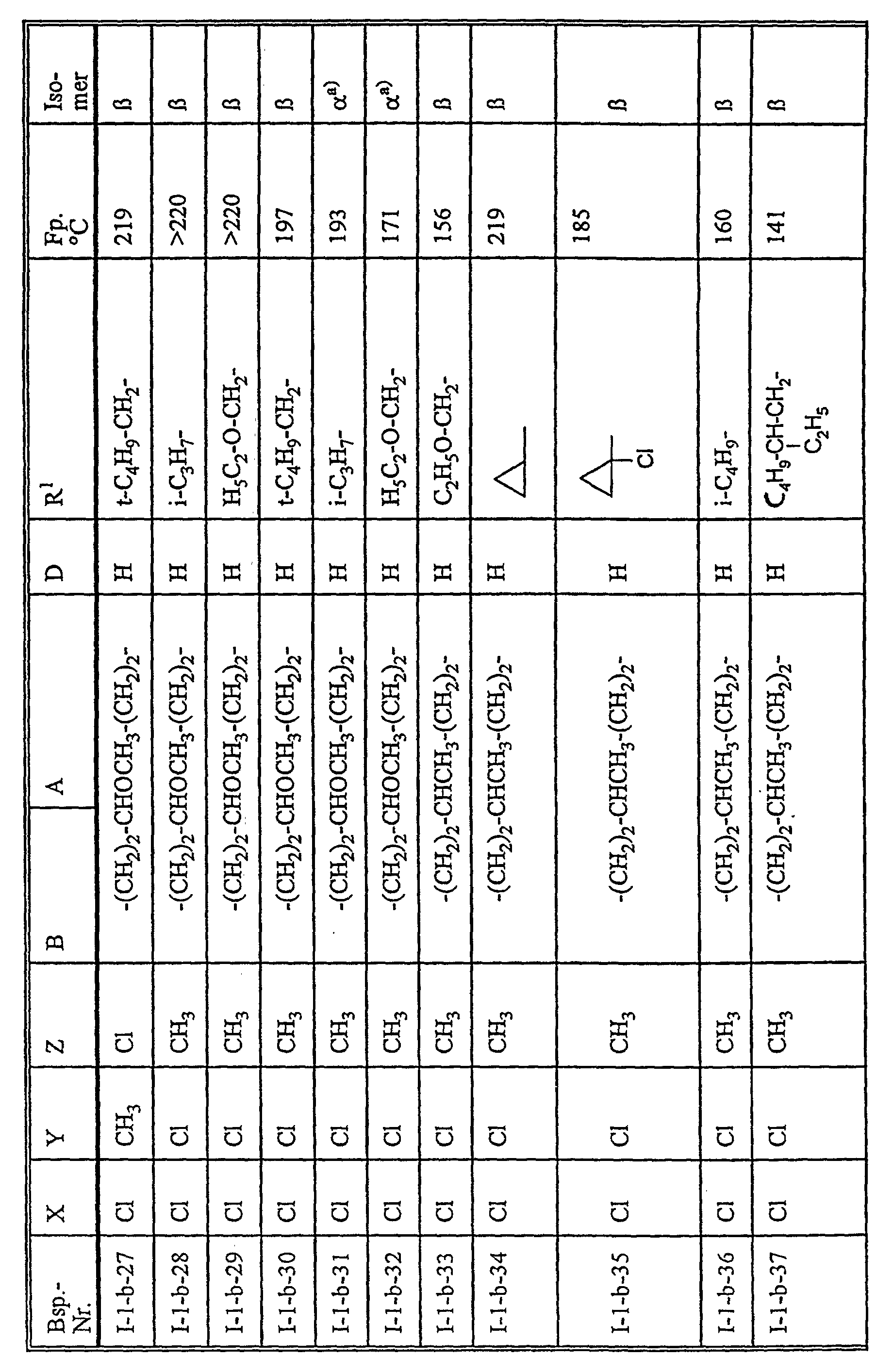

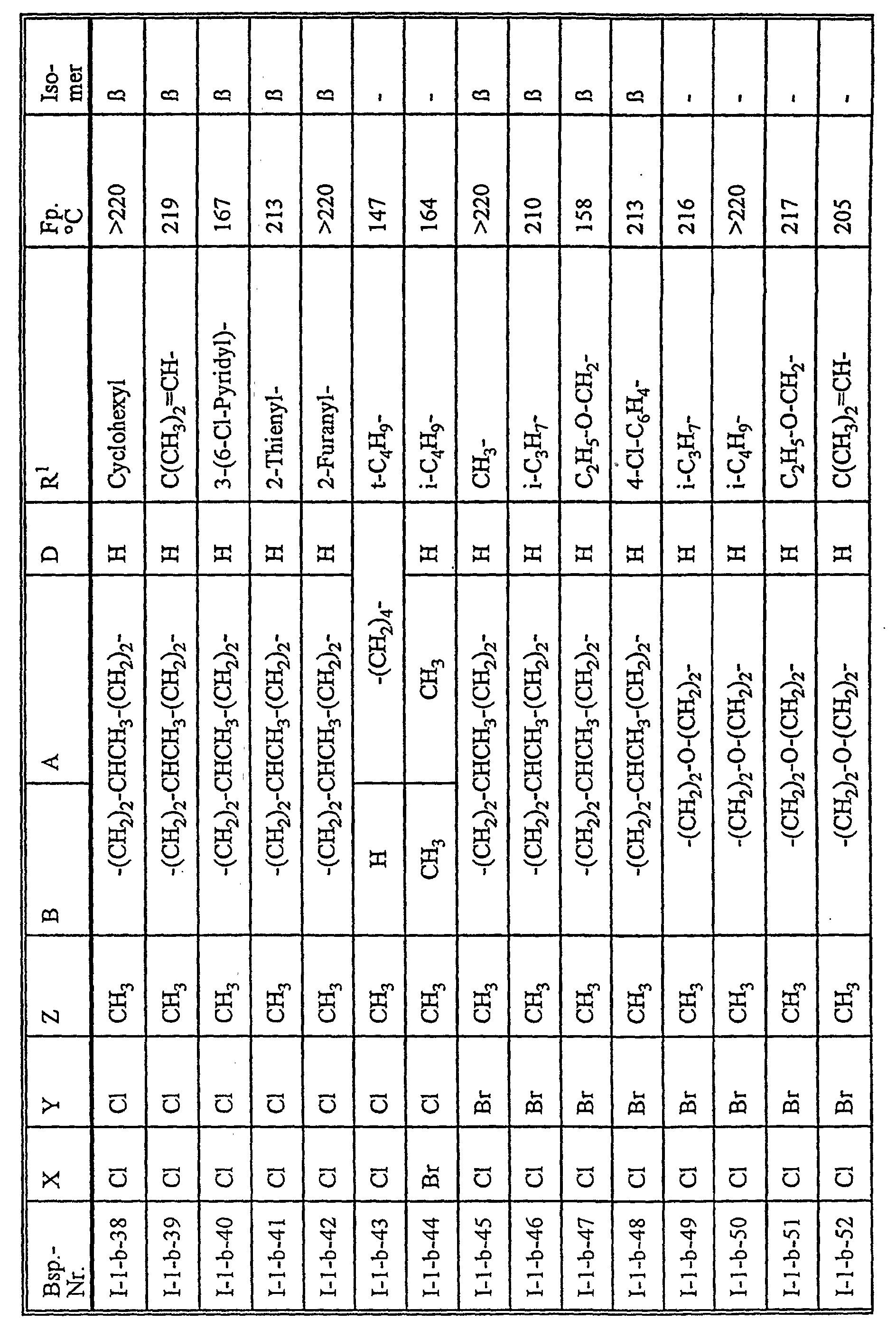

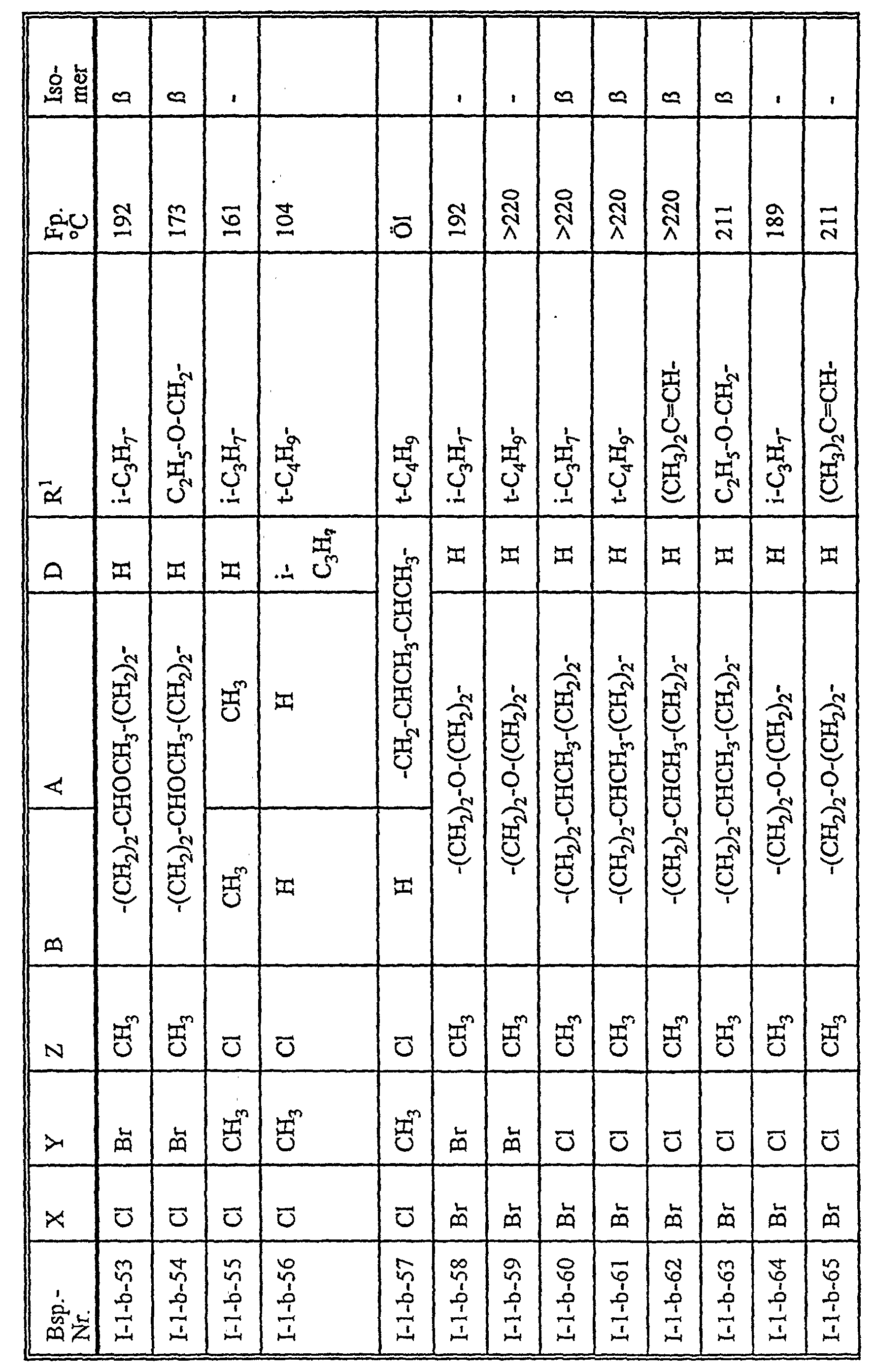

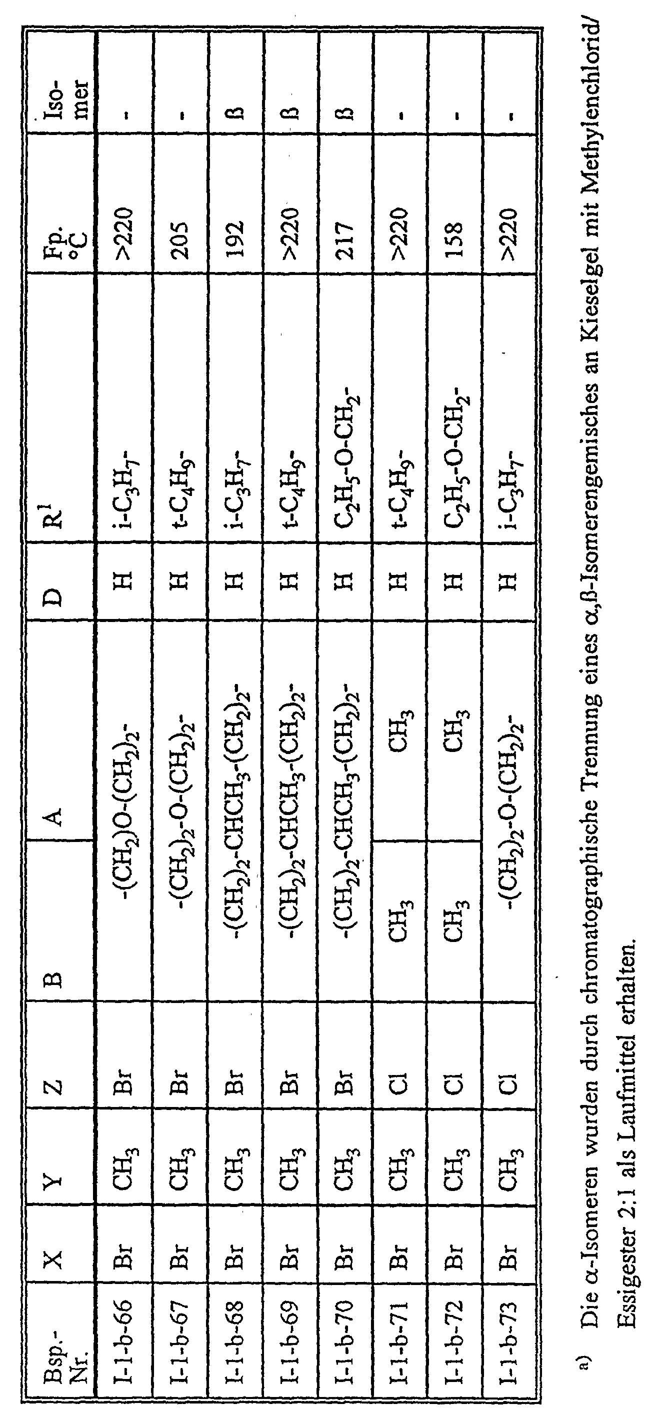

### Beispiel (I-1-c-1)

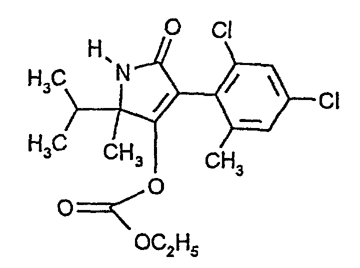

Zu 4,7 g (0,015 Mol) der Verbindung gemäß Beispiel (I-1-a-1) und 2,1 ml Triethylamin in 70 ml absolutem THF tropft man bei 0 bis 10°C 1,8 ml (18 mMol) Chlorameisensäureethylester in 5 ml absolutem Methylenchlorid und rührt bei Raumtemperatur, bis die Reaktion nach dünnschichtchromatographischer Kontrolle beendet ist. Zur Aufarbeitung wird 2 mal mit 50 ml 0,5 N Natronlauge gewaschen, über Magnesiumsulfat getrocknet und eingedampft.
Ausbeute: 4,0 g (69 % der Theorie), Fp.: >220°C.
Analog bzw. gemäß den allgemeinen Angaben zur Herstellung erhält man die folgenden Verbindungen der Formel (I-1-c):

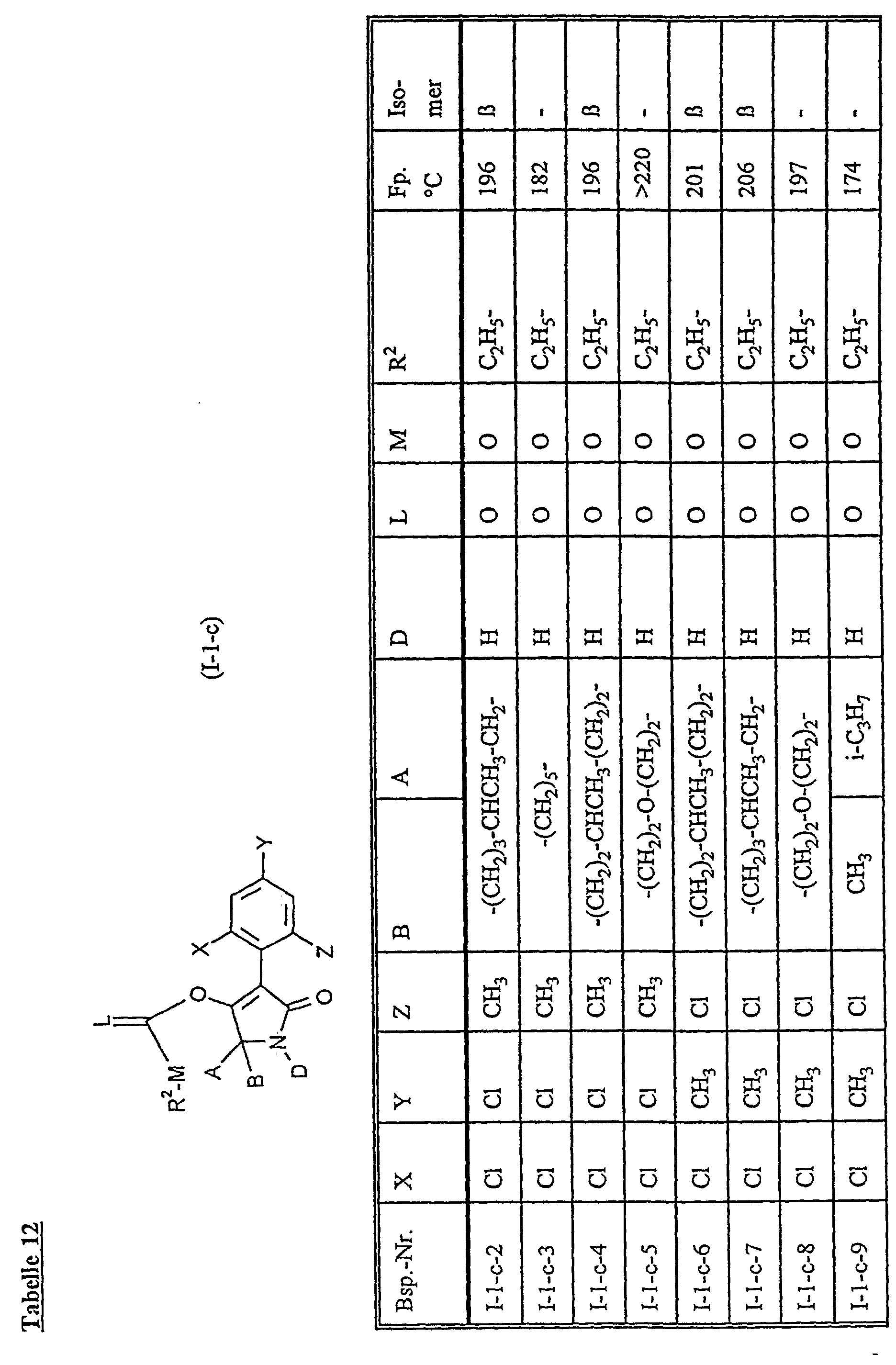

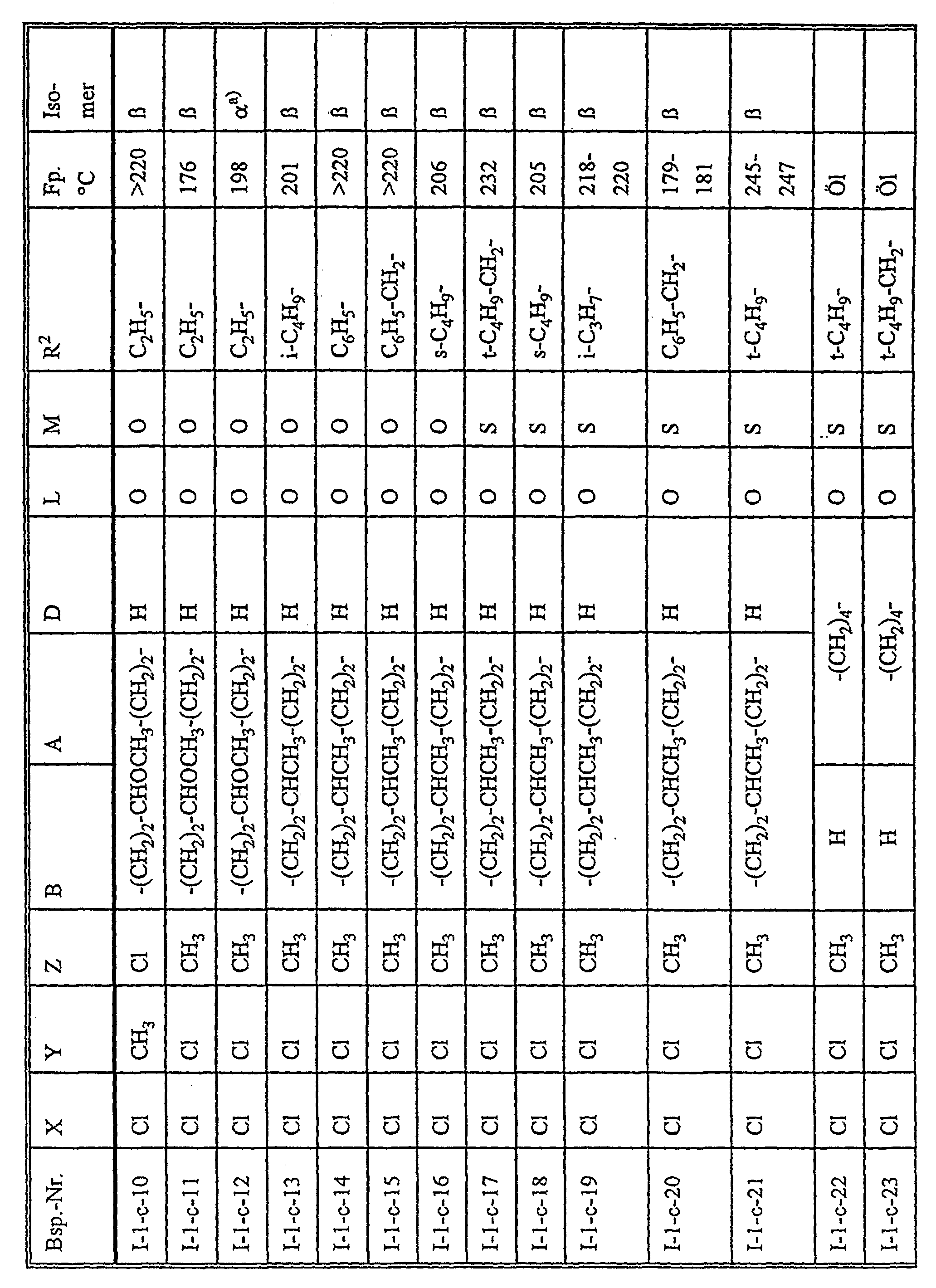

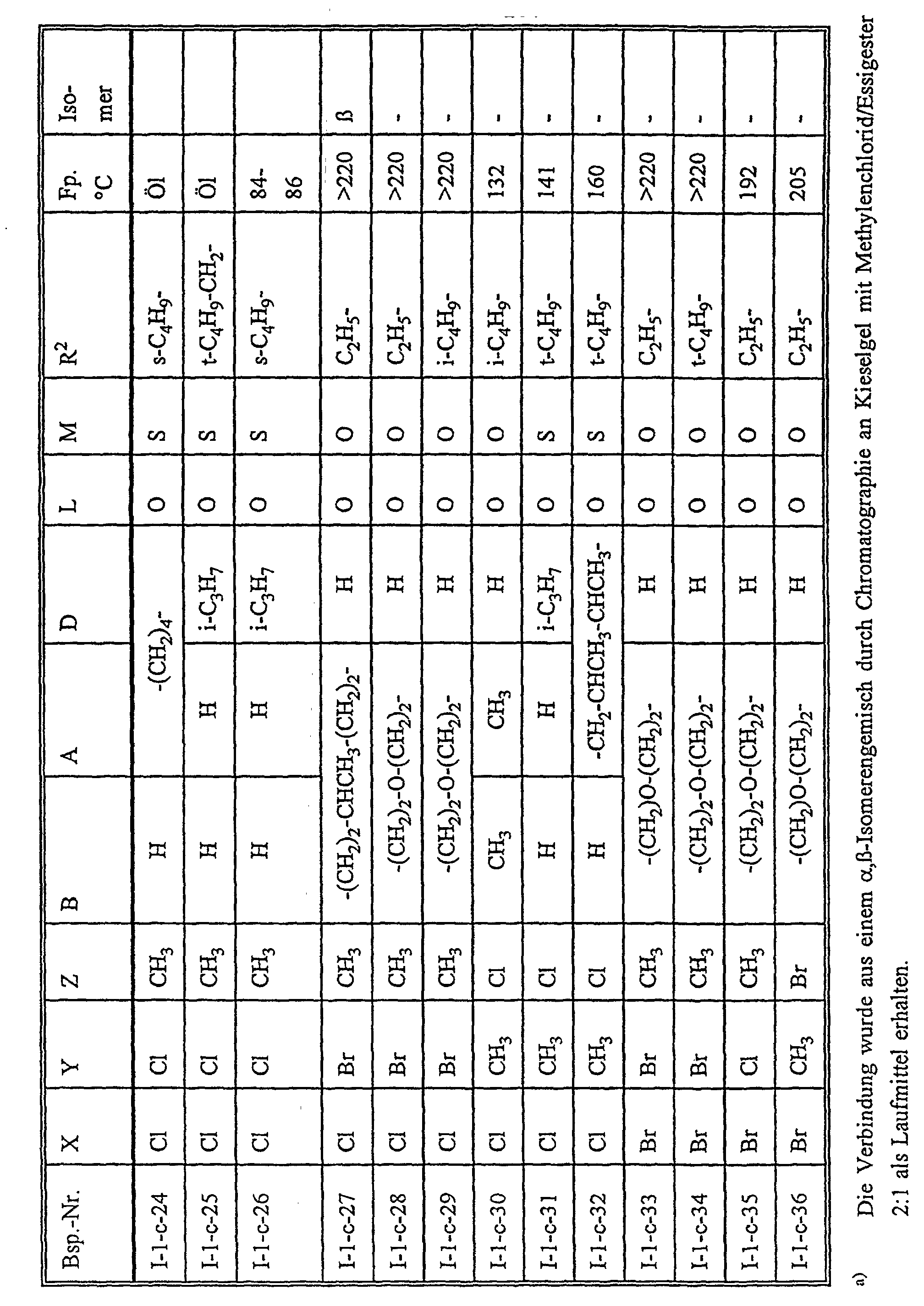

### Beispiel (I-1-d-1)

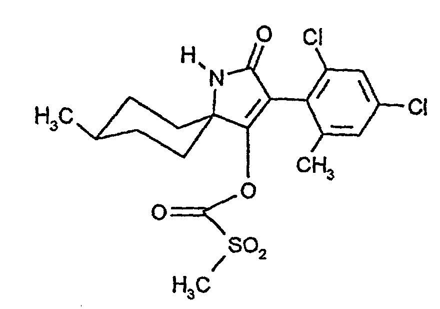

Zu 4,08 g der Verbindung gemäß Beispiel I-1-a-4 und 1,7 ml (0,012 Mol) Triethylamin in 70 ml abs. Methylenchlorid tropft man bei 0°C bis 10°C 0,93 ml (0,012 Mol) Methansulfonsäurechlorid in 5 ml abs. Methylenchlorid und rührt bei Raumtemperatur bis die Reaktion beendet ist (DC-Kontrolle). Man wäscht 2 x mit 50 ml 0,5 N NaOH, trocknet, engt ein und kristallisiert den Rückstand aus Methyltert.-butylether (MTB-Ether)/n-Hexan um.
Ausbeute: 2,80 g (52 % der Theorie), Fp.: 211°C.

### Beispiel (I-1-f-1)

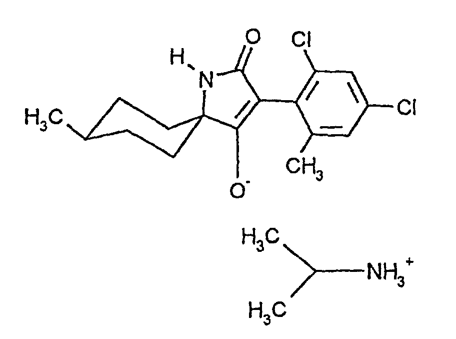

Zu 2,04 g der Verbindung gemäß Beispiel I-1-a-4 in 40 ml MTB-Ether gibt man 0,85 ml (0,01 Mol) wasserfreies Isopropylamin und rührt 10 Minuten bei Raumtemperatur. Anschließend wird im Vakuum zur Trockne eingeengt.
Ausbeute: 2,20 g (91 % der Theorie), Fp.: >220°C.

### Beispiel (I-1-g-1)

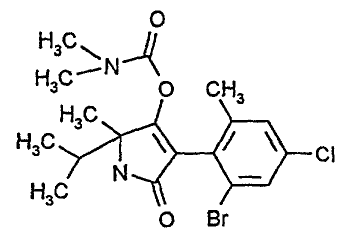

2,2 g (6,1 mmol) der Verbindung gemäß Beispiel I-1-a-19 und 1,3 ml (9,2 mmol) Triethylamin werden in 50 ml wasserfreiem Essigester gelöst und unter Rückfluß erhitzt. Dazu werden 0,85 ml (9,2 mmol) N,N-Dimethylcarbamidsäurechlorid in 5 ml wasserfreiem Essigester gegeben und unter dünnschichtchromatografischer Kontrolle rückflussiert. Die Lösung wird eingeengt, der Rückstand in Methylenchlorid aufgenommen, zweimal mit 40 ml 0,5 N NaOH gewaschen, getrocknet und eingeengt. Nach Kristallisation aus Methyl-tert.-butylether/n-Hexan erhält man 0,4 g (Δ 15 % der Theorie) der Verbindung I-1-g-1 von Fp. 140 bis 143°C.

In Analogie zu Beispiel I-1-g-1 erhält man Beispiel I-1-g-2 vom Fp. 163 bis 166°C

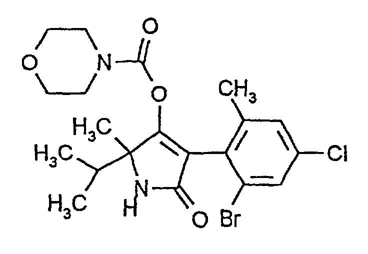

### Beispiel (II-1)

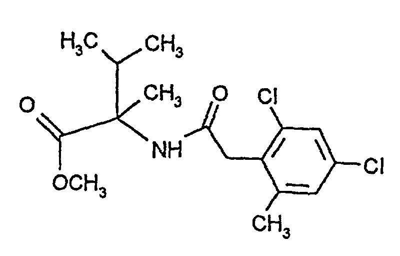

Zu 48,4 g (0,493 Mol) konz. Schwefelsäure tropft man bei 30 bis 40°C vorsichtig 30,8 g der Verbindung gemäß Beispiel (XXI-1) und rührt 2 Stunden bei dieser Temperatur. Dann tropft man 67 ml absolutes Methanol so zu, daß sich eine Innentemperatur von etwa 40°C einstellt und rührt noch 6 Stunden bei 40 bis 70°C.

Zur Aufarbeitung gießt man auf 0,48 kg Eis, extrahiert mit Methylenchlorid, wäscht mit wäßriger Natriumhydrogencarbonatlösung, trocknet und dampft ein. Das Rohprodukt wird aus MTB-Ether/n-Hexan umkristallisiert.
Ausbeute: 25,1 g (73 % der Theorie), Fp.: 114°C.

### Beispiel (II-2)

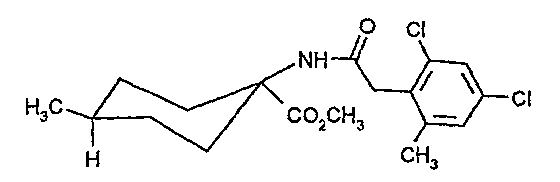

26,3 g (0,12 Mol) 2,4-Dichlor-6-methyl-phenylessigsäure gemäß Beispiel (XXV-2) und 17,7 ml (0,24 Mol) Thionylchlorid werden bei 80°C gerührt, bis die Gasentwicklung beendet ist. Überschüssiges Thionylchlorid wird bei 50°C im Vakuum entfernt. Dann gibt man 50 ml absolutes Toluol zu und dampft erneut ein. Der Rückstand wird in 100 ml absolutem THF aufgenommen (Lösung 1).

Zu 25,03 g 4-Methylcyclohexylamin-1-carbonsäuremethylester und 37 ml (0,264 Mol) Triethylamin in 250 ml absolutem THF tropft man bei 0 bis 10°C Lösung 1 und rührt anschließend 1 Stunde bei Raumtemperatur. Dann wird abgesaugt, mit absolutem THF gewaschen und eingedampft. Der Rückstand wird in Methylenchlorid aufgenommen, mit 0,5 N HCl gewaschen, getrocknet und eingedampft. Das Rohprodukt wird aus MTB-Ether/n-Hexan umkristallisiert.
Ausbeute: 34,6 g (77 % der Theorie), Fp.: 145°C.

Analog zu den Beispielen (II-1) und (II-2) bzw. gemäß den allgemeinen Angaben zur Herstellung werden die folgenden Verbindungen der Formel (II) hergestellt.

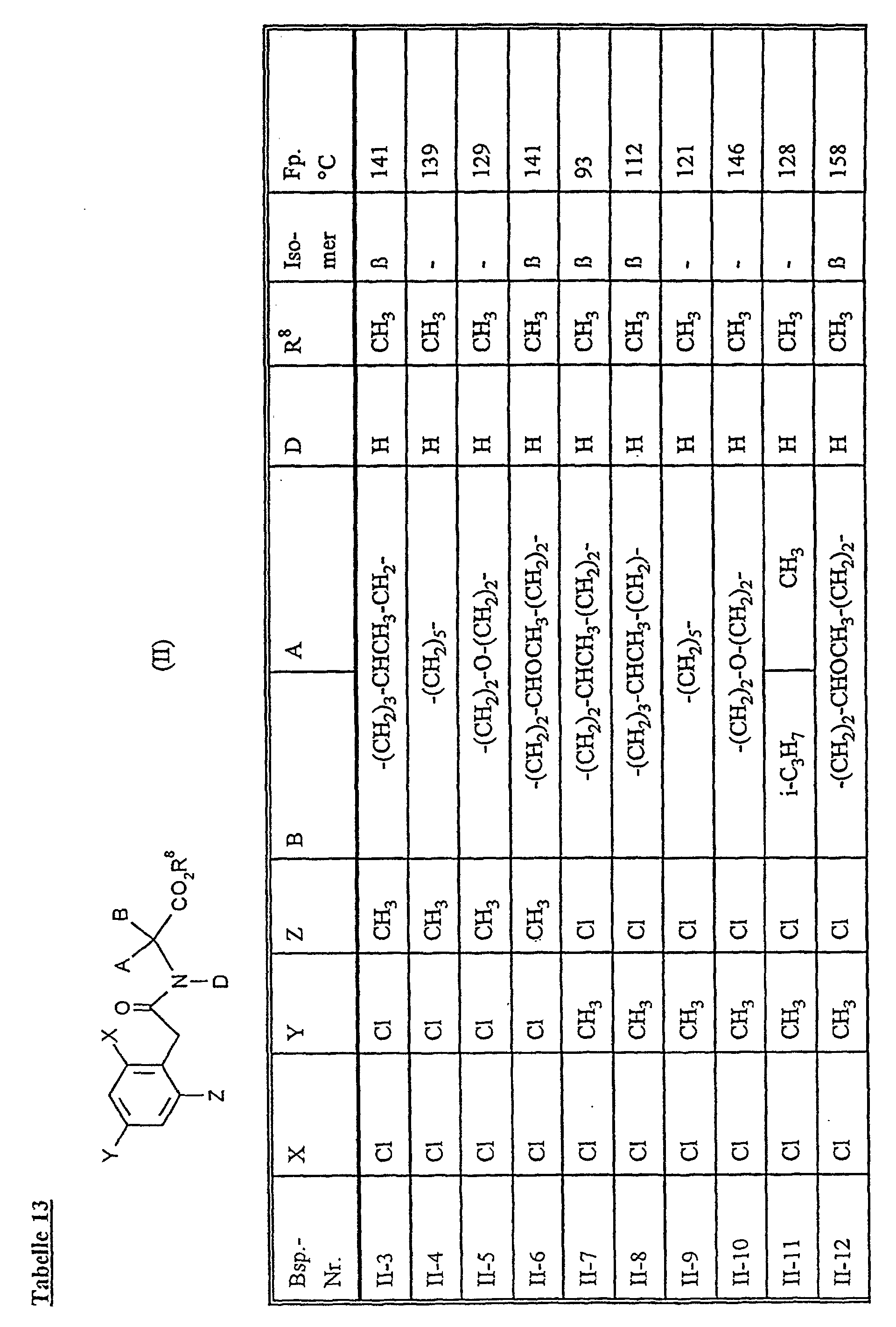

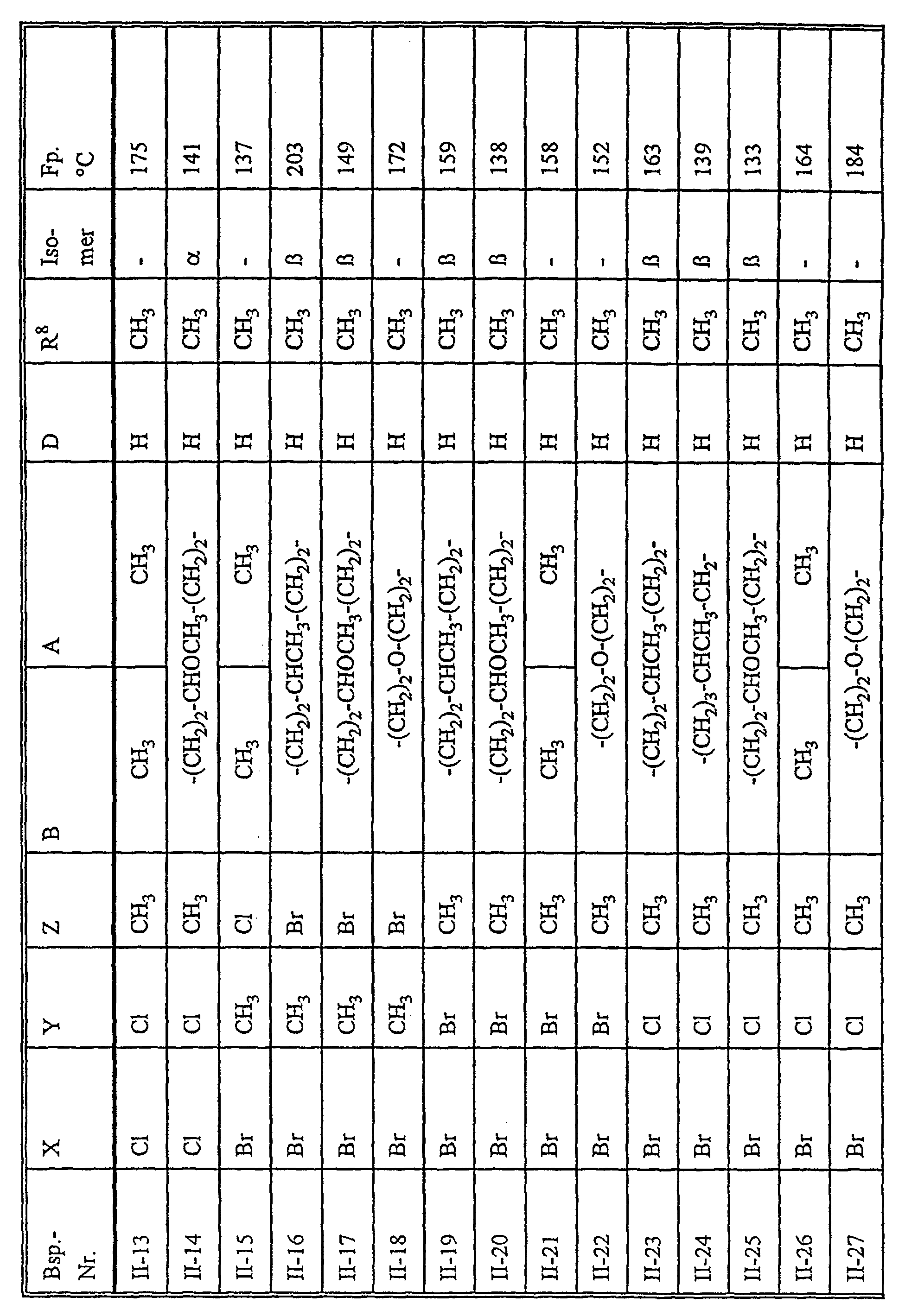

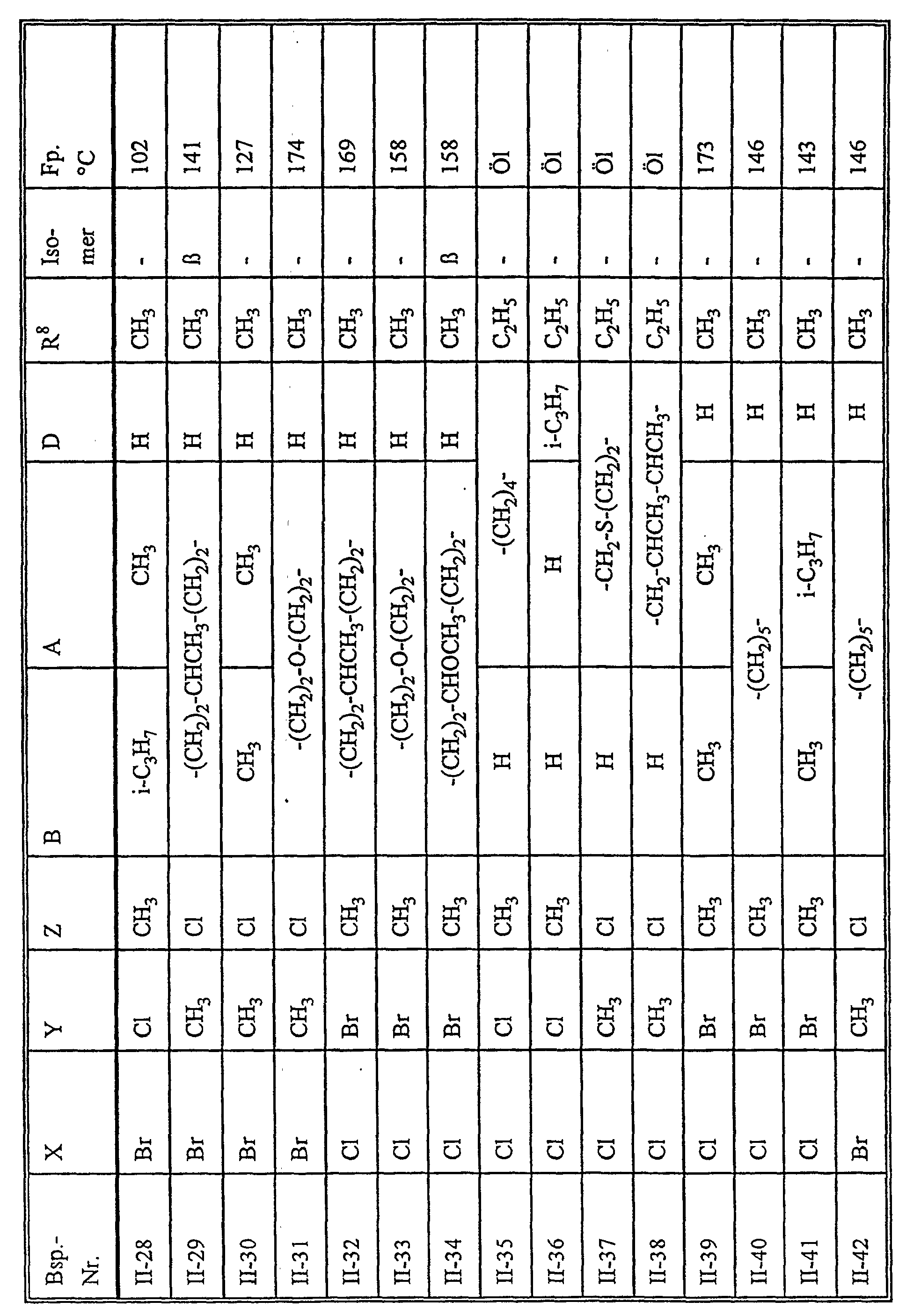

### Beispiel (XXXI-1)

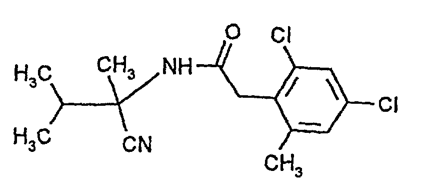

Ausgehend von 26,3 g 2,4-Dichlor-6-methyl-phenylessigsäure wird wie in Beispiel (II-2) Lösung 1 hergestellt.

Zu 13,46 g 2-Amino-2,3-dimethylbutyronitril und 18,5 ml (0,132 Mol) Triethylamin in 150 ml absolutem THF tropft man bei 0 bis 10°C Lösung 1 und rührt noch 1 Stunde bei Raumtemperatur. Dann wird abgesaugt, mit absolutem THF gewaschen und eingedampft. Man nimmt den Rückstand in Methylenchlorid auf, wäscht mit 0,5 N HCl, trocknet und dampft ein. Das Rohprodukt wird aus MTB-Ether/n-Hexan umkristallisiert.
Ausbeute: 30,8 g (81 % der Theorie), Fp.: 112°C.

Analog zu Beispiel (XXXI-1) bzw. gemäß den allgemeinen Angaben zur Herstellung wurden die folgenden Verbindungen der Formel (XXXI) hergestellt.

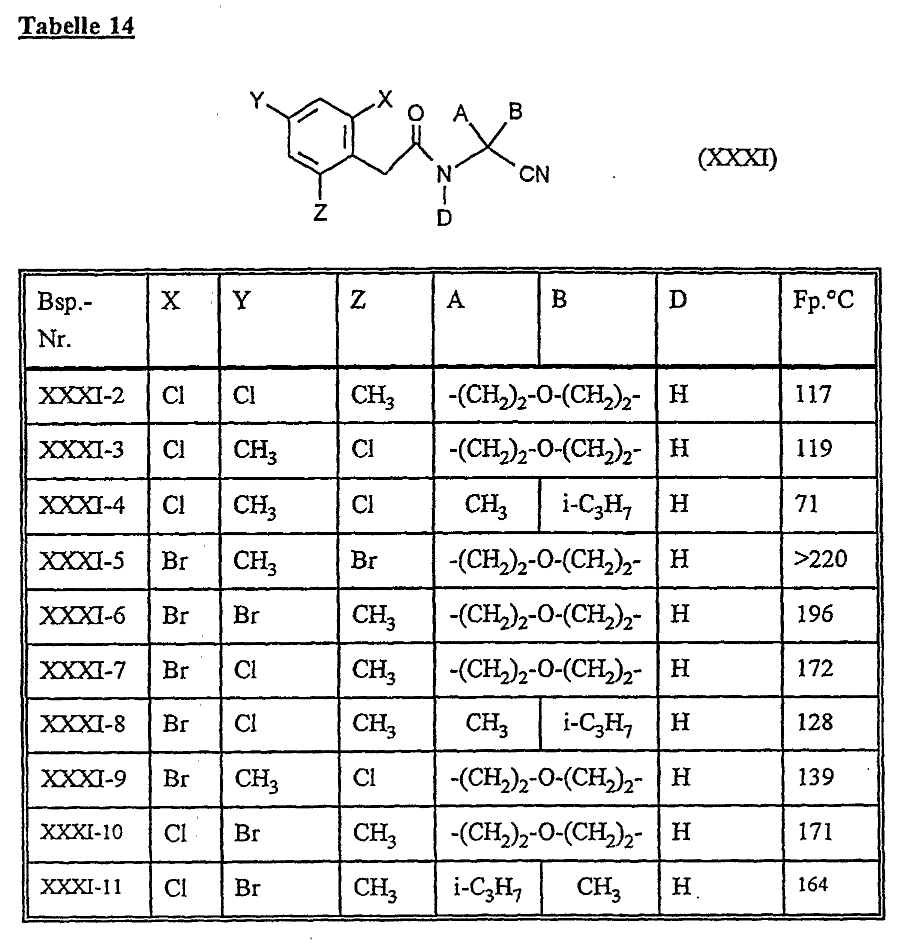

### Beispiel (I-2-a-1)

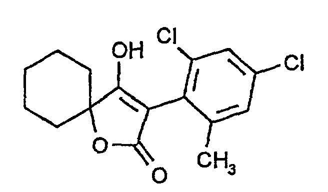

8,42 g (75 mMol) Kalium-tert.-butylat werden in 50 ml DMF vorgelegt, bei 0 bis 10°C eine Lösung von 18,6 g (50 mMol) 2,4-Dichlor-6-methyl-phenylessigsäure-(1-ethyloxycarbonyl-cyclohexyl)-ester gemäß Beispiel (III-1) in 50 ml DMF zugetropft und über Nacht bei Raumtemperatur gerührt.

Zur Aufarbeitung tropft man das Reaktionsgemisch in 500 ml eiskalte 1 N HCl, saugt das ausgefallene Produkt ab, wäscht mit Wasser und trocknet im Vakuumtrockenschrank. Zur weiteren Reinigung wird das Rohprodukt noch mit n-Hexan/Aceton ausgekocht.
Ausbeute: 6,71 g (41 % der Theorie) 3-(2,4-Dichlor-6-methyl-phenyl)-5-spirocyclohexyltetronsäure, Fp.: >240°C.

Analog bzw. gemäß den allgemeinen Angaben zur Herstellung erhält man die folgenden Verbindungen der Formel (I-2-a):

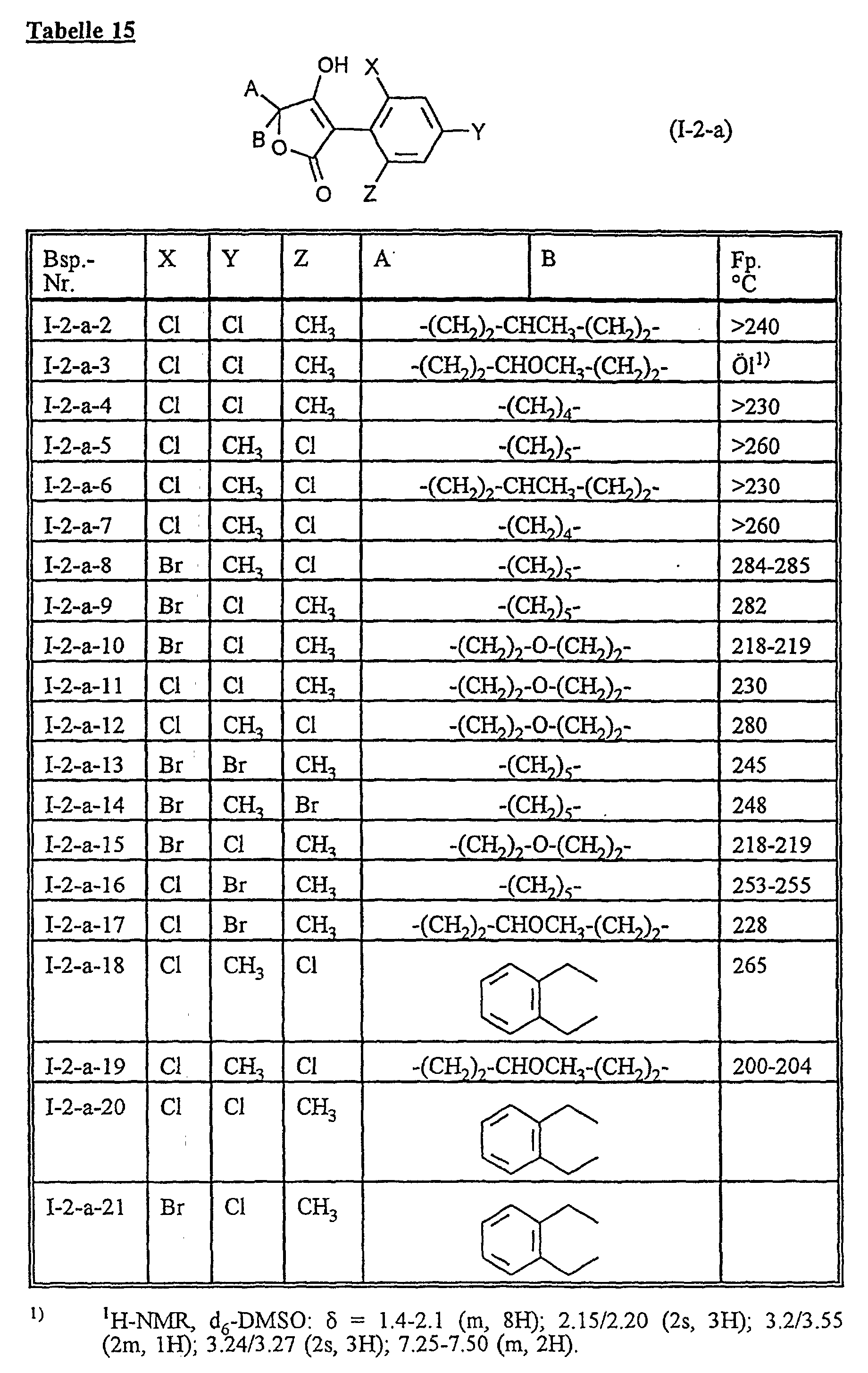

### Beispiel (I-2-b-1)

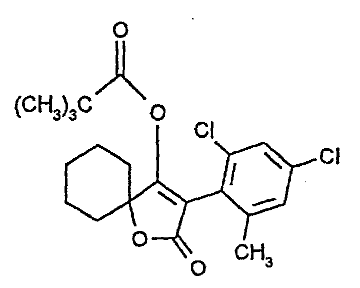

3,27 g (10 mMol) der Verbindung gemäß Beispiel I-2-a-1 werden in 40 ml Dichlormethan vorgelegt, 1,52 g (15 mMol) Triethylamin zugesetzt, unter Eiskühlung eine Lösung von 1,57 g (13 mMol) Pivaloylchlorid in 40 ml Dichlormethan zugetropft und 1 bis 2 h bei Raumtemperatur nachgerührt. Zur Aufarbeitung wäscht man nacheinander mit 10 %iger Citronensäure, 1N NaOH und NaCl-Lösung, trocknet die organische Phase über MgSO₄ und dampft ein. Das Rohprodukt wird zur weiteren Aufreinigung noch mit wenig Petrolether verrührt.
Ausbeute: 3,6 g (88 % der Theorie) 4-Pivaloyl-oxy-3-(2,4-dichlor-6-methyl-phenyl)-5-spiro-cyclohexyl-tetronsäure, Fp.: 137°C.

Analog bzw. gemäß den allgemeinen Angaben zur Herstellung erhält man die folgenden Verbindungen der Formel (I-2-b):

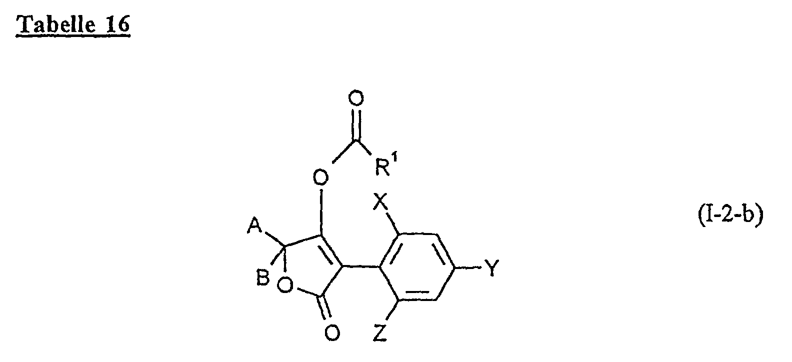

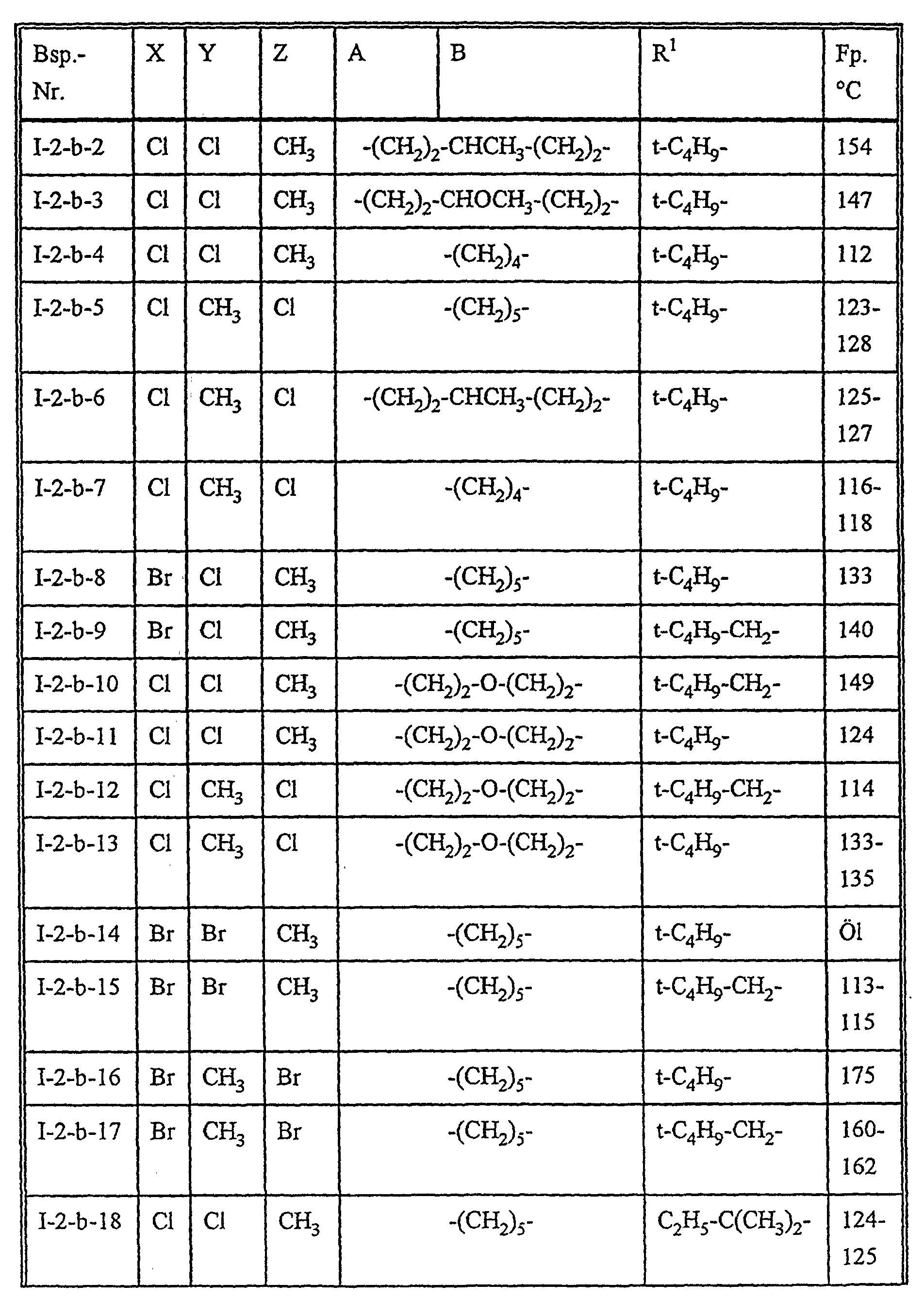

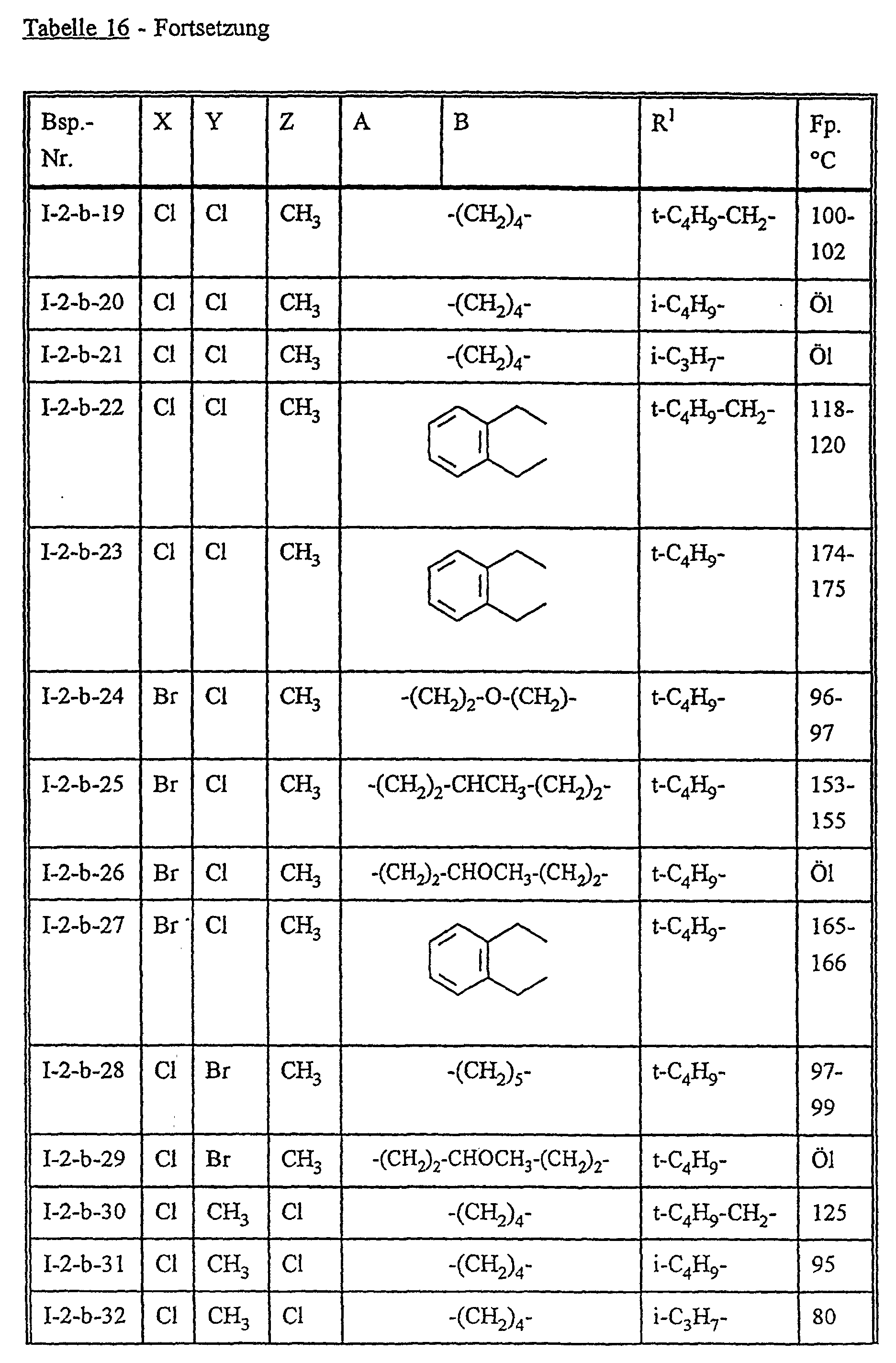

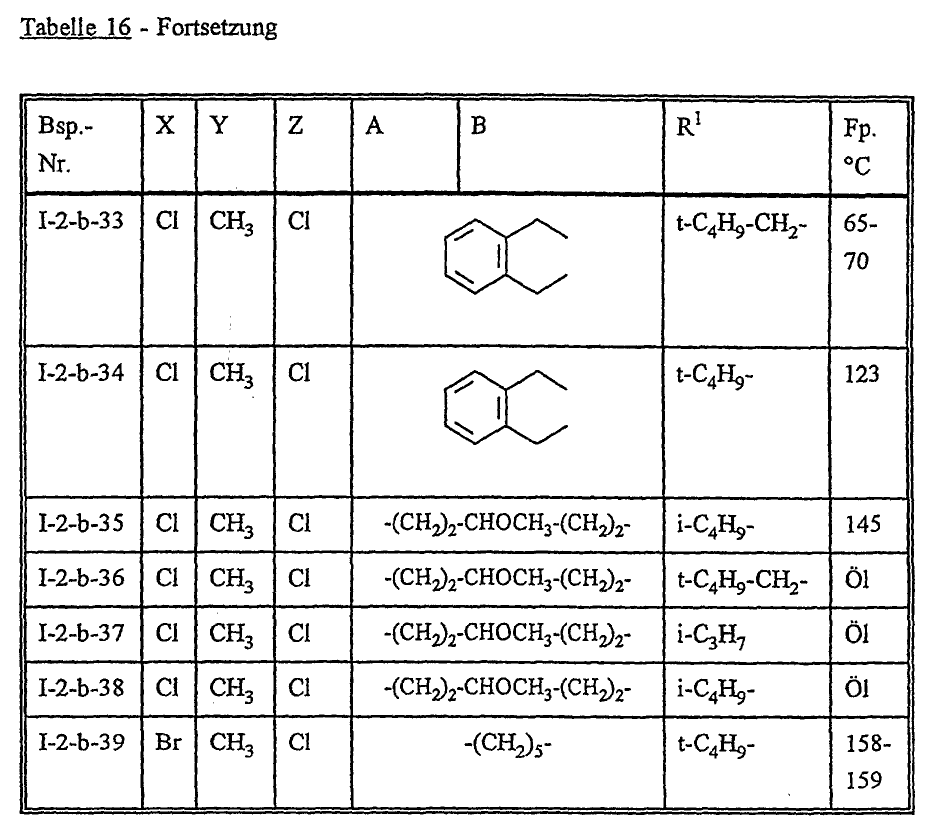

### Beispiel (III-1)

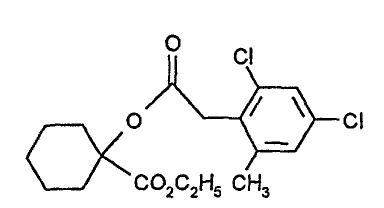

10,95 g (50 mMol) 2,4-Dichlor-6-methyl-phenylessigsäure werden in 50 ml Toluol vorgelegt, 11,9 g (100 mMol) Thionylchlorid zugegeben, bis zur Beendigung der Chlorwasserstoffentwicklung bei 80°C gerührt und eingedampft. Das rohe Säurechlorid wird zusammen mit 8,6 g (50 mMol) 1-Hydroxy-cyclohexancarbonsäureethylester über Nacht in 50 ml Toluol gekocht, anschließend wird eingedampft.
Ausbeute: 18,6 g (quant.) 2,4-Dichlor-6-methyl-phenylessigsäure(1-ethoxycarbonylcyclohexyl)-ester als farbloses Öl.
¹H-NMR (CDCl₃): δ = 1.2 (t, 3H); 1.3-2.0 (m, 9H); 2.3 (s, 3H); 3.8 (s, 2H); 4.15 (q, 2H); 7.3 (m, 2H).

Analog bzw. gemäß den allgemeinen Vorschriften zur Herstellung erhält man die folgenden Verbindungen der Formel (III):

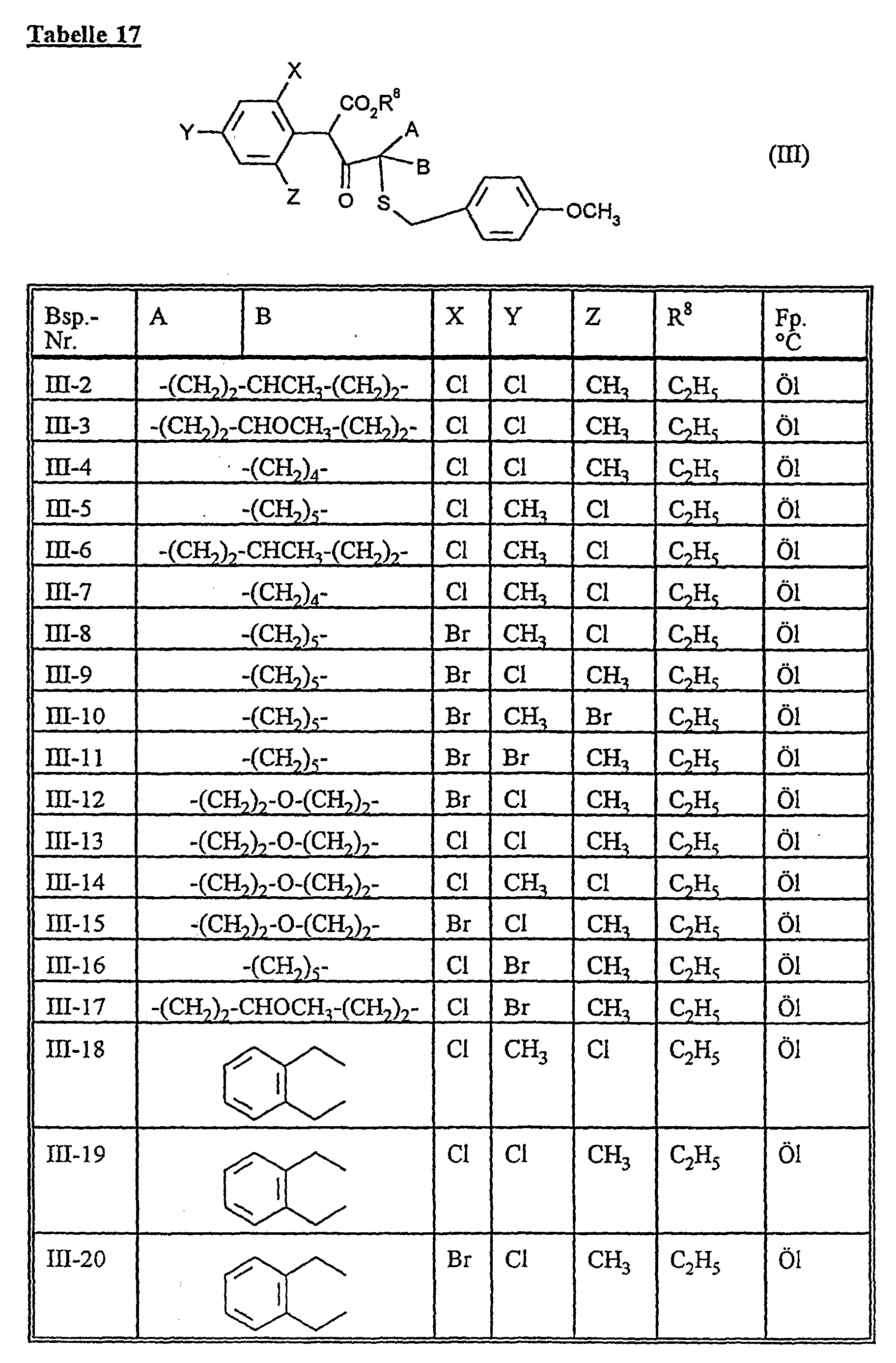

### Beispiel (I-3-a-1)

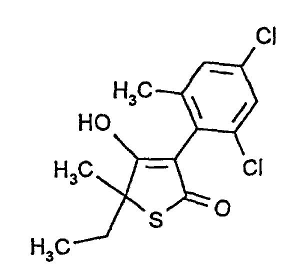

10,8 g (23 mMol) der Verbindung gemäß Beispiel (IV-1) werden in 25 ml Trifluoressigsäure und 50 ml Toluol 5 Stunden unter Rückfluß erhitzt. Es wird zur Trockne eingeengt, der Rückstand mit 120 ml Wasser und 40 ml MTB-Ether versetzt und es wird so lange NaOH zugegeben, bis pH 14 erreicht ist. Es wird 2 mal mit MTB-Ether extrahiert. Dann wird die wäßrige Phase angesäuert und 3 mal mit MTB-Ether extrahiert. Die organische Phase wird getrocknet, eingeengt, der Rückstand mit Cyclohexan verrührt und abgesaugt.
Ausbeute: 0,60 g (8 % der Theorie), Fp.: 222°C.

Analog bzw. gemäß den allgemeinen Angaben zur Herstellung wurden die folgenden Verbindungen der Formel (I-3-a) erhalten:

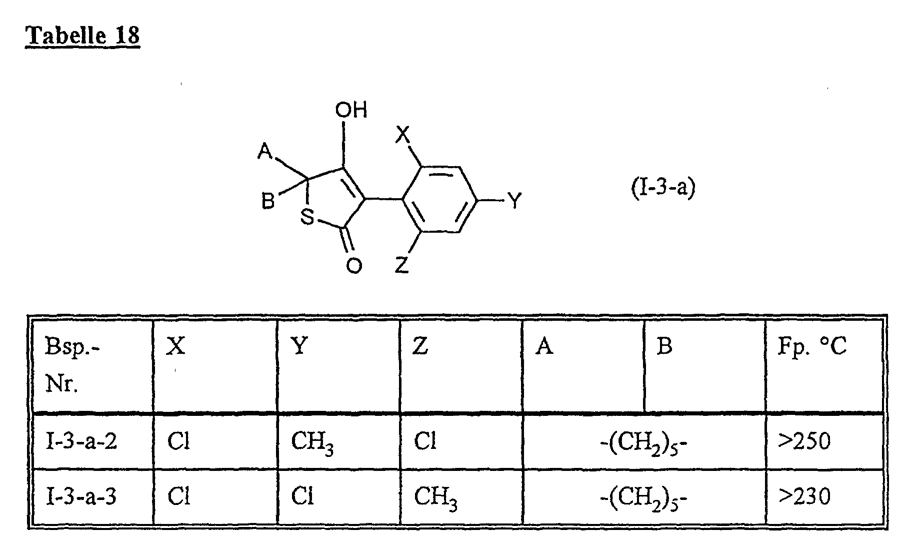

### Beispiel (I-3-b-1)

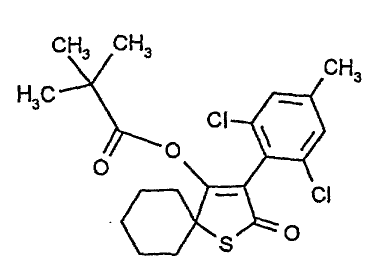

1 g (2,9 mMol) der Verbindung gemäß Beispiel (I-3-a-2) werden in 12 ml abs. Methylenchlorid vorgelegt und mit 0,6 ml (1,5 eq) Triethylamin versetzt. Eine Lösung von 0,47 ml (0,45 g, 3,77 mMol) Pivaloylchlorid in 3 ml abs. Methylenchlorid werden unter Eiskühlung zugetropft. Der Ansatz wird 1 bis 2 Stunden bei Raumtemperatur gerührt. Wegen unvollständigem Umsatz werden 0,3 ml Triethylamin und 0,24 ml Säurechlorid nachdosiert. Anschließend wird 2 mal mit 10 %iger Citronensäure gewaschen und die vereinigten wäßrigen Phasen mit Methylenchlorid extrahiert. Die vereinigten organischen Phasen werden 2 mal mit 1 N NaOH gewaschen und die wäßrigen Phasen mit Methylenchlorid extrahiert. Die vereinigten organischen Phasen werden getrocknet und eingeengt. Der Rückstand wird in Petrolether verrührt, abgesaugt und getrocknet.
Ausbeute: 0,70 g (56 % der Theorie), Fp.: 100-108°C.

### Beispiel (I-3-c-1)

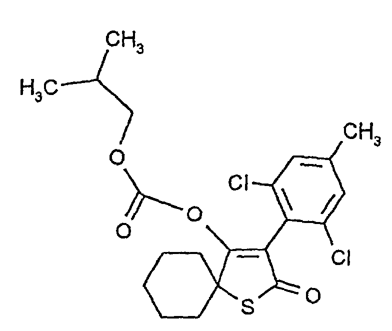

1 g (2,9 mMol) der Verbindung gemäß Beispiel (I-3-a-2) werden in 12 ml abs. Methylenchlorid vorgelegt und mit 0,6 ml (1,5 eq) Triethylamin versetzt. Eine Lösung von 0,49 ml (0,51 g, 3,77 mMol) Chlorameisensäureisobutylester in 3 ml abs. Methylenchlorid wird unter Eiskühlung zugetropft. Der Ansatz wird 1 bis 2 Stunden bei Raumtemperatur gerührt. Aufarbeitung wie bei Beispiel (I-3-b-1).
Ausbeute: 0,60 g (47 % der Theorie), Fp.: 174-176°C.

### Beispiel (I-6-a-1)

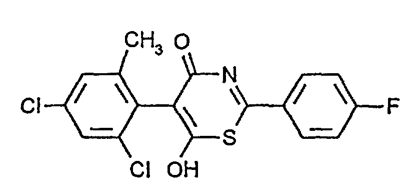

2,6 g (10 mmol) der Verbindung gemäß Beispiel V-1 werden in 80 ml wasserfreiem Toluol vorgelegt. Nach Zugabe von 1,6 g (10 mmol) 4-Fluor-thiobenzamid rührt man 6 h bei 50°C. Beim Abkühlen fällt ein Niederschlag aus. Nach Absaugen, zweimaligem Waschen mit Cyclohexan und säulenchromatografischer Trennung an Kieselgel mit Toluol/Ethanol 20:1 erhält man 1,7 g (44 % der Theorie) der oben gezeigten Verbindung vom Fp. 276 bis 278°C.

### Beispiel (IV-1)

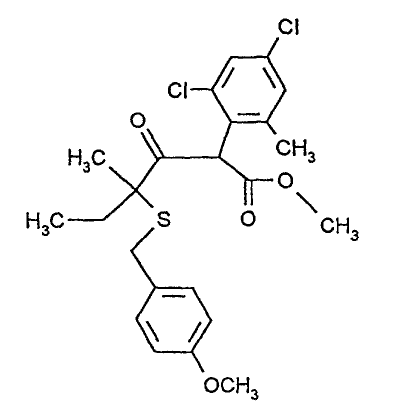

**A:** 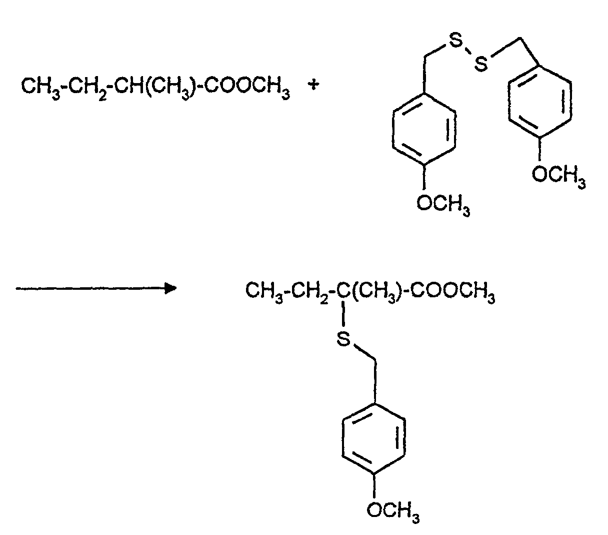 Zu 17,65 g (173,2 mMol) Diisopropylamin in 130 ml abs. THF tropft man bei -78°C (100 ml (160 mMol) n-Butyllithium (1,6 N in n-Hexan) und rührt 20 Minuten. Dann tropft man 15,5 g (133,2 mMol) Methyl-2-methylbutyrat in 30 ml abs. THF zu und rührt 60 Minuten. Anschließend tropft man 40,63 g (133,2 mMol) Bis-(4-methoxybenzyl)-disulfid, gelöst in 120 ml THF, zu, läßt die Mischung auf Raumtemperatur kommen und rührt 1 Stunde. Man versetzt mit 300 ml MTB-Ether, wäscht mit 150 ml Wasser, trocknet und engt ein.
   Ausbeute: 35,1 g.
**B:** 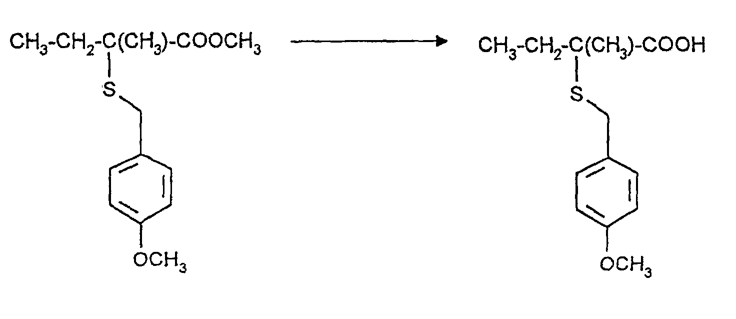 1,5 g (5,6 mMol) der oben gemäß A hergestellten Verbindung werden mit 2,0 g KOH in 20 ml Methanol 48 Stunden bei 40°C gerührt. Nach üblicher Aufarbeitung und säulenchromatographischer Reinigung des Rohproduktes (Laufmittel Cyclohexan/Essigester 1/1) erhält man 0,6 g (42 % der Theorie) der Säure.
C:
   25 g (98 mMol) der oben gemäß B hergestellten Carbonsäure in 107 ml Toluol werden mit einem Tropfen Dimethylformamid (DMF) und 17,5 g (147 mMol) Thionylchlorid versetzt und bei 100°C bis zum Ende der Gasentwicklung gerührt. Anschließend wird zur Trockne eingedampft und das Säurechlorid in 40 ml abs. THF gelöst (Säurechloridlösung).
   13,0 g (129 mMol) Diisopropylamin in 100 ml THF werden tropfenweise bei 0°C mit 71,6 ml (118 mMol) Butyllithium (1.6 M in n-Hexan) versetzt. Nach 15 Minuten tropft man bei 0°C 25,05 g (108 mMol) 2,4-Dichlor-6-methylphenylessigsäuremethylester in 40 ml THF zu und rührt 30 Minuten. Anschließend tropft man bei 0°C die oben hergestellte Säurechloridlösung zu und rührt eine Stunde bei Raumtemperatur. Man versetzt mit 350 ml MTB-Ether, wäscht 2 mal mit 10 %iger Ammoniumchloridlösung, trocknet die organische Phase und engt ein. Nach säulenchromatographischer Reinigung des Rohprodukts (Laufmittel Cyclohexan/Essigester 20/1 bis 10/1) erhält man 11,3 g (25 % der Theorie) der Verbindung der Formel (IV-1).

Analog bzw. gemäß den allgemeinen Angaben zur Herstellung erhält man die in der folgenden Tabelle aufgeführten Verbindungen der Formel (IV):

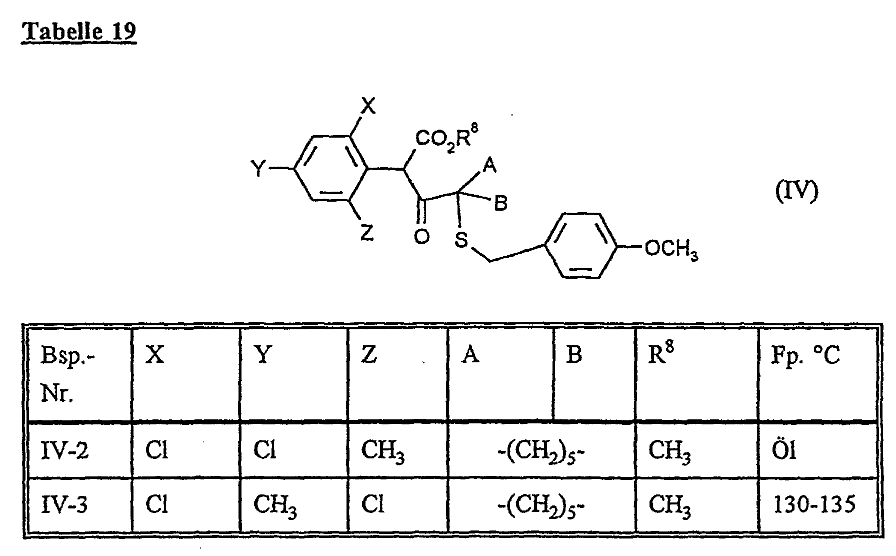

### Beispiel (I-5-a-1)

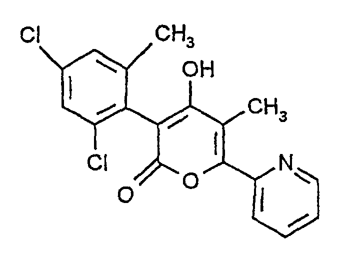

2,6 g (10 mMol) 2,4-Dichlor-6-methyl-phenylchlorcarbonylketen legt man in 20 ml abs. Toluol vor, gibt bei 20°C 1,3 g (10 mMol) Ethyl-2-pyridylketen zu und erhitzt 8 Stunden unter Rückfluß. Der Niederschlag wird in der Kälte abgetrennt und an 200 g Kieselgel (35 bis 70 µm) chromatographiert. (Laufmittel Toluol/Ethanol 10:1)
Man erhält 1,0 g (28 %) Feststoff vom Fp.: 96-100°C.
R_{F}= 0,14 (Toluol/Ethanol = 10/1).

Analog bzw. gemäß den allgemeinen Angaben zur Herstellung erhält man die in der folgenden Tabelle aufgeführten Verbindungen der Formel (I-5-a):

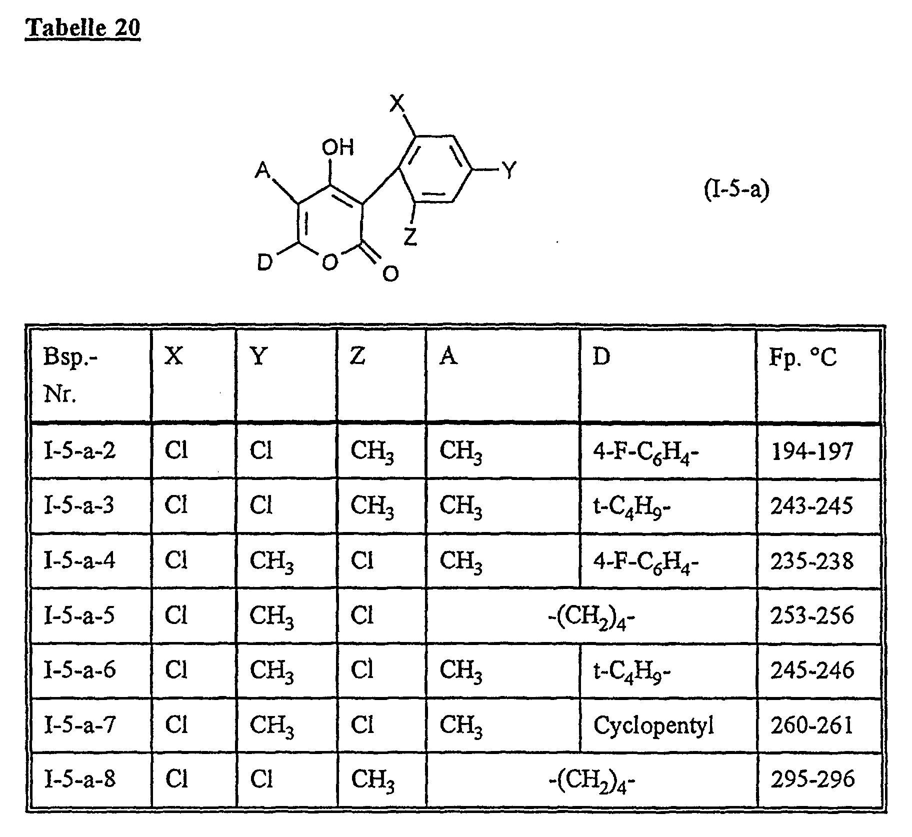

### Beispiel (V-1)

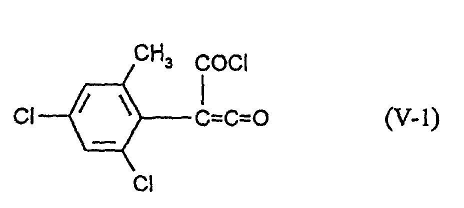

26 g (0,1 Mol) der Verbindung gemäß Beispiel (XXXV-1) in 115 ml Toluol werden bei Raumtemperatur mit 85 ml Thionylchlorid versetzt Anschließend wird über Nacht unter Rückfluß erhitzt. Man läßt abkühlen und engt zur Trockne ein.
Ausbeute: 26,2 g (99 % der Theorie).
¹H-NMR (200 MHz, CDCl₃): δ = 2.35, 2.37 (2s, 3H, Ar-CH₃),
7.05-7.45 (m, 2H Ar-H).

### Beispiel (V-2)

Analog erhält man ausgehend von der Verbindung gemäß Beispiel (XXXV-2) 2-(2,6-Dichlor-4-methylphenyl)chlorcarbonylketen als Öl.

### Beispiel (XXXV-1)

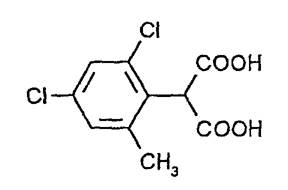

10 g (0,034 Mol) der Verbindung gemäß Beispiel (VI-1) in 20 ml Methanol werden bei Raumtemperatur tropfenweise mit einer Lösung von 6,4 g (0,114 Mol) KOH in 8,6 ml Wasser versetzt. Anschließend wird 10 Stunden unter Rückfluß erhitzt.

Nach üblicher Aufarbeitung erhält man 4,7 g (53 % der Theorie) eines Feststoffs vom Fp.: 176-179°C.

### Beispiel (XXXV-2)

Analog erhält man ausgehend von der Verbindung gemäß Beispiel (VI-2) 2-(2,6-Dichlor-4-methylphenyl)malonsäure vom Fp.: 199-207°C.

### Beispiel (VI-1)

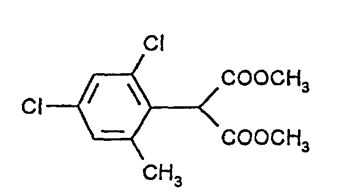

Zu 820 ml Dimethylcarbonat gibt man bei Raumtemperatur 21,7 g (0,7 Mol) 80 %iges Natriumhydrid. Anschließend tropft man bei 80 bis 90°C 125 g (0,54 Mol) 2,4-Dichlor-6-methylphenylessigsäuremethylester zu und rührt über Nacht bei dieser Temperatur. Man versetzt mit wenig Methanol, gibt auf Eiswasser und säuert mit 20 %iger HCl an. Die Phasen werden getrocknet, mehrfach mit Methylenchlorid extrahiert, die vereinigten organischen Phasen werden getrocknet und eingedampft.
Ausbeute: 132,1 g (84 % der Theorie), Fp.: 96-99°C.

### Beispiel (VI-2)

Analog erhält man 2-(2,6-Dichlor-4-methylphenyl)-malonsäuredimethylester als Öl.
¹H-NMR (200 MHz, CDCl₃): δ = 2.31 (s, 3H, 4-CH₃-Ar), 2.77 (s, 6H, OCH₃),
5.43 (s, 1H, CH), 7.18 (s, 1H, Ar-H).

### Beispiel (XXII-1)

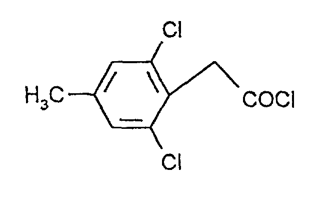

Zu 38,9 g (0,177 Mol) 2,6-Dichlor-4-methylphenylessigsäure werden 63 g (0,531 Mol) Thionylchlorid bei Raumtemperatur zugetropft und anschließend auf 70°C erwärmt, bis die Gasentwicklung beendet ist. Überschüssiges Thionylchlorid wird im Vakuum abdestilliert und der Rückstand im Hochvakuum destilliert.

Man erhält 37,8 g (92 % der Theorie) des Phenylessigsäurechlorids (XXII-1) vom Kp._{0.06-0.03 mbar} 109-115°C.

Analog bzw. gemäß den allgemeinen Angaben zur Herstellung wurden die in der folgenden Tabelle aufgeführten Verbindungen der Formel (XXII) hergestellt:

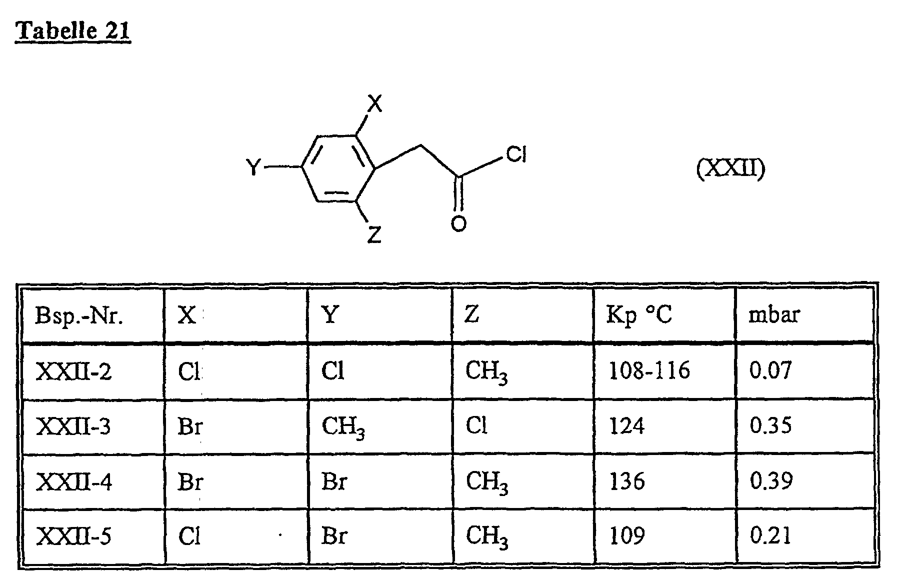

Alle anderen Säurechloride, die für die Synthese der Verbindungen (II), (III) und (XXXI) eingesetzt wurden, wurden als Rohprodukte eingesetzt und nicht näher charakterisiert.

### Beispiel (XXV-1)

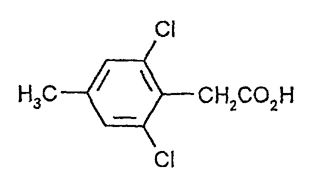

Zu einer Mischung aus 88,51 g (1,58 Mol) KOH in 115 ml Wasser und 230 ml Methanol tropft man bei Raumtemperatur 228,6 g (0,98 Mol) der Verbindung gemäß Beispiel (XXVI-1) (94 %ig) und erhitzt 5 Stunden unter Rückfluß. Nach dem Abkühlen wird mit 300 ml Wasser verdünntn und mit Essigsäureethylester extrahiert. Die wäßrige Phase wird mit halbkonzentrierter Salzsäure angesäuert, der Niederschlag abgesaugt und getrocknet.
Ausbeute: 183 g (90 % der Theorie), Fp.: 201-203°C.

Analog bzw. gemäß den allgemeinen Angaben zur Herstellung wurden die in der folgenden Tabelle aufgeführten Verbindungen der Formel (XXV) erhalten:

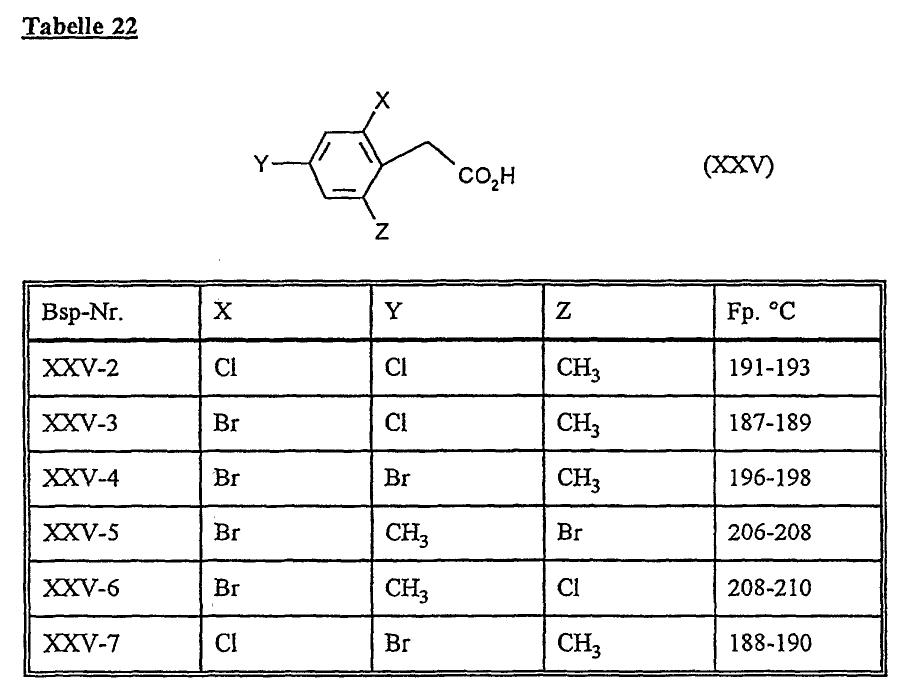

### Beispiel (XXVI-1)

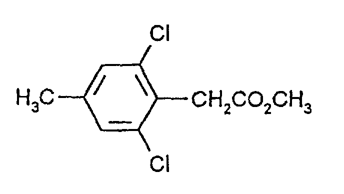

Zur Lösung von 283,5 g (0,97 Mol) der Verbindung gemäß Beispiel (XXVII-1) in 410 ml Methanol tropft man bei Raumtemperatur 740 ml einer 30 %igen methanolischen Lösung von Natriummethylat, erhitzt 5 Stunden unter Rückfluß, kühlt auf Raumtemperatur ab und tropft 110 ml konz. Schwefelsäure zu. Man kocht eine Stunde unter Rückfluß, destilliert das Methanol ab und nimmt den festen Rückstand in Wasser auf. Man trennt die organische Phase ab und extrahiert die wäßrige Phase 2 mal mit 1,5 l Methylenchlorid. Die vereinigten organischen Phasen werden über MgSO₄ getrocknet und eingeengt.
Ausbeute: 228,6 g eines Öls (Ausbeute quant.) vom Kp._{0,1 mbar} 104-108°C.

Analog bzw. gemäß den allgemeinen Angaben zur Herstellung wurden die in der folgenden Tabelle aufgeführten Verbindungen der Formel (XXVI) erhalten:

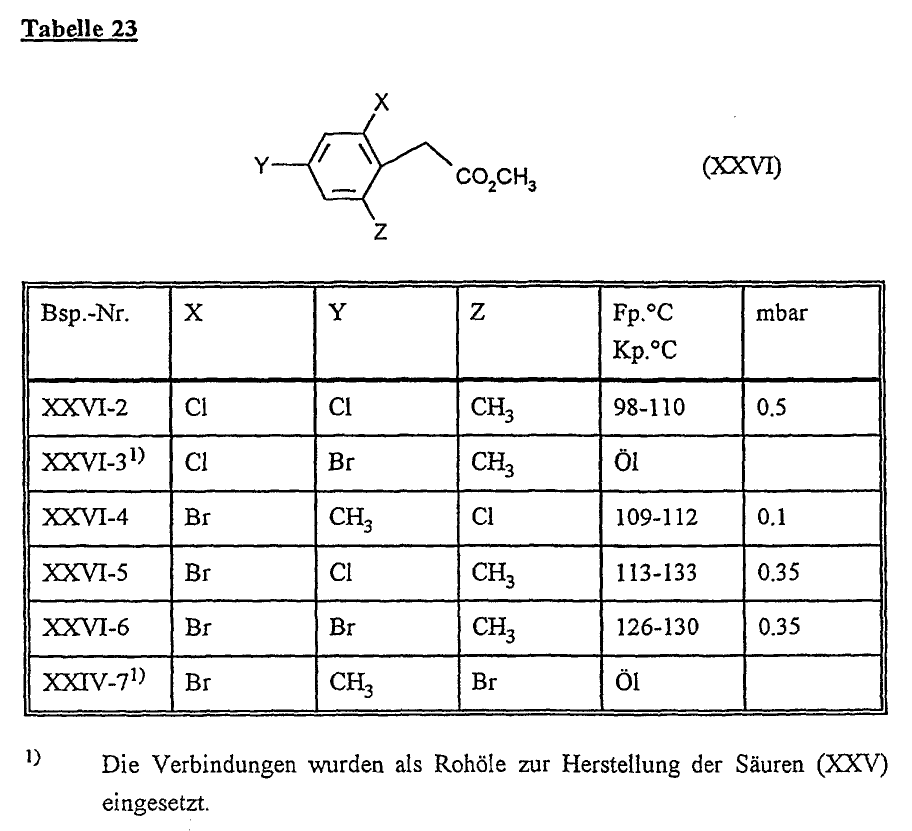

### Beispiel (XXVII-1)

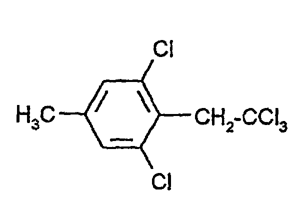

Zur gut gekühlten Mischung von 92,37 g (0,914 Mol) tert.-Butylnitrit und 102,5 g (0,715 Mol) wasserfreiem Kupfer-II-chlorid in 360 ml wasserfreiem Acetonitril tropft man 886,5 g (9,135 Mol) 1,1-Dichlorethen, wobei man die Mischung auf Raumtemperatur hält. Dann wird bei einer Temperatur von unter 30°C eine Mischung aus 106,5 g (0,6 Mol) 2,6-Dichlor-4-methylanilin und 600 ml wasserfreiem Acetonitril zugetropft. Bei Raumtemperatur wird bis zum Ende der Gasentwicklung gerührt, die Mischung dann vorsichtig in 2,4 l 20 %ige HCl gegossen und mehrfach mit insgesamt 2,5 l Methyl-tert.-butylether (MTBE) extrahiert. Der vereinigten organischen Phasen werden mit 20 %iger HCl gewaschen, getrocknet und eingeengt. Das zurückbleibende Öl wird rektifiziert.
Ausbeute: 72 g (42 % der Theorie), Kp._{0.8} 130-135°C.

Analog bzw. gemäß den allgemeinen Angaben zur Herstellung wurden die in der folgenden Tabelle aufgeführten Verbindungen der Formel (XXVII) erhalten:

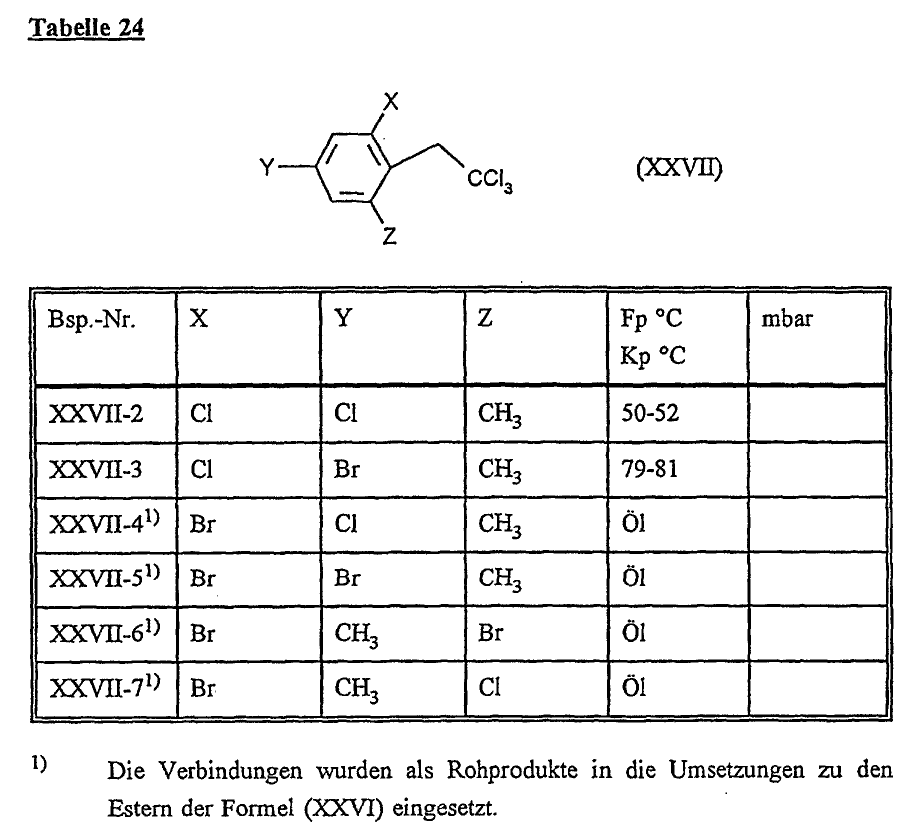

### Anwendungsbeispiele

### Beispiel 1

| Phaedon-Larven-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

In diesem Test bewirkten z.B. die Verbindungen gemäß den Herstellungsbeispielen (I-3-a-1), (I-3-a-2), (I-3-a-3), (I-5-a-3), (I-5-a-4), (I-2-b-5), (I-2-a-6), (I-1-a-2), (I-1-b-7), (I-1-a-8), (I-1-a-9), (I-1-a-10) und (I-1-a-11) bei einer beispielhaften Wirkstoffkonzentration von 0,1 % eine Abtötung von 100 % nach 7 Tagen und die Verbindungen gemäß den Herstellungsbeispielen (I-3-b-1) und (I-3-c-1) eine Abtötung von mindestens 80 %.

### Beispiel 2

| Plutella-Test | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet; daß keine Raupen abgetötet wurden.

In diesem Test bewirkten z.B. die Verbindungen gemäß den Herstellungsbeispielen (I-3-a-3), (I-3-b-1), (I-3-a-1), (I-5-a-3), (I-5-a-4), (I-1-a-3), (I-1-a-4), (I-1-b-2), (I-1-c-2), (I-1-a-1) und (I-1-a-7) bei einer beispielhaften Wirkstoffkonzentration von 0,1 % eine Abtötung von 100 % nach 7 Tagen.

### Beispiel 3

| Nephotettix-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet; daß keine Zikaden abgetötet wurden.

In diesem Test bewirkten z.B. die Verbindungen gemäß den Herstellungsbeispielen (I-3-a-2), (I-5-a-3), (I-5-a-4), (I-5-a-8), (I-3-b-1), (I-5-a-4), (I-1-a-2), (I-1-a-3), (I-1-a-4), (I-1-c-3), (I-1-a-1), (I-1-b-10), (I-1-c-4), (I-1-b-13), (I-1-a-7), (I-1-a-8), (I-1-a-9) und (I-1-a-11) bei einer beispielhaften Wirkstoffkonzentration von 0,1 % eine Abtötung von 100 % nach 6 Tagen.

### Beispiel 4

| Spodoptera-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkte z.B. die Verbindung gemäß Herstellungsbeispiel (I-1-b-5) bei einer beispielhaften Wirkstoffkonzentration von 0,1 % eine Abtötung von 100 % nach 7 Tagen.

### Beispiel 5

| Myzus-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der grünen Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

In diesem Test bewirkten z.B. die Verbindungen gemäß den Herstellungsbeispielen (I-2-a-5), (I-2-b-5), (I-2-a-6), (I-1-b-7) und (I-1-a-1) bei einer beispielhaften Wirkstoffkonzentration von 0,1 % eine Abtötung von 100 % nach 6 Tagen.

### Beispiel 6

| Panonychus-Test | |
|---|---|
| Lösungsmittel | 3 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen

Ca. 30 cm hohe Pflaumenbäumchen (Prunus domestica), die stark von allen Stadien der Obstbaumspinnmilbe (Panonychus ulmi) befallen sind, werden mit einer Wirkstoffzubereitung der gewünschten Konzentration gespritzt.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

In diesem Test bewirkten z.B. die Verbindungen gemäß den Herstellungsbeispielen (I-2-a-1), (I-2-b-1), (I-2-a-2), (I-2-b-2), (I-2-b-4) und (I-2-b-5) bei einer beispielhaften Wirkstoffkonzentration von 0,004 % eine Abtötung von mindestens 95 % nach 7 Tagen.

### Beispiel 7

| Tetranychus-Test (OP-resistent/Spritzbehandlung) | | |
|---|---|---|
| Lösungsmittel | 3 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Bohnenpflanzen (Phaseolus vulgaris), die stark von allen Entwicklungsstadien der gemeinen Spinnmilbe oder Bohnenspinnmilbe (Tetranychus urticae) befallen sind, werden mit einer Wirkstoffzubereitung der gewünschten Konzentration gespritzt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

In diesem Test hatten z.B. die Verbindungen gemäß den Herstellungsbeispielen (I-2-b-1), (I-2-b-4) und (I-2-b-5) bei einer beispielhaften Wirkstoffkonzentration von 0,004 % eine Wirkung von 100 % nach 7 Tagen.

### Beispiel 8

| Tetranychus-Test (OP-resistent/Tauchbehandlung) | | |
|---|---|---|
| Lösungsmittel | 3 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Bohnenpflanzen (Phaseolus vulgaris), die stark von allen Entwicklungsstadien der gemeinen Spinnmilbe oder Bohnenspinnmilbe (Tetranychus urticae) befallen sind, werden in eine Wirkstoffzubereitung der gewünschten Konzentration getaucht.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

In diesem Test hatten z.B. die Verbindungen gemäß den Herstellungsbeispielen (I-2-b-2), (I-2-b-6), (I-1-b-7), (I-1-b-10), (I-1-c-4), (I-1-a-7), (I-1-a-11), (I-3-a-2), (I-3-b-1), (I-3-c-1), (I-5-a-4), (I-5-a-3) und (I-5-a-8) bei einer beispielhaften Wirkstoffkonzentration von 0,1 % eine Wirkung von 100 % nach 13 Tagen.

### Beispiel 9

| Pre-emergence-Test | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden im normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
- 0 % =: keine Wirkung (wie unbehandelte Kontrolle)
- 100 % =: totale Vernichtung

### Beispiel 9a

| Post-emergence-Test | | |
|---|---|---|
| Lösungsmittel | 5 Gewichtsteile | Aceton |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben, so daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
- 0 %: = keine Wirkung (wie unbehandelte Kontrolle)
- 100 %: = totale Vernichtung

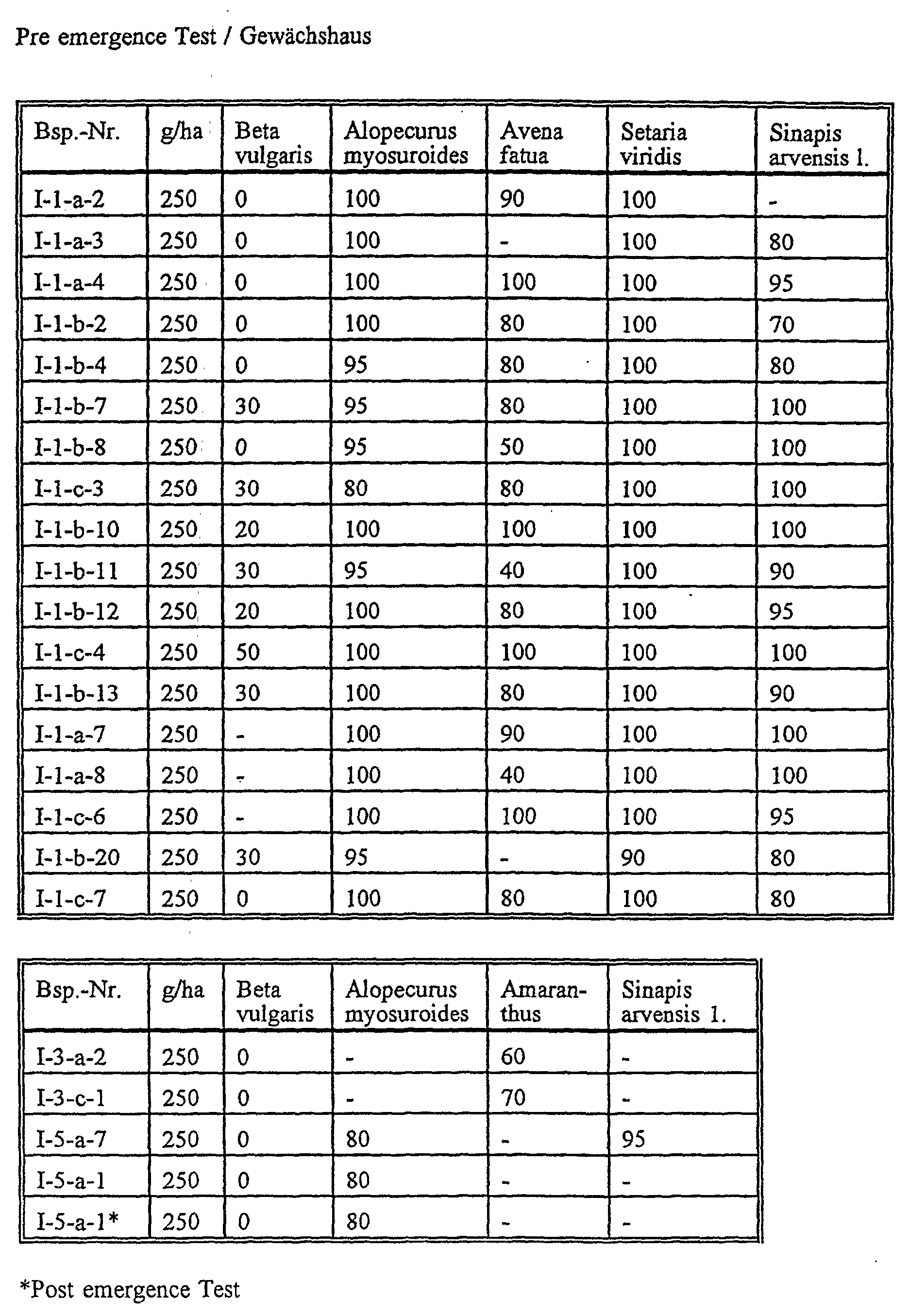

### Beispiel 10

| Test mit Boophilus microplus resistent / SP resistenter Parkhurst-Stamm | |
|---|---|
| Testtiere | Adulte gesogene Weibchen |
| Lösungsmittel | Dimethylsulfoxid |

20 mg Wirkstoff werden in 1 ml Dimethylsulfoxid gelöst, geringere Konzentrationen werden durch Verdünnen mit dem gleichen Lösungsmittel hergestellt.

Der Test wird in 5-fach Bestimmung durchgeführt. 1 µl der Lösungen wird in das Abdomen injiziert, die Tiere in Schalen überführt und in einem klimatisierten Raum aufbewahrt. Die Wirkung wird über die Hemmung der Eiablage bestimmt. Dabei bedeutet 100 %, daß keine Zecke gelegt hat.

In diesem Test zeigten z.B. die Verbindungen gemäß den Herstellungsbeispielen (I-2-b-5), (I-2-a-6), (I-1-b-3) und (I-1-a-5) bei einer beispielhaften Wirkstoffkonzentration von 20 µg/Tier eine Wirkung von 100 %.

### Beispiel 11

| Test mit Fliegenlarven / Entwicklungshemmende Wirkung | |
|---|---|
| Testtiere | Alle larvalen Stadien von Lucilia cuprina (OP-resistent) [Puppen und Adulte (ohne Kontakt zum Wirkstoff)] |
| Lösungsmittel: | 35 Gewichtsteile Ethylenglykolmonomethylether |
| Emulgator | 35 Gewichtsteile Nonylphenolpolyglykolether |

Zwecks Herstellung einer geeigneten Formulierung vermischt man 3 Gewichtsteile Wirkstoff mit 7 Teilen des oben angegebenen Lösungsmittel-Emulgator-Gemisches und verdünnt das so erhaltene Emulsionskonzentrat mit Wasser auf die jeweils gewünschte Konzentration.

30 bis 50 Larven je Konzentration werden auf in Glasröhrchen befindliches Pferdefleisch (1 cm³) gebracht, auf welches 500 µl der zu testenden Verdünnung pipettiert werden. Die Glasröhrchen werden in Kunststoffbecher gestellt, deren Boden mit Seesand bedeckt ist, und im klimatisierten Raum (26°C ± 1,5°C, 70 % rel. Feuchte ± 10 %) aufbewahrt. Die Wirkungskontrolle erfolgt nach 24 Stunden und 48 Stunden (larvizide Wirkung). Nach dem Auswandern der Larven (ca. 72 h) werden die Glasröhrchen entfernt und gelochte Kunststoffdeckel auf die Becher gesetzt. Nach 1½-facher Entwicklungsdauer (Schlupf der Kontrollfliegen) werden die geschlüpften Fliegen und die Puppen/Puppenhüllen ausgezählt.

Als Kriterium für die Wirkung gilt der Eintritt des Todes bei den behandelten Larven nach 48 h (larvizider Effekt), bzw. die Hemmung des Adultschlupfes aus den Puppen bzw. die Hemmung der Puppenbildung. Als Kriterium für die in-vitro-Wirkung einer Substanz gilt die Hemmung der Fliegenentwicklung, bzw. ein Entwicklungsstillstand vor dem Adulten-Stadium. Dabei bedeutet 100 % larvizide Wirkung, daß nach 48 Stunden alle Larven abgestoben sind. 100 % entwicklungsinhibitorische Wirkung bedeutet, daß keine adulte Fliegen geschlüpft sind.

In diesem Test zeigten z.B. die Verbindungen gemäß den Herstellungsbeispielen (I-2-b-5), (I-2-a-6), (I-1-b-3) und (I-1-a-5) bei einer beispielhaften Wirkstoffkonzentration von 1000 ppm eine Wirkung von 100 %.

### Beispiel 12

| Grenzkonzentrations-Test / Wurzelsystemische Wirkung | |
|---|---|
| Testinsekt | Aphis fabae |
| Lösungsmittel | 4 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Ackerbohnen (Vicia fabae). Der Wirkstoff kann so von den Pflanzenwurzeln aus dem Boden aufgenommen und in die Blätter transportiert werden.

Für den Nachweis des wurzelsystemischen Effektes werden nach 8 Tagen die Blätter mit den obengenannten Testtieren besetzt. Nach weiteren 6 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

In diesem Test hatten die Verbindungen gemäß den Herstellungsbeispielen (I-2-a-10), (I-1-c-28) und (I-1-b-15) bei einer beispielhaften Wirkstoffkonzentration von 20 ppm eine Wirkung von 100 %.

## Patentansprüche

1. Verbindungen der Formel (I) 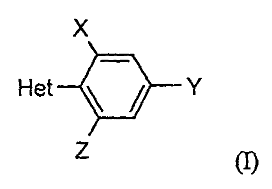 in welcher
X für Halogen.
Y für Halogen oder C₁-C₆-Alkyl.
Z für Halogen oder C₁-C₆-Alkyl.
wobei immer einer der Substituenten Y und Z für Halogen und der andere fiir Alkyl steht,
Het für eine der Gruppen 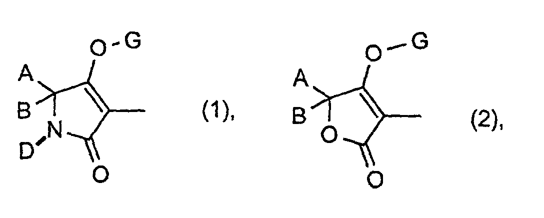 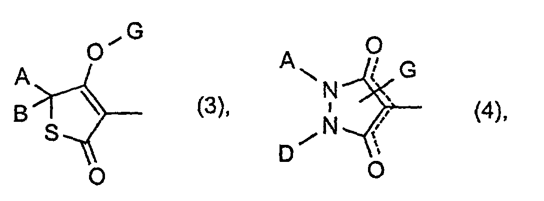 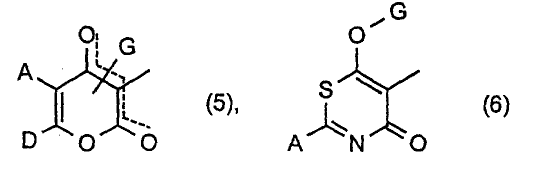
steht,
A für Wasserstoff, für jeweils gegebenenfalls durch Halogen substituiertes C₁-C₁₂-Alkyl, C₂-C₈-Alkenyl, C₁-C₁₀-Alkoxy-C₁-C₈-alkyl, Poly-C₁-C₈-alkoxy-C₁-C₈-alkyl oder C₁-C₁₀-Alkylthio-C₁-C₆-alkyl, für jeweils gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Naphthyl, Phenyl-C₁-C₆-alkyl, Naphthyl-C₁-C₆-alkyl oder Hetaryl mit 5 oder 6 Ringatomen und ein bis drei Heteroatomen aus der Reihe Sauerstoff, Schwefel und Stickstoff steht,
B für Wasserstoff, C₁-C₁₂-Alkyl oder C₁-C₈-Alkoxy-C₁-C₆-alkyl steht oder
A, B und das Kohlenstoffatom, an das sie gebunden sind, für C₃-C₁₀-Cycloalkyl oder C₅-C₁₀-Cycloalkenyl stehen, worin jeweils gegebenenfalls eine Methylengruppe durch Sauerstoff oder Schwefel ersetzt ist und welche gegebenenfalls durch C₁-C₈-Alkyl, C₃-C₁₀-Cycloalkyl, C₁-C₈-Halogenalkyl, C₁-C₈-Alkoxy, C₁-C₈-Alkylthio, Halogen oder Phenyl substituiert sind oder
A, B und das Kohlenstoffatom, an das sie gebunden sind, für C₅-C₆-Cycloalkyl stehen, welches durch eine gegebenenfalls ein oder zwei Sauerstoff- und/oder Schwefelatome enthaltende Alkylendiyl-, oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Ring bildet oder
A, B und das Kohlenstoffatom, an das sie gebunden sind, für C₃-C₈-Cycloalkyl oder C₅-C₈-Cycloalkenyl stehen, in welchen zwei Substituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₃-C₆-Alkandiyl, C₃-C₆-Alkendiyl oder C₄-C₆-Alkandiendiyl stehen, worin jeweils gegebenenfalls eine Methylengruppe durch Sauerstoff oder Schwefel ersetzt ist,
D für Wasserstoff, für jeweils gegebenenfalls durch Halogen substituiertes C₁-C₁₂-Alkyl, C₃-C₈-Alkenyl, C₃-C₈-Alkinyl, C₁-C₁₀-Alkoxy-C₂-C₈-alkyl, Poly-C₁-C₈-alkoxy-C₂-C₈-alkyl oder C₁-C₁₀-Alkylthio-C₂-C₈-alkyl, für gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Hetaryl mit 5 bis 6 Ringatomen und ein oder zwei Heteroatomen aus der Reihe Sauerstoff, Schwefel und Stickstoff, Phenyl-C₁-C₆-alkyl oder Hetaryl-C₁-C₆-alkyl mit 5 bis 6 Ringatomen und ein oder zwei Heteroatomen aus der Reihe Sauerstoff, Schwefel und Stickstoff steht oder
A und D gemeinsam für eine C₃-C₆-Alkandiyl-, C₃-C₆-Alkendiyl- oder C₄-C₆-Alkandiendiylgruppe stehen, in welchen jeweils gegebenenfalls eine Methylengruppe durch Sauerstoff oder Schwefel ersetzt ist und welche jeweils gegebenenfalls substituiert sind durch Halogen, Hydroxy, Mercapto oder jeweils gegebenenfalls durch Halogen substituiertes C₁-C₁₀-Alkyl, C₁-C₆-Alkoxy, C₁-C₆-Alkylthio, C₃-C₇-Cycloalkyl, Phenyl oder Benzyloxy oder durch eine weitere, einen ankondensierten Ring bildende C₃-C₆-Alkandiyl-, C₃-C₆-Alkendiyl- oder C₄-C₆-Alkandien-diylgruppe, in welchen gegebenenfalls jeweils eine Methylengruppe durch Sauerstoff oder Schwefel ersetzt ist und welche gegebenenfalls durch C₁-C₆-Alkyl substituiert sind oder in welchen gegebenenfalls zwei benachbarte Substituenten gemeinsam mit den Kohlenstoffatomen, an die sie gebunden sind, einen weiteren gesättigten oder ungesättigten Carbocyclus mit 5 oder 6 Ringatomen bilden oder
A und D gemeinsam für eine C₃-C₆-Alkandiyl- oder C₃-C₆-Alkendiylgruppe stehen, worin jeweils gegebenenfalls eine der folgenden Gruppen 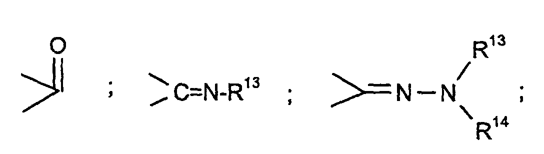 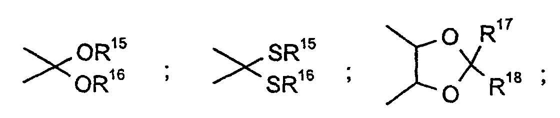 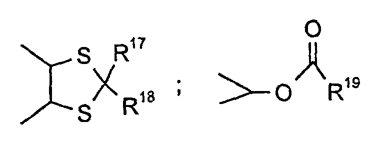 oder 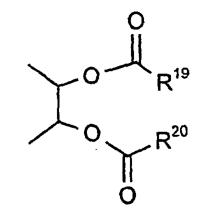 enthalten ist,
G im Fall, daß Het für einen der Reste (1), (2), (3), (5) oder (6) steht, für Wasserstoff (a) oder, im Fall, daß Het für einen der Reste (1), (2), (3), (4), (5) oder (6) steht, für eine der Gruppen 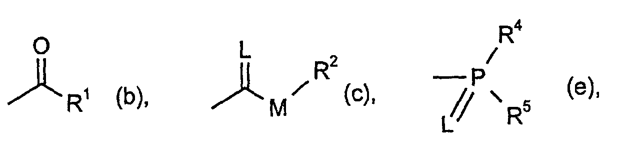 E (f) oder 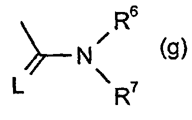 steht, oder im Fall, dass
Het für einen der Reste (2), (3), (4), (5) oder (6) steht, für die Gruppe ―SO₂―R³ (d) steht,
in welchen
Het für einen der Reste (2), (3), (4), (5) oder (6) steht, für die Gruppe ―SO₂―R³ (d) steht,
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈--alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff steht,
R² für jeweils gegebenenfalls durch Halogen substituiertes C₁-C₂₀-Alkyl, C₂-C₂₀-Alkenyl, C₁-C₈-Alkoxy-C₂-C₈-alkyl oder Poly-C₁-C₈-alkoxy-C-C8-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht,
R³ für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl,
R⁴ und R⁵ unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio oder C₂-C₈-Alkenylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen,
R⁶ und R⁷ unabhängig voneinander für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Alkylenrest stehen, in welchem gegebenenfalls eine Methylengruppe durch Sauerstoff oder Schwefel ersetzt ist,
R¹³ für Wasserstoff, für jeweils gegebenenfalls durch Halogen substituiertes C₁-C₈-Alkyl oder C₁-C₈-Alkoxy, für gegebenenfalls durch Halogen, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt ist, oder für jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenyl-C₁-C₄-alkyl oder Phenyl-C₁-C₄-alkoxy steht,
R¹⁴ für Wasserstoffoder C₁-C₈-Alkyl steht oder
R¹³ und R¹⁴ gemeinsam für C₄-C₆-Alkandiyl stehen,
R¹⁵ und R¹⁶ gleich oder verschieden sind und für C₁-C₆-Alkyl stehen oder
R¹⁵ und R¹⁶ gemeinsam für einen C₂-C₄-Alkandiylrest stehen, der gegebenenfalls durch C₁-C₆-Alkyl oder durch gegebenenfalls durch Halogen, C₁-C₄-Alkyl, C₁-C₄-Halogenalkyl, C₁-C₄-Alkoxy, C₁-C₄-Halogenalkoxy, Nitro oder Cyano substituiertes Phenyl substituiert ist,
R¹⁷ und R¹⁸ unabhängig voneinander für Wasserstoff, für gegebenenfalls durch Halogen substituiertes C₁-C₈-Alkyl oder für gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl stehen oder
R¹⁷ und R¹⁸ gemeinsam mit dem Kohlenstoffatom, an das sie gebunden sind, für gegebenenfalls durch C₁-C₄-Alkyl oder C₁-C₄-Alkoxy substituiertes C₅-C₇-Cycloalkyl stehen, in dem gegebenenfalls eine Methylengruppe durch Sauerstoff oder Schwefel ersetzt ist und
R¹⁹ und R²⁰ unabhängig voneinander für C₁-C₁₀-Alkyl, C₂-C₁₀-Alkenyl, C₁-C₁₀-Alkoxy, C₁-C₁₀-Alkylamino, C₃-C₁₀-Alkenylamino, Di-(C₁-C₁₀-alkyl)-amino oder Di-(C₃-C₁₀-alkenyl)amino stehen.

2. Verbindungen der Formel (I) gemäß Anspruch 1, in welcher
X für Fluor, Chlor oder Brom steht,
Y für Fluor, Chlor, Brom oder C₁-C₄-Alkyl steht,
Z für Fluor, Chlor, Brom oder C₁-C₄-Alkyl steht,
wobei immer einer der Reste Y und Z für Halogen und der andere für Alkyl steht,
Het für eine der Gruppen 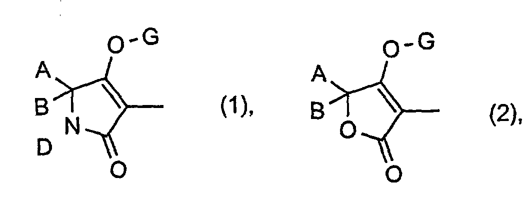 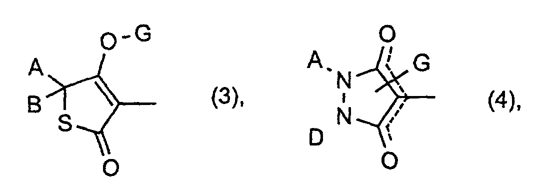 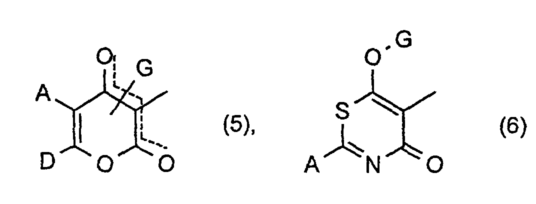
steht,
A für Wasserstoff, für jeweils gegebenenfalls durch Fluor oder Chlor substituiertes C₁-C₁₀-Alkyl, C₂-C₆-Alkenyl, C₁-C₈-Alkoxy-C₁-C₆-alkyl, Poly-C₁-C₆-alkoxy-C₁-C₆-alkyl oder C₁-C₈-Alkylthio-C₁-C₆-alkyl oder für gegebenenfalls durch Fluor, Chlor, C₁-C₄-Alkyl oder C₁-C₄-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, C₁-C₄-Alkyl, C₁-C₄-Halogenalkyl, C₁-C₄-Alkoxy, C₁-C₄-Halogenalkoxy, Cyano oder Nitro substituiertes Phenyl, Furanyl, Pyridyl, Imidazolyl, Triazolyl, Pyrazolyl, Indolyl, Thiazolyl, Thienyl oder Phenyl-C₁-C₄-alkyl steht,
B für Wasserstoff, C₁-C₁₀-Alkyl oder C₁-C₆-Alkoxy-C₁-C₄-alkyl steht oder
A, B und das Kohlenstoffatom an das sie gebunden sind, für C₃-C₈-Cycloalkyl oder C₅-C₈-Cycloalkenyl stehen, worin jeweils gegebenenfalls eine Methylengruppe durch Sauerstoff oder Schwefel ersetzt ist und welche gegebenenfalls durch C₁-C₆-Alkyl, C₃-C₈-Cycloalkyl, C₁-C₃-Halogenalkyl, C₁-C₆-Alkoxy, C₁-C₆-Alkylthio, Fluor, Chlor oder Phenyl substituiert sind oder
A, B und das Kohlenstoffatom, an das sie gebunden sind, für C₅-C₆-Cycloalkyl stehen, welches durch eine gegebenenfalls ein oder zwei Sauerstoff- oder Schwefelatome enthaltende Alkylendiyl- oder durch eine Alkylendioxyl- oder durch eine Alkylendithiol-Gruppe substituiert ist, die mit dem Kohlenstoffatom, an das sie gebunden ist, einen weiteren fünf- bis siebengliedrigen Ring bildet oder
A, B und das Kohlenstoffatom, an das sie gebunden sind, für C₃-C₆-Cycloalkyl oder C₅-C₆-Cycloalkenyl stehen, in welchen zwei Substituenten gemeinsam mit den Kohlenstoffatomen, an die sie gebunden sind, für jeweils gegebenenfalls durch C₁-C₅-Alkyl, C₁-C₅-Alkoxy, Fluor, Chlor oder Brom substituiertes C₃-C₅-Alkandiyl, C₃-C₅-Alkendiyl oder Butadiendiyl stehen, worin jeweils gegebenenfalls eine Methylengruppe durch Sauerstoff oder Schwefel ersetzt ist,
D für Wasserstoff, für jeweils gegebenenfalls durch Fluor oder Chlor substituiertes C₁-C₁₀-Alkyl, C₃-C₆-Alkenyl, C₃-C₆-Alkinyl, C₁-C₈-Alkoxy-C₂-C₆-alkyl, Poly-C₁-C₆-alkoxy-C₂-C₆-alkyl oder C₁-C₈-Alkylthio-C₂-C₆-alkyl, für gegebenenfalls durch Fluor, Chlor, C₁-C₄-Alkyl, C₁-C₄-Alkoxy oder C₁-C₂-Halogenalkyl substituiertes C₃-C₇-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, C₁-C₄-Alkyl, C₁-C₄-Halogenalkyl, C₁-C₄-Alkoxy, C₁-C₄-Halogenalkoxy, Cyano oder Nitro substituiertes Phenyl, Furanyl, Imidazolyl, Pyridyl, Thiazolyl, Pyrazolyl, Pyrimidyl, Pyrrolyl, Thienyl, Triazolyl oder Phenyl-C₁-C₄-alkyl steht oder
A und D gemeinsam für eine C₃-C₅-Alkandiyl- oder C₃-C₅-Alkendiylgruppe stehen, worin jeweils gegebenenfalls eine Methylengruppe durch Sauerstoff oder Schwefel ersetzt ist und welche gegebenenfalls substituiert sind durch Fluor, Chlor, Hydroxy, Mercapto oder durch jeweils gegebenenfalls durch Fluor oder Chlor substituiertes C₁-C₆-Alkyl, C₁-C₄-Alkoxy, C₁-C₄-Alkylthio, C₃-C₆-Cycloalkyl, Phenyl oder Benzyloxy oder
worin jeweils gegebenenfalls eine der folgenden Gruppen: 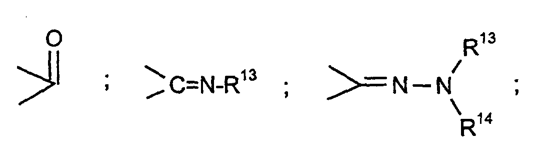 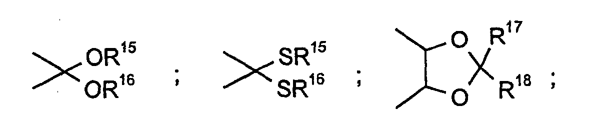 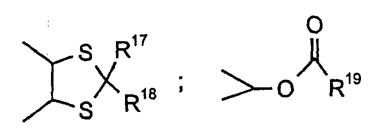 oder 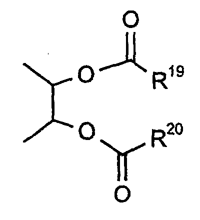 enthalten ist;
oder A und D (im Fall der Verbindungen der Formel (I-1)) gemeinsam mit den Atomen, an die sie gebunden sind, für eine der Gruppen AD-1 bis AD-27 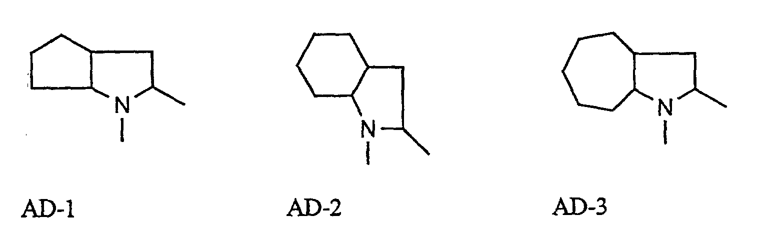 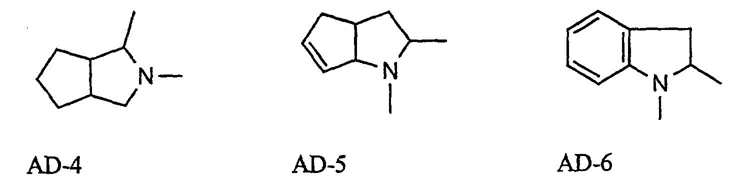 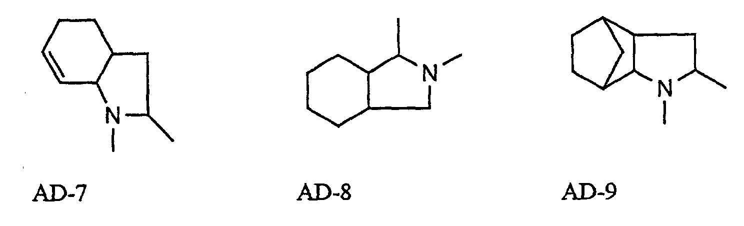 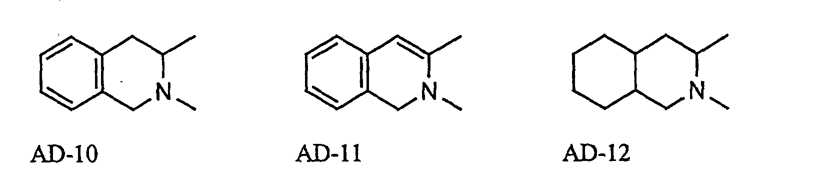 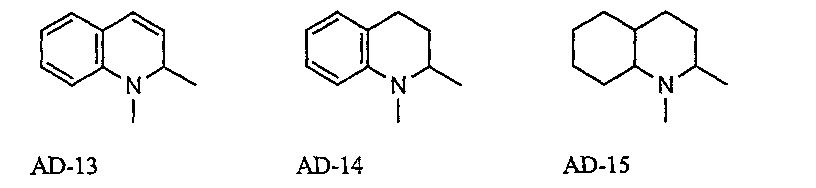 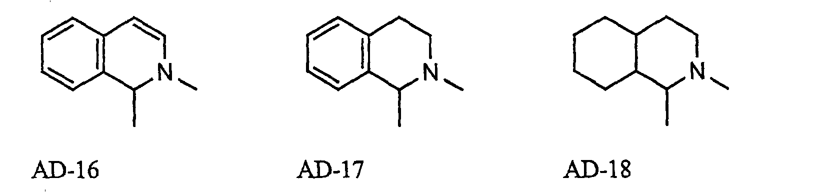 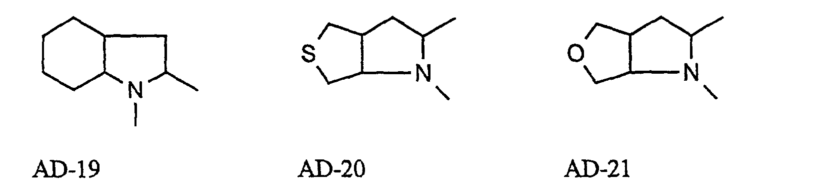 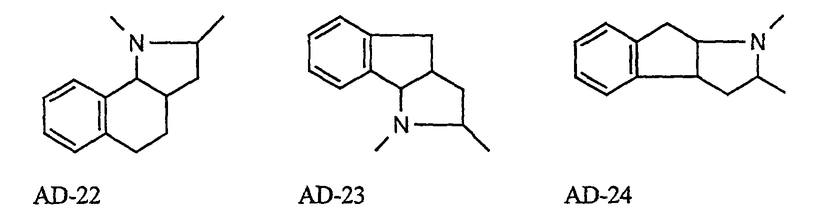 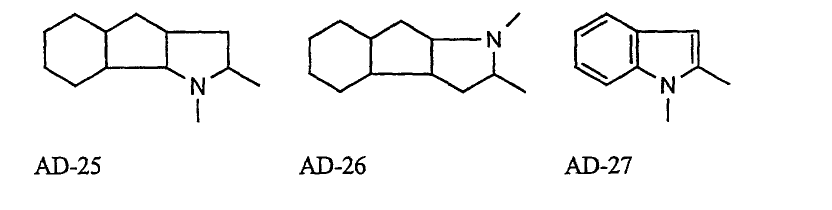 stehen,
G im Fall, daß Het für einen der Reste (1), (2), (3), (5) oder (6) steht, für Wasserstoff (a) oder im Fall, daß Het für einen der Reste (1), (2), (3), (4), (5) oder (6) steht, für eine der Gruppen 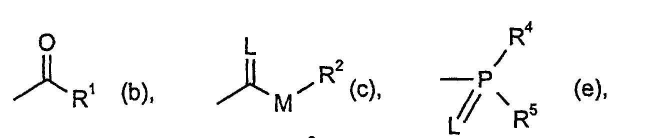 E (f) oder 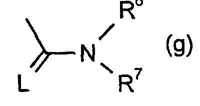 steht, oder im Fall, dass
Het für einen der Reste (2), (3), (4), (5) oder (6) steht, für die Gruppe ―SO₂―R³ (d) steht,
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Fluor oder Chlor substituiertes C₁-C₁₆-Alkyl, C₂-C₁₆-Alkenyl, C₁-C₆-Alkoxy-C₁-C₆-alkyl, C₁-C₆-Alkylthio-C₁-C₆-alkyl oder Poly-C₁-C₆-alkoxy-C₁-C₆-alkyl oder für gegebenenfalls durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls durch Fluor, Chlor, Brom oder C₁-C₄-Alkyl substituiertes Phenoxy-C₁-C₅-alkyl oder
für jeweils gegebenenfalls durch Fluor, Chlor, Brom, Amino oder C₁-C₄-Alkyl substituiertes Pyridyloxy-C₁-C₅-alkyl, Pyrimidyloxy-C₁-C ₅-alkyl oder Thiazolyloxy-C₁-C₅-alkyl steht,
R² für jeweils gegebenenfalls durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls durch Fluor, Chlor, C₁-C₄-Alkyl oder C₁-C₄-Alkoxy substituiertes C₃-C₇-Cycloalkyl oder
für jeweils gegebenenfalls durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl steht,
R³ für gegebenenfalls durch Fluor oder Chlor substituiertes C₁-C₆-Alkyl oder für jeweils gegebenenfalls durch Fluor, Chlor, Brom, C₁-C₅-Alkyl, C₁-C₅-Alkoxy, C₁-C₃-Halogenalkyl, C₁-C₃-Halogenalkoxy, Cyano oder Nitro substituiertes Phenyl oder Benzyl steht,
R⁴ und R⁵ unabhängig voneinander für jeweils gegebenenfalls durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio oder C₃-C₄-Alkenylthio oder für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio stehen,
R⁶ und R⁷ unabhängig voneinander fiir Wasserstoff, für jeweils gegebenenfalls durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest stehen, in welchem gegebenenfalls eine Methylengruppe durch Sauerstoff oder Schwefel ersetzt ist,
R¹³ für Wasserstoff, für jeweils gegebenenfalls durch Fluor oder Chlor substituiertes C₁-C₆-Alkyl oder C₁-C₆-Alkoxy, für gegebenenfalls durch Fluor, C₁-C₂-Alkyl oder C₁-C₂-Alkoxy substituiertes C₃-C₇-Cycloalkyl, in welchem gegebenenfalls eine Methylengruppe durch Sauerstoff oder Schwefel ersetzt ist oder für jeweils gegebenenfalls durch Fluor, Chlor, Brom, C₁-C₅-Alkyl, C₁-C₅-Alkoxy, C₁-C₂-Halogenalkyl, C₁-C₂-Halogenalkoxy, Nitro oder Cyano substituiertes Phenyl, Phenyl-C₁-C₃-alkyl oder Phenyl-C₁-C₂-alkyloxy steht,
R¹⁴ für Wasserstoff oder C₁-C₆-Alkyl steht oder
R¹³ und R¹⁴ gemeinsam für C₄-C₆-Alkandiyl stehen,
R¹⁵ und R¹⁶ gleich oder verschieden sind und für C₁-C₄-Alkyl stehen oder
R¹⁵ und R¹⁶ zusammen für einen C₂-C₃-Alkandiylrest stehen, der gegebenenfalls durch C₁-C₄-Alkyl oder durch gegebenenfalls durch Fluor, Chlor, Brom, C₁-C₂-Alkyl, C₁-C₂-Halogenalkyl, C₁-C₂-Alkoxy, C₁-C₂-Halogenalkoxy, Nitro oder Cyano substituiertes Phenyl substituiert ist,
R¹⁷ und R¹⁸ unabhängig voneinander für Wasserstoff, für gegebenenfalls durch Fluor oder Chlor substituiertes C₁-C₈-Alkyl oder für gegebenenfalls durch Fluor, Chlor, C₁-C₄-Alkyl, C₁-C₄-Alkoxy, C₁-C₂-Halogenalkyl, C₁-C₂-Halogenalkoxy, Nitro oder Cyano substituiertes Phenyl stehen oder
R¹⁷ und R¹⁸ gemeinsam mit dem Kohlenstoffatom, an das sie gebunden sind, für gegebenenfalls durch C₁-C₃-Alkyl oder C₁-C₃-Alkoxy substituiertes C₅-C₆-Cycloalkyl stehen, in dem gegebenenfalls eine Methylengruppe durch Sauerstoff oder Schwefel ersetzt ist und
R¹⁹ und R²⁰ unabhängig voneinander für C₁-C₆-Alkyl, C₂-C₆-Alkenyl, C₁-C₆-Alkoxy, C₁-C₆-Alkylamino, C₃-C₆-Alkenylamino, Di-(C₁-C₆-alkyl)-amino oder Di-(C₃-C₆-alkenyl)amino stehen.

3. Verbindungen der Formel (I) gemäß Anspruch 1, in welcher
X für Fluor, Chlor oder Brom steht,
Y für Fluor, Chlor, Brom, Methyl, Ethyl, n-Propyl oder iso-Propyl steht,
Z für Fluor, Chlor, Brom, Methyl, Ethyl, n-Propyl oder iso-Propyl steht,
wobei immer einer der Reste Y und Z für Halogen und der andere für Alkyl steht,
Het für eine der Gruppen 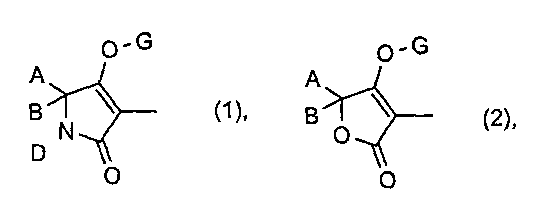 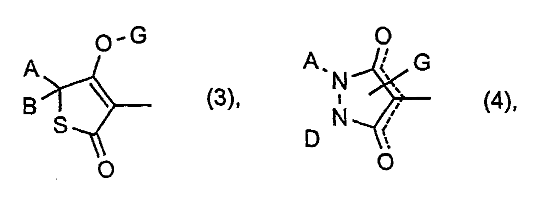 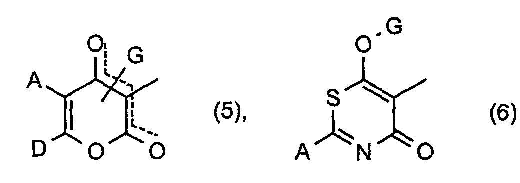
steht,
A für Wasserstoff, für jeweils gegebenenfalls durch Fluor oder Chlor substituiertes C₁-C₈-Alkyl, C₂-C₄-Alkenyl, C₁-C₆-Alkoxy-C₁-C₄-alkyl, Poly-C₁-C₄-alkoxy-C₁-C₄-alkyl oder C₁-C₆-Alkylthio-C₁-C₄-alkyl, oder für gegebenenfalls durch Fluor, Chlor, Methyl oder Methoxy substituiertes C₃-C₆-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Pyridyl oder Benzyl steht,
B für Wasserstoff, C₁-C₈-Alkyl oder C₁-C₄-Alkoxy-C₁-C₂-alkyl steht oder
A, B und das Kohlenstoffatom an das sie gebunden sind, für C₃-C₈-Cycloalkyl oder C₅-C₈-Cycloalkenyl stehen, worin jeweils gegebenenfalls eine Methylengruppe durch Sauerstoff oder Schwefel ersetzt ist und welche gegebenenfalls durch Methyl, Ethyl, n-Propyl, iso-Ppropyl, Butyl, iso-Butyl, sec.-Butyl, tert.-Butyl, Cyclohexyl, Trifluormethyl, Methoxy, Ethoxy, n-Propoxy, iso-Propoxy, Butoxy, iso-Butoxy, sek.-Butoxy, tert.-Butoxy, Methylthio, Ethylthio, Fluor, Chlor oder Phenyl substituiert sind oder
A, B und das Kohlenstoffatom, an das sie gebunden sind, für C₅-C₆-Cycloalkyl stehen, welches durch eine gegebenenfalls ein Sauerstoff- oder Schwefelatom enthaltende Alkylendiyl- oder durch eine Alkylendioxyl-Gruppe substituiert ist, die mit dem Kohlenstoffatom, an das sie gebunden ist, einen weiteren fünf- oder sechsgliedrigen Ring bildet oder
A, B und das Kohlenstoffatom, an das sie gebunden sind, für C₃-C₆-Cycloalkyl oder C₅-C₆-Cycloalkenyl stehen, in welchen zwei Substituenten gemeinsam mit den Kohlenstoffatomen, an die sie gebunden sind, für C₃-C₄-Alkandiyl, C₃-C₄-Alkendiyl oder Butadiendiyl stehen, worin jeweils gegebenenfalls eine Methylengruppe durch Sauerstoff oder Schwefel ersetzt ist,
D für Wasserstoff, für jeweils gegebenenfalls durch Fluor oder Chlor substituiertes C₁-C₈-Alkyl, C₃-C₄-Alkenyl, C₃-C₄-Aüdnyl, C₁-C₆-Alkoxy-C₂-C₄-alkyl, Poly-C₁-C₄-alkoxy-C₂-C₄-alkyl, C₁-C₄-Alkylthio-C₂-C₄-alkyl oder C₃-C₆-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Furanyl, Pyridyl, Thienyl oder Benzyl steht,
oder
A und D gemeinsam für eine C₃-C₅-Alkandiyl- oder C₃-C₅-Alkendiylgruppe stehen, worin jeweils gegebenenfalls eine Methylengruppe durch Sauerstoff oder Schwefel ersetzt ist und welche gegebenenfalls durch Fluor, Chlor, Hydroxy, Mercapto oder durch jeweils gegebenenfalls durch Fluor oder Chlor substituiertes C₁-C₆-Alkyl, C₁-C₄-Alkoxy, C₁-C ₄-Alkylthio, C₃-C₆-Cycloalkyl, Phenyl oder Benzyloxy substituiert sind oder
worin jeweils gegebenenfalls eine der folgenden Gruppen 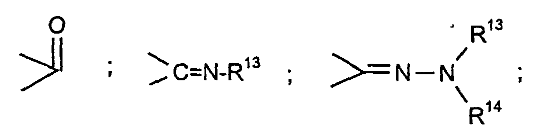 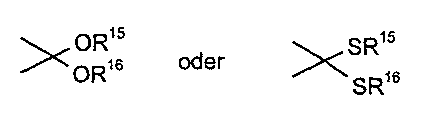 enthalten ist,
oder A und D im Fall der Verbindungen der Formel (I-1) gemeinsam mit den Atomen, an die sie gebunden sind, für eine der folgenden Gruppen: 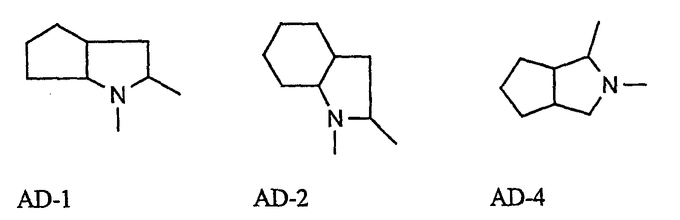 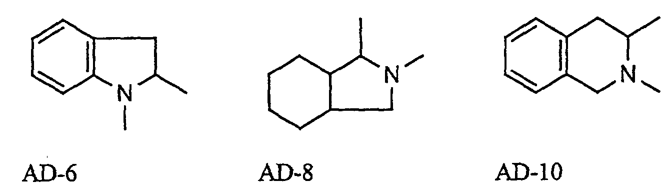 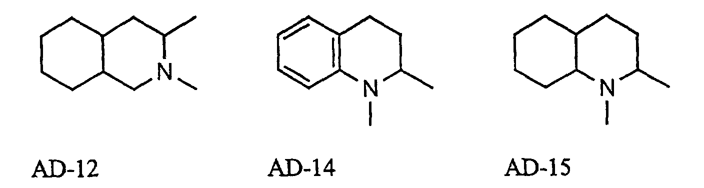 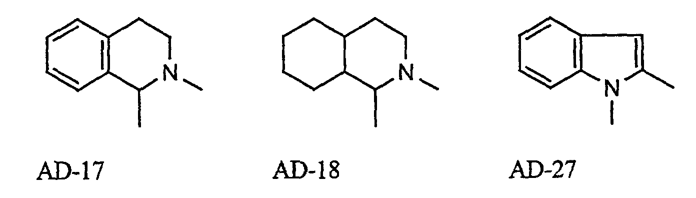 stehen
G im Fall, daß Het für einen der Reste (1), (2), (3), (5) oder (6) steht, für Wasserstoff (a) oder, im Fall, daß Het für einen der Reste (1), (2), (3), (4), (5) oder (6) steht, für eine der Gruppen 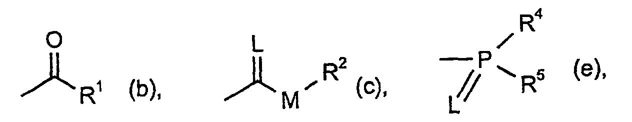 E (f) oder 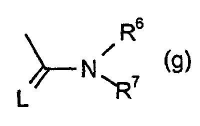 steht, oder, im Fall, dass
Het für einen der Reste (2), (3), (4), (5) oder (6) steht, für die Gruppe ―SO₂―R³ (d) steht,
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, Poly-C₁-C₄-alkoxy-C₁-C₄-alkyl oder für gegebenenfalls durch Fluor, Chlor, Methyl, Ethyl, n-Propyl, i-Propyl, n-Butyl, i-Butyl, tert.-Butyl, Methoxy, Ethoxy, n-Propoxy oder iso-Propoxy substituiertes C₃-C₆-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, Methyl, Ethyl, n-Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für gegebenenfalls durch Fluor, Chlor, Brom, Methyl, Ethyl, n-Propyl, i-Propyl, Methoxy, Ethoxy, Trifluormethyl oder Trifluormethoxy substituiertes Benzyl,
für jeweils gegebenenfalls durch Fluor, Chlor, Brom, Methyl oder Ethyl substituiertes Furanyl, Thienyl oder Pyridyl,
für gegebenenfalls durch Fluor, Chlor, Methyl oder Ethyl substituiertes Phenoxy-C₁-C₄-alkyl oder
für jeweils gegebenenfalls durch Fluor, Chlor, Amino, Methyl oder Ethyl substituiertes Pyridyloxy-C₁-C₄-alkyl, Pyrimidyloxy-C₁-C₄-alkyl oder Thiazolyloxy-C₁-C₄-alkyl steht,
R² für jeweils gegebenenfalls durch Fluor oder Chlor substituiertes C₁-C₁₄-Alkyl, C₂-C₁₄-Alkenyl, C₁-C₄-Alkoxy-C₂-C₆-alkyl oder Poly-C₁-C₄-alkoxy-C₂-C₆-alkyl,
für gegebenenfalls durch Fluor, Chlor, Methyl, Ethyl, n-Propyl, iso-Propyl oder Methoxy substituiertes C₃-C₆-Cycloalkyl,
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl steht,
R³ für gegebenenfalls durch Fluor oder Chlor substituiertes Methyl, Ethyl, Propyl, Isopropyl oder jeweils gegebenenfalls durch Fluor, Chlor, Brom, Methyl, Ethyl, Propyl, iso-Propyl, tert.-Butyl, Methoxy, Ethoxy, Isopropoxy, tert.-Butoxy, Trifluormethyl, Trifluormethoxy, Cyano oder Nitro substituiertes Phenyl oder Benzyl steht,
R⁴ und R⁵ unabhängig voneinander für jeweils gegebenenfalls durch Fluor oder Chlor substituiertes C₁-C₄-Alkyl, C₁-C₄-Alkoxy, C₁-C₄-Alkylamino, Di-(C₁-C₄-alkyl)amino oder C₁-C₄-Alkylthio oder für jeweils gegebenenfalls durch Fluor, Chlor, Brom, Nitro, Cyano, Methyl, Methoxy, Trifluormethyl oder Trifluormethoxy substituiertes Phenyl, Phenoxy oder Phenylthio stehen,
R⁶ und R⁷ unabhängig voneinander für Wasserstoff, für jeweils gegebenenfalls durch Fluor oder Chlor substituiertes C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für jeweils gegebenenfalls durch Fluor, Chlor, Brom, Methyl, Methoxy oder Trifluormethyl substituiertes Phenyl oder Benzyl, oder zusammen für einen gegebenenfalls durch Methyl oder Ethyl substituierten C₅-C₆-Alkylenrest stehen, in welchem gegebenenfalls eine Methylengruppe durch Sauerstoff oder Schwefel ersetzt ist,
R¹³ für Wasserstoff, für jeweils gegebenenfalls durch Fluor oder Chlor substituiertes C₁-C₄-Alkyl oder C₁-C₄-Alkoxy, für C₃-C₆-Cycloalkyl oder für jeweils gegebenenfalls durch Fluor, Chlor, Brom, Methyl, Ethyl, iso-Propyl, tert-Butyl, Methoxy, Ethoxy, iso-Propoxy, tert.-Butoxy, Trifluormethyl, Trifluormethoxy, Nitro oder Cyano substituiertes Phenyl, Phenyl-C₁-C₂-alkyl oder Benzyloxy steht,
R¹⁴ für Wasserstoffoder C₁-C₄-Alkyl steht oder
R¹³ und R¹⁴ gemeinsam für C₄-C₆-Alkandiyl stehen,
R¹⁵ und R¹⁶ gleich oder verschieden sind für Methyl oder Ethyl stehen oder
R¹⁵ und R¹⁶ zusammen für einen C₂-C₃-Alkandiylrest stehen, der gegebenenfalls durch Methyl, Ethyl, n-Propyl, iso-Propyl, n-Butyl, iso-Butyl, sek.-Butyl, tert.-Butyl oder durch gegebenenfalls durch Fluor, Chlor, Methoxy, Trifluormethyl, Trifluormethoxy, Nitro oder Cyano substi-tuiertes Phenyl substituiert ist.

4. Verfahren zur Herstellung von Verbindungen der Formel (I) gemäß Anspruch 1, **dadurch gekennzeichnet, daß** man
(A) Verbindungen der Formel (I-1-a) 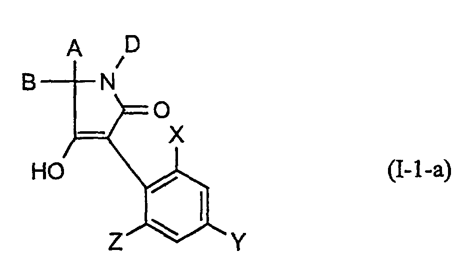 in welcher
A, B, D, X, Y und Z die oben angegebenen Bedeutungen haben,
erhält, wenn man
Verbindungen der Formel (II) 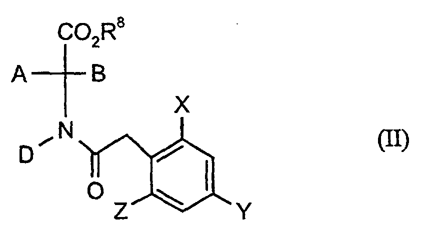 in welcher
A, B, D, X, Y und Z die oben angegebenen Bedeutungen haben,
und
R⁸ für Alkyl steht,
in Gegenwart eines Verdünnungsmittels und in Gegenwart einer Base
intramolekular kondensiert,
(B) Verbindungen der Formel (I-2-a) 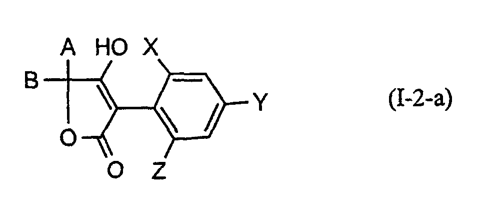 in welcher
A, B, X, Y und Z die oben angegebenen Bedeutungen haben,
erhält, wenn man
Verbindungen der Formel (III) 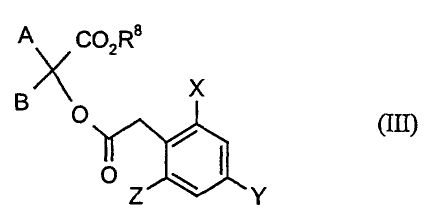 in welcher
A, B, X, Y, Z und R⁸ die oben angegebenen Bedeutungen haben,
in Gegenwart eines Verdünnungsmittels und in Gegenwart einer Base intramolekular kondensiert,
(C) Verbindungen der Formel (I-3-a) 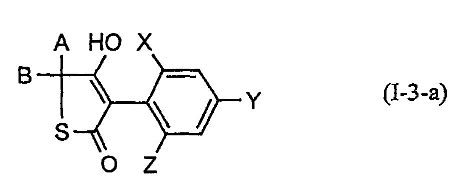 in welcher
A, B, X, Y und Z die oben angegebenen Bedeutungen haben,
erhält, wenn man
Verbindungen der Formel (IV) 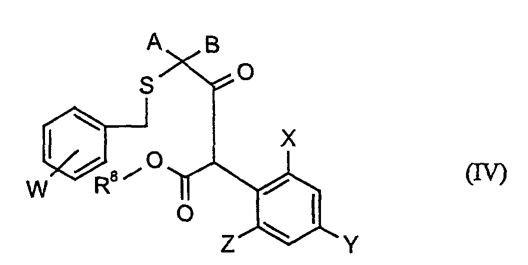 in welcher
A, B, X, Y, Z und R⁸ die oben angegebenen Bedeutungen haben und
W für Wasserstoff, Halogen, Alkyl oder Alkoxy steht,
gegebenenfalls in Gegenwart eines Verdünnungsmittels und in Gegenwart einer Säure intramolekular cyclisiert,
(E) die Verbindungen der Formel (I-5-a) 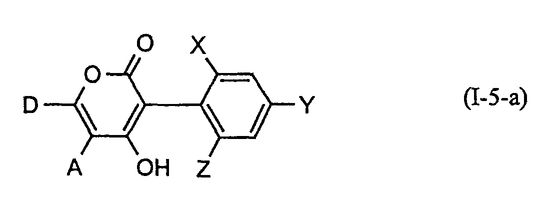 in welcher
A, D, X, Y und Z die oben angegebenen Bedeutungen haben,
erhält, wenn man
Verbindungen der Formel (VIII) 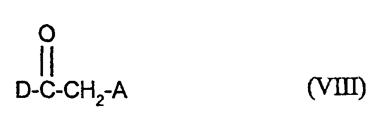 in welcher
A und D die oben angegebenen Bedeutungen haben,
mit Verbindungen der Formel (V) 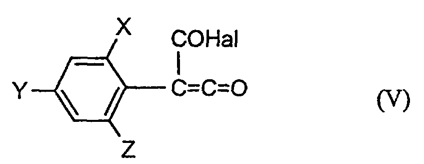 in welcher
X, Y und Z die oben angegebenen Bedeutungen haben und
Hal fiir Halogen steht,
gegebenenfalls in Gegenwart eines Verdünnungsmittels und gegebenenfalls in Gegenwart eines Säureakzeptors umsetzt,
(F) die Verbindungen der Formel (I-6-a) 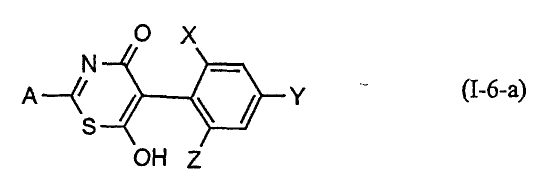 in welcher
A, X, Y und Z die oben angegebenen Bedeutungen haben,
erhält, wenn man Verbindungen der Formel (IX) 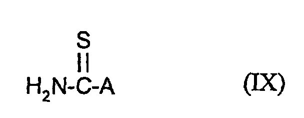 in welcher
A die oben angegebene Bedeutung hat,
mit Verbindungen der Formel (V) 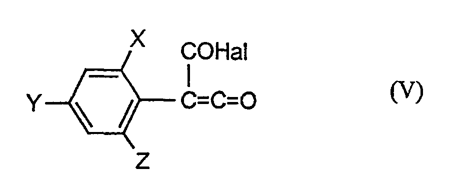 in welcher
Hal, X, Y und Z die oben angegebenen Bedeutungen haben,
gegebenenfalls in Gegenwart eines Verdünnungsmittels und gegebenenfalls in Gegenwart eines Säureakzeptors umsetzt,
und gegebenenfalls anschließend die so erhaltenen Verbindungen der Formeln (I-1-a), (I-2-a) bis (I-3-a), (I-5-a) und (I-6-a), oder Verbindungen der Formel (I-4-a) 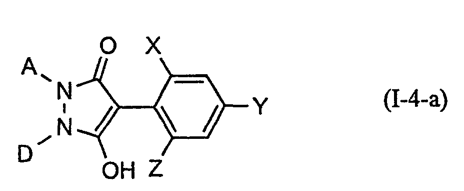 in welcher
A, D, X, Y und Z die oben angegebene Bedeutung haben, jeweils
(G)
α) mit Säurehalogeniden der Formel (X) 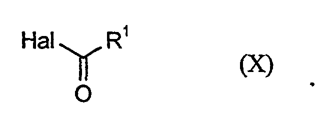 in welcher
R¹ die oben angegebene Bedeutung hat und
Hal für Halogen steht
oder
β) mit Carbonsäureanhydriden der Formel (XI)
R¹-CO-O-CO-R¹ (XI)
in welcher
R¹ die oben angegebene Bedeutung hat,
gegebenenfalls in Gegenwart eines Verdünnungsmittels und gegebenenfalls in Gegenwart eines Säurebindemittels umsetzt,
oder
(H) mit Chlorameisensäureestern oder Chlorameisensäurethioestem der Formel (XII)
R²-M-CO-Cl (XII)
in welcher
R² und M die oben angegebenen Bedeutungen haben,
gegebenenfalls in Gegenwart eines Verdünnungsmittels und gegebenenfalls in Gegenwart eines Säurebindemittels umsetzt,
oder
(I)
α) mit Chlormonothioameisensäureestern oder Chlordithioameisen säureestern der Formel (XIII) 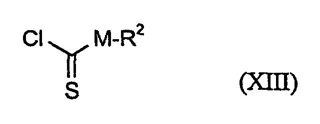 in welcher
M und R² die oben angegebenen Bedeutungen haben,
gegebenenfalls in Gegenwart eines Verdünnungsmittels und gegebenenfalls in Gegenwart eines Säurebindemittels umsetzt
oder
β) mit Schwefelkohlenstoff und anschließend mit Verbindungen der Formel (XIV)
R²-Hal (XIV)
in welcher
R² die oben angegebene Bedeutung hat und
Hal für Chlor, Brom oder Iod steht,
gegebenenfalls in Gegenwart eines Verdünnungsmittels und gegebenenfalls in Gegenwart einer Base umsetzt,
oder
(J) mit Sulfonsäurechloriden der Formel (XV)
R³-SO₂-Cl (XV)
in welcher
R³ die oben angegebene Bedeutung hat,
gegebenenfalls in Gegenwart eines Verdünnungsmittels und gegebenenfalls in Gegenwart eines Säurebindemittels umsetzt,
oder
(K) mit Phosphorverbindungen der Formel (XVI) 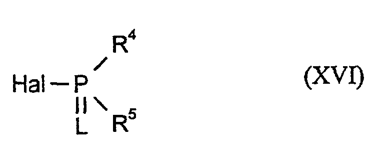 in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
oder
(L) mit Metallverbindungen oder Aminen der Formeln (XVII) oder (XVIII)
Me(OR¹⁰)ₜ (XVII) 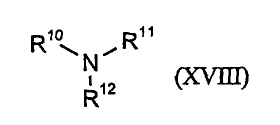
in welchen
Me für ein ein- oder zweiwertiges Metall,
t für die Zahl 1 oder 2 und
R¹⁰, R¹¹, R¹² unabhängig voneinander fiir Wasserstoff oder Alkyl stehen,
gegebenenfalls in Gegenwart eines Verdünnungsmittels umsetzt
oder
(M)
α) mit Isocyanaten oder Isothiocyanaten der Formel (XIX)
R⁶-N=C=L (XIX)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
β) mit Carbamidsäurechloriden oder Thiocarbamidsäurechloriden der Formel (XX) 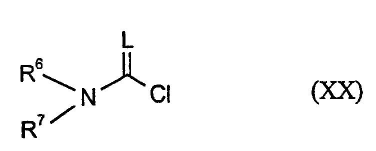 in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt.

5. Verbindungen der Formel (II) 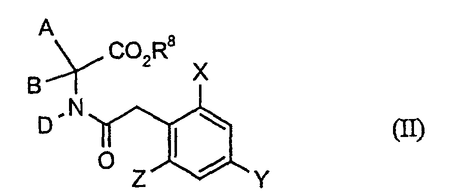 in welcher
A, B, D, X, Y und Z die in Anspruch 1 angegebene Bedeutungen haben und
R⁸ für Alkyl steht.

6. Verbindungen der Formel (XXIII) 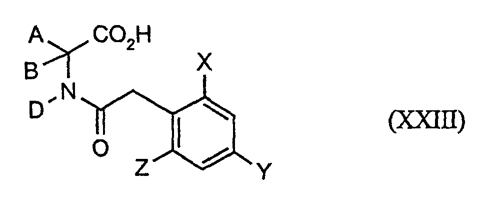 in welcher
A, B, D, X, Y und Z die in Anspruch 1 angegebenen Bedeutungen haben.

7. Verbindungen der Formel (XXII) 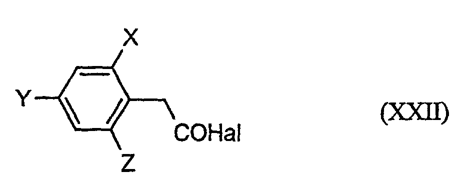 in welcher
X, Y und Z die in Anspruch 1 angegebenen Bedeutungen haben und
Hal für Chlor oder Brom steht.

8. Verbindungen der Formel (XXV) 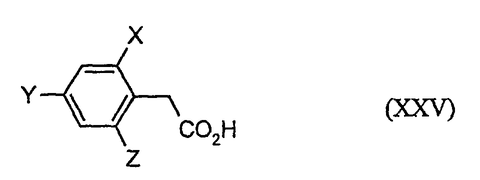 in welcher
X, Y und Z die in Anspruch 1 angegebene Bedeutung haben, ausgenommen 2,4-Dichlor-6-methylphenylessigsäure.

9. Verbindungen der Formel (XXVI) 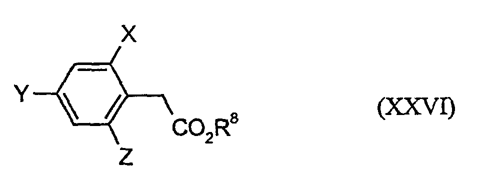 in welcher
X, Y und Z die oben angegebene Bedeutung haben, und
R⁸ für Alkyl steht.

10. Verbindungen der Formel (XXVII) 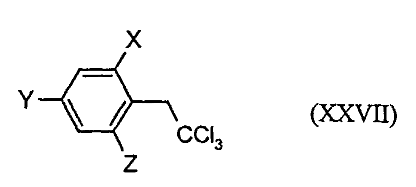 in welcher
X, Y und Z die in Anspruch 1 angegebene Bedeutung haben.

11. Verbindungen der Formel (XXXI) 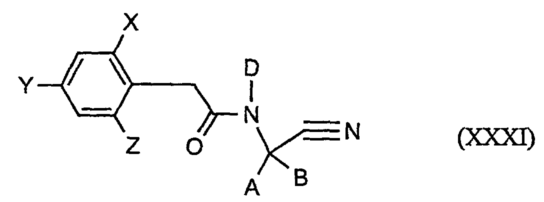 in welcher
A, B, D, X, Y und Z die in Anspruch 1 angegebenen Bedeutungen haben.

12. Verbindungen der Formel (III) 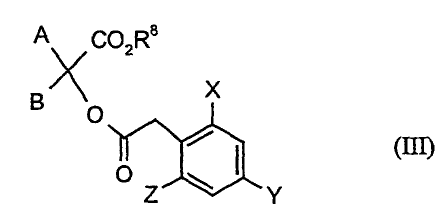 in welcher
A, B, X, Y und Z die oben angegebenen Bedeutungen haben, und
R⁸ für Alkyl steht.

13. Verbindungen der Formel (TV) 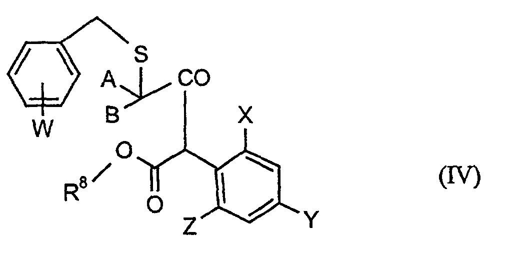 in welcher
A, B, W, X, Y und Z die in Anspruch 1 angegebenen Bedeutungen haben,
R⁸ für Alkyl steht.

14. Verbindungen der Formel (V) 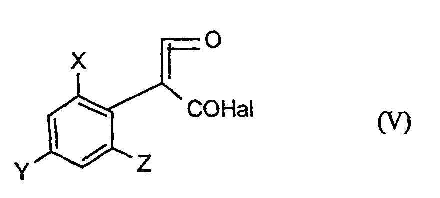 in welcher
X, Y und Z die in Anspruch 1 angegebenen Bedeutungen haben und
Hal für Chlor oder Brom steht.

15. Verbindungen der Formel (XXXV) 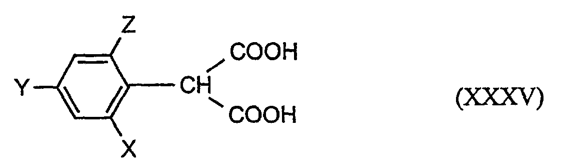 in welcher
X, Y und Z die in Anspruch 1 angegebenen Bedeutungen haben.

16. Schädlingsbekämpfungsmittel und Unkrautbekämpfungsmittel, **gekennzeichnet durch** einen Gehalt an mindestens einer Verbindung der Formel (I) gemäß Anspruch 1.

17. Verwendung von Verbindungen der Formel (I) gemäß Anspruch 1 zur Bekämpfung von Schädlingen und Unkräutern.

18. Verfahren zur Bekämpfung von Schädlingen und Unkräutern, **dadurch gekennzeichnet, daß** man Verbindungen der Formel (I) gemäß Anspruch 1 auf Schädlinge und/oder ihren Lebensraum einwirken läßt oder auf Unkräuter und/oder ihren Lebensraum einwirken läßt.

19. Verfahren zur Herstellung von Schädlingsbekämpfungsmitteln und Unkrautbekämpfungsmitteln, **dadurch gekennzeichnet, daß** man Verbindungen der Formel (I) gemäß Anspruch 1 mit Streckmitteln und/oder oberflächenaktiven Mitteln vermischt.

20. Verwendung von Verbindungen der Formel (I) gemäß Anspruch 1 zur Herstellung von Schädlingsbekämpfungsmitteln und Unkrautbekämpfungsmitteln.

## Claims

1. Compounds of the formula (I) 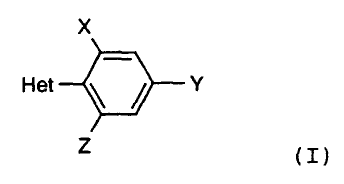 in which
X represents halogen,
Y represents halogen or C₁-C₆-alkyl,
Z represents halogen or C₁-C₆-alkyl,
where always one of the substituents Y and Z represents halogen, while the other represents alkyl,
Het represents one of the groups 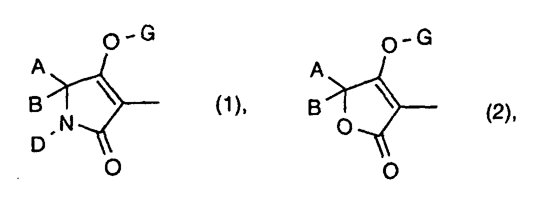 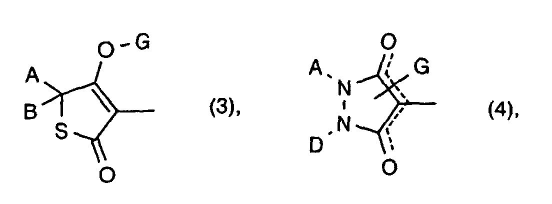 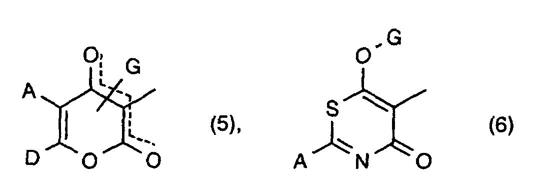
A represents hydrogen, or represents C₁-C₁₂-alkyl, C₂-C₈-alkenyl, C₁-C₁₀-alkoxy-C₁-C₈-alkyl, poly-C₁-C₈-alkoxy-C₁-C₈-alkyl or C₁-C₁₀-alkylthio-C₁-C₆-alkyl, each of which is optionally substituted by halogen, or represents C₃-C₈-cycloalkyl which is optionally substituted by halogen, C₁-C₆-alkyl or C₁-C₆-alkoxy and in which one or two methylene groups which are not directly adjacent are optionally replaced by oxygen and/or sulphur, or represents phenyl, naphthyl, phenyl-C₁-C₆-alkyl, naphthyl-C₁-C₆-alkyl or hetaryl having 5 or 6 ring atoms and one to three hetero atoms from the series consisting of oxygen, sulphur and nitrogen, in each case optionally substituted by halogen, C₁-C₆-alkyl, C₁-C₆-halogenoalkyl, C₁-C₆-alkoxy, C₁-C₆-halogenoalkoxy, cyano or nitro,
B represents hydrogen, C₁-C₁₂-alkyl or C₁-C₈-alkoxy-C₁-C₆-alkyl, or
A, B and the carbon atom to which they are bonded represent C₃-C₁₀-cycloalkyl or C₅-C₁₀-cycloalkenyl in each of which a methylene group is optionally replaced by oxygen or sulphur and which are optionally substituted by C₁-C₈-alkyl, C₃-C₁₀-cycloalkyl, C₁-C₈-halogenoalkyl, C₁-C₈-alkoxy, C₁-C₈-alkylthio, halogen or phenyl, or
A, B and the carbon atom to which they are bonded represent C₅-C₆-cycloalkyl which is substituted by an alkylenediyl group which optionally contains one or two oxygen and/or sulphur atoms or by an alkylenedioxy or by an alkylenedithioyl group, this group, together with the carbon atom to which it is bonded forming a further five to eight-membered ring, or
A, B and the carbon atom to which they are bonded represent C₃-C₈-cycloalkyl or C₅-C₈-cycloalkenyl in which two substituents together with the carbon atoms to which they are bonded represent C₃-C₆-alkanediyl, C₃-C₆-alkenediyl or C₄-C₆-alkanedienediyl, each of which is optionally substituted by C₁-C₆-alkyl, C₁-C₆-alkoxy or halogen and in which in each case one methylene group is optionally replaced by oxygen or sulphur,
D represents hydrogen, or represents C₁-C₁₂-alkyl, C₃-C₈-alkenyl, C₃-C₈-alkinyl, C₁-C₁₀-alkoxy-C₂-C₈-alkyl, poly-C₁-C₈-alkoxy-C₂-C₈-alkyl or C₁-C₁₀-alkylthio-C₂-C₈-alkyl, each of which is optionally substituted by halogen, or represents C₃-C₈-cycloalkyl which is optionally substituted by halogen, C₁-C₄-alkyl, C₁-C₄-alkoxy or C₁-C₄-halogenoalkyl and in which one or two methylene groups which are not directly adjacent are optionally replaced by oxygen and/or sulphur, or represents phenyl, hetaryl having 5 to 6 ring atoms and one or two hetero atoms from the series consisting of oxygen, sulphur and nitrogen, phenyl-C₁-C₆-alkyl or hetaryl-C₁-C₆-alkyl having 5 to 6 ring atoms and one or two hetero atoms from the series consisting of oxygen, sulphur and nitrogen, in each case optionally substituted by halogen, C₁-C₆-alkyl, C₁-C₆-halogenoalkyl, C₁-C₆-alkoxy, C₁-C₆-halogenoalkoxy, cyano or nitro, or
A and D together represent a C₃-C₆-alkanediyl, C₃-C₆-alkenediyl or C₄-C₆-alkanedienediyl group in each of which one methylene group is optionally replaced by oxygen or sulphur and which is in each case optionally substituted by halogen, hydroxyl, mercapto, or by C₁-C₁₀-alkyl, C₁-C₆-alkoxy, C₁-C₆-alkylthio, C₃-C₇-cycloalkyl, phenyl or benzyloxy, each of which is optionally substituted by halogen, or by a further C₃-C₆-alkanediyl, C₃-C₆-alkenediyl or C₄-C₆-alkanedienediyl group which forms a fused ring and in each of which one methylene group is optionally replaced by oxygen or sulphur and which is optionally substituted by C₁-C₆-alkyl or in which two adjacent substituents together with the carbon atoms to which they are bonded optionally form a further saturated or unsaturated carbocycle having 5 or 6 ring atoms, or
A and D together represent a C₃-C₆-alkanediyl or C₃-C₆-alkenediyl group in each of which one of the following groups 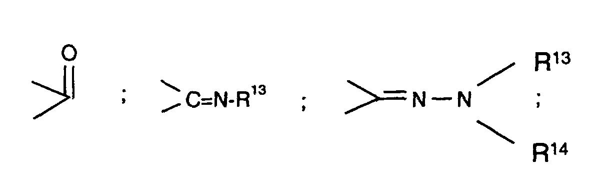 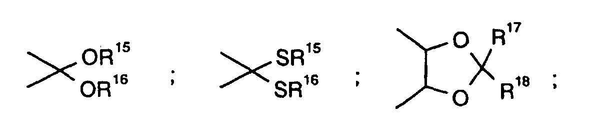 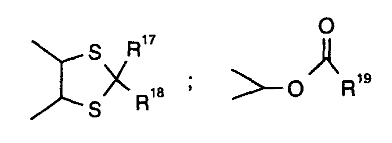 or 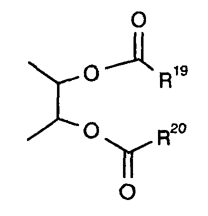 is optionally present,
G in the event that Het represents one of the radicals (1), (2), (3), (5) or (6), represents hydrogen (a) or, in the event that Het represents one of the radicals (1), (2), (3), (4), (5) or (6), represents one of the groups 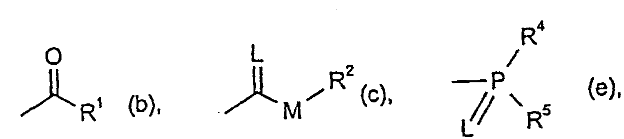 E (f) or 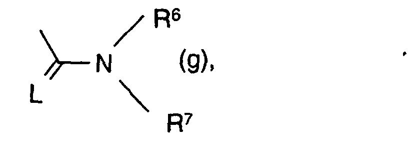 or, in the event that Het represents one of the radicals (2), (3), (4), (5) or (6), represents the group -SO₂-R³(d) in which
E represents a metal ion equivalent or an ammonium ion,
L represents oxygen or sulphur and
M represents oxygen or sulphur,
R¹ represents C₁-C₂₀-alkyl, C₂-C₂₀-alkenyl, C₁-C₈-alkoxy-C₁-C₈-alkyl, C₁-C₈-alkylthio-C₁-C₈-alkyl or poly-C₁-C₈-alkoxy-C₁-C₈-alkyl, each of which is optionally substituted by halogen, or represents C₃-C₈-cycloalkyl which is optionally substituted by halogen, C₁-C₆-alkyl or C₁-C₆-alkoxy and in which one or two methylene groups which are not directly adjacent are optionally replaced by oxygen and/or sulphur,
or represents phenyl which is optionally substituted by halogen, cyano, nitro, C₁-C₆-alkyl, C₁-C₆-alkoxy, C₁-C₆-halogenoalkyl, C₁-C₆-halogenoalkoxy, C₁-C₆-alkylthio or C₁-C₆-alkylsulphonyl,
or represents phenyl-C₁-C₆-alkyl which is optionally substituted by halogen, nitro, cyano, C₁-C₆-alkyl, C₁-C₆-alkoxy, C₁-C₆-halogenoalkyl or C₁-C₆-halogenoalkoxy,
or represents 5- or 6-membered hetaryl having one or two hetero atoms from the series consisting of oxygen, sulphur and nitrogen which is optionally substituted by halogen or C₁-C₆-alkyl,
or represents phenoxy-C₁-C₆-alkyl which is optionally substituted by halogen or C₁-C₆-alkyl,
or represents 5- or 6-membered hetaryloxy-C₁-C₆-alkyl having one or two hetero atoms from the series consisting of oxygen, sulphur and nitrogen which is optionally substituted by halogen, amino or C₁-C₆-alkyl,
R² represents C₁-C₂₀-alkyl, C₂-C₂₀-alkenyl, C₁-C₈-alkoxy-C₂-C₈-alkyl or poly-C₁-C₈-alkoxy-C₂-C₈-alkyl, each of which is optionally substituted by halogen,
or represents C₃-C₈-cycloalkyl which is optionally substituted by halogen, C₁-C₆-alkyl or C₁-C₆-alkoxy, or
represents phenyl or benzyl, each of which is optionally substituted by halogen, cyano, nitro, C₁-C₆-alkyl, C₁-C₆-alkoxy, C₁-C₆-halogenoalkyl or C₁-C₆-halogenoalkoxy,
R³ represents C₁-C₈-alkyl which is optionally substituted by halogen, or represents phenyl or benzyl, each of which is optionally substituted by halogen, C₁-C₆-alkyl, C₁-C₆-alkoxy, C₁-C₄-halogenoalkyl, C₁-C₄-halogenoalkoxy, cyano or nitro,
R⁴ and R⁵ independently of one another represent C₁-C₈-alkyl, C₁-C₈-alkoxy, C₁-C₈-alkylamino, di-(C₁-C₈-alkyl)amino, C₁-C₈-alkylthio or C₂-C₈-alkenylthio, each of which is optionally substituted by halogen, or represent phenyl, phenoxy or phenylthio, each of which is optionally substituted by halogen, nitro, cyano, C₁-C₄-alkoxy, C₁-C₄-halogenoalkoxy, C₁-C₄-alkylthio, C₁-C₄-halogenoalkylthio, C₁-C₄-alkyl or C₁-C₄-halogenoalkyl,
R⁶ and R⁷ independently of one another represent hydrogen, or represent C₁-C₈-alkyl, C₃-C₈-cycloalkyl, C₁-C₈-alkoxy, C₃-C₈-alkenyl or C₁-C₈-alkoxy-C₂-C₈-alkyl, each of which is optionally substituted by halogen, or represent phenyl or benzyl, each of which is optionally substituted by halogen, C₁-C₈-alkyl, C₁-C₈-halogenoalkyl or C₁-C₈-alkoxy, or together represent a C₃-C₆-alkylene radical which is optionally substituted by C₁-C₆-alkyl and in which one methylene group is optionally replaced by oxygen or sulphur,
R¹³ represents hydrogen, or represents C₁-C₈-alkyl or C₁-C₈-alkoxy, each of which is optionally substituted by halogen, or represents C₃-C₈-cycloalkyl which is optionally substituted by halogen, C₁-C₄-alkyl or C₁-C₄-alkoxy and in which one methylene group is optionally replaced by oxygen or sulphur, or represents phenyl, phenyl-C₁-C₄-alkyl or phenyl-C₁-C₄-alkoxy, each of which is optionally substituted by halogen, C₁-C₆-alkyl, C₁-C₆-alkoxy, C₁-C₄-halogenoalkyl, C₁-C₄-halogenoalkoxy, nitro or cyano,
R¹⁴ represents hydrogen or C₁-C₈-alkyl or
R¹³ and R¹⁴ together represent C₄-C₆-alkanediyl,
R¹⁵ and R¹⁶ are identical or different and represent C₁-C₆-alkyl or
R¹⁵ and R¹⁶ together represent a C₂-C₄-alkanediyl radical which is optionally substituted by C₁-C₆-alkyl or by phenyl which is optionally substituted by halogen, C₁-C₄-alkyl, C₁-C₄-halogenoalkyl, C₁-C₄-alkoxy, C₁-C₄-halogenoalkoxy, nitro or cyano,
R¹⁷ and R¹⁸ independently of one another represent hydrogen, or represent C₁-C₈-alkyl which is optionally substituted by halogen, or represent phenyl which is optionally substituted by halogen, C₁-C₆-alkyl, C₁-C₆-alkoxy, C₁-C₄-halogenoalkyl, C₁-C₄-halogenoalkoxy, nitro or cyano, or
R¹⁷ and R¹⁸ together with the carbon atom to which they are bonded represent C₅-C₇-cycloalkyl which is optionally substituted by C₁-C₄-alkyl or C₁-C₄-alkoxy and in which one methylene group is optionally replaced by oxygen or sulphur and
R¹⁹ and R²⁰ independently of one another represent C₁-C₁₀-alkyl, C₂-C₁₀-alkenyl, C₁-C₁₀-alkoxy, C₁-C₁₀-alkylamino, C₃-C₁₀-alkenylamino, di-(C₁-C₁₀-alkyl)amino or di-(C₃-C₁₀-alkenyl)amino.

2. Compounds of the formula (I) according to Claim 1 in which
X represents fluorine, chlorine or bromine,
Y represents fluorine, chlorine, bromine or C₁-C₄-alkyl,
Z represents fluorine, chlorine, bromine or C₁-C₄-alkyl,
where always one of the radicals Y and Z represents halogen while the other represents alkyl,
Het represents one of the groups 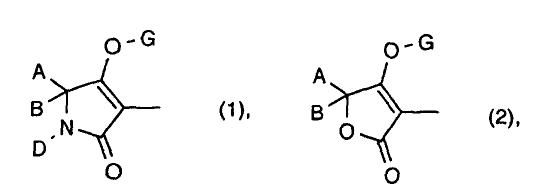 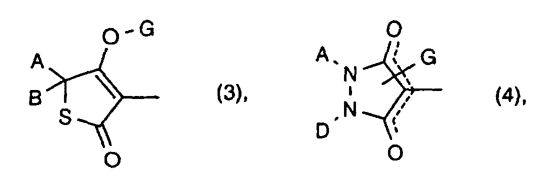 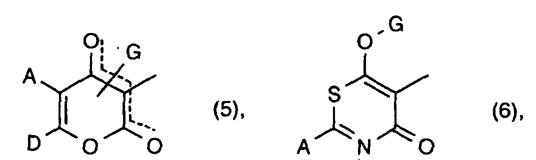
A represents hydrogen, or represents C₁-C₁₀-alkyl, C₂-C₆-alkenyl, C₁-C₈-alkoxy-C₁-C₆-alkyl, poly-C₁-C₆-alkoxy-C₁-C₆-alkyl or C₁-C₈-alkylthio-C₁-C₆-alkyl, each of which is optionally substituted by fluorine or chlorine, or represents C₃-C₇-cycloalkyl which is optionally substituted by fluorine, chlorine, C₁-C₄-alkyl or C₁-C₄-alkoxy and in which one or two methylene groups which are not directly adjacent are optionally replaced by oxygen and/or sulphur, or represents phenyl, furanyl, pyridyl, imidazolyl, triazolyl, pyrazolyl, indolyl, thiazolyl, thienyl or phenyl-C₁-C₄-alkyl, each of which is optionally substituted by fluorine, chlorine, bromine, C₁-C₄-alkyl, C₁-C₄-halogenoalkyl, C₁-C₄-alkoxy, C₁-C₄-halogenoalkoxy, cyano or nitro,
B represents hydrogen, C₁-C₁₀-alkyl or C₁-C₆-alkoxy-C₁-C₄-alkyl, or
A, B and the carbon atom to which they are bonded represent C₃-C₈-cycloalkyl or C₅-C₈-cycloalkenyl in each of which one methylene group is optionally replaced by oxygen or sulphur and which are optionally substituted by C₁-C₆-alkyl, C₃-C₈-cycloalkyl, C₁-C₃-halogenoalkyl, C₁-C₆-alkoxy, C₁-C₆-alkylthio, fluorine, chlorine or phenyl, or
A, B and the carbon atom to which they are bonded represent C₅-C₆-cycloalkyl which is substituted by an alkylenediyl group which optionally contains one or two oxygen or sulphur atoms or by an alkylenedioxy or by an alkylenedithioyl group, this group, together with the carbon atom to which it is bonded forming a further five to seven-membered ring, or
A, B and the carbon atom to which they are bonded represent C₃-C₆-cycloalkyl or C₅-C₆-cycloalkenyl in which two substituents together with the carbon atoms to which they are bonded represent C₃-C₅-alkanediyl, C₃-C₅-alkenediyl or butadienediyl, each of which is optionally substituted by C₁-C₅-alkyl, C₁-C₅-alkoxy, fluorine, chlorine or bromine and in which in each case one methylene group is optionally replaced by oxygen or sulphur,
D represents hydrogen, or represents C₁-C₁₀-alkyl, C₃-C₆-alkenyl, C₃-C₆-alkinyl, C₁-C₈-alkoxy-C₂-C₆-alkyl, poly-C₁-C₆-alkoxy-C₂-C₆-alkyl or C₁-C₈-alkylthio-C₂-C₆-alkyl, each of which is optionally substituted by fluorine or chlorine, or represents C₃-C₇-cycloalkyl which is optionally substituted by fluorine, chlorine, C₁-C₄-alkyl, C₁-C₄-alkoxy or C₁-C₂-halogenoalkyl and in which one or two methylene groups which are not directly adjacent are optionally replaced by oxygen and/or sulphur, or represents phenyl, furanyl, imidazolyl, pyridyl, thiazolyl, pyrazolyl, pyrimidyl, pyrrolyl, thienyl, triazolyl or phenyl-C₁-C₄-alkyl, each of which is optionally substituted by fluorine, chlorine, bromine, C₁-C₄-alkyl, C₁-C₄-halogenoalkyl, C₁-C₄-alkoxy, C₁-C₄-halogenoalkoxy, cyano or nitro, or
A and D together represent a C₃-C₅-alkanediyl or C₃-C₅-alkenediyl group in each of which one methylene group is optionally replaced by oxygen or sulphur and which are optionally substituted by fluorine, chlorine, hydroxyl, mercapto, or by C₁-C₆-alkyl, C₁-C₄-alkoxy, C₁-C₄-alkylthio, C₃-C₆-cycloalkyl, phenyl or benzyloxy, each of which is optionally substituted by fluorine or chlorine, or
in which in each case one of the following groups 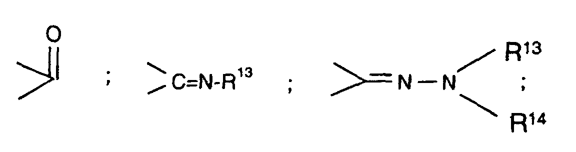 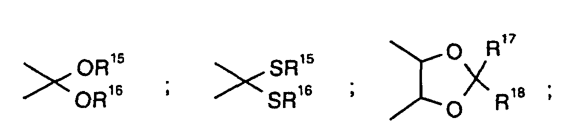 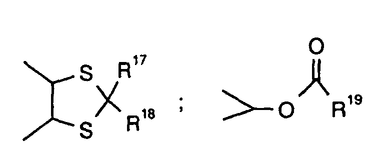 or 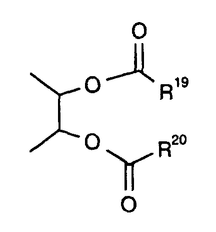 is optionally present;
or A and D (in the case of the compounds of the formula (I-1)) together with the atoms to which they are bonded represent one of the groups AD-1 to AD-27 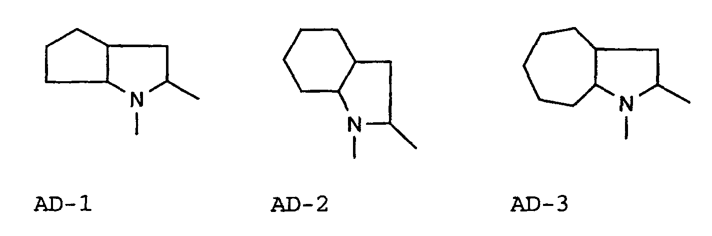 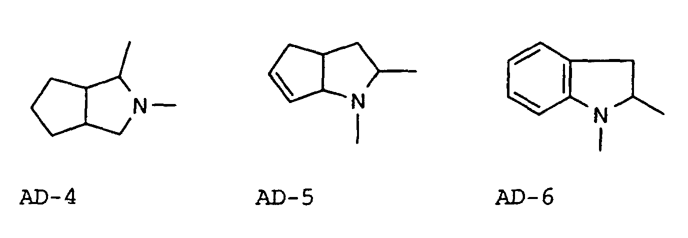 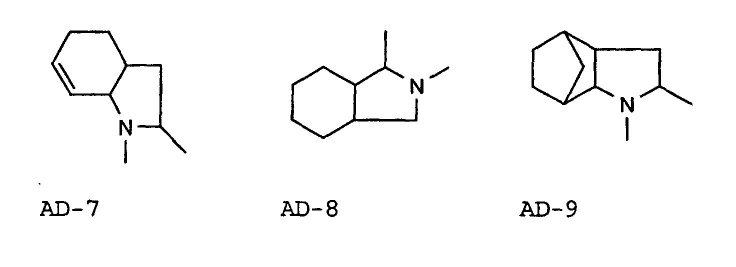 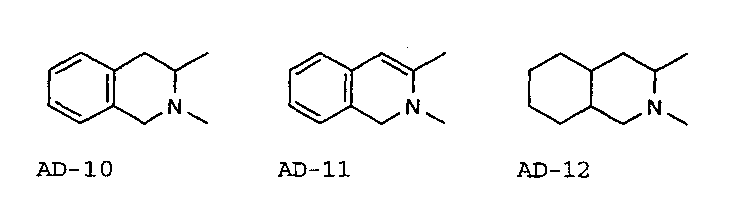 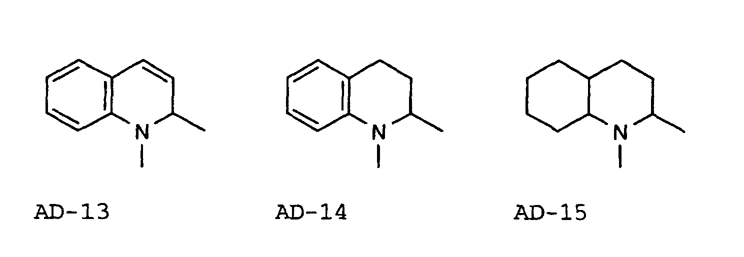 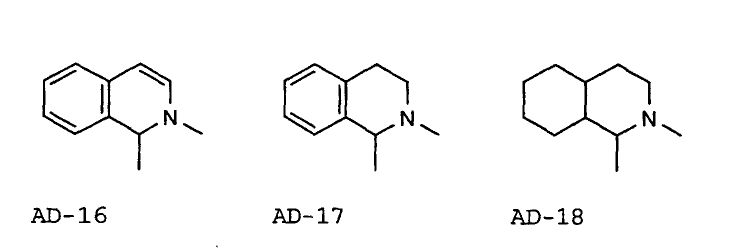 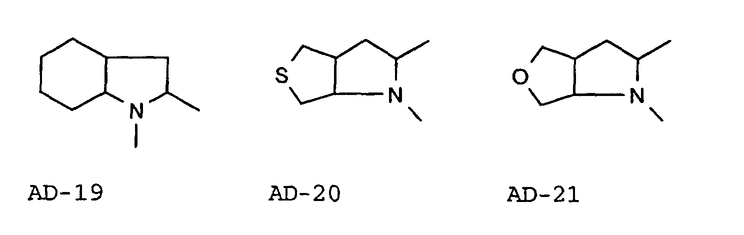 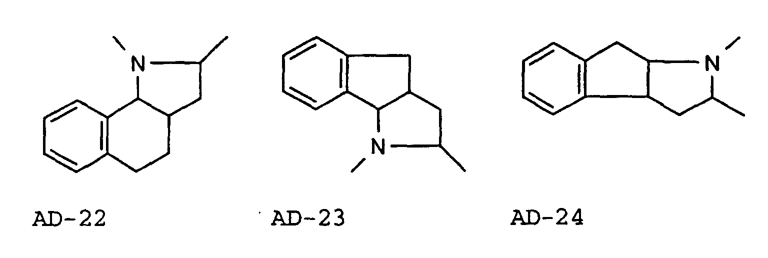 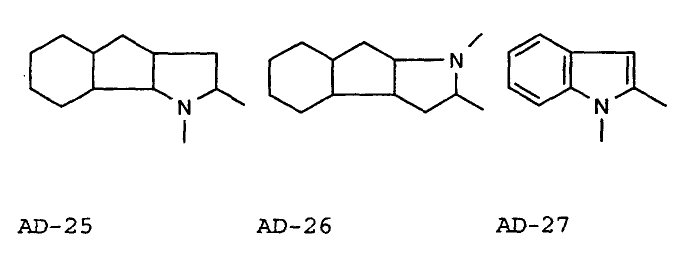
G in the event that Het represents one of the radicals (1), (2), (3), (5) or (6), represents hydrogen (a) or, in the event that Het represents one of the radicals (1), (2), (3), (4), (5) or (6), represents one of the groups 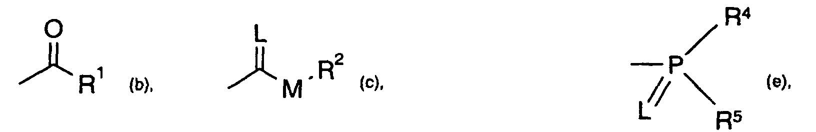 E (f) or 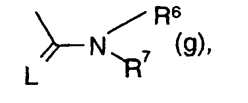 or, in the event that Het represents one of the radicals (2), (3), (4), (5) or (6), represents the group -SO₂-R³ (d)
in which
E represents a metal ion equivalent or an ammonium ion,
L represents oxygen or sulphur and
M represents oxygen or sulphur,
R¹ represents C₁-C₁₆-alkyl, C₂-C₁₆-alkenyl, C₁-C₆-alkoxy-C₁-C₆-alkyl, C₁-C₆-alkylthio-C₁-C₆-alkyl or poly-C₁-C₆-alkoxy-C₁-C₆-alkyl, each of which is optionally substituted by fluorine or chlorine, or represents C₃-C₇-cycloalkyl which is optionally substituted by fluorine, chlorine, C₁-C₅-alkyl or C₁-C₅-alkoxy and in which one or two methylene groups which are not directly adjacent are optionally replaced by oxygen and/or sulphur,
or represents phenyl which is optionally substituted by fluorine, chlorine, bromine, cyano, nitro, C₁-C₄-alkyl, C₁-C₄-alkoxy, C₁-C₃-halogenoalkyl, C₁-C₃-halogenoalkoxy, C₁-C₄-alkylthio or C₁-C₄-alkylsulphonyl,
or represents phenyl-C₁-C₄-alkyl which is optionally substituted by fluorine, chlorine, bromine, C₁-C₄-alkyl, C₁-C₄-alkoxy, C₁-C₃-halogenoalkyl or C₁-C₃-halogenoalkoxy,
or represents pyrazolyl, thiazolyl, pyridyl, pyrimidyl, furanyl or thienyl, each of which is optionally substituted by fluorine, chlorine, bromine or C₁-C₄-alkyl,
or represents phenoxy-C₁-C₅-alkyl which is optionally substituted by fluorine, chlorine, bromine or C₁-C₄-alkyl, or
represents pyridyloxy-C₁-C₅-alkyl, pyrimidyloxy-C₁-C₅-alkyl or thiazolyloxy-C₁-C₅-alkyl, each of which is optionally substituted by fluorine, chlorine, bromine, amino or C₁-C₄-alkyl,
R² represents C₁-C₁₆-alkyl, C₂-C₁₆-alkenyl, C₁-C₆-alkoxy-C₂-C₆-alkyl or poly-C₁-C₆-alkoxy-C₂-C₆-alkyl, each of which is optionally substituted by fluorine or chlorine,
or represents C₃-C₇-cycloalkyl which is optionally substituted by fluorine, chlorine, C₁-C₄-alkyl or C₁-C₄-alkoxy, or
represents phenyl or benzyl, each of which is optionally substituted by fluorine, chlorine, bromine, cyano, nitro, C₁-C₄-alkyl, C₁-C₃-alkoxy, C₁-C₃-halogenoalkyl or C₁-C₃-halogenoalkoxy,
R³ represents C₁-C₆-alkyl which is optionally substituted by fluorine or chlorine, or represents phenyl or benzyl, each of which is optionally substituted by fluorine, chlorine, bromine, C₁-C₅-alkyl, C₁-C₅-alkoxy, C₁-C₃-halogenoalkyl, C₁-C₃-halogenoalkoxy, cyano or nitro,
R⁴ and R⁵ independently of one another represent C₁-C₆-alkyl, C₁-C₆-alkoxy, C₁-C₆-alkylamino, di-(C₁-C₆-alkyl)amino, C₁-C₆-alkylthio or C₃-C₄-alkenylthio, each of which is optionally substituted by fluorine or chlorine, or represent phenyl, phenoxy or phenylthio, each of which is optionally substituted by fluorine, chlorine, bromine, nitro, cyano, C₁-C₃-alkoxy, C₁-C₃-halogenoalkoxy, C₁-C₃-alkylthio, C₁-C₃-halogenoalkylthio, C₁-C₃-alkyl or C₁-C₃-halogenoalkyl,
R⁶ and R⁷ independently of one another represent hydrogen, or represent C₁-C₆-alkyl, C₃-C₆-cycloalkyl, C₁-C₆-alkoxy, C₃-C₆-alkenyl or C₁-C₆-alkoxy-C₂-C₆-alkyl, each of which is optionally substituted by fluorine or chlorine, or represent phenyl or benzyl, each of which is optionally substituted by fluorine, chlorine, bromine, C₁-C₅-halogenoalkyl, C₁-C₅-alkyl or C₁-C₅-alkoxy, or together represent a C₃-C₆-alkylene radical which is optionally substituted by C₁-C₄-alkyl and in which one methylene group is optionally replaced by oxygen or sulphur,
R¹³ represents hydrogen, or represents C₁-C₆-alkyl or C₁-C₆-alkoxy, each of which is optionally substituted by fluorine or chlorine, or represents C₃-C₇-cycloalkyl which is optionally substituted by fluorine, C₁-C₂-alkyl or C₁-C₂-alkoxy and in which one methylene group is optionally replaced by oxygen or sulphur, or represents phenyl, phenyl-C₁-C₃-alkyl or phenyl-C₁-C₂-alkoxy, each of which is optionally substituted by fluorine, chlorine, bromine, C₁-C₅-alkyl, C₁-C₅-alkoxy, C₁-C₂-halogenoalkyl, C₁-C₂-halogenoalkoxy, nitro or cyano,
R¹⁴ represents hydrogen or C₁-C₆-alkyl or
R¹³ and R¹⁴ together represent C₄-C₆-alkanediyl,
R¹⁵ and R¹⁶ are identical or different and represent C₁-C₄-alkyl or
R¹⁵ and R¹⁶ together represent a C₂-C₃-alkanediyl radical which is optionally substituted by C₁-C₄-alkyl or by phenyl which is optionally substituted by fluorine, chlorine, bromine, C₁-C₂-alkyl, C₁-C₂-halogenoalkyl, C₁-C₂-alkoxy, C₁-C₂-halogenoalkoxy, nitro or cyano,
R¹⁷ and R¹⁸ independently of one another represent hydrogen, or represent C₁-C₈-alkyl which is optionally substituted by fluorine or chlorine, or represent phenyl which is optionally substituted by fluorine, chlorine, C₁-C₄-alkyl, C₁-C₄-alkoxy, C₁-C₂-halogenoalkyl, C₁-C₂-halogenoalkoxy, nitro or cyano, or
R¹⁷ and R¹⁸ together with the carbon atom to which they are bonded represent C₅-C₆-cycloalkyl which is optionally substituted by C₁-C₃-alkyl or C₁-C₃-alkoxy and in which one methylene group is optionally replaced by oxygen or sulphur, and
R¹⁹ and R²⁰ independently of one another represent C₁-C₆-alkyl, C₂-C₆-alkenyl, C₁-C₆-alkoxy, C₁-C₆-alkylamino, C₃-C₆-alkenylamino, di-(C₁-C₆-alkyl)amino or di-(C₃-C₆-alkenyl)amino.

3. Compounds of the formula (I) according to Claim 1, in which
X represents fluorine, chlorine or bromine,
Y represents fluorine, chlorine, bromine, methyl, ethyl, n-propyl or iso-propyl,
Z represents fluorine, chlorine, bromine, methyl, ethyl, n-propyl or iso-propyl,
where always one of the radicals Y and Z represents halogen while the other represents alkyl,
Het represents one of the groups 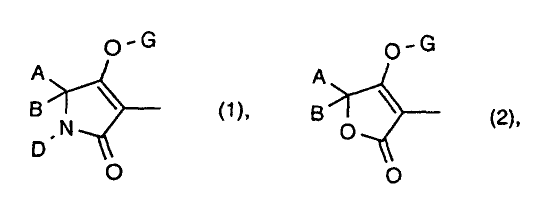 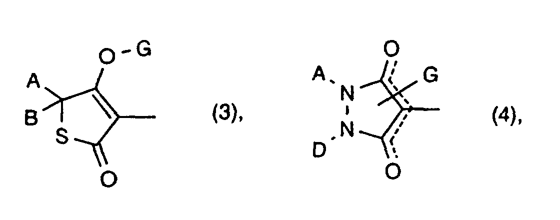 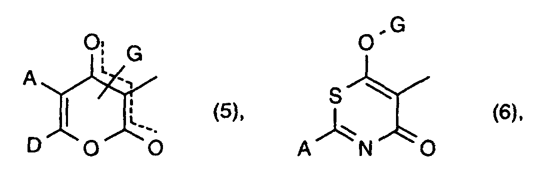
A represents hydrogen, or represents C₁-C₈-alkyl, C₂-C₄-alkenyl, C₁-C₆-alkoxy-C₁-C₄-alkyl, poly-C₁-C₄-alkoxy-C₁-C₄-alkyl or C₁-C₆-alkylthio- C₁-C₄-alkyl, each of which is optionally substituted by fluorine or chlorine, or represents C₃-C₆-cycloalkyl which is optionally substituted by fluorine, chlorine, methyl or methoxy and in which one or two methylene groups which are not directly adjacent are optionally replaced by oxygen and/or sulphur, or represents phenyl, pyridyl or benzyl, each of which is optionally substituted by fluorine, chlorine, bromine, methyl, ethyl, n-propyl, iso-propyl, methoxy, ethoxy, trifluoromethyl, trifluoromethoxy, cyano or nitro,
B represents hydrogen, C₁-C₈-alkyl or C₁-C₄-alkoxy-C₁-C₂-alkyl, or
A, B and the carbon atom to which they are bonded represent C₃-C₈-cycloalkyl or C₅-C₈-cycloalkenyl in each of which one methylene group is optionally replaced by oxygen or sulphur and which are optionally substituted by methyl, ethyl, n-propyl, iso-propyl, butyl, iso-butyl, sec-butyl, tert-butyl, cyclohexyl, trifluoromethyl, methoxy, ethoxy, n-propoxy, iso-propoxy, butoxy, iso-butoxy, sec-butoxy, tert-butoxy, methylthio, ethylthio, fluorine, chlorine or phenyl, or
A, B and the carbon atom to which they are bonded represent C₅-C₆-cycloalkyl which is substituted by an alkylenediyl group which optionally contains an oxygen or sulphur atom or by an alkylenedioxy group, this alkylenediyl or alkylenedioxy group together with the carbon atom to which it is bonded forming a further five to six-membered ring, or
A, B and the carbon atom to which they are bonded represent C₃-C₆-cycloalkyl or C₅-C₆-cycloalkenyl in which two substituents together with the carbon atoms to which they are bonded represent C₃-C₄-alkanediyl, C₃-C₄-alkenediyl or butadienediyl, in each of which one methylene group is optionally replaced by oxygen or sulphur,
D represents hydrogen, or represents C₁-C₈-alkyl, C₃-C₄-alkenyl, C₃-C₄-alkinyl, C₁-C₆-alkoxy-C₂-C₄-alkyl, poly-C₁-C₄-alkoxy-C₂-C₄-alkyl, C₁-C₄-alkylthio-C₂-C₄-alkyl or C₃-C₆-cycloalkyl, in which one or two methylene groups which are not directly adjacent are replaced by oxygen and/or sulphur, in each case optionally substituted by fluorine or chlorine, or represents phenyl, furanyl, pyridyl, thienyl or benzyl, each of which is optionally substituted by fluorine, chlorine, bromine, methyl, ethyl, n-propyl, iso-propyl, methoxy, ethoxy, trifluoromethyl, trifluoromethoxy, cyano or nitro,
or
A and D together represent a C₃-C₅-alkanediyl or C₃-C₅-alkenediyl group in each of which one methylene group is optionally replaced by oxygen or sulphur and which are optionally substituted by fluorine, chlorine, hydroxyl, mercapto, or by C₁-C₆-alkyl, C₁-C₄-alkoxy, C₁-C₄-alkylthio, C₃-C₆-cycloalkyl, phenyl or benzyloxy, each of which is optionally substituted by fluorine or chlorine, or
in which in each case one of the following groups 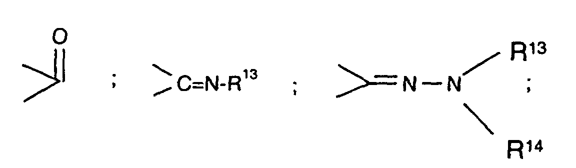 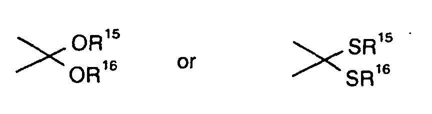 is optionally present,
or A and D, in the case of the compounds of the formula (I-1), together with the atoms to which they are bonded represent one of the following groups: 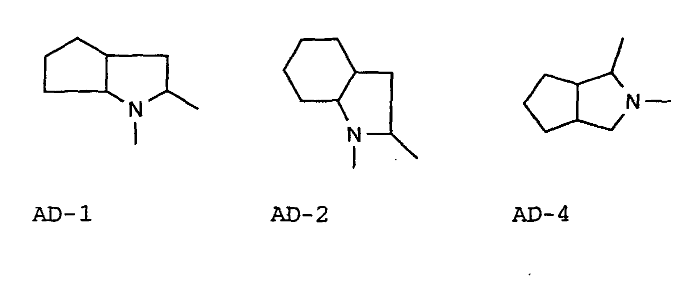 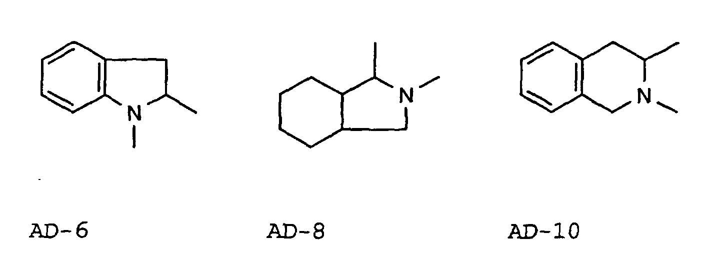 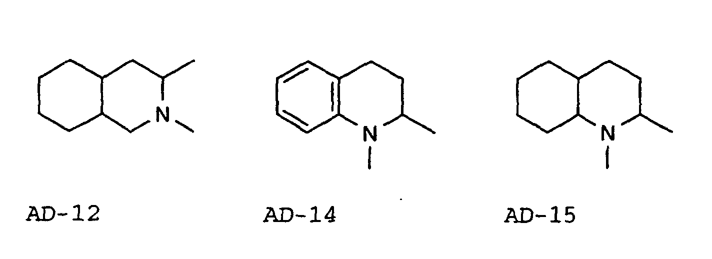 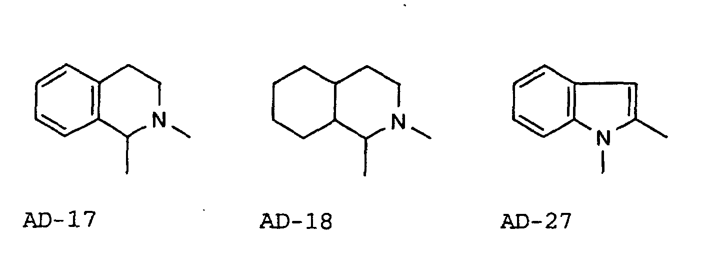
G in the event that Het represents one of the radicals (1), (2), (3), (5) or (6), represents hydrogen (a) or, in the event that Het represents one of the radicals (1), (2), (3), (4), (5) or (6), represents one of the groups 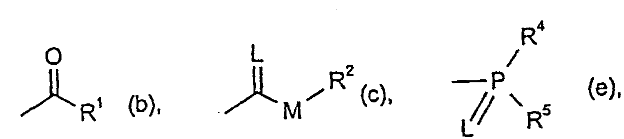 E (f) or 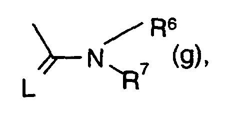 or, in the event that Het represents one of the radicals (2), (3), (4), (5) or (6), represents the group -SO₂-R³ (d) in which
E represents a metal ion equivalent or an ammonium ion,
L represents oxygen or sulphur and
M represents oxygen or sulphur,
R¹ represents C₁-C₁₄-alkyl, C₂-C₁₄-alkenyl, C₁-C₄-alkoxy-C₁-C₆-alkyl, C₁-C₄-alkylthio-C₁-C₆-alkyl or poly-C₁-C₄-alkoxy-C₁-C₄-alkyl, each of which is optionally substituted by fluorine or chlorine, or represents C₃-C₆-cycloalkyl which is optionally substituted by fluorine, chlorine, methyl, ethyl, n-propyl, i-propyl, n-butyl, i-butyl, tert-butyl, methoxy, ethoxy, n-propoxy or iso-propoxy and in which one or two methylene groups which are not directly adjacent are optionally replaced by oxygen and/or sulphur, or represents phenyl which is optionally substituted by fluorine, chlorine, bromine, cyano, nitro, methyl, ethyl, n-propyl, i-propyl, methoxy, ethoxy, trifluoromethyl, trifluoromethoxy, methylthio, ethylthio, methylsulphonyl or ethylsulphonyl,
or represents benzyl which is optionally substituted by fluorine, chlorine, bromine, methyl, ethyl, n-propyl, i-propyl, methoxy, ethoxy, trifluoromethyl or trifluoromethoxy,
or represents furanyl, thienyl or pyridyl, each of which is optionally substituted by fluorine, chlorine, bromine, methyl or ethyl,
or represents phenoxy-C₁-C₄-alkyl which is optionally substituted by fluorine, chlorine, methyl or ethyl, or
represents pyridyloxy-C₁-C₄-alkyl, pyrimidyloxy-C₁-C₄-alkyl or thiazolyloxy-C₁-C₄-alkyl, each of which is optionally substituted by fluorine, chlorine, amino, methyl or ethyl,
R² represents C₁-C₁₄-alkyl, C₂-C₁₄-alkenyl, C₁-C₄-alkoxy-C₂-C₆-alkyl or poly-C₁-C₄-alkoxy-C₂-C₆-alkyl, each of which is optionally substituted by fluorine or chlorine,
or represents C₃-C₆-cycloalkyl which is optionally substituted by fluorine, chlorine, cyano, nitro, methyl, ethyl, n-propyl, iso-propyl, or methoxy, ethoxy, trifluoromethyl or trifluoromethoxy,
or represents phenyl or benzyl, each of which is optionally substituted by fluorine, chlorine, methyl, ethyl, n-propyl, iso-propyl or methoxy, cyano, nitro, ethoxy, trifluoromethyl or trifluoromethoxy,
R³ represents methyl, ethyl, propyl or isopropyl, each of which is optionally substituted by fluorine or chlorine, or represents phenyl or benzyl, each of which is optionally substituted by fluorine, chlorine, bromine, methyl, ethyl, propyl, iso-propyl, tert-butyl, methoxy, ethoxy, isopropoxy, tert-butoxy, trifluoromethyl, trifluoromethoxy, cyano or nitro,
R⁴ and R⁵ independently of one another represent C₁-C₄-alkyl, C₁-C₄-alkoxy, C₁-C₄-alkylamino, di-(C₁-C₄-alkyl)amino or C₁-C₄-alkylthio, each of which is optionally substituted by fluorine or chlorine, or represent phenyl, phenoxy or phenylthio, each of which is optionally substituted by fluorine, chlorine, bromine, nitro, cyano, methyl, methoxy, trifluoromethyl or trifluoromethoxy,
R⁶ and R⁷ independently of one another represent hydrogen, or represent C₁-C₄-alkyl, C₃-C₆-cycloalkyl, C₁-C₄-alkoxy, C₃-C₄-alkenyl or C₁-C₄-alkoxy-C₂-C₄-alkyl, each of which is optionally substituted by fluorine or chlorine, or represent phenyl or benzyl, each of which is optionally substituted by fluorine, chlorine, bromine, methyl, methoxy or trifluoromethyl, or together represent a C₅-C₆-alkylene radical which is optionally substituted by methyl or ethyl and in which one methylene group is optionally replaced by oxygen or sulphur,
R¹³ represents hydrogen, or represents C₁-C₄-alkyl or C₁-C₄-alkoxy, each of which is optionally substituted by fluorine or chlorine, or represents C₃-C₆-cycloalkyl, or represents phenyl, phenyl-C₁-C₂-alkyl or benzyloxy, each of which is optionally substituted by fluorine, chlorine, bromine, methyl, ethyl, iso-propyl, tert-butyl, methoxy, ethoxy, iso-propoxy, tert-butoxy, trifluoromethyl, trifluoromethoxy, nitro or cyano,
R¹⁴ represents hydrogen or C₁-C₄-alkyl, or
R¹³ and R¹⁴ together represent C₄-C₆-alkanediyl,
R¹⁵ and R¹⁶ are identical or different and represent methyl or ethyl, or
R¹⁵ and R¹⁶ together represent a C₂-C₃-alkanediyl radical which is optionally substituted by methyl, ethyl, n-propyl, iso-propyl, n-butyl, iso-butyl, sec-butyl or tert-butyl, or by phenyl which is optionally substituted by fluorine, chlorine, methoxy, trifluoromethyl, trifluoromethoxy, nitro or cyano.

4. Process for the preparation of compounds of the formula (I) according to Claim 1, **characterized in that**
(A)compounds of the formula (I-1-a) 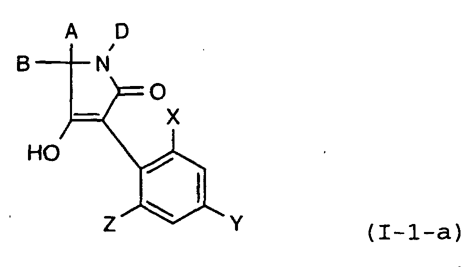 in which
A, B, D, X, Y and Z have the abovementioned meanings,
are obtained when
compounds of the formula (II) 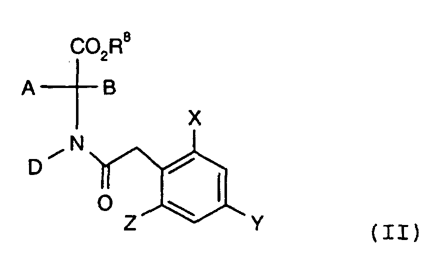 in which
A, B, D, X, Y and Z have the abovementioned meanings
and
R⁸ represents alkyl,
are subjected to intramolecular condensation in the presence of a diluent and in the presence of a base,
(B)compounds of the formula (I-2-a) 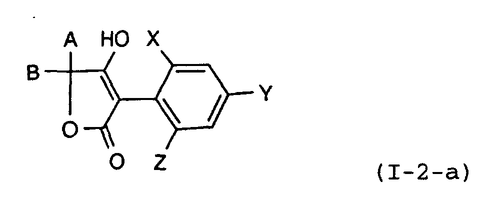 in which
A, B, X, Y and Z have the abovementioned meanings,
are obtained when
compounds of the formula (III) 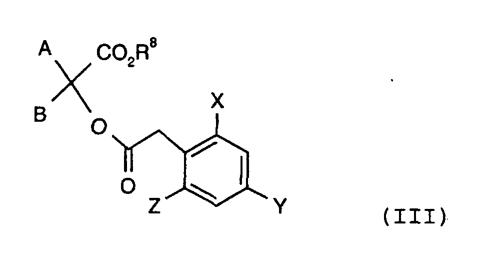 in which
A, B, X, Y, Z and R⁸ have the abovementioned meanings,
are subjected to intramolecular condensation in the presence of a diluent and in the presence of a base,
(C) compounds of the formula (I-3-a) 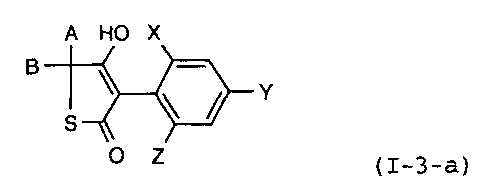 in which
A, B, X, Y and Z have the abovementioned meanings,
are obtained when
compounds of the formula (IV) 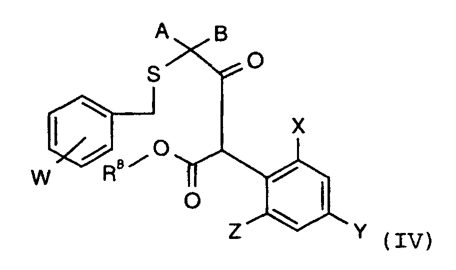 in which
A, B, X, Y, Z and R⁸ have the abovementioned meanings and
W represents hydrogen, halogen, alkyl or alkoxy,
are subjected to intramolecular cyclization, if appropriate in the presence of a diluent and in the presence of an acid,
(E)compounds of the formula (I-5-a) 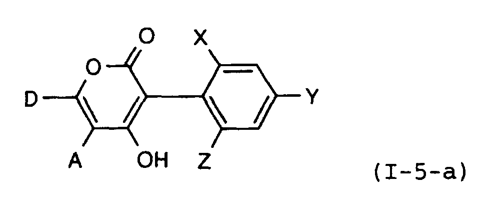 in which
A, D, X, Y and Z have the abovementioned meanings
are obtained when
compounds of the formula (VIII) 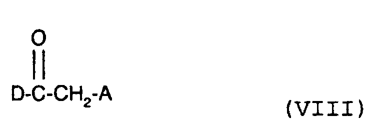 in which
A and D have the abovementioned meanings,
are reacted with compounds of the formula (V) 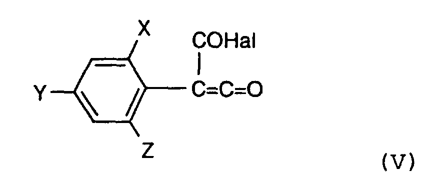 in which
X, Y and Z have the abovementioned meanings and
Hal represents halogen,
if appropriate in the presence of a diluent and if appropriate in the presence of an acid acceptor,
(F) the compounds of the formula (I-6-a) 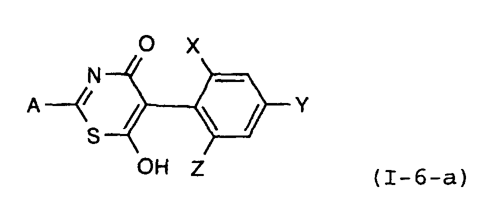 in which
A, X, Y and Z have the abovementioned meanings,
are obtained when compounds of the formula (IX) 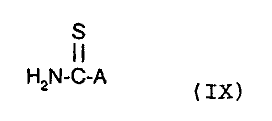 in which
A has the abovementioned meaning,
are reacted with compounds of the formula (V) 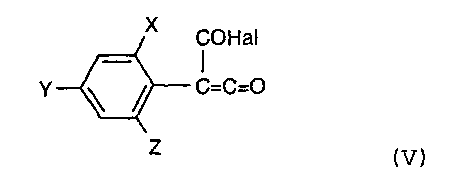 in which
Hal, X, Y and Z have the abovementioned meanings,
if appropriate in the presence of a diluent and if appropriate in the presence of an acid acceptor,
and, if appropriate, the resulting compounds of the formulae (I-1-a), (I-2-a) to (I-3-a), (I-5-a) and (I-6-a), or compounds of the formula (I-4-a) 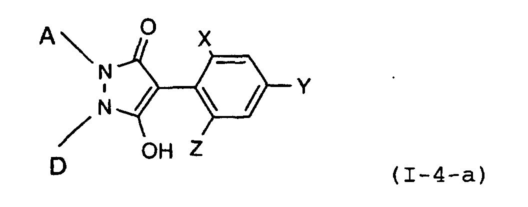 in which
A, D, X, Y and Z have the abovementioned meanings, are in each case reacted
(G)
α) with acid halides of the formula (X) 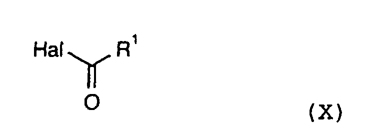 in which
R¹ has the abovementioned meaning and
Hal represents halogen
or
β) with carboxylic anhydrides of the formula (XI)
R¹-CO-O-CO-R¹ (XI)
in which
R¹ has the abovementioned meaning,
if appropriate in the presence of a diluent and if appropriate in the presence of an acid-binding agent,
or
(H) are reacted with chloroformic esters or chloroformic thioesters of the formula (XII)
R²-M-CO-Cl (XII)
in which
R² and M have the abovementioned meanings,
if appropriate in the presence of a diluent and if appropriate in the presence of an acid-binding agent,
or
(I)
α) are reacted with chloromonothioformic esters or chlorodithioformic esters of the formula (XIII) 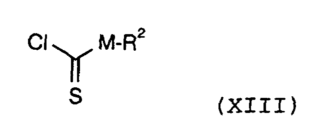 in which
M and R² have the abovementioned meanings,
if appropriate in the presence of a diluent and if appropriate in the presence of an acid-binding agent,
or
β) are reacted with carbon disulphide and subsequently with compounds of the formula (XIV)
R²-Hal (XIV)
in which
R² has the abovementioned meaning and
Hal represents chlorine, bromine or iodine,
if appropriate in the presence of a diluent and if appropriate in the presence of a base,
or
(J) are reacted with sulphonyl chlorides of the formula (XV)
R³-SO₂-Cl (XV)
in which
R³ has the abovementioned meaning,
if appropriate in the presence of a diluent and if appropriate in the presence of an acid-binding agent,
or
(K) are reacted with phosphorus compounds of the formula (XVI) 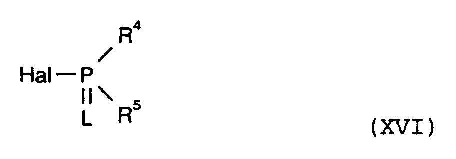 in which
L, R⁴ and R⁵ have the abovementioned meanings and
Hal represents halogen,
if appropriate in the presence of a diluent and if appropriate in the presence of an acid-binding agent,
or
(L) are reacted with metal compounds or amines of the formulae (XVII) or (XVIII)
Me(OR¹⁰)ₜ (XVII) 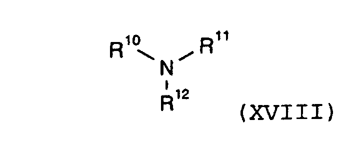
in which
Me represents a mono- or divalent metal,
t represents the number 1 or 2 and
R¹⁰, R¹¹ and R¹² independently of one another represent hydrogen or alkyl,
if appropriate in the presence of a diluent,
or
(M)
α) are reacted with isocyanates or isothiocyanates of the formula (XIX)
R⁶-N=C=L (XIX)
in which
R⁶ and L have the abovementioned meanings,
if appropriate in the presence of a diluent and if appropriate in the presence of a catalyst, or
β) are reacted with carbamoyl chlorides or thiocarbamoyl chlorides of the formula (XX) 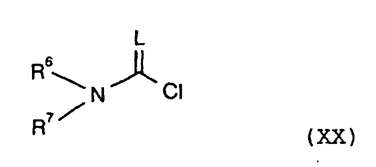 in which
L, R⁶ and R⁷ have the abovementioned meanings,
if appropriate in the presence of a diluent and if appropriate in the presence of an acid-binding agent.

5. Compounds of the formula (II) 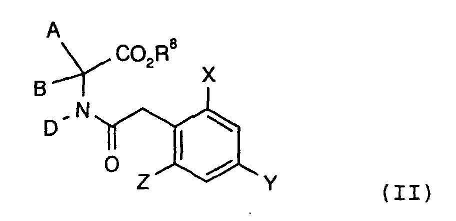 in which
A, B, D, X, Y and Z have the meanings given in Claim 1 and
R⁸ represents alkyl.

6. Compounds of the formula (XXIII) 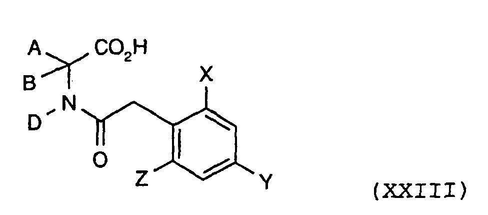 in which
A, B, D, X, Y and Z have the meanings given in Claim 1.

7. Compounds of the formula (XXII) 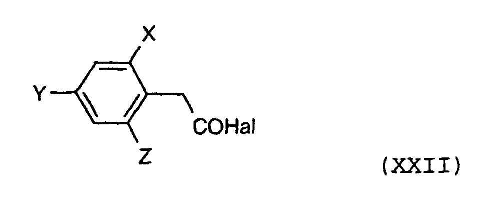 in which
X, Y and Z have the meanings given in Claim 1 and
Hal represents chlorine or bromine.

8. Compounds of the formula (XXV) 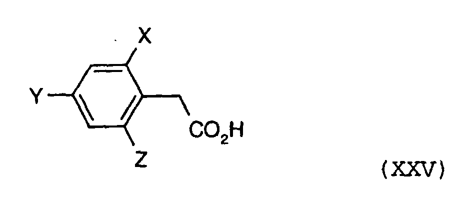 in which
X, Y and Z have the meanings given in Claim 1, with the exception of 2,4-dichloro-6-methylphenylacetic acid.

9. Compounds of the formula (XXVI) 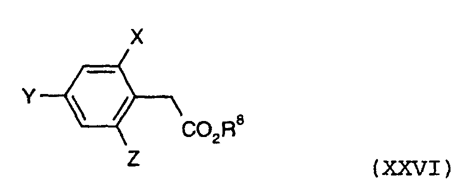 in which
X, Y and Z have the abovementioned meanings and
R⁸ represents alkyl.

10. Compounds of the formula (XXVII) 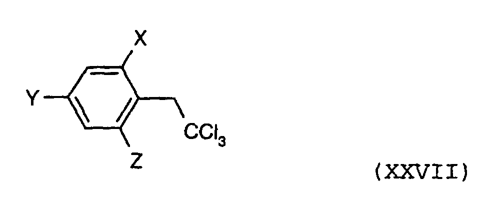 in which
X, Y and Z have the meanings given in Claim 1.

11. Compounds of the formula (XXXI) 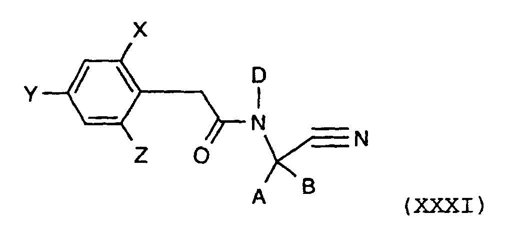 in which
A, B, D, X, Y and Z have the meanings given in Claim 1.

12. Compounds of the formula (III) 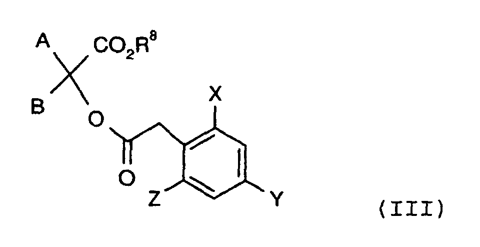 in which
A, B, X, Y and Z have the abovementioned meanings and
R⁸ represents alkyl.

13. Compounds of the formula (IV) 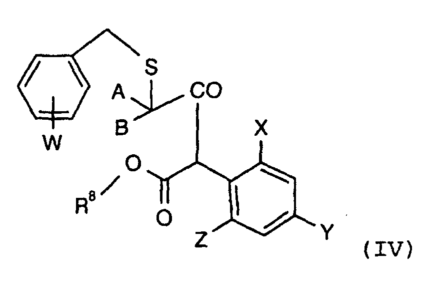 in which
A, B, W, X, Y and Z have the meanings given in Claim 1 and
R⁸ represents alkyl.

14. Compounds of the formula (V) 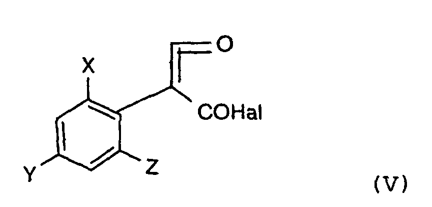 in which
X, Y and Z have the meanings given in Claim 1 and
Hal represents chlorine or bromine.

15. Compounds of the formula (XXXV) 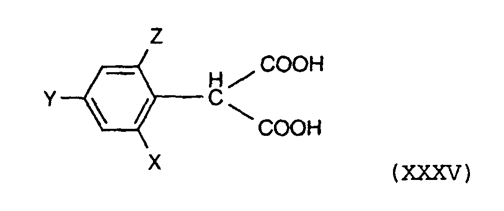 in which
X, Y and Z have the meanings given in Claim 1.

16. Pesticides and herbicides, **characterized in that** they comprise at least one compound of the formula (I) according to Claim 1.

17. Use of compounds of the formula (I) according to Claim 1 for combating pests and weeds.

18. Method of combating pests and weeds, **characterized in that** compounds of the formula (I) according to Claim 1 are allowed to act on pests and/or their environment or on weeds and/or their environment.

19. Process for the preparation of pesticides and herbicides, **characterized in that** compounds of the formula (I) according to Claim 1 are mixed with extenders and/or surface-active agents.

20. Use of compounds of the formula (I) according to Claim 1 for the preparation of pesticides and herbicides.

## Revendications

1. Composés de formule (I) 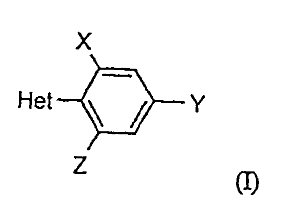 dans laquelle
X représente un halogène,
Y est un halogène ou un groupe alkyle en C₁ à C₆,
Z est un halogène ou un groupe alkyle en C₁ à C₆,
avec, dans tous les cas, l'un des substituants Y et Z représentant un halogène et l'autre représentant un groupe alkyle,
Het représente l'un des groupes 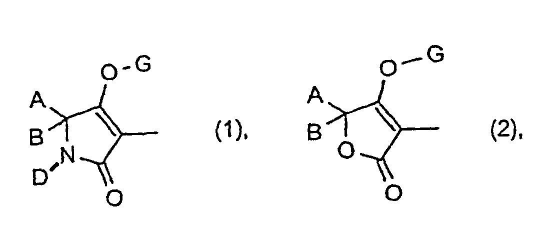 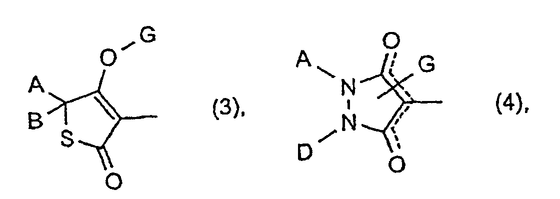 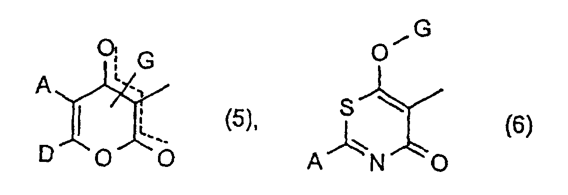
A représente l'hydrogène, un groupe alkyle en C₁ à C₁₂, alcényle en C₂ à C₈, (alkoxy en C₁ à C₁₀) - (alkyle en C₁ à C₈), poly - (alkoxy en C₁ à C₈) - (alkyle en C₁ à C₈) ou (alkylthio en C₁ à C₁₀) - (alkyle en C₁ à C₆) dont chacun est éventuellement substitué par un halogène, un groupe cycloalkyle en C₃ à C₈ éventuellement substitué dans chaque cas par un radical halogéno, alkyle en C₁ à C₆ ou alkoxy en C₁ à C₆ et dans lequel, le cas échéant, un ou deux groupes méthylène non contigus sont éventuellement remplacés par de l'oxygène et/ou par du soufre, ou un groupe phényle, naphtyle, phényl-(alkyle en C₁ à C₆), naphtyl-(alkyle en C₁ à C₆) ou hétaryle à noyau de 5 ou 6 atomes contenant un à trois hétéroatomes de la série oxygène, soufre et azote, éventuellement substitué dans chaque cas par un radical halogéno, alkyle en C₁ à C₆, halogénalkyle en C₁ à C₆, alkoxy en C₁ à C₆, halogénalkoxy en C₁ à C₆, cyano ou nitro,
B représente l'hydrogène, un groupe alkyle en C₁ à C₁₂ ou un groupe (alkoxy en C₁ à C₈) - (alkyle en C₁ à C₆),
ou bien
A, B et l'atome de carbone auquel ils sont liés forment un groupe cycloalkyle en C₃ à C₁₀ ou cycloalcényle en C₅ à C₁₀, où dans chaque cas un groupe méthylène est éventuellement remplacé par de l'oxygène ou du soufre et qui sont éventuellement substitués par un radical alkyle en C₁ à C₈, cycloalkyle en C₃ à C₁₀, halogénalkyle en C₁ à C₈, alkoxy en C₁ à C₈, alkoxy en C₁ à C₈, alkylthio en C₁ à C₈, halogéno ou phényle, ou bien
A, B et l'atome de carbone auquel ils sont liés forment un groupe cycloalkyle en C₅ ou C₆ qui est substitué par un groupe alkylènediyle contenant éventuellement un ou deux atomes d'oxygène et/ou de soufre ou par un groupe alkylènedioxyle ou par un groupe alkylènedithioyle formant avec l'atome de carbone auquel il est lié un autre noyau pentagonal à octogonal, ou bien
A, B et l'atome de carbone auquel ils sont liés forment un groupe cycloalkyle en C₃ à C₈ ou un groupe cycloalcényle en C₅ à C₈ dans lesquels deux substituants forment en commun avec les atomes de carbone auxquels ils sont liés un groupe alcanediyle en C₃ à C₆, alcène-diyle en C₃ à C₆ ou alcanediènediyle en C₄ à C₆ dont chacun est éventuellement substitué par un groupe cycloalkyle en C₁ à C₆, alkoxy en C₁ à C₆ ou un halogène et dans chacun desquels un groupe méthylène est éventuellement remplacé par de l'oxygène ou du soufre,
D représente l'hydrogène, un groupe alkyle en C₁ à C₁₂, alcényle en C₃ à C₈, alcynyle en C₃ à C₈, (alkoxy en C₁ à C₁₀) - (alkyle en C₂ à C₈), poly - (alkoxy en C₁ à C₈) - (alkyle en C₂ à C₈) ou (alkylthio en C₁ à C₁₀) - (alkyle en C₂ à C₈) dont chacun est éventuellement substitué par un halogène, un groupe cycloalkyle en C₃ à C₈ éventuellement substitué par un radical halogéno, alkyle en C₁ à C₄, alkoxy en C₁ à C₄ ou halogénalkyle en C₁ à C₄, dans lequel, le cas échéant, un ou deux groupes méthylène non contigus sont remplacés par de l'oxygène et/ou du soufre ou un groupe phényle, hétaryle à noyau de 5 ou 6 atomes avec un ou deux hétéroatomes de la série oxygène, soufre et azote, phényl-(alkyle en C₁ à C₆) ou hétaryl-(alkyle en C₁ à C₆) à noyau de 5 ou 6 atomes avec un ou deux hétéroatomes de la série oxygène, soufre et azote, dont chacun est éventuellement substitué par un radical halogéno, alkyle en C₁ à C₆, halogénalkyle en C₁ à C₆, alkoxy en C₁ à C₆, halogénalkoxy en C₁ à C₆, cyano ou nitro, ou bien
A et D forment ensemble un groupe alcanediyle en C₃ à C₆, un groupe alcènediyle en C₃ à C₆ ou un groupe alcanediènediyle en C₄ à C₆ dans chacun desquels un groupe méthylène est éventuellement remplacé par de l'oxygène ou du soufre et dont chacun est éventuellement substitué par un radical halogéno, hydroxy, mercapto ou par un radical alkyle en C₁ à C₁₀, alkoxy en C₁ à C₆, alkylthio en C₁ à C₆, cycloalkyle en C₃ à C₇, phényle ou benzyloxy dont chacun est éventuellement substitué par un radical halogéno, ou par un autre groupe alcanediyle en C₃ à C₆, alcènediyle en C₃ à C₆ ou alcanediènediyle en C₄ à C₆ formant un noyau condensé, dans chacun desquels, le cas échéant, un groupe méthylène est remplacé par de l'oxygène ou du soufre et qui est substitué par un radical alkyle en C₁ à C₆, ou dans lesquels, le cas échéant, deux substituants voisins forment conjointement avec les atomes de carbone auxquels ils sont liés un autre cycle carboné saturé ou non saturé à noyau de 5 ou 6 atomes, ou bien
A et D forment ensemble un groupe alcanediyle en C₃ à C₆ ou alcènediyle en C₃ à C₆ contenant chacun, le cas échéant, l'un des groupes suivants : 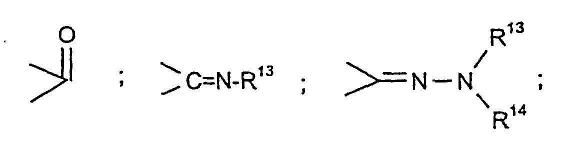 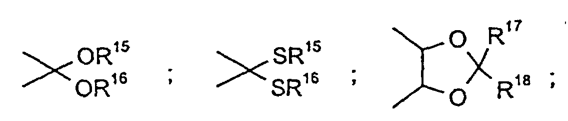 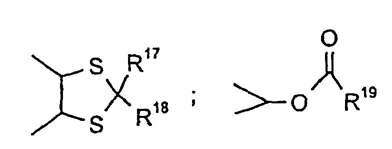 ou 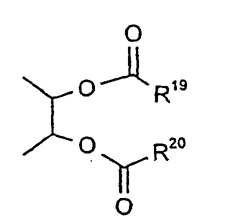
G représente, au cas où Het est l'un des restes (1), (2), (3), (5) ou (6), l'hydrogène (a) ou bien, au cas. où Het représente l'un des restes (1), (2), (3), (4), (5) ou (6), l'un des groupes 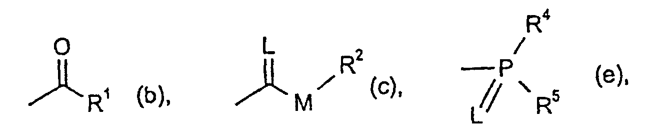 E (f) ou 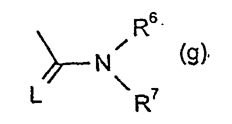 ou bien, au cas où Het représente l'un des restes (2), (3), (4), (5) ou (6), le groupe -SO₂-R³ (d),
où
E est un équivalent d'ion métallique ou un ion ammonium,
L est l'oxygène ou le soufre et
M est l'oxygène ou le soufre,
R¹ représente un groupe alkyle en C₁ à C₂₀, alcényle en C₂ à C₂₀, (alkoxy en C₁ à C₈) - (alkyle en C₁ à C₈), (alkylthio en C₁ à C₈) - (alkyle en C₁ à C₈) ou poly-(alkoxy en C₁ à C₈) - (alkyle en C₁ à C₈) dont chacun est éventuellement substitué par un halogène, ou un groupe cycloalkyle éventuellement substitué par un radical halogéno, alkyle en C₁ à C₆ ou alkoxy en C₁ à C₆ et dans lequel, le cas échéant, un ou deux groupes méthylène non contigus sont remplacés par de l'oxygène et/ou du soufre,
un groupe phényle éventuellement substitué par un radical halogéno, cyano, nitro, alkyle en C₁ à C₆, alkoxy en C₁ à C₆, halogénalkyle en C₁ à C₆, halogénalkoxy en C₁ à C₆, alkylthio en C₁ à C₆ ou alkylsulfonyle en C₁ à C₆,
un groupe phényl-(alkyle en C₁ à C₆) éventuellement substitué par un radical halogéno, nitro, cyano, alkyle en C₁ à C₆, alkoxy en C₁ à C₆, halogénalkyle en C₁ à C₆ ou halogénalkoxy en C₁ à C₆,
un groupe hétaryle pentagonal ou hexagonal ayant un ou deux hétéroatomes de la série oxygène, soufre et azote, éventuellement substitué par un radical halogéno ou alkyle en C₁ à C₆,
un groupe phénoxy-(alkyle en C₁ à C₆) éventuellement substitué par un radical halogéno ou alkyle en C₁ à C₆,
un groupe hétaryloxy-(alkyle en C₁ à C₆) à noyau de 5 ou 6 atomes ayant un ou deux hétéroatomes de la série oxygène, soufre et azote, éventuellement substitué par un radical halogéno, amino ou alkyle en C₁ à C₆,
R² est un groupe alkyle en C₁ à C₂₀, alcényle en C₂ à C₂₀, (alkoxy en C₁ à C₈) - (alkyle en C₂ à C₈) ou poly - (alkoxy en C₁ à C₈) - (alkyle en C₁ à C₈) éventuellement substitué par un radical halogéno,
un groupe cycloalkyle en C₃ à C₈ éventuellement substitué par un radical halogéno, alkyle en C₁ à C₆ ou alkoxy en C₁ à C₆,
un groupe phényle ou un groupe benzyle dont chacun est éventuellement substitué par un radical halogéno, cyano, nitro, alkyle en C₁ à C₆, alkoxy en C₁ à C₆, halogénalkyle en C₁ à C₆ ou halogénalkoxy en C₁ à C₆,
R³ est un groupe alkyle en C₁ à C₈ éventuellement substitué par un halogène, ou bien un groupe phényle ou un groupe benzyle dont chacun est éventuellement substitué par un radical halogéno, alkyle en C₁ à C₆, alkoxy en C₁ à C₆, halogénalkyle en C₁ à C₄, halogénalkoxy en C₁ à C₄, cyano ou nitro,
R⁴ et R⁵ représentent, indépendamment l'un de l'autre, un groupe alkyle en C₁ à C₈, alkoxy en C₁ à C₈, alkylamino en C₁ à C₈, di-(alkyle en C₁ à C₈)amino, alkylthio en C₁ à C₈ ou alcénylthio en C₂ à C₈ dont chacun est éventuellement substitué par un halogène, ou bien un groupe phényle, phénoxy ou phénylthio dont chacun est éventuellement substitué par un radical halogéno, nitro, cyano, alkoxy en C₁ à C₄, halogénalkoxy en C₁ à C₄, alkylthio en C₁ à C₄, halogénalkylthio en C₁ à C₄, alkyle en C₁ à C₄ ou halogénalkyle en C₁ à C₄,
R⁶ et R⁷ représentent, indépendamment l'un de l'autre, l'hydrogène, un groupe alkyle en C₁ à C₈, cycloalkyle en C₃ à C₈, alkoxy en C₁ à C₈, alcényle en C₃ à C₈, ou (alkoxy en C₁ à C₈)-(alkyle en C₁ à C₈) dont chacun est éventuellement substitué par un radical halogéno, un groupe phényle ou un groupe benzyle dont chacun est éventuellement substitué par un radical halogéno, alkyle en C₁ à C₈, halogénalkyle en C₁ à C₈ ou alkoxy en C₁ à C₈ ou forment ensemble un reste alkylène en C₃ à C₆ éventuellement substitué par un radical alkyle en C₁ à C₆ et dans lequel, le cas échéant, un groupe méthylène est remplacé par l'oxygène ou le soufre,
R¹³ représente l'hydrogène, un groupe alkyle en C₁ à C₈ ou alkoxy en C₁ à C₈ dont chacun est éventuellement substitué par un halogène, un groupe cycloalkyle en C₃ à C₈ éventuellement substitué par un radical halogéno, alkyle en C₁ à C₄ ou alkoxy en C₁ à C₄, dans lequel un groupe méthylène est éventuellement remplacé par de l'oxygène ou du soufre, ou bien un groupe phényle, phényl-(alkyle en C₁ à C₄) ou phényl-(alkoxy en C₁ à C₄) dont chacun est éventuellement substitué par un radical halogéno, alkyle en C₁ à C₆, alkoxy en C₁ à C₆, halogénalkyle en C₁ à C₄, halogénalkoxy en C₁ à C₄, nitro ou cyano,
R¹⁴ représente l'hydrogène ou un groupe alkyle en C₁ à C₈, ou bien
R¹³ et R¹⁴ forment ensemble un groupe alcanediyle en C₄ à C₆,
R¹⁵ et R¹⁶ sont identiques ou différents et représentent un groupe alkyle en C₁ à C₆, ou bien
R¹⁵ et R¹⁶ forment ensemble un reste alcanediyle en C₂ à C₄ qui est éventuellement substitué par un radical alkyle en C₁ à C₆ ou par un radical phényle éventuellement substitué par un radical halogéno, alkyle en C₁ à C₄, halogénalkyle en C₁ à C₄, alkoxy en C₁ à C₄ halogénalkoxy en C₁ à C₄, nitro ou cyano,
R¹⁷ et R¹⁸ représentent, indépendamment l'un de l'autre, l'hydrogène, un groupe alkyle en C₁ à C₈ éventuellement substitué par un halogène ou un groupe phényle éventuellement substitué par un radical halogéno, alkyle en C₁ à C₆, alkoxy en C₁ à C₆, halogénalkyle en C₁ à C₄, halogénalkoxy en C₁ à C₄, nitro ou cyano, ou bien
R¹⁷ et R¹⁸ forment conjointement avec l'atome de carbone auquel ils sont liés un groupe cycloalkyle en C₅ à C₇ éventuellement substitué par un radical alkyle en C₁ à C₄ ou alkoxy en C₁ à C₄ et dans lequel, le cas échéant, un groupe méthylène est remplacé par l'oxygène ou le soufre, et
R¹⁹ et R²⁰ forment, indépendamment l'un de l'autre, un groupe alkyle en C₁ à C₁₀, alcényle en C₂ à C₁₀, alkoxy en C₁ à C₁₀, alkylamino en C₁ à C₁₀, alcénylamino en C₃ à C₁₀, di-(alkyle en C₁ à C₁₀)amino ou di-(alcényle en C₃ à C₁₀)amino.

2. Composés de formule (I) suivant la revendication 1, dans laquelle
X est le fluor, le chlore ou le brome,
Y est le fluor, le chlore, le brome ou un groupe alkyle en C₁ à C₄,
Z est le fluor, le chlore, le brome ou un groupe alkyle en C₁ à C₄,
dans tous les cas l'un des restes Y et Z étant un halogène et l'autre étant un groupe alkyle,
Het représente l'un des groupes 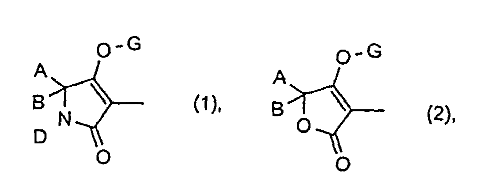 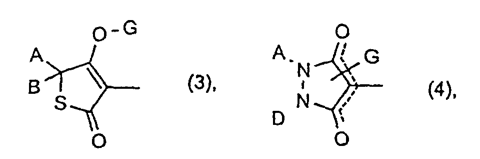 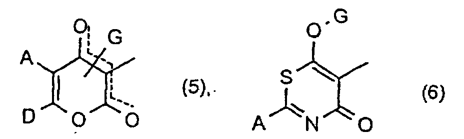
A est l'hydrogène, un groupe alkyle en C₁ à C₁₀, alcényle en C₂ à C₆, (alkoxy en C₁ à C₈) - (alkyle en C₁ à C₆), poly- (alkoxy en C₁ à C₆) - (alkyle en C₁ à C₆) ou (alkylthio en C₁ à C₈) - (alkyle en C₁ à C₆) dont chacun est éventuellement substitué par du fluor ou du chlore, ou bien un groupe cycloalkyle en C₃ à C₇ éventuellement substitué par du fluor, du chlore, un radical alkyle en C₁ à C₄ ou alkoxy en C₁ à C₄, dans lequel, le cas échéant, un ou deux groupes méthylène non contigus sont remplacés par de l'oxygène et/ou par du soufre, ou bien un groupe phényle, furannyle, pyridyle, imidazolyle, triazolyle, pyrazolyle, indolyle, thiazolyle, thiényle ou phényl-(alkyle en C₁ à C₄) dont chacun est éventuellement substitué par un radical fluoro, chloro, bromo, alkyle en C₁ à C₄, halogénalkyle en C₁ à C₄, alkoxy en C₁ à C₄, halogénalkoxy en C₁ à C₄, cyano ou nitro,
B représente l'hydrogène, un groupe alkyle en C₁ à C₁₀ ou (alkoxy en C₁ à C₆) - (alkyle en C₁ à C₄), ou bien
A, B et l'atome de carbone auquel ils sont liés représentent un groupe cycloalkyle en C₃ à C₈ ou un groupe cycloalcényle en C₅ à C₈, dans chacun desquels, le cas échéant, un groupe méthylène est remplacé par de l'oxygène ou du soufre et qui sont éventuellement substitués par un radical alkyle en C₁ à C₆, cycloalkyle en C₃ à C₈, halogénalkyle en C₁ à C₃, alkoxy en C₁ à C₆, alkylthio en C₁ à C₆, fluoro, chloro ou phényle, ou bien
A, B et l'atome de carbone auquel ils sont liés représentent un groupe cycloalkyle en C₅ ou C₆ qui est substitué par un groupe alkylènediyle contenant éventuellement un ou deux atomes d'oxygène ou de soufre ou par un groupe alkylènedioxyle ou par un groupe alkylènedithiol qui forme conjointement avec l'atome de carbone auquel il est lié un autre noyau pentagonal à heptagonal, ou bien
A, B et l'atome de carbone auquel ils sont liés forment un groupe cycloalkyle en C₃ à C₆ ou un groupe cycloalcényle en C₅ ou C₆ dans lesquels deux substituants représentent conjointement avec l'atome de carbone auquel ils sont liés un groupe alcanediyle en C₃ à C₅, alcènediyle en C₃ à C₅ ou butadiènediyle éventuellement substitué dans chaque cas par un radical alkyle en C₁ à C₅, alkoxy en C₁ à C₅, fluoro, chloro ou bromo, dans lequel un groupe méthylène est éventuellement remplacé dans chaque cas par de l'oxygène ou du soufre,
D représente l'hydrogène, un groupe alkyle en C₁ à C₁₀, alcényle en C₃ à C₆, alcynyle en C₃ à C₆, (alkoxy en C₁ à C₈) - (alkyle en C₂ à C₆), poly - (alkoxy en C₁ à C₆) - (alkyle en C₂ à C₆) ou (alkylthio en C₁ à C₈) - (alkyle en C₂ à C₆) dont chacun est éventuellement substitué par du fluor ou du chlore, un groupe cycloalkyle en C₃ à C₇ éventuellement substitué par un radical fluoro, chloro, alkyle en C₁ à C₄, alkoxy en C₁ à C₄ ou halogénalkyle en C₁ ou C₂, et dans lequel, le cas échéant, un ou deux groupes méthylène non contigus sont remplacés par de l'oxygène et/ou du soufre, ou un groupe phényle, furannyle, imidazolyle, pyridyle, thiazolyle, pyrazolyle, pyrimidyle, pyrrolyle, thiényle, triazolyle ou phényl-(alkyle en C₁ à C₄) dont chacun est éventuellement substitué par un radical fluoro, chloro, bromo, alkyle en C₁ à C₄, halogénalkyle en C₁ à C₄, alkoxy en C₁ à C₄, halogénalkoxy en C₁ à C₄, cyano ou nitro, ou bien
A et D forment ensemble un groupe alcanediyle en C₃ à C₅ ou alcènediyle en C₃ à C₅ dans chacun desquels, le cas échéant, un groupe méthylène est remplacé par de l'oxygène ou du soufre et qui sont éventuellement substitués par un radical fluoro, chloro, hydroxy, mercapto ou par un radical alkyle en C₁ à C₆, alkoxy en C₁ à C₄, alkylthio en C₁ à C₄, cycloalkyle en C₃ à C₇, phényle ou benzyloxy dont chacun est éventuellement substitué par du fluor ou du chlore, ou bien
contenant chacun, le cas échéant, l'un des groupes suivants : 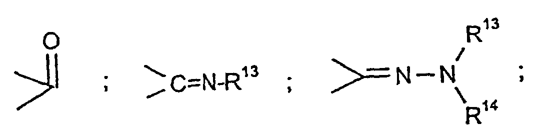 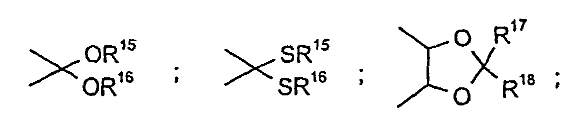 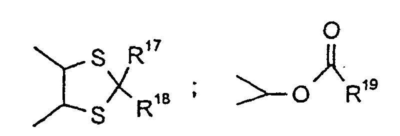 ou 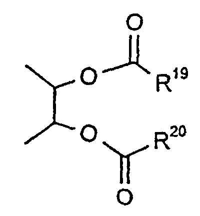 ou bien A et D (dans le cas des composés de formule (I-1)) forment conjointement avec les atomes auxquels ils sont liés l'un des groupes AD-1 à AD-27 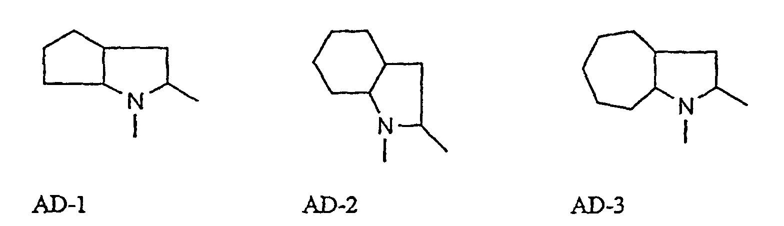 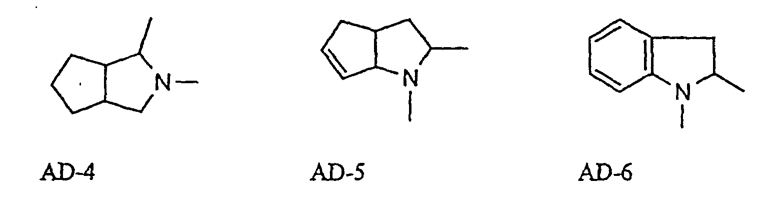 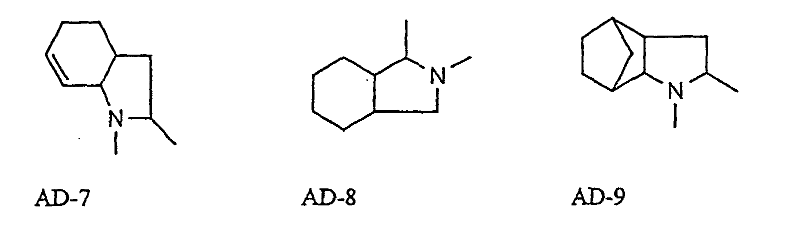 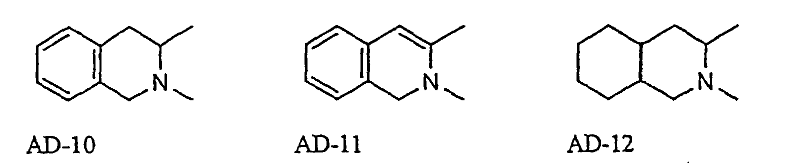 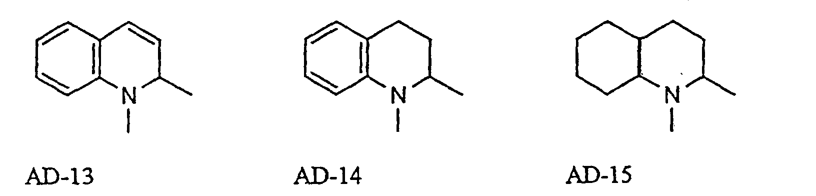 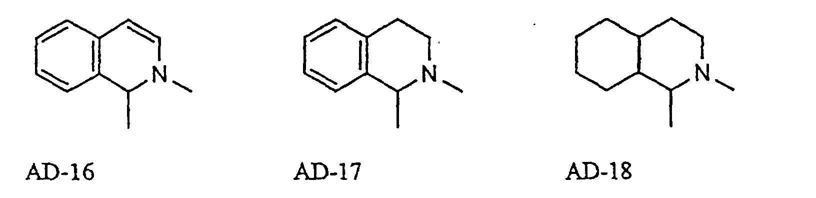 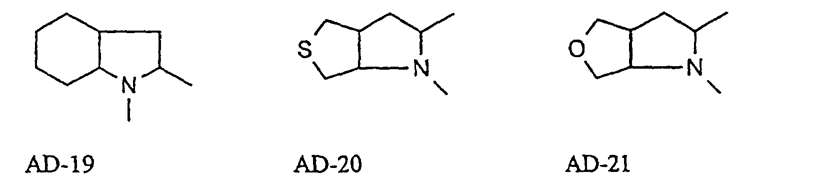 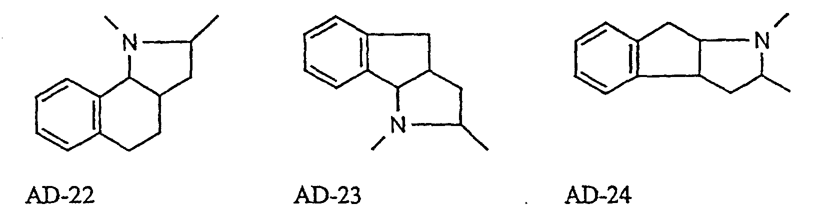 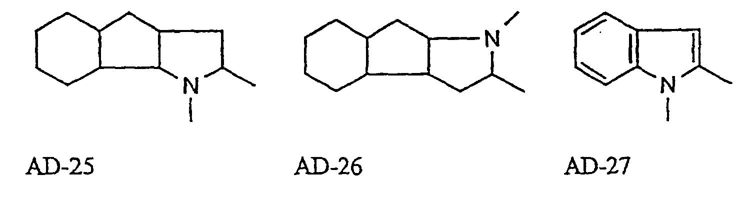
G au cas où Het est l'un des restes (1), (2), (3), (5) ou (6), représente l'hydrogène (a) ou bien, au cas où Het est l'un des restes (1), (2), (3), (4), (5) ou (6), représente l'un des groupes 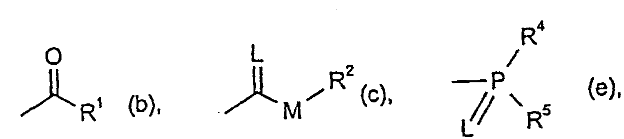 E (f) ou 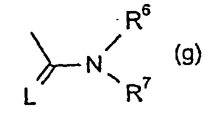 ou bien, au cas où Het est l'un des restes (2), (3), (4), (5) ou (6), représente le groupe -SO₂-R³ (d),
où
E est un équivalent d'ion métallique ou un ion ammonium,
L est l'oxygène ou le soufre et
M est l'oxygène ou le soufre,
R¹ représente un groupe alkyle en C₁ à C₁₆, alcényle en C₂ à C₁₆, (alkoxy en C₁ à C₆) - (alkyle en C₁ à C₆), (alkylthio en C₁ à C₆) - (alkyle en C₁ à C₆) ou poly-(alkoxy en C₁ à C₆) - (alkyle en C₁ à C₆) dont chacun est éventuellement substitué par du fluor ou du chlore, ou un groupe cycloalkyle éventuellement substitué par un radical fluoro, chloro, alkyle en C₁ à C₅ ou alkoxy en C₁ à C₅ et dans lequel, le cas échéant, un ou deux groupes méthylène non contigus sont remplacés par de l'oxygène et/ou du soufre,
un groupe phényle éventuellement substitué par un radical fluoro, chloro, bromo, cyano, nitro, alkyle en C₁ à C₄, alkoxy en C₁ à C₄, halogénalkyle en C₁ à C₃, halogénalkoxy en C₁ à C₃, alkylthio en C₁ à C₄ ou alkylsulfonyle en C₁ à C₄,
un groupe phényl-(alkyle en C₁ à C₄) éventuellement substitué par un radical fluoro, chloro, bromo, alkyle en C₁ à C₄, alkoxy en C₁ à C₄, halogénalkyle en C₁ à C₃ ou halogénalkoxy en C₁ à C₃,
un groupe pyrazolyle, thiazolyle, pyridyle, pyrimidyle, furannyle ou thiényle éventuellement substitué dans chaque cas par un radical fluoro, chloro, bromo ou alkyle en C₁ à C₄,
un groupe phénoxy- (alkyle en C₁ à C₅) éventuellement substitué par un radical fluoro, chloro, bromo ou alkyle en C₁ à C₄,
un groupe pyridyloxy- (alkyle en C₁ à C₅), pyrimidyloxy- (alkyle en C₁ à C₅) ou thiazolyloxy-(alkyle en C₁ à C₅) dont chacun est éventuellement substitué par un radical fluoro, chloro, bromo, amino ou alkyle en C₁ à C₄,
R² représente un groupe alkyle en C₁ à C₁₆, alcényle en C₂ à C₁₆, (alkoxy en C₁ à C₆) - (alkyle en C₂ à C₆) ou poly-(alkoxy en C₁ à C₆) - (alkyle en C₂ à C₆) dont chacun est éventuellement substitué par du fluor ou du chlore,
un groupe cycloalkyle en C₃ à C₇ éventuellement substitué par du fluor, du chlore, un radical alkyle en C₁ à C₄ ou alkoxy en C₁ à C₄,
un groupe phényle ou un groupe benzyle dont chacun est éventuellement substitué par un radical fluoro, chloro, bromo, cyano, nitro, alkyle en C₁ à C₄, alkoxy en C₁ à C₃, halogénalkyle en C₁ à C₃ ou halogénalkoxy en C₁ à C₃,
R³ est un groupe alkyle en C₁ à C₆ éventuellement substitué par du fluor ou chlore, ou un groupe phényle ou benzyle dont chacun est éventuellement substitué par un radical fluoro, chloro, bromo, alkyle en C₁ à C₅, alkoxy en C₁ à C₅, halogénalkyle en C₁ à C₃, halogénalkoxy en C₁ à C₃, cyano ou nitro,
R⁴ et R³ forment, indépendamment l'un de l'autre, un groupe alkyle en C₁ à C₆, alkoxy en C₁ à C₆, alkylamino en C₁ à C₆, di-(alkyle en C₁ à C₆) amino, alkylthio en C₁ à C₆ ou alcénylthio en C₃ ou C₄ dont chacun est éventuellement substitué par du fluor ou du chlore, ou bien un groupe phényle, phénoxy ou phénylthio dont chacun est éventuellement substitué par un radical fluoro, chloro, bromo, nitro, cyano, alkoxy en C₁ à C₃, halogénalkoxy en C₁ à C₃, alkylthio en C₁ à C₃, halogénalkylthio en C₁ à C₃, alkyle en C₁ à C₃ ou halogénalkyle en C₁ à C₃,
R⁶ et R⁷ représentent, indépendamment l'un de l'autre, l'hydrogène, un groupe alkyle en C₁ à C₆, cycloalkyle en C₃ à C₆, alkoxy en C₁ à C₆, alcényle en C₃ à C₆, ou (alkoxy en C₁ à C₆) - (alkyle en C₂ à C₆) dont chacun est éventuellement substitué par du fluor ou du chlore, un groupe phényle ou un groupe benzyle dont chacun est éventuellement substitué par un radical fluoro, chloro, bromo, halogénalkyle en C₁ à C₅, alkyle en C₁ à C₅ ou alkoxy en C₁ à C₅ ou forment ensemble un reste alkylène en C₃ à C₆ éventuellement substitué par un radical alkyle en C₁ à C₄ et dans lequel, le cas échéant, un groupe méthylène est remplacé par l'oxygène ou du soufre,
R¹³ est l'hydrogène, un groupe alkyle en C₁ à C₆ ou alkoxy en C₁ à C₆ éventuellement substitué par du fluor ou du chlore, un groupe cycloalkyle en C₃ à C₇ éventuellement substitué par un radical fluoro, alkyle en C₁ ou C₂ ou alkoxy en C₁ ou C₂, et dans lequel, le cas échéant, un groupe méthylène est remplacé par de l'oxygène ou du soufre, ou bien un groupe phényle, phényl-(alkyle en C₁ à C₃) ou phényl-(alkyloxy en C₁ ou C₂) dont chacun est éventuellement substitué par un radical fluoro, chloro, bromo, alkyle en C₁ à C₅, alkoxy en C₁ à C₅, halogénalkyle en C₁ ou C₂, halogénalkoxy en C₁ ou C₂, nitro ou cyano,
R¹⁴ représente l'hydrogène ou un groupe alkyle en C₁ à C₆, ou bien
R¹³ et R¹⁴ forment ensemble un groupe alcanediyle en C₄ à C₆,
R¹⁵ et R¹⁶ sont identiques ou différents et représentent un groupe alkyle en C₁ à C₄, ou bien
R¹⁵ et R¹⁶ forment ensemble un reste alcanediyle en C₂ ou C₃ qui est éventuellement substitué par un radical alkyle en C₁ à C₄ ou par un reste phényle éventuellement substitué par un radical fluoro, chloro, bromo, alkyle en C₁ ou C₂, halogénalkyle en C₁ ou C₂, alkoxy en C₁ ou C₂, halogénalkoxy en C₁ ou C₂, nitro ou cyano,
R¹⁷ et R¹⁸ représentent, indépendamment l'un de l'autre, l'hydrogène, un groupe alkyle en C₁ à C₈ éventuellement substitué par du fluor ou du chlore ou un reste phényle éventuellement substitué par un radical fluoro, chloro, alkyle en C₁ à C₄, alkoxy en C₁ à C₄, halogénalkyle en C₁ ou C₂, halogénalkoxy en C₁ ou C₂, nitro ou cyano, ou bien
R¹⁷ et R¹⁸ forment conjointement avec l'atome de carbone auquel ils sont liés un groupe cycloalkyle en C₅ ou C₆ éventuellement substitué par un radical alkyle en C₁ à C₃ ou alkoxy en C₁ à C₃, et dans lequel, le cas échéant, un groupe méthylène est remplacé par de l'oxygène ou du soufre, et
R¹⁹ et R²⁰ représentent, indépendamment l'un de l'autre, un groupe alkyle en C₁ à C₆, alcényle en C₂ à C₆, alkoxy en C₁ à C₆, alkylamino en C₁ à C₆, alcénylamino en C₃ à C₆, di-(alkyle en C₁ à C₆)amino ou di-(alcényle en C₃ à C₁₀) amino.

3. Composés de formule (I) suivant la revendication 1, dans laquelle
X est le fluor, le chlore ou le brome,
Y est le fluor, le chlore, le brome, un groupe méthyle, éthyle, n-propyle ou isopropyle,
Z est le fluor, le chlore, le brome, un groupe méthyle, éthyle, n-propyle ou isopropyle,
avec toujours l'un des restes Y et Z représentant un halogène et l'autre représentant un groupe alkyle,
Het représente l'un des groupes 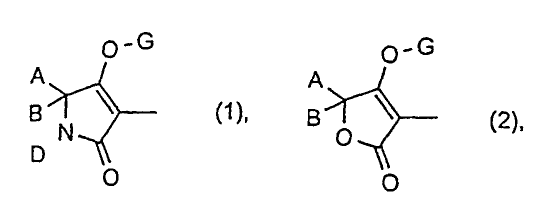 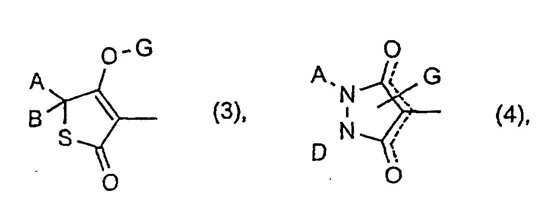 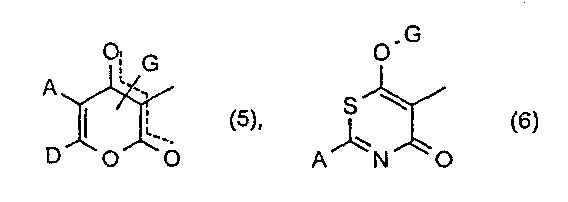
A est l'hydrogène, un groupe alkyle en C₁ à C₈, alcényle en C₂ à C₄, (alkoxy en C₁ à C₆) - (alkyle en C₁ à C₄), poly-(alkoxy en C₁ à C₄) - (alkyle en C₁ à C₄) ou (alkylthio en C₁ à C₆) - (alkyle en C₁ à C₄) dont chacun est éventuellement substitué par du fluor ou du chlore, un groupe cycloalkyle en C₃ à C₆ éventuellement substitué par un radical fluoro, chloro, méthyle ou méthoxy, dans lequel, le cas échéant, un ou deux groupes méthylène non directement continus sont remplacés par de l'oxygène et/ou du soufre, ou bien un groupe phényle, pyridyle ou benzyle dont chacun est éventuellement substitué par un radical fluoro, chloro, bromo, méthyle, éthyle, n-propyle, isopropyle, méthoxy, éthoxy, trifluorométhyle, trifluorométhoxy, cyano ou nitro,
B représente l'hydrogène, un groupe alkyle en C₁ à C₈ ou un groupe (alkoxy en C₁ à C₄) - (alkyle en C₁ ou C₂), ou bien
A, B et l'atome de carbone auquel ils sont liés forment un groupe cycloalkyle en C₃ à C₈ ou cycloalcényle en C₅ à C₈, dans chacun desquels, le cas échéant, un groupe méthylène est remplacé par de l'oxygène ou du soufre et qui sont éventuellement substitués par un radical méthyle, éthyle, n-propyle, isopropyle, butyle, isobutyle, sec.-butyle, tertio-butyle, cyclohexyle, trifluorométhyle, méthoxy, éthoxy, n-propoxy, isopropoxy, butoxy, isobutoxy, sec.-butoxy, tertio-butoxy, méthylthio, éthylthio, fluoro, chloro ou phényle, ou bien
A, B et l'atome de carbone auquel ils sont liés représentent un groupe cycloalkyle en C₅ ou C₆ qui est substitué par un groupe alkylènediyle contenant éventuellement un atome d'oxygène ou de soufre ou par un groupe alkylènedioxyle qui forme avec l'atome de carbone auquel il est lié un autre noyau pentagonal à heptagonal, ou bien
A, B et l'atome de carbone auquel ils sont liés représentent un groupe cycloalkyle en C₃ à C₆ ou un groupe cycloalcényle en C₅ ou C₆ dans lesquels deux substituants forment conjointement avec les atomes de carbone auxquels ils sont liés un groupe alcanediyle en C₃ ou C4, alcènediyle en C₃ ou C₄ ou butadiènediyle, dans chacun desquels, le cas échéant, un groupe méthylène est remplacé par de l'oxygène ou du soufre,
représente l'hydrogène, un groupe alkyle en C₁ à C₈, alcényle en C₃ ou C₄, alcynyle en C₃ ou C₄, (alkoxy en C₁ à C₆) - (alkyle en C₂ à C₄), poly-(alkoxy en C₁ à C₄) - (alkyle en C₂ à C₄), (alkylthio en C₁ à C₄)-(alkyle en C₂ à C₄) ou cycloalkyle en C₃ à C₆ dont chacun est éventuellement substitué par du fluor ou du chlore, où, le cas échéant, un groupe méthylène ou deux groupes méthylène non contigus sont remplacés par de l'oxygène et/ou du soufre, ou bien un groupe phényle, furannyle, pyridyle, thiényle ou benzyle dont chacun est éventuellement substitué par un radical fluoro, chloro, bromo, méthyle, éthyle, n-propyle, isopropyle, méthoxy, éthoxy, trifluorométhyle, trifluorométhoxy, cyano ou nitro, ou bien
A et D forment ensemble un groupe alcanediyle en C₃ à C₅ ou alcènediyle en C₃ à C₅ dans chacun desquels, le cas échéant, un groupe méthylène est remplacé par de l'oxygène ou du soufre et qui sont éventuellement substitués par du fluor, du chlore, un radical hydroxy, mercapto ou par un radical alkyle en C₁ à C₆, alkoxy en C₁ à C₄, alkylthio en C₁ à C₄, cycloalkyle en C₃ à C₆, phényle ou benzyloxy dont chacun est substitué par du fluor ou du chlore, ou bien chacun contient, le cas échéant, l'un des groupes suivants : 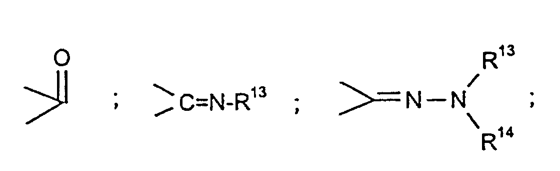 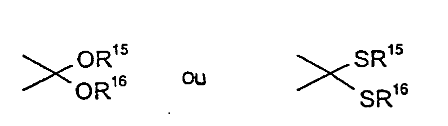 ou bien A et D, dans le cas des composés de formule (I-1), forment conjointement avec les atomes auxquels ils sont liés, l'un des groupes suivants : 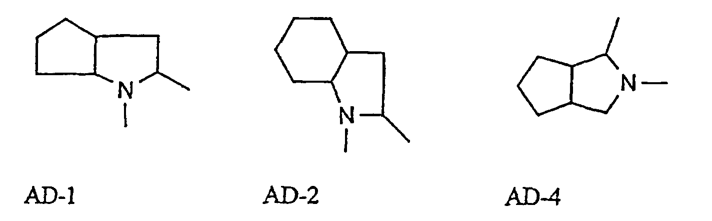 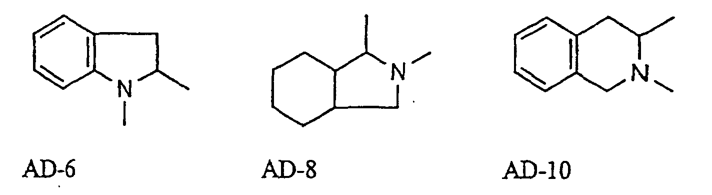 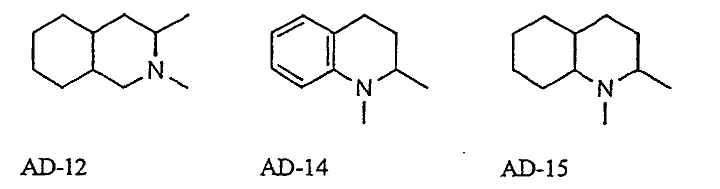 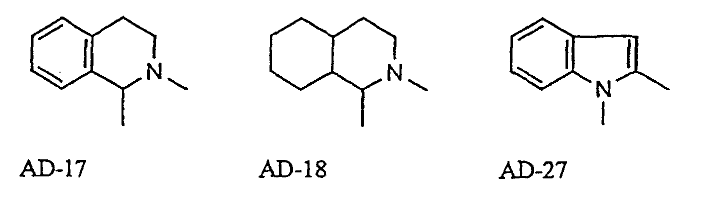
G au cas où Het est l'un des restes (1), (2), (3), (5) ou (6), représente l'hydrogène (a) ou bien, au cas où Het est l'un des restes (1), (2), (3), (4), (5) ou (6), représente l'un des groupes 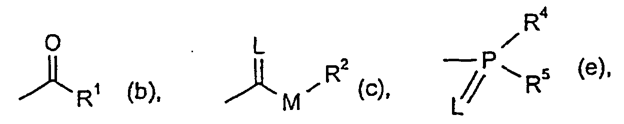 E (f) ou 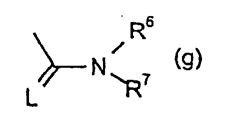 ou bien, au cas où Het est l'un des restes (2), (3), (4), (5) ou (6), représente un groupe -SO₂-R³ (d), où
E est un équivalent d'ion métallique ou un ion ammonium,
L est l'oxygène ou le soufre et
M est l'oxygène ou le soufre,
R¹ représente un groupe alkyle en C₁ à C₁₄, alcényle en C₂ à C₁₄, (alkoxy en C₁ à C₄) - (alkyle en C₁ à C₆), (alkylthio en C₁ à C₄) - (alkyle en C₁ à C₆), poly(alkoxy en C₁ à C₄) - (alkyle en C₁ à C₄) dont chacun est éventuellement substitué par du fluor ou du chlore, ou un groupe cycloalkyle en C₃ à C₆ éventuellement substitué par un radical fluoro, chloro, méthyle, éthyle, n-propyle, isopropyle, n-butyle, isobutyle, tertio-butyle, méthoxy, éthoxy, n-propoxy ou isopropoxy et dans lequel, le cas échéant, un groupe méthylène ou deux groupes méthylène non contigus sont remplacés par de l'oxygène et/ou du soufre,
un groupe phényle éventuellement substitué par un radical fluoro, chloro, bromo, cyano, nitro, méthyle, éthyle, n-propyle, isopropyle, méthoxy, éthoxy, trifluorométhyle, trifluorométhoxy, méthylthio, éthylthio, méthylsulfonyle ou éthylsulfonyle,
un groupe benzyle éventuellement substitué par un radical fluoro, chloro, bromo, méthyle, éthyle, n-propyle, isopropyle, méthoxy, éthoxy, trifluorométhyle ou trifluorométhoxy,
un groupe furannyle, thiényle ou pyridyle éventuellement substitué par un radical fluoro, chloro, bromo, méthyle ou éthyle, ou bien
un groupe phénoxy-(alkyle en C₁ à C₄) éventuellement substitué par un radical fluoro, chloro, méthyle ou éthyle, ou bien
un groupe pyridyloxy - (alkyle en C₁ à C₄), pyrimidyloxy - (alkyle en C₁ à C₄) ou thiazolyloxy-(alkyle en C₁ à C₄) dont chacun est éventuellement substitué par un radical fluoro, chloro, amino, méthyle ou éthyle,
R² est un groupe alkyle en C₁ à C₁₄, alcényle en C₂ à C₁₄, (alkoxy en C₁ à C₄) - (alkyle en C₂ à C₆) ou poly-(alkoxy en C₁ à C₄) - (alkyle en C₂ à C₆) dont chacun est éventuellement substitué par du fluor ou du chlore,
un groupe cycloalkyle en C₃ à C₆ éventuellement substitué par un radical fluoro, chloro, méthyle, éthyle, n-propyle, isopropyle ou méthoxy,
ou bien un groupe phényle ou benzyle dont chacun est éventuellement substitué par un radical fluoro, chloro, bromo, cyano, nitro, méthyle, éthyle, n-propyle, isopropyle, méthoxy, éthoxy, trifluorométhyle ou trifluorométhoxy,
R³ est un groupe méthyle, éthyle, propyle, isopropyle éventuellement substitué par du fluor ou chlore, ou un groupe phényle ou benzyle dont chacun est éventuellement substitué par un radical fluoro, chloro, bromo, méthyle, éthyle, propyle, isopropyle, tertio-butyle, méthoxy, éthoxy, isopropoxy, tertio-butoxy, trifluorométhyle, trifluorométhoxy, cyano ou nitro,
R⁴ et R⁵ représentent, indépendamment l'un de l'autre, un groupe alkyle en C₁ à C₄, alkoxy en C₁ à C₄, alkylamino en C₁ à C₄, di-(alkyle en C₁ à C₄)amino ou alkylthio en C₁ à C₄ dont chacun est éventuellement substitué par du fluor ou du chlore, ou un groupe phényle, phénoxy ou phénylthio dont chacun est éventuellement substitué par un radical fluoro, chloro, bromo, nitro, cyano, méthyle, méthoxy, trifluorométhyle ou trifluorométhoxy,
R⁶ et R⁷ représentent, indépendamment l'un de l'autre, l'hydrogène, un groupe alkyle en C₁ à C₄, cycloalkyle en C₃ à C₆, alkoxy en C₁ à C₄, alcényle en C₃ ou C₄, ou (alkoxy en C₁ à C₄) - (alkyle en C₂ à C₄) dont chacun est éventuellement substitué par du fluor ou du chlore, un groupe phényle ou un groupe benzyle dont chacun est éventuellement substitué par un radical fluoro, chloro, bromo, méthyle, méthoxy ou trifluorométhyle, ou forment ensemble un reste alkylène en C₅ ou C₆ éventuellement substitué par un radical méthyle ou éthyle et dans lequel, le cas échéant, un groupe méthylène est remplacé par de l'oxygène ou du soufre,
R¹³ représente l'hydrogène, un groupe alkyle en C₁ à C₄ ou alkoxy en C₁ à C₄ éventuellement substitué par du fluor ou du chlore, un groupe cycloalkyle en C₃ à C₆ ou bien un groupe phényle, phényl-(alkyle en C₁ ou C₂) ou benzyloxy dont chacun est éventuellement substitué par un radical fluoro, chloro, bromo, méthyle, éthyle, isopropyle, tertio-butyle, méthoxy, éthoxy, iso-propoxy, tertio-butoxy, trifluorométhyle, trifluorométhoxy, nitro ou cyano,
R¹⁴ est l'hydrogène ou un groupe alkyle en C₁ à C₄,
R¹³ et R¹⁴ forment ensemble un groupe alcanediyle en C₄ à C₆,
R¹⁵ et R¹⁶ sont identiques ou différents et représentent un groupe méthyle ou éthyle, ou bien
R¹⁵ et R¹⁶ forment ensemble un reste alcanediyle en C₂ ou C₃ qui est éventuellement substitué par un radical méthyle, éthyle, n-propyle, isopropyle, n-butyle, isobutyle, sec.-butyle, tertio-butyle ou par un reste phényle éventuellement substitué par un radical fluoro, chloro, méthoxy, trifluorométhyle, trifluorométhoxy, nitro ou cyano.

4. Procédé de production de composés de formule (I) suivant la revendication 1, **caractérisé en ce que** :
(A) on obtient des composés de formule (I-1-a) 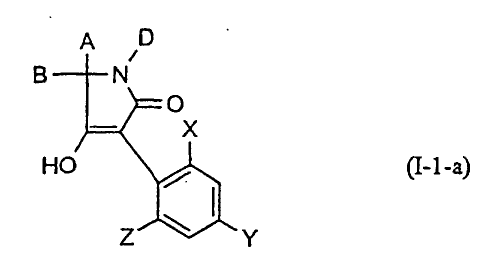 dans laquelle
A, B, D, X, Y et Z ont les définitions indiquées ci-dessus,
lorsque :
on effectue la condensation intermoléculaire de composés de formule (II) 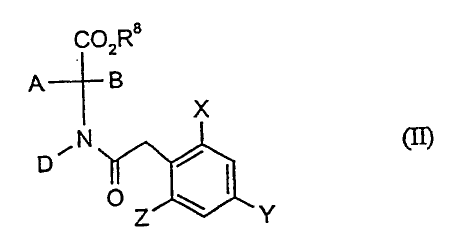 dans laquelle
A, B, D, X, Y et Z ont les définition indiquées ci-dessus,
et
R⁸ est un groupe alkyle,
en présence d'un diluant et en présence d'une base,
(B) on obtient des composés de formule (I-2-a) 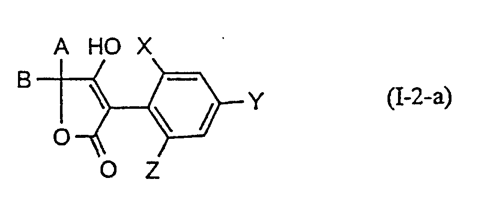 dans laquelle
A, B, X, Y et Z ont les définitions indiquées ci-dessus,
lorsque :
on effectue la condensation intramoléculaire de composés de formule (III) 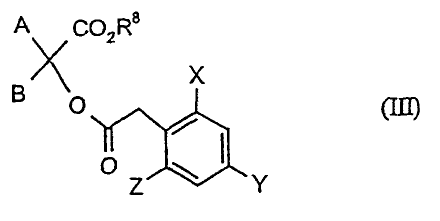 dans laquelle
A, B, X, Y, Z et R⁸ ont les définitions indiquées ci-dessus,
en présence d'un diluant et en présence d'une base,
(C) on obtient des composés de formule (I-3-a) 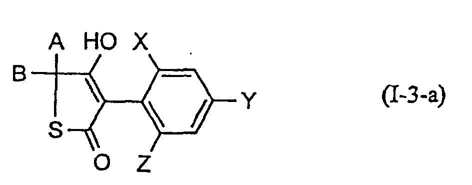 dans laquelle
A, B, X, Y et Z ont les définitions indiquées ci-dessus,
lorsque :
on effectue la cyclisation intramoléculaire de composés de formule (IV) 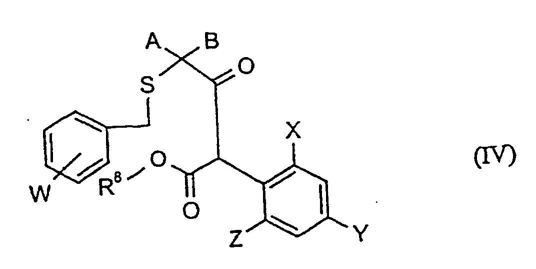 dans laquelle
A, B, X, Y, Z et R⁸ ont les définitions indiquées ci-dessus et
W est l'hydrogène, un halogène un groupe alkyle ou alkoxy,
éventuellement en présence d'un diluant et en présence d'un acide,
(E) on obtient des composés de formule (I-5-a) 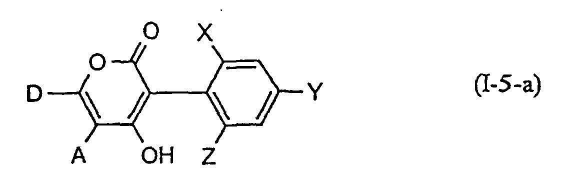 dans laquelle
A, D, X, Y et Z ont les définitions indiquées ci-dessus,
lorsque :
on fait réagir des composés de formule (VIII) 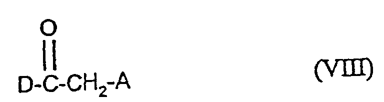 dans laquelle
A et D ont les définitions indiquées ci-dessus, avec des composés de formule (V) 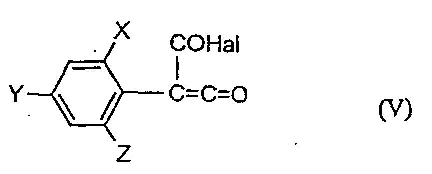 dans laquelle
X, Y et Z ont les définitions indiquées ci-dessus et
Hal représente un halogène,
le cas échéant en présence d'un diluant et en présence éventuelle d'un accepteur d'acide,
(F) on obtient des composés de formule (I-6-a) 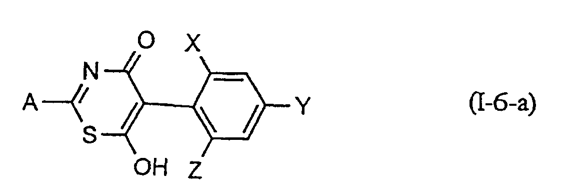 dans laquelle
A, X, Y et Z ont les définitions indiquées ci-dessus, lorsqu'on fait réagir des composés de formule (IX) 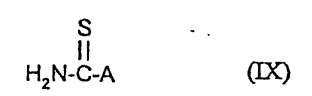 dans laquelle
A a la définition indiquée ci-dessus,
avec des composés de formule (V) 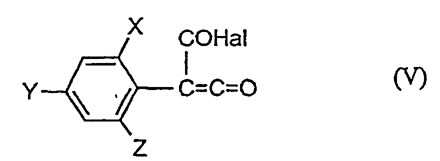 dans laquelle
Hal, X, Y et Z ont les définitions indiquées ci-dessus,
le cas échéant en présence d'un diluant et en présence éventuelle d'un accepteur d'acide,
puis, le cas échéant, on fait réagir les composés ainsi obtenus de formules (I-1-a), (I-2-a) à (I-3-a), (I-5-a) et (I-6-a) ou des composés de formule (I-4-a) 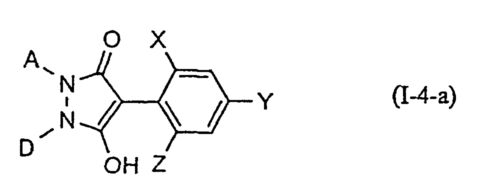 dans laquelle
A, D, X, Y et Z ont les définitions indiquées ci-dessus, dans chaque cas
(G)
α) avec des halogénures d'acides de formule (X) 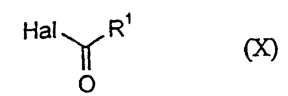 dans laquelle
R¹ a la définition indiquée ci-dessus et
Hal représente un halogène,
ou bien
β) avec des anhydrides d'acides carboxyliques de formule (XI)
R¹-CO-O-CO-R¹ (XI)
dans laquelle
R¹ a la définition indiquée ci-dessus,
le cas échéant en présence d'un diluant et en présence éventuelle d'un accepteur d'acide,
ou bien
(H) avec des esters d'acide chloroformique ou des thioesters d'acide chloroformique de formule (XII)
R²-M-CO-Cl (XII)
dans laquelle
R² et M ont les définitions indiquées ci-dessus,
le cas échéant en présence d'un diluant et en présence éventuelle d'un accepteur d'acide,
ou bien
I)
α) avec des esters d'acide chloromonothioformique ou des esters d'acide chlorodithioformique de formule (XIII) 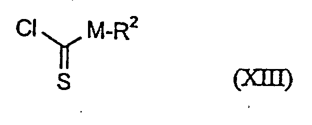 dans laquelle
M et R² ont les définitions indiquées ci-dessus,
le cas échéant en présence d'un diluant et en présence éventuelle d'un accepteur d'acide, ou bien
β) avec le sulfure de carbone, puis avec des composés de formule (XIV)
R²-Hal (XIV)
dans laquelle
R² a la définition indiquée ci-dessus et
Hal représente le chlore, le brome ou l'iode,
le cas échéant en présence d'un diluant et en présence éventuelle d'une base,
ou bien
(J) avec des chlorures d'acide sulfonique de formule (XV)
R³-SO₂-Cl (XV)
dans laquelle
R¹ a la définition indiquée ci-dessus,
le cas échéant en présence d'un diluant et en présence éventuelle d'un accepteur d'acide,
ou bien
(K) avec des composés phosphorés de formule (XVI) 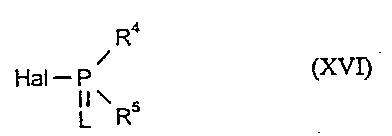 dans laquelle
L, R⁴ et R⁵ ont les définitions indiquées ci-dessus et
Hal représente un halogène,
le cas échéant en présence d'un diluant et en présence éventuelle d'un accepteur d'acide,
ou bien
(L) avec des composés métalliques ou des amines de formules (XVII) ou (XVIII)
Me(OR¹⁰)ₜ (XVII) 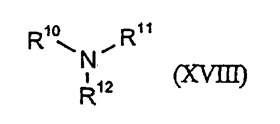
dans lesquelles
Me représente un métal monovalent ou divalent,
t représente le nombre 1 ou 2 et
R¹⁰, R¹¹, R¹² représentent, indépendamment les uns des autres, l'hydrogène ou un groupe alkyle,
le cas échéant en présence d'un diluant,
ou bien
(M)
α) avec des isocyanates ou des isothiocyanates de formule (XIX)
R⁶-N=C=L (XIX)
dans laquelle
R⁶ et L ont les définitions indiquées ci-dessus,
le cas échéant en présence d'un diluant et en présence éventuelle d'un catalyseur, ou bien
β) avec des chlorures d'acide carbamique ou des chlorures d'acide thiocarbamique de formule (XX) 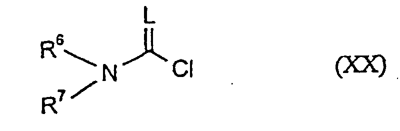 dans laquelle
L, R⁶ et R⁷ ont les définitions indiquées ci-dessus,
le cas échéant en présence d'un diluant et en présence éventuelle d'un accepteur d'acide.

5. Composés de formule (II) 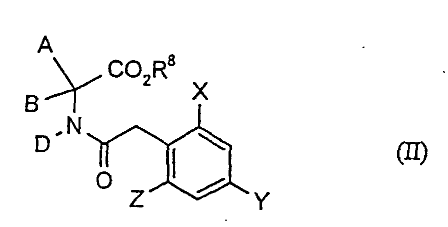 dans laquelle
A, B, D, X, Y et Z ont les définitions indiquées dans la revendication 1 et
R⁸ est un groupe alkyle.

6. Composés de formule (XXIII) 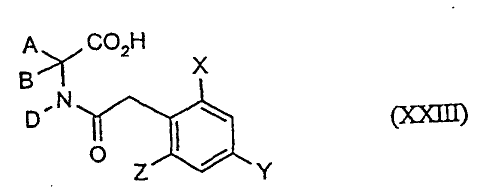 dans laquelle
A, B, D, X, Y et Z ont les définitions indiquées dans la revendication 1.

7. Composés de formule (XXII) 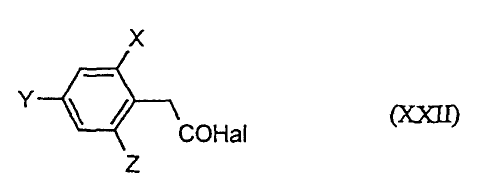 dans laquelle
X, Y et Z ont les définitions indiquées dans la revendication 1 et
Hal représente le chlore ou le brome.

8. Composés de formule (XXV) 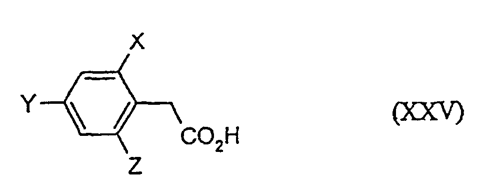 dans laquelle
X, Y et Z ont la définition indiquée dans la revendication 1, excepté l'acide 2,4-dichloro-6-méthylphénylacétique.

9. Composés de formule (XXVI) 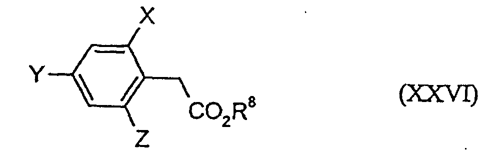 dans laquelle
X, Y et Z ont la définition indiquée ci-dessus et
R⁸ est un groupe alkyle.

10. Composés de formule (XXVII) 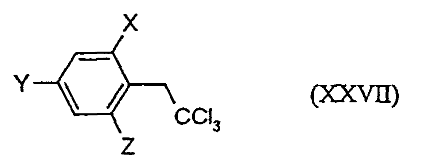 dans laquelle
X, Y et Z ont la définition indiquée dans la revendication 1.

11. Composés de formule (XXXI) 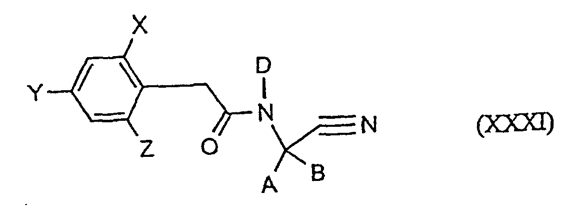 dans laquelle
A, B, D, X, Y et Z ont la définition indiquée dans la revendication 1.

12. Composés de formule (III) 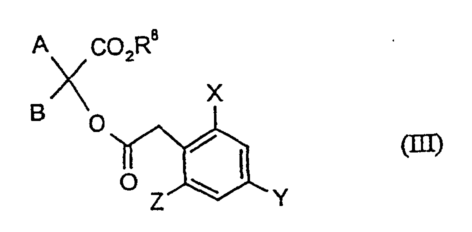 dans laquelle
A, B, X, Y et Z ont les définitions indiquées ci-dessus et
R⁸ est un groupe alkyle.

13. Composés de formule (IV) 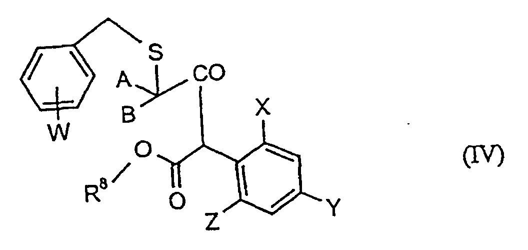 dans laquelle
A, B, W, X, Y et Z ont les définitions indiquées dans la revendication 1 et
R⁸ est un groupe alkyle.

14. Composés de formule (V) 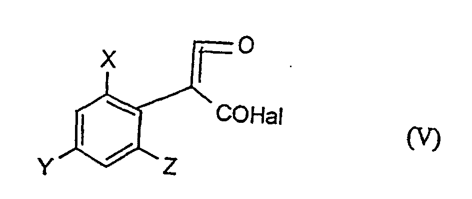 dans laquelle
X, Y et Z ont les définitions indiquées dans la revendication 1 et
Hal représente le chlore ou le brome.

15. Composés de formule (XXXV) 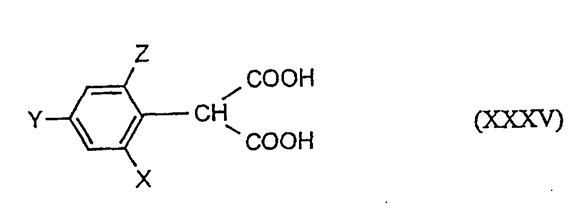 dans laquelle
X, Y et Z ont les définitions indiquées dans la revendication 1.

16. Compositions parasiticides et compositions herbicides, **caractérisées par** une teneur en au moins un composé de formule (I) suivant la revendication 1.

17. Utilisation de composés de formule (I) suivant la revendication 1 pour combattre des parasites et des mauvaises herbes.

18. Procédé pour combattre des parasites et des mauvaises herbes, **caractérisé en ce qu'**on fait agir des composés de formule (I) suivant la revendication 1 sur les parasites et/ou sur leur milieu ou **en ce qu'**on les fait agir sur des mauvaises herbes et/ou sur leur milieu.

19. Procédé de préparation de compositions parasiticides et de compositions herbicides, **caractérisé en ce qu'**on mélange des composés de formule (I) suivant la revendication 1 avec des diluants et/ou des agents tensioactifs.

20. Utilisation de composés de formule (I) suivant la revendication 1, pour la préparation de compositions parasiticides et de compositions herbicides.
